# EUROPÄISCHE PATENTSCHRIFT

(11) **EP 2 334 175 B1**
(45) Veröffentlichungstag und Bekanntmachung des Hinweises auf die Patenterteilung: **01.03.2017**
(21) Anmeldenummer: 09778755.0
(22) Anmeldetag: 29.09.2009
(51) Int. Cl.: A01N 35/06, A01N 37/50, A01N 43/18, A01N 43/32, C07D 317/72, A01N 25/22, A01N 35/10, A01N 43/24, A01N 43/30, A01P 13/00, C07C 43/225, C07C 309/66, C07C 69/757, C07D 319/08, C07D 327/04, C07D 327/06, C07D 339/06, C07D 339/08, C07C 69/013, C07C 309/73, C07C 251/44, C07C 22/04, C07D 321/10, C07C 69/96, C07C 49/683

(54) **PHENYLSUBSTITUIERTE BICYCLOOKTAN-1,3-DION-DERIVATE**
PHENYL-SUBSTITUTED BICYCLOOCTANE 1,3 DIONE DERIVATIVES
DÉRIVÉS DE BICYCLO-OCTANE-1,3-DIONE PHÉNYLE SUBSTITUÉS

(30) Priorität: 10.10.2008 EP 08166352
(43) Veröffentlichungstag der Anmeldung: 22.06.2011
(73) Patentinhaber: Bayer Intellectual Property GmbH, 40789 Monheim am Rhein (DE)
(72) Erfinder: ANGERMANN, Alfred, 65830 Kriftel (DE); BOJACK, Guido, 65207 Wiesbaden-Naurod (DE); FISCHER, Reiner, 40789 Monheim (DE); LEHR, Stefan, 65835 Liederbach (DE); DITTGEN, Jan, 60316 Frankfurt (DE); FEUCHT, Dieter, 65760 Eschborn (DE); HÄUSER-HAHN, Isolde, 51375 Leverkusen (DE); ROSINGER, Christopher, Hugh, 65719 Hofheim (DE); VOERSTE, Arnd, 50674 Köln (DE)
(74) Vertreter: BIP Patents
(86) Internationale Anmeldenummer: PCT/EP2009/006985
(87) Internationale Veröffentlichungsnummer: WO 2010/040460

(56) Entgegenhaltungen:
- EP-A1- 0 773 920
- WO-A-2007/080066
- WO-A1-2006/056281
- WO-A1-2007/096058
- US-A- 5 808 135
- DATABASE BEILSTEIN BEILSTEIN INSTITUTE FOR ORGANIC CHEMISTRY, FRANKFURT-MAIN, DE; XP002561862 gefunden im XFIRE Database accession no. 2000530 & CHEMISTRY OF NATURAL COMPOUNDS, Bd. 21, Nr. 4, 1985, Seiten 502-509,
- DATABASE BEILSTEIN BEILSTEIN INSTITUTE FOR ORGANIC CHEMISTRY, FRANKFURT-MAIN, DE; XP002561863 gefunden im XFIRE Database accession no. 21994417 & CHEM. ZENTRALBL., Bd. 106, Nr. I, 1935, Seite 2807,

## Beschreibung

Die vorliegende Erfindung betrifft neue phenylsubstituierte Bicyclooktan-1,3-dion-Derivate, mehrere Verfahren zu ihrer Herstellung und ihre Verwendung als Schädlingsbekämpfungsmittel und/oder Herbizide.

Die Erfindung betrifft außerdem neue selektiv-herbizide Wirkstoffkombinationen, die phenylsubstituierte Bicyclooktan-1,3-dion-Derivate einerseits und zumindest eine die Kulturpflanzenverträglichkeit verbessernde Verbindung andererseits enthalten und mit besonders gutem Erfolg zur selektiven Unkrautbekämpfung in verschiedenen Nutzpflanzenkulturen verwendet werden können.

Die vorliegende Erfindung betrifft weiterhin die Steigerung der Wirkung von Pflanzenschutzmitteln enthaltend insbesondere phenylsubstituierte Bicyclooktan-1,3-dion-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Schädlingsbekämpfungsmittel und/oder zur Verhinderung von unerwünschtem Pflanzenwuchs.

Es ist bekannt, dass bestimmte substituierte 2-Arylcyclopentandione, herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO99/48869, WO 99/55673, WO 01/17972, WO 01/74770, WO 03/062244, WO 04/080962, WO04/111042, WO05/092897, WO06/029799, WO07/080066, WO07/096058, WO 09/019005 und WO 09/019015). Außerdem sind ähnlich substituierte Verbindungen bekannt: 3-Hydroxy-5,5-dimethyl-2-phenylcyclopent-2-en-1-on aus der Publikation Micklefield et. al, Tetrahedron, (1992), 7519-26 sowie der Naturstoff Involution (-)-cis-5-(3,4-Dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66 (1973), 584 und der Offenlegungsschrift DE-A 2 361 084 bekannt, mit Angabe von herbiziden und akariziden Wirkungen.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 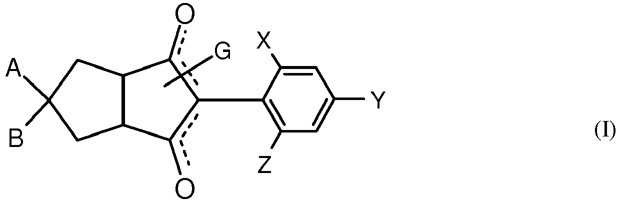 in welcher
- X: für Alkyl, Cycloalkyl oder Alkoxy steht,
- Y: für Wasserstoff, Alkyl oder Alkoxy steht,
- Z: für Wasserstoff, Alkyl oder Cycloalkyl steht,
wobei
- A und B: gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
oder
- A und B: gemeinsam mit dem Kohlenstoffatom an das sie gebunden sind für eine C₁-C₄-Alkylen-Gruppe oder eine =N-OR⁹-Gruppe stehen,
- G: für Wasserstoff (a) oder für eine der Gruppen 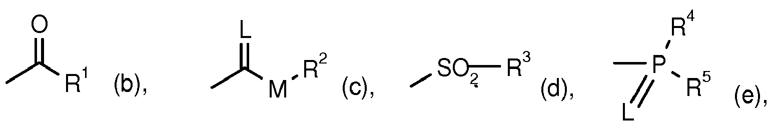 E(f) oder 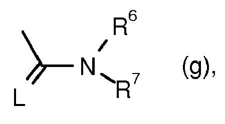 steht,
worin
E für ein Metallionäquivalent oder ein Ammoniumion steht,
L für Sauerstoff oder Schwefel steht,
M für Sauerstoff oder Schwefel steht,
- R¹: für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen,
- R⁹: für Wasserstoff, für jeweils gegebenenfalls substituiertes Alkyl, Cycloalkyl, CH₂ Cycloalkyl, Alkenyl, Alkinyl, Arylalkyl oder Hetarylalkyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Die Verbindungen der Formel (I) können in Abhängigkeit von der Stellung des Substituenten G in den zwei isomeren Formen der Formeln (I-A) und (I-B) vorliegen, 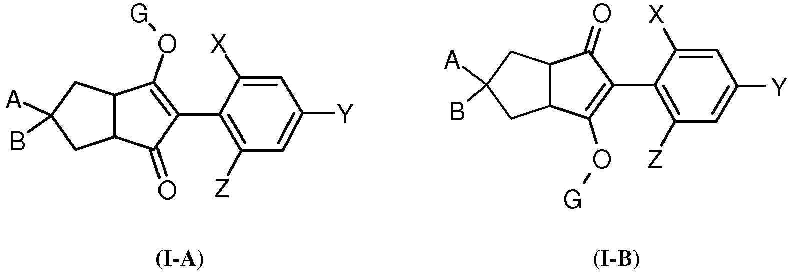 was durch die gestrichelte Linie in der Formel (I) zum Ausdruck gebracht werden soll.

Die Verbindungen der Formeln (I-A) und (I-B) können sowohl als Gemische als auch in Form ihrer reinen Isomeren vorliegen. Gemische der Verbindungen der Formeln (I-A) und (I-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-a) bis (I-g): 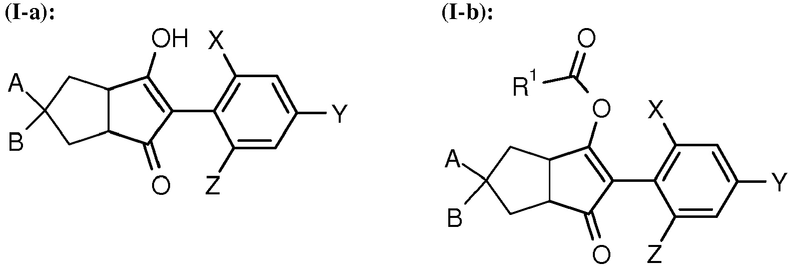 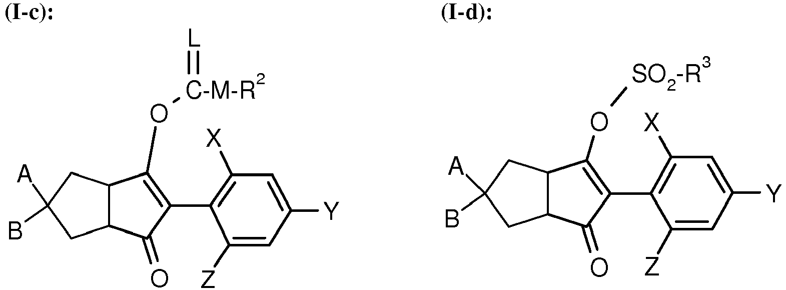 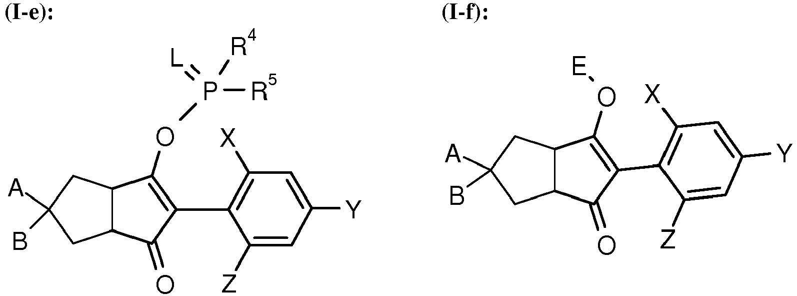 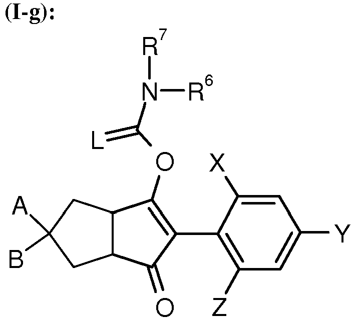 worin
A, B, E, L, M, X, Y, Z, R¹, R², R³, R⁴, R⁵, R⁶ und R⁷ die oben angegebenen Bedeutungen besitzen.

Eine weitere Form von Stereoisomerie ergibt sich aus der cis-Verknüpfung der beiden carbacyclischen Fünfringe und zwar für den Fall, daß speziell die beiden Substituenten A und B in der Formel (Ia) nicht identisch sind.

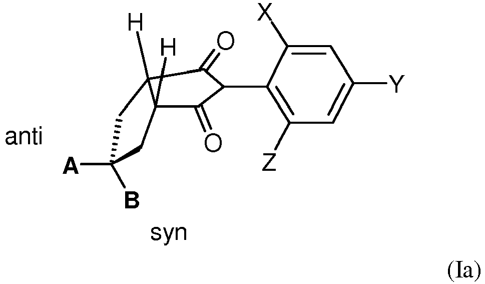

Die dann auftretenden Isomeren werden im Folgenden mit "syn" bzw "anti" bezeichnet, je nachdem ob der gemäß Cahn-Ingold-Prelog-Regeln zu priorisierende Substituent A oder B in anti- oder syn-Position zum Cyclopentandion-Ring steht. Nachfolgend werden zwei Beispiele für derartige Isomerieformen bei den Verbindungen der Formel (I-a) genannt:

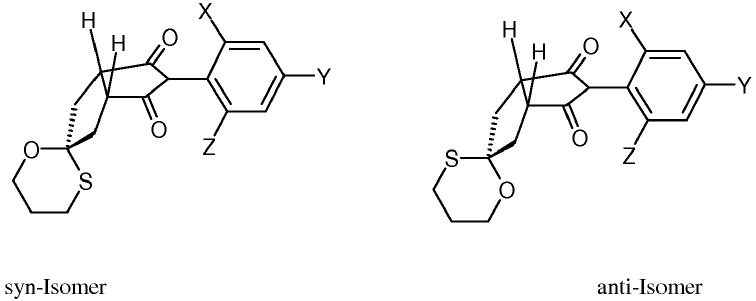

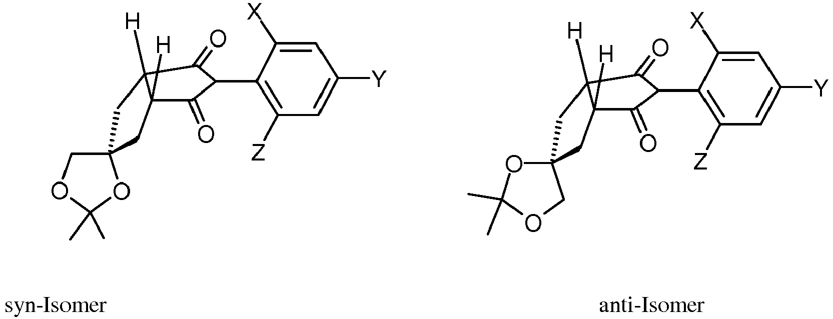

Weiterhin wurde gefunden, dass man die neuen Verbindungen der Formel (I) nach einem der im folgenden beschriebenen Verfahren erhält:
(A) Man erhält Verbindungen der Formel (I-a) 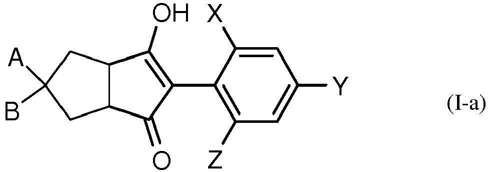 in welcher
   A, B, X, Y und Z die oben angegebene Bedeutung haben,
   wenn man
   Ketocarbonsäureester der Formel (II) 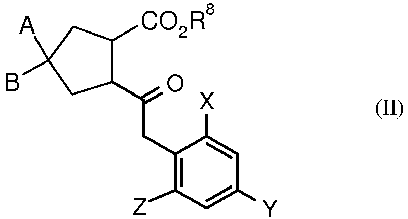 in welcher
   A, B, X, Y und Z die oben angegebene Bedeutung haben, und
   R⁸ für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden
(B) dass man die Verbindungen der oben gezeigten Formel (I-b), in welcher A, B, R¹, X, Y und Z die oben angegebenen Bedeutungen haben, erhält, wenn man Verbindungen der oben gezeigten Formel (I-a), in welcher A, B, X, Y und Z die oben angegebenen Bedeutungen haben, jeweils
   (α) mit Säurehalogeniden der Formel (III) 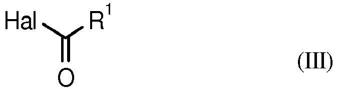 in welcher
      R¹ die oben angegebene Bedeutung hat und
      Hal für Halogen (insbesondere Chlor oder Brom) steht
      oder
   (ß) mit Carbonsäureanhydriden der Formel (IV)

      R¹-CO-O-CO-R¹ (IV)

      in welcher
      R¹ die oben angegebene Bedeutung hat,
      gegebenenfalls in Gegenwart eines Verdünnungsmittels und gegebenenfalls in Gegenwart eines Säurebindemittels umsetzt;
(C) dass man die Verbindungen der oben gezeigten Formel (I-c), in welcher A, B, R², M, X, Y und Z die oben angegebenen Bedeutungen haben und L für Sauerstoff steht, erhält, wenn man Verbindungen der oben gezeigten Formel (I-a), in welcher A, B, X, Y und Z die oben angegebenen Bedeutungen haben, jeweils
   mit Chlorameisensäureestern oder Chlorameisensäurethioestern der Formel (V)

   R²-M-CO-Cl (V)

   in welcher
   R² und M die oben angegebenen Bedeutungen haben, gegebenenfalls in Gegenwart eines Verdünnungsmittels und gegebenenfalls in Gegenwart eines Säurebindemittels umsetzt;
(D) dass man Verbindungen der oben gezeigten Formel (I-c), in welcher A, B, R², M, X, Y und Z die oben angegebenen Bedeutungen haben und L für Schwefel steht, erhält, wenn man Verbindungen der oben gezeigten Formel (I-a), in welchen A, B, X, Y und Z die oben angegebenen Bedeutungen haben, jeweils
   mit Chlormonothioameisensäureestern oder Chlordithioameisensäureestern der Formel (VI) 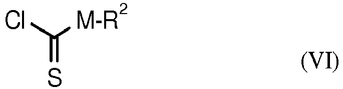 in welcher
   M und R² die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formel (I-d), in welcher A, B, R³, X, Y und Z die oben angegebenen Bedeutungen haben, erhält, wenn man Verbindungen der oben gezeigten Formel (I-a), in welcher A, B, X, Y und Z die oben angegebenen Bedeutungen haben, jeweils
   mit Sulfonsäurechloriden der Formel (VII)

   R³-SO₂-Cl (VII)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(F) dass man Verbindungen der oben gezeigten Formel (I-e), in welcher A, B, L, R⁴, R⁵, X, Y und Z die oben angegebenen Bedeutungen haben, erhält, wenn man Verbindungen der oben gezeigten Formel (I-a), in welchen A, B, X, Y und Z die oben angegebenen Bedeutungen haben, jeweils
   mit Phosphorverbindungen der Formel (VIII) 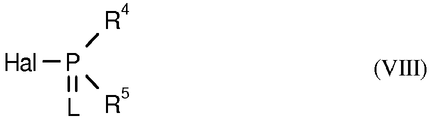 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt,
(G) dass man Verbindungen der oben gezeigten Formel (I-f), in welcher A, B, E, X, Y und Z die oben angegebenen Bedeutungen haben, erhält, wenn man Verbindungen der Formel (I-a), in welcher A, B, X, Y und Z die oben angegebenen Bedeutungen haben, jeweils mit Metallverbindungen oder Aminen der Formeln (IX) oder (X)

   Me(OR¹⁰)ₜ (IX)

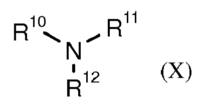

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(H) dass man Verbindungen der oben gezeigten Formel (I-g), in welcher A, B, L, R⁶, R⁷, X, Y und Z die oben angegebenen Bedeutungen haben, erhält, wenn man Verbindungen
   der oben gezeigten Formel (I-a), in welcher A, B, X, Y und Z die oben angegebenen Bedeutungen haben, jeweils
   (α) mit Isocyanaten oder Isothiocyanaten der Formel (XI)

      R⁶-N=C=L (XI)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   (ß) mit Carbamidsäurechloriden oder Thiocarbamidsäurechloriden der Formel (XII) 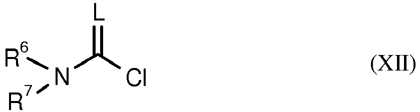 in welcher
      L, R⁶ und R⁷ die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safener/Antidots) ausgesprochen gut die Schädigung der Kulturpflanzen verhindert und besonders vorteilhaft als breit wirksame Kombinationspräparate zur selektiven Bekämpfung von unerwünschten Pflanzen in Nutzpflanzenkulturen, wie z.B. in Getreide aber auch Mais, Kartoffeln, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher A, B, G, X, Y und Z die oben angegebene Bedeutung haben
   und
(b') zumindest eine die Kulturpflanzen-Verträglichkeit verbessernde Verbindung (Safener).
   Die Safener sind vorzugsweise ausgewählt aus der Gruppe bestehend aus: 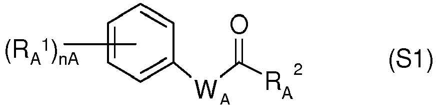
   S1) Verbindungen der Formel (S1),
      wobei die Symbole und Indizes folgende Bedeutungen haben:
      - n_{A}: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl;
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),

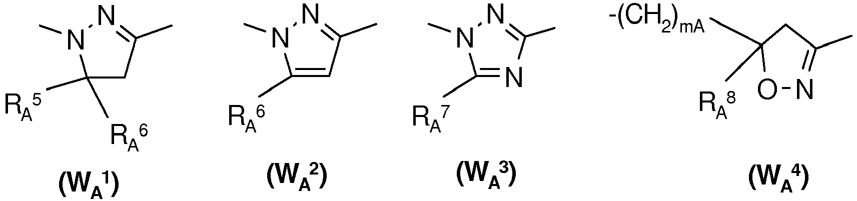

      - m_{A}: ist 0 oder 1;
      - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
      - R_{A}³: ist Wasserstoff oder ein unsubstituierter oder substituierter aliphatischer Kohlenwasserstoffrest, vorzugsweise mit insgesamt 1 bis 18 C-Atomen;
      - R_{A}⁴: ist Wasserstoff, (C₁-C₆)Alkyl, (C₁-C₆)Alkoxy oder substituiertes oder unsubstituiertes Phenyl;
      - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
      - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
      vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
   S2) Chinolinderivate der Formel (S2), 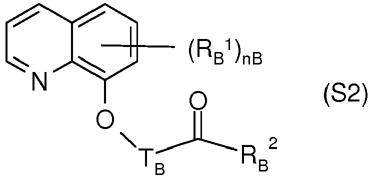 wobei die Symbole und Indizes folgende Bedeutungen haben:
      - R_{B}¹: ist Halogen, (C₁-C₄)Alkyl, (C₁-C₄)Alkoxy, Nitro oder (C₁-C₄)Haloalkyl;

      - n_{B}: ist eine natürliche Zahl von 0 bis 5, vorzugsweise 0 bis 3;
      - R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
      - R_{B}³: ist Wasserstoff oder ein unsubstituierter oder substituierter aliphatischer Kohlenwasserstoffrest, vorzugsweise mit insgesamt 1 bis 18 C-Atomen;
      - R_{B}⁴: ist Wasserstoff, (C₁-C₆)Alkyl, (C₁-C₆)Alkoxy oder substituiertes oder unsubstituiertes Phenyl;
      - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
      vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolin-oxy)malonsäure-methyl-ethylester und verwandte Verbindungen, wie sie in EP-A-0 582 198 beschrieben sind.
   S3) Verbindungen der Formel (S3) 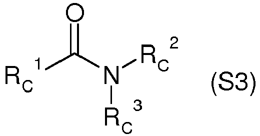 wobei die Symbole und Indizes folgende Bedeutungen haben:
      R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
      R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
      vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4),
      "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8), "Diclonon" (Dicyclonon) oder "BAS145138" oder "LAB145138" (S3-9)
      (3-Dichloracetyl-2,5,5-trimethyl-1,3-diazabicyclo[4.3.0]nonan) der Firma BASF, "Furilazol" oder "MON 13900" ((RS)-3-Dichloracetyl-5-(2-furyl)-2,2-dimethyl-oxazolidin) (S3-10); sowie dessen (R)-Isomer (S3-11).
   S4) N-Acylsulfonamide der Formel (S4) und ihre Salze, 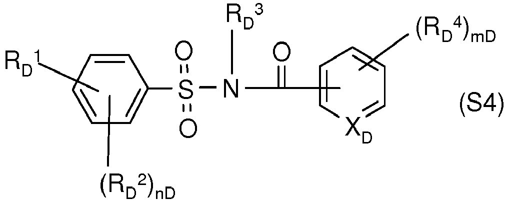 worin die Symbole und Indizes folgende Bedeutungen haben:
      - X_{D}: ist CH oder N;
      - R_{D}¹: ist CO-NR_{D}⁵R_{D}⁶ oder NHCO-R_{D}⁷;
      - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
      - R_{D}³: ist Wasserstoff, (C₁-C₄)Alkyl, (C₂-C₄)Alkenyl oder (C₂-C₄)Alkinyl;
      - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;

      - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
      - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
      - R_{D}⁵ und R_{D}⁶: gemeinsam mit dem dem sie tragenden Stickstoffatom einen Pyrrolidinyl- oder Piperidinyl-Rest bilden;
      - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - n_{D}: ist 0, 1 oder 2;
      - m_{D}: ist 1 oder 2;
      - v_{D}: ist 0, 1, 2 oder 3;
      davon bevorzugt sind Verbindungen von Typ der N-Acylsulfonamide, z.B. der nachfolgenden Formel (S4^{a}), die z. B. bekannt sind aus WO-A-97/45016 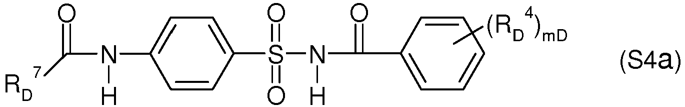 worin
      - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - R_{D}⁴: Halogen, (C₁-C₄)Alkyl, (C₁-C₄)Alkoxy, CF_{3;}
      - m_{D}: 1 oder 2;
      - v_{D}: ist 0, 1, 2 oder 3 bedeutet;
      sowie
      Acylsulfamoylbenzoesäureamide, z.B. der nachfolgenden Formel (S4^{b}), die z.B. bekannt sind aus WO-A-99/16744, 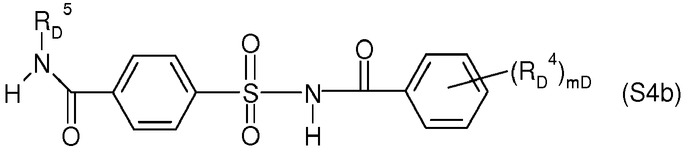
      - z.B.: solche worin
      - R_{D}⁵: = Cyclopropyl und (R_{D}⁴) = 2-OMe ist ("Cyprosulfamide", S4-1),
      - R_{D}⁵: = Cyclopropyl und (R_{D}⁴) = 5-Cl-2-OMe ist (S4-2),
      - R_{D}⁵: = Ethyl und (R_{D}⁴) = 2-OMe ist (S4-3),
      - R_{D}⁵: = Isopropyl und (R_{D}⁴) = 5-Cl-2-OMe ist (S4-4) und
      - R_{d}⁵: = Isopropyl und (R_{D}⁴) = 2-OMe ist (S4-5)
      sowie
      Verbindungen vom Typ der N-Acylsulfamoylphenylharnstoffe der Formel (S4^{c}), die z.B. bekannt sind aus der EP-A-365484, 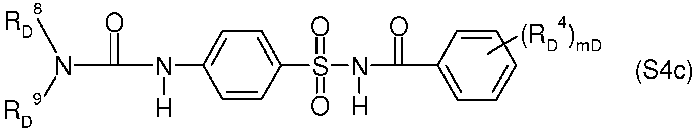 worin
      - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
      - R_{D}⁴: Halogen, (C₁-C₄)Alkyl, (C₁-C₄)Alkoxy, CF₃
      - m_{D}: 1 oder 2 bedeutet;
      beispielsweise
      1-[4-(N-2-Methoxybenzoylsulfamoyl)phenyl]-3-methylharnstoff, 1-[4-(N-2-Methoxybenzoylsulfamoyl)phenyl]-3,3-dimethylharnstoff, 1-[4-(N-4,5-Dimethylbenzoylsulfamoyl)phenyl] -3-methylharnstoff.
   S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
   S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
   S7) Verbindungen der Formel (S7),wie sie in der WO-A-1998/38856 beschrieben sind 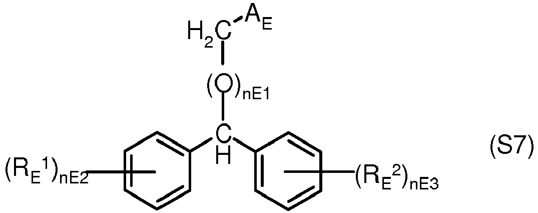 worin die Symbole und Indizes folgende Bedeutungen haben:
      - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
      - A_{E}: ist COOR_{E}³ oder COSR_{E}⁴
      - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
      - n_{E}¹: ist 0 oder 1
      - n_{E}², n_{E}³: sind unabhängig voneinander 0, 1 oder 2,

      vorzugsweise:
         Diphenylmethoxyessigsäure,
         Diphenylmethoxyessigsäureethylester,
         Diphenylmethoxyessigsäuremethylester (CAS-Reg.Nr. 41858-19-9) (S7-1).
   S8) Verbindungen der Formel (S8),wie sie in der WO-A-98/27049 beschrieben sind 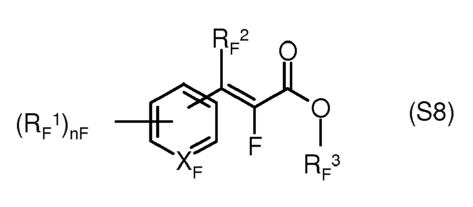 worin
      - X_{F}: CH oder N,
      - n_{F}: für den Fall, dass X_{F}=N ist, eine ganze Zahl von 0 bis 4 und für den Fall, dass X_{F}=CH ist, eine ganze Zahl von 0 bis 5 ,
      - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,

      - R_{f}²: Wasserstoff oder (C₁-C₄)Alkyl
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
      vorzugsweise Verbindungen worin
      - X_{F}: CH,
      - n_{F}: eine ganze Zahl von 0 bis 2 ,
      - R_{F}¹: Halogen, (C₁-C₄)Alkyl, (C₁-C₄)Haloalkyl, (C₁-C₄)Alkoxy, (C₁-C₄)Haloalkoxy,
      - R_{F}²: Wasserstoff oder (C₁-C₄)Alkyl,
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
   S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
   S10) Verbindungen der Formeln (S10^{a}) oder (S10^{b})
      wie sie in der WO-A-2007/023719 und WO-A-2007/023764 beschrieben sind 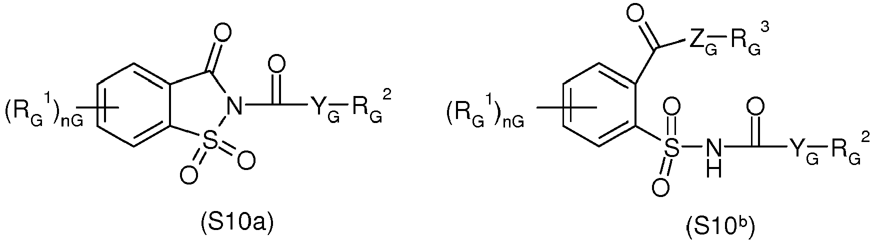 worin
      - R_{G}¹: Halogen, (C₁-C₄)Alkyl, Methoxy, Nitro, Cyano, CF₃, OCF₃
      - Y_{G}, Z_{G}: unabhängig voneinander O oder S,
      - n_{G}: eine ganze Zahl von 0 bis 4,
      - R_{G}²: (C₁-C₁₆)Alkyl, (C₂-C₆)Alkenyl, (C₃-C₆)Cycloalkyl, Aryl; Benzyl, Halogenbenzyl,
      - R_{G}³: Wasserstoff oder (C₁-C₆)Alkyl bedeutet.
   S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
      "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
      "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
   S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
   S13) Eine oder mehrere Verbindungen aus Gruppe (S13):
      "Naphthalic anhydrid" (1,8-Naphthalindicarbonsäureanhydrid) (S13-1), das als Saatbeiz-Safener für Mais gegen Schäden von Thiocarbamatherbiziden bekannt ist,
      "Fenclorim" (4,6-Dichlor-2-phenylpyrimidin) (S13-2), das als Safener für Pretilachlor in gesätem Reis bekannt ist,
      "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
      "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
      "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
      "MG-838" (CAS-Reg.Nr. 133993-74-5) (2-propenyl 1-oxa-4-azaspiro[4.5]decane-4-carbodithioate) (S 13-6) der Firma Nitrokemia,
      "Disulfoton" (O,O-Diethyl S-2-ethylthioethyl phosphordithioat) (S13-7),
      "Dietholate" (O,O-Diethyl-O-phenylphosphorotioat) (S13-8),
      "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
      "Daimuron" oder "SK 23" (1-(1-Methyl-1-phenylethyl)-3-p-tolyl-harnstoff), das als Safener für Reis gegen Schäden des Herbizids Imazosulfuron bekannt ist,
      "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
      "Methoxyphenon" oder "NK 049" (3,3'-Dimethyl-4-methoxy-benzophenon), das als Safener für Reis gegen Schäden einiger Herbizide bekannt ist,
      "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
   S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Cloquintocet-mexyl und Mefenpyr-diethyl besonders hervorgehoben seien. Cyprosulfamide (S4-1) ist ebenfalls hervorgehoben.

Beispiele für die erfindungsgemäßen selektiv herbiziden Kombinationen aus jeweils einem Wirkstoff der Formel (I) und jeweils einem der oben definierten Safener sind nachstehend aufgeführt. Kombinationen aus jeweils einem Wirkstoff der Formel (I), wobei A und B gemeinsam mit dem Kohlenstoffatom an das sie gebunden sind für eine Carbonylgruppe stehen, sind nicht erfindungsgemäß.

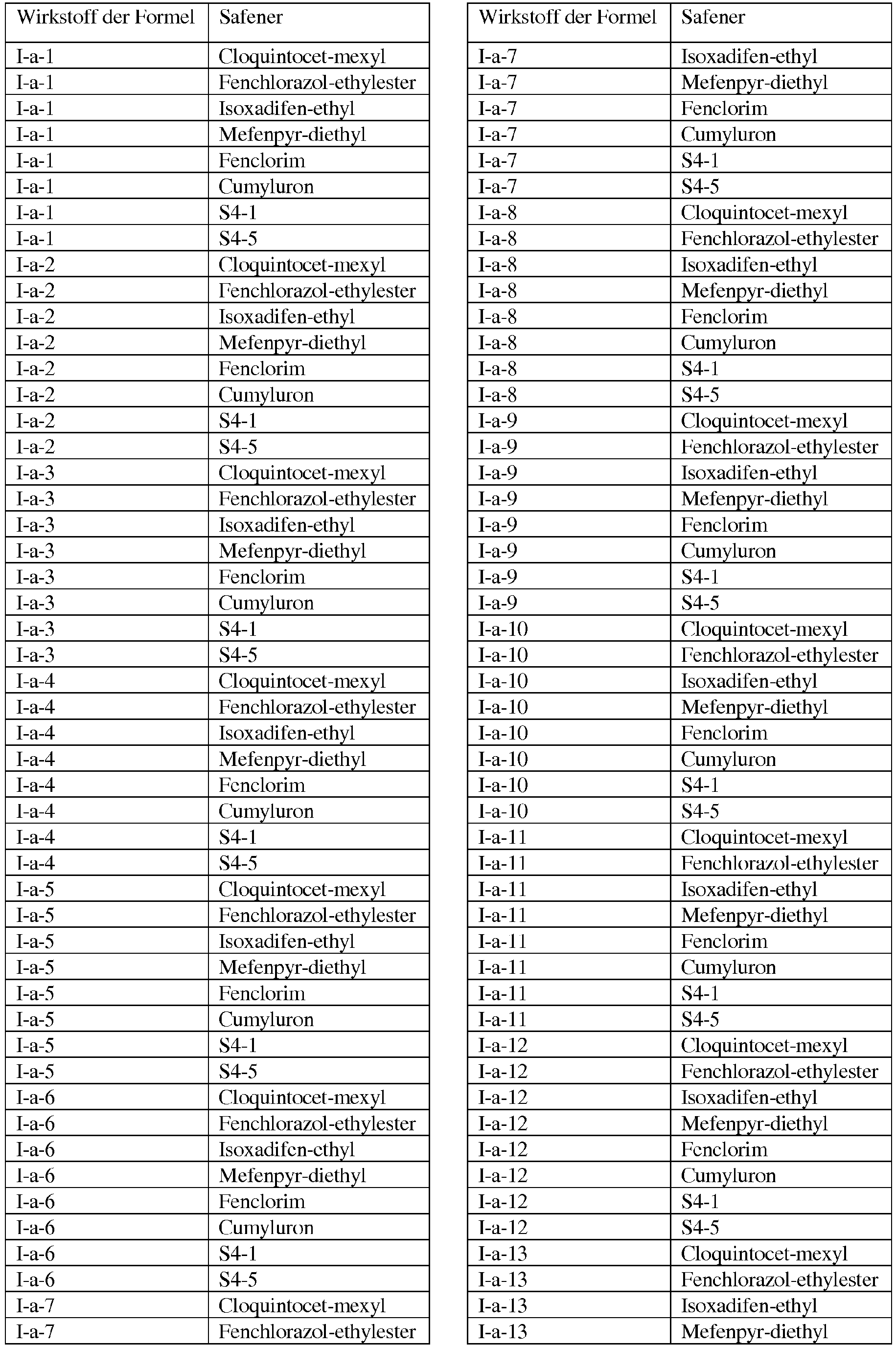

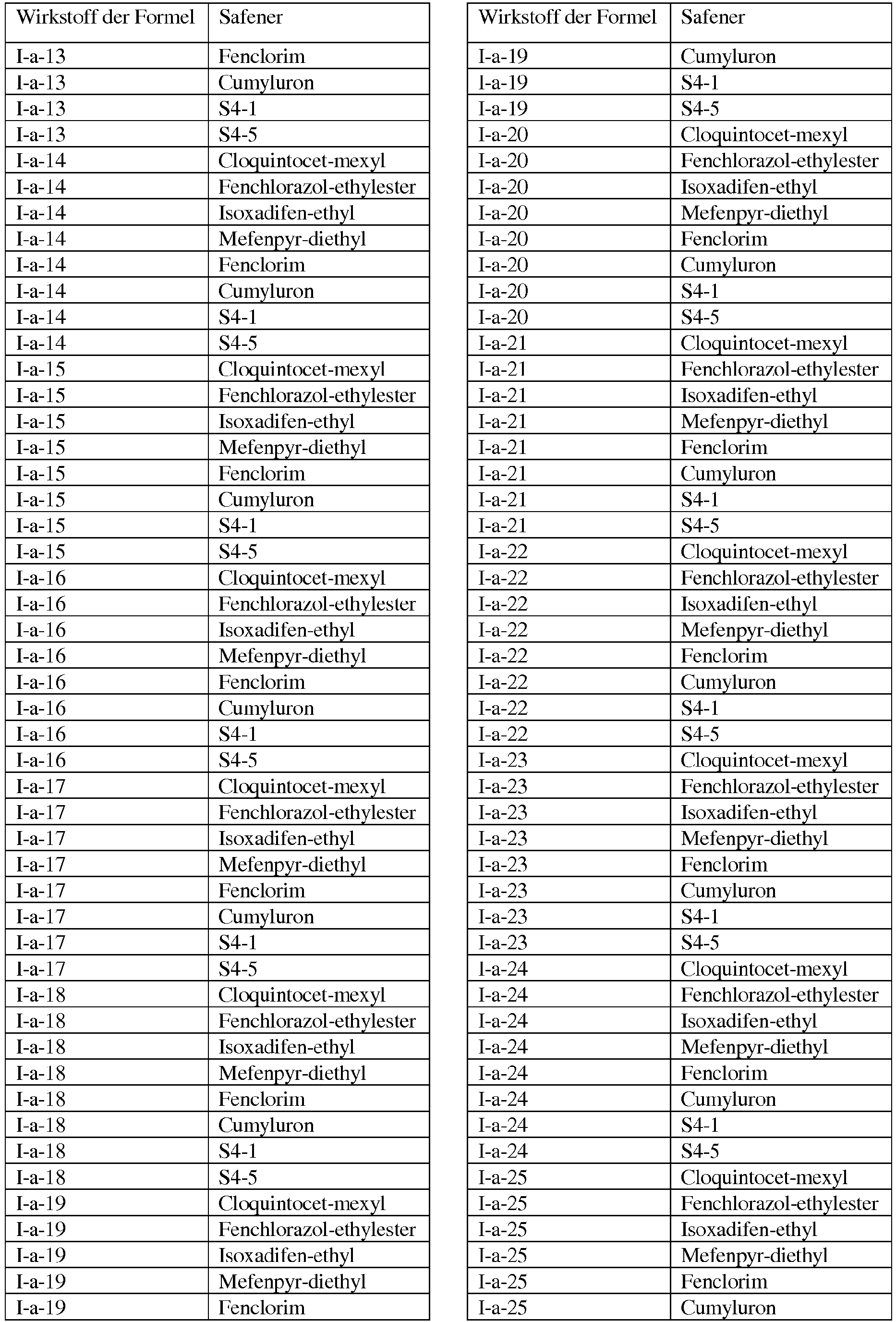

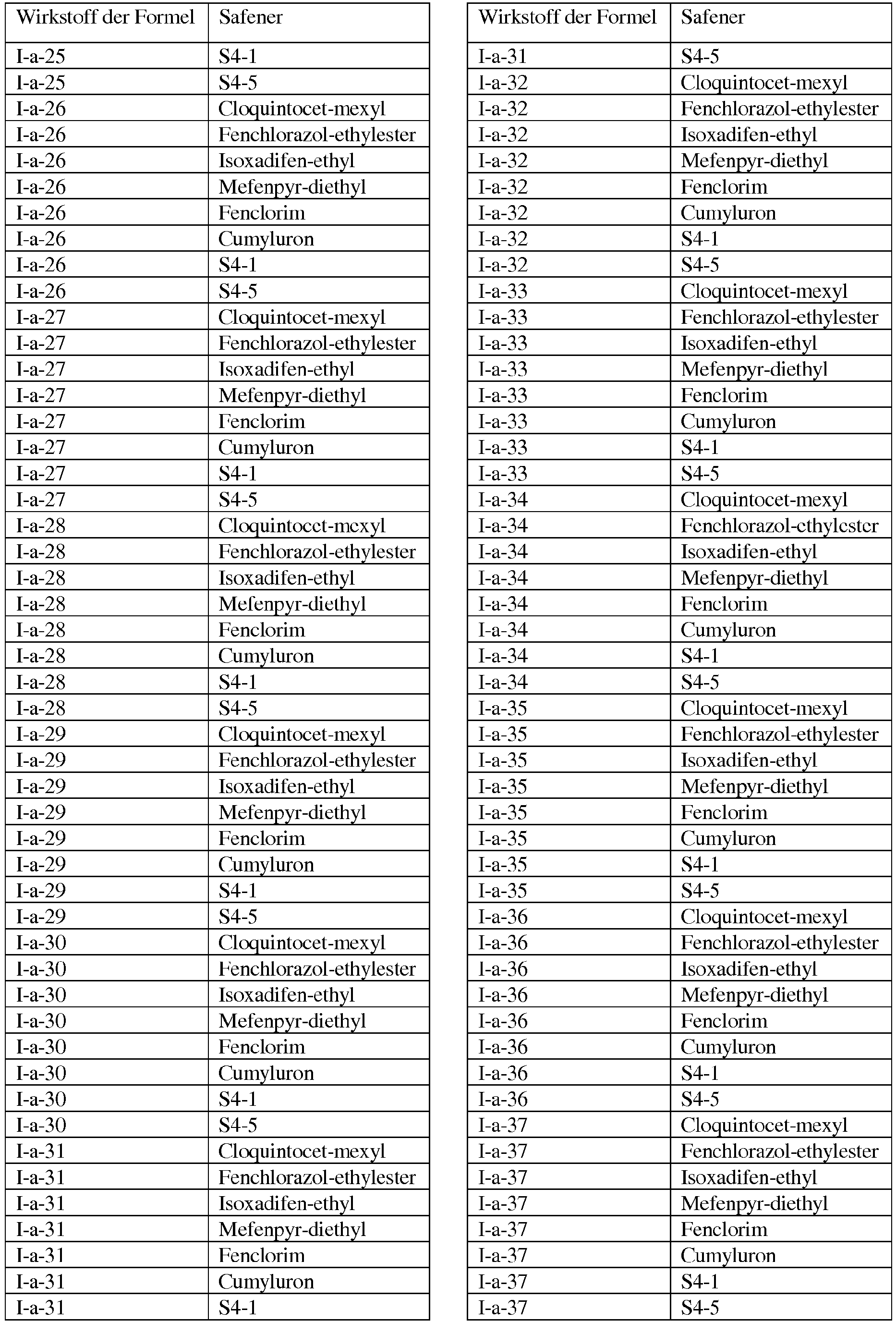

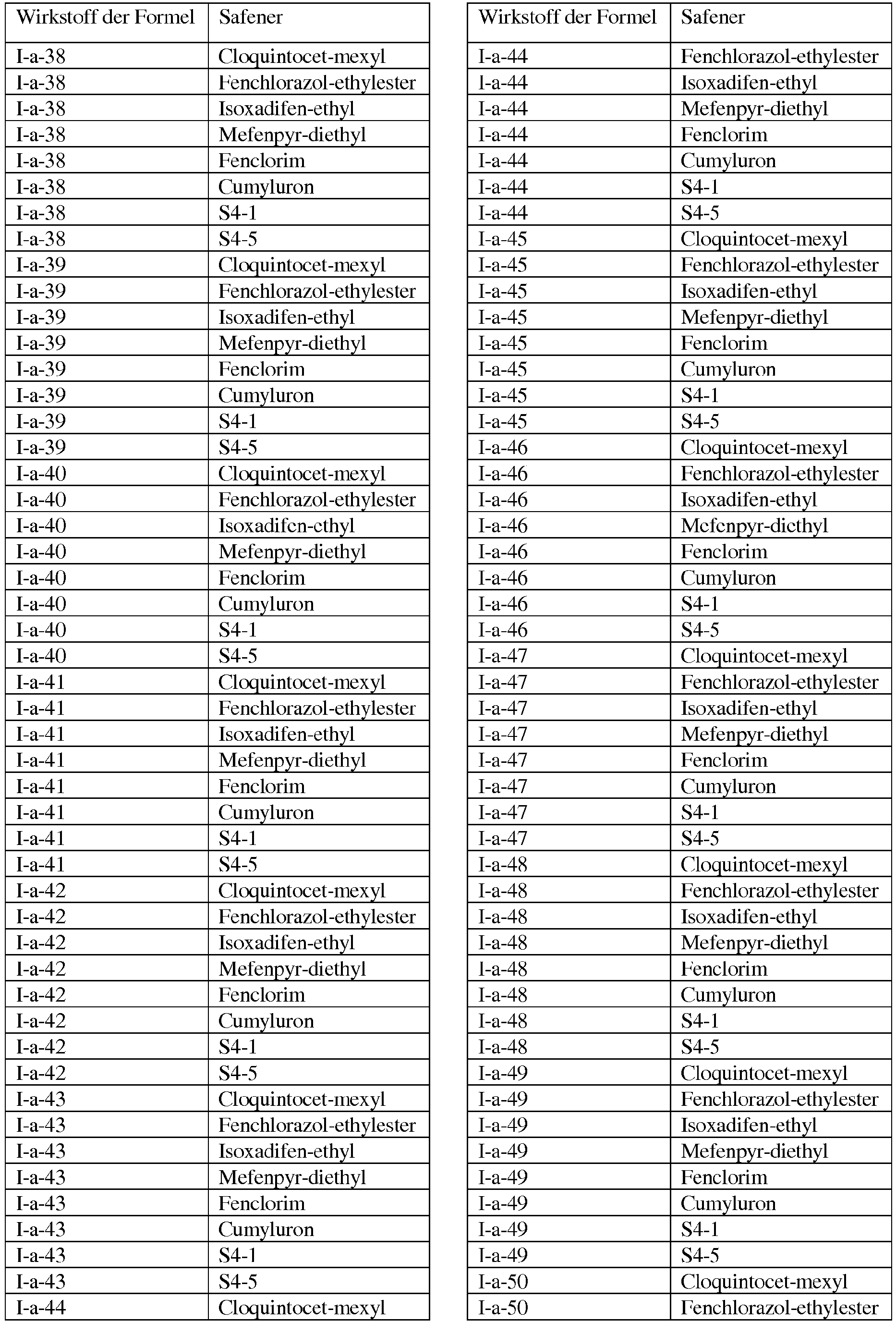

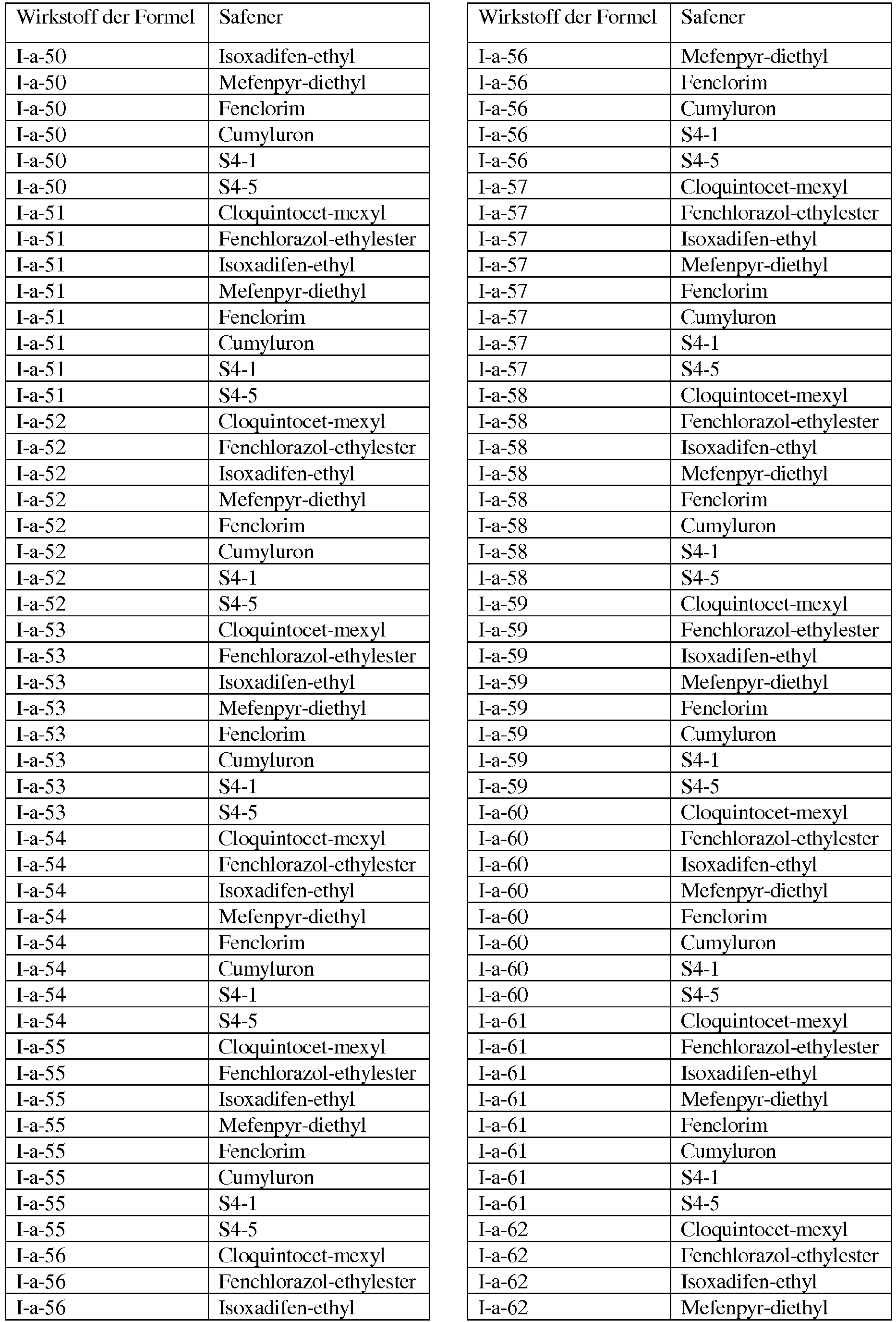

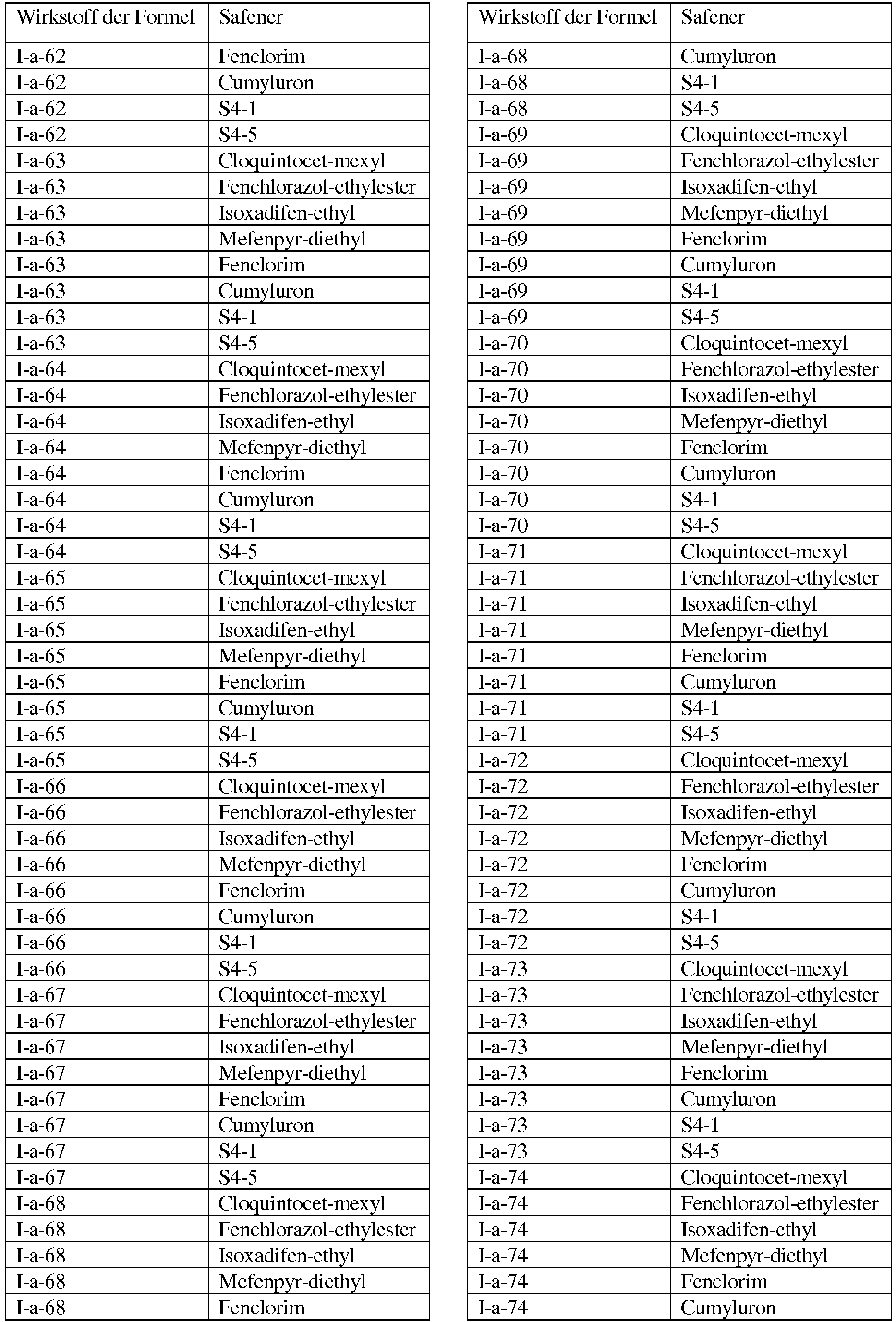

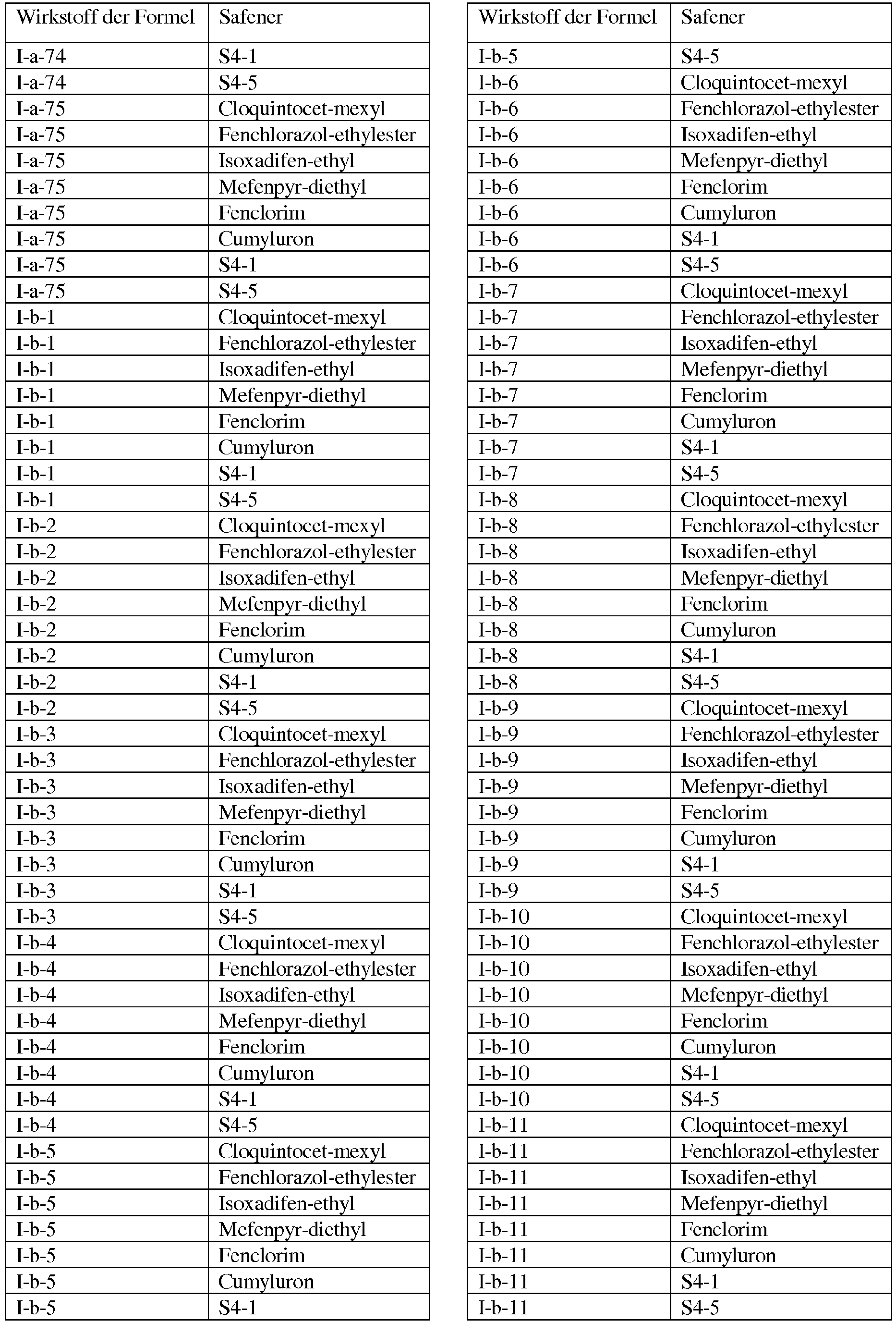

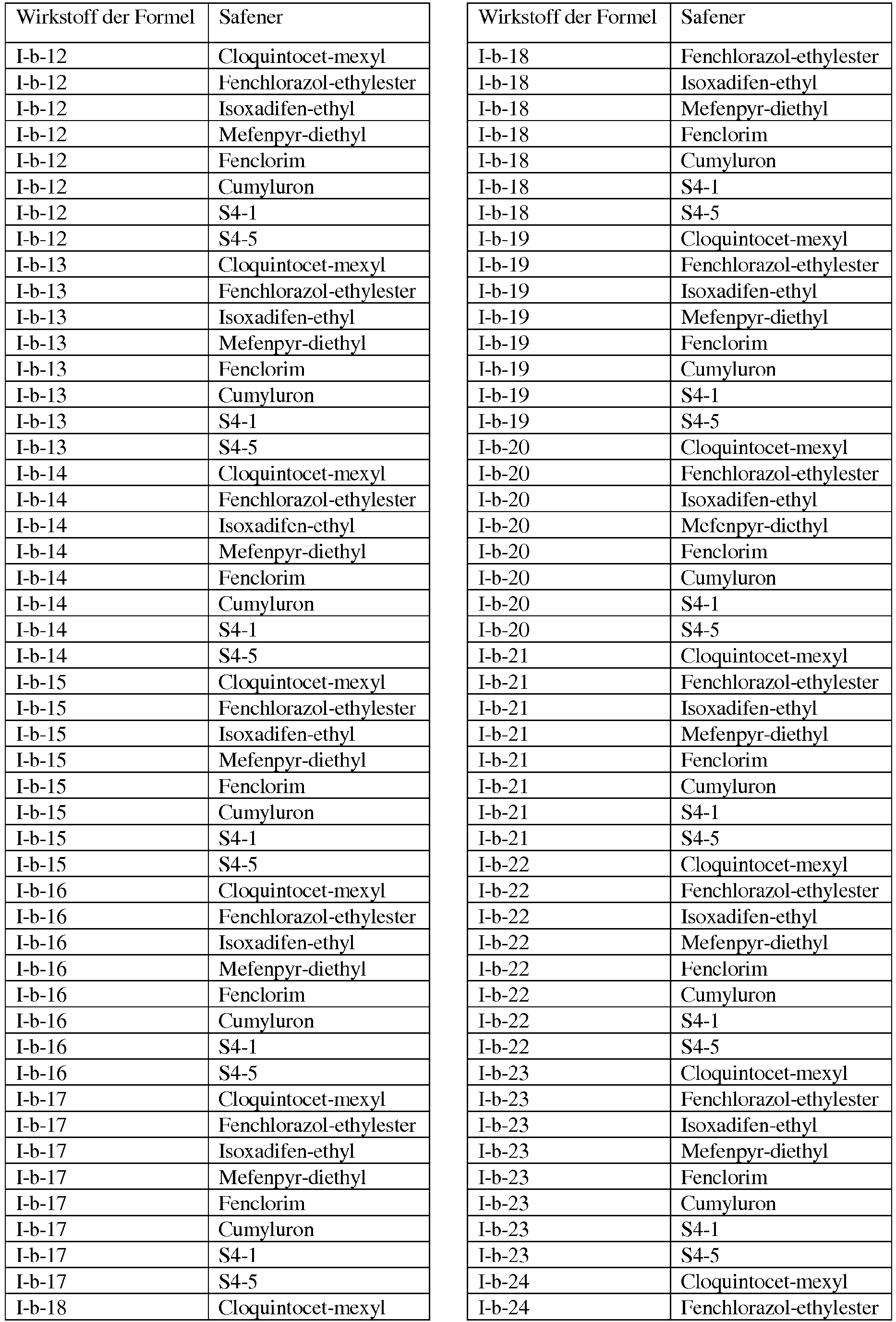

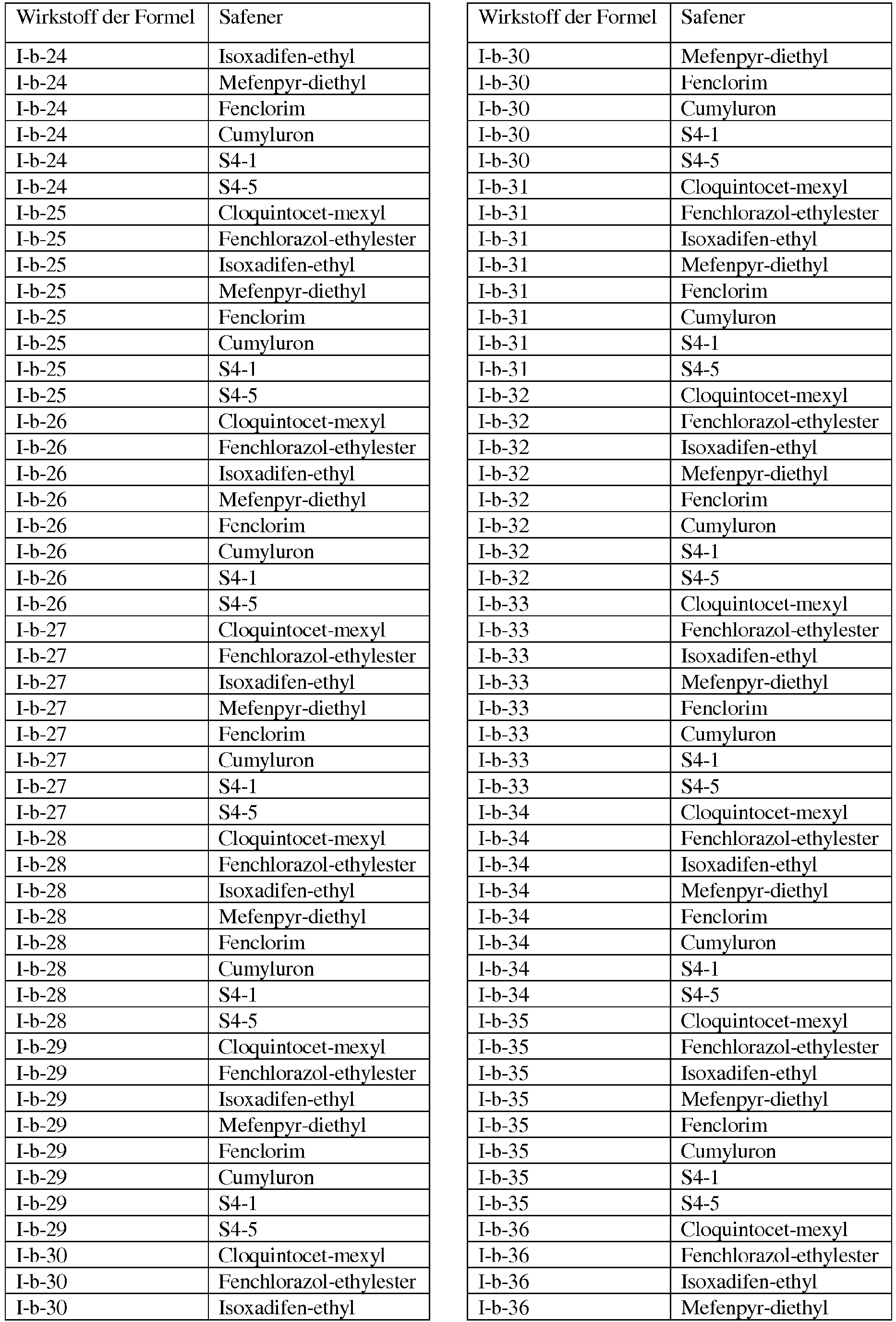

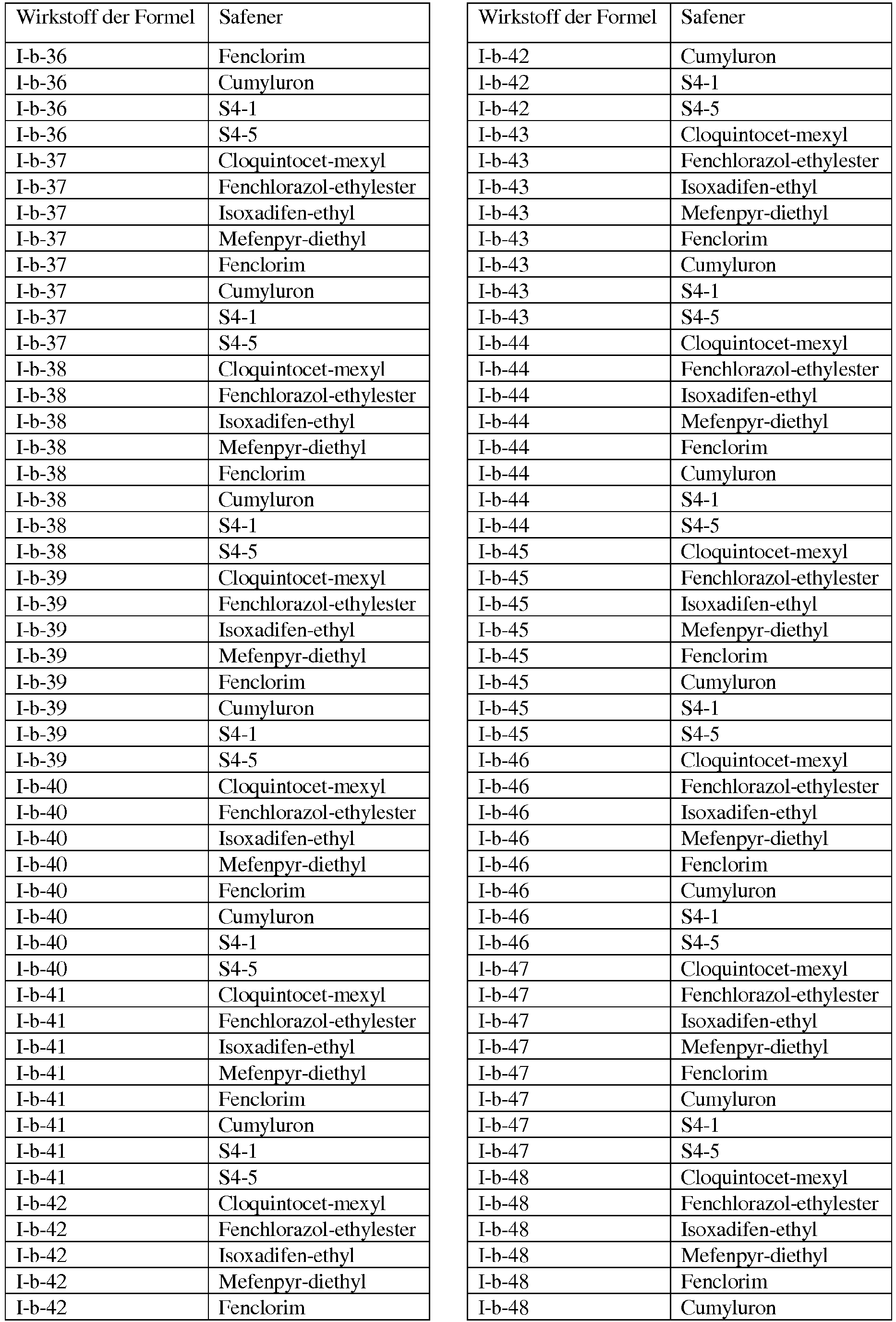

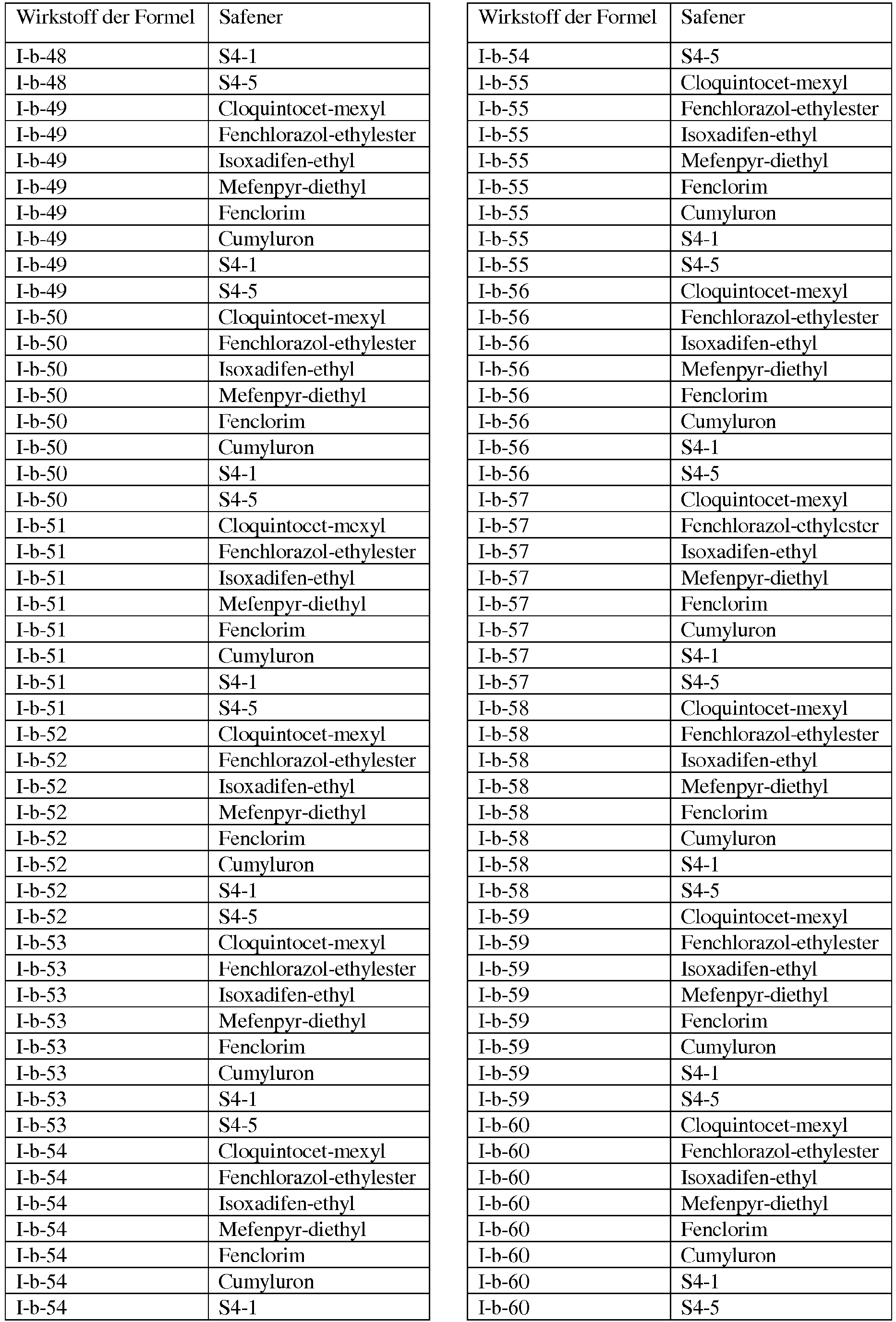

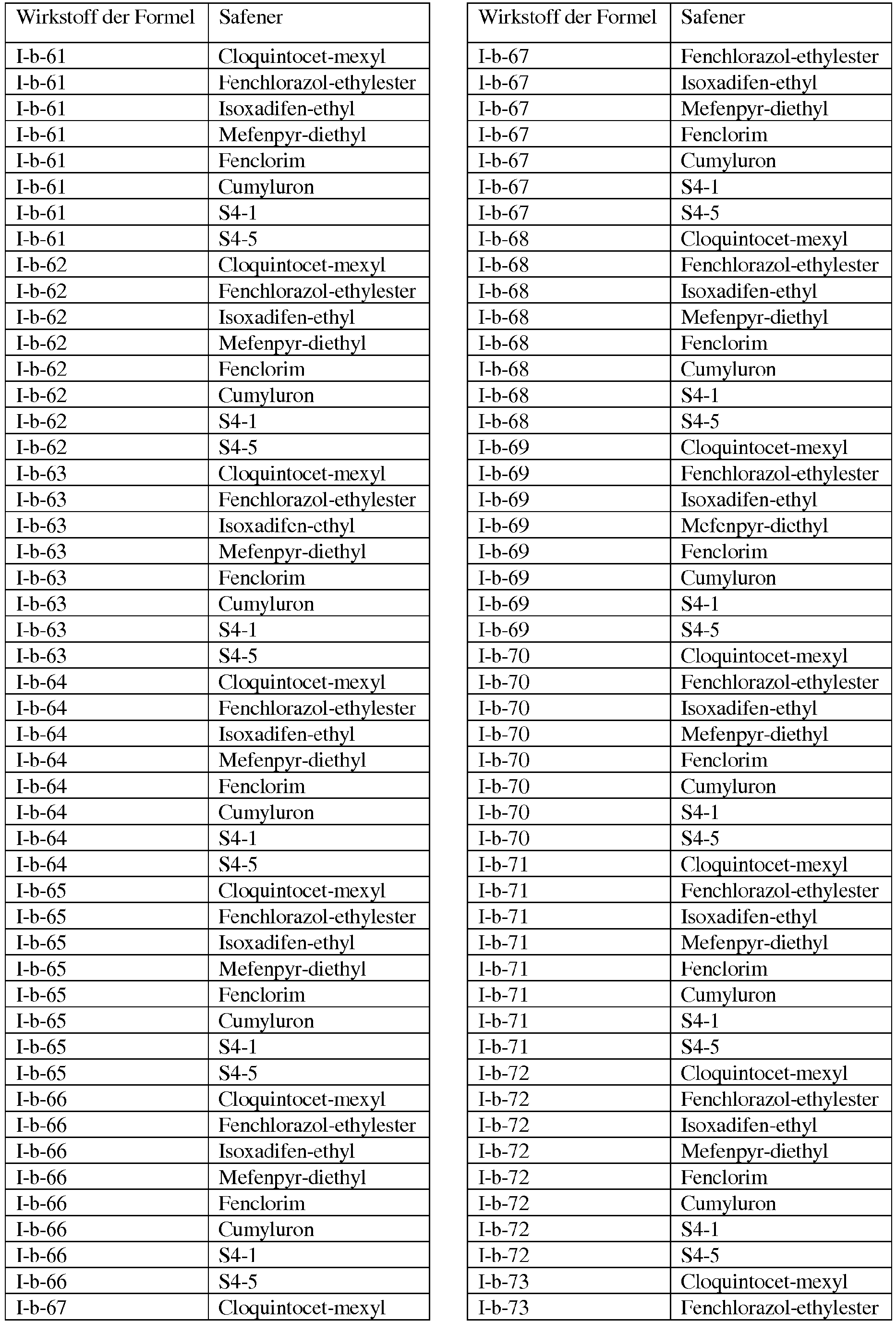

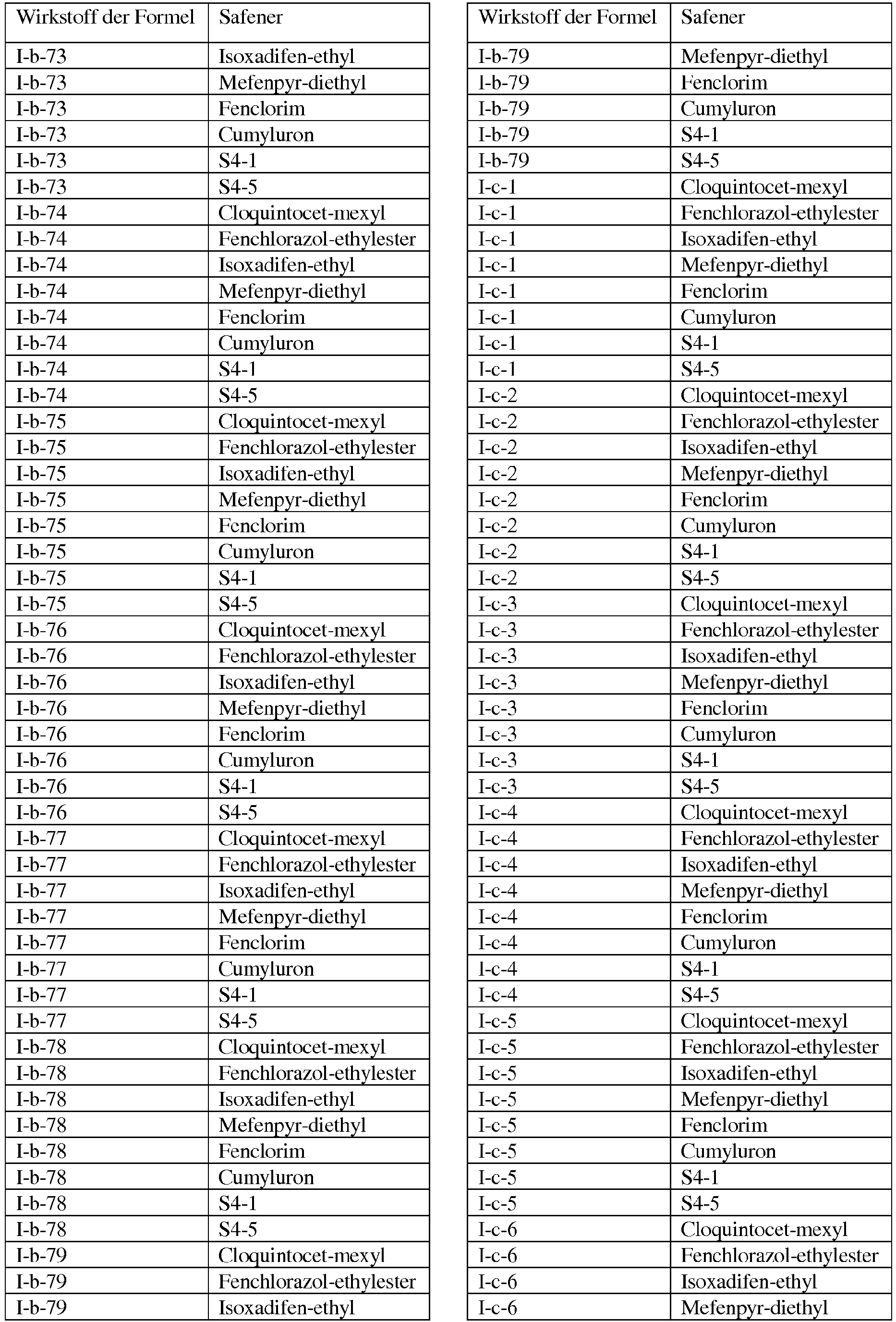

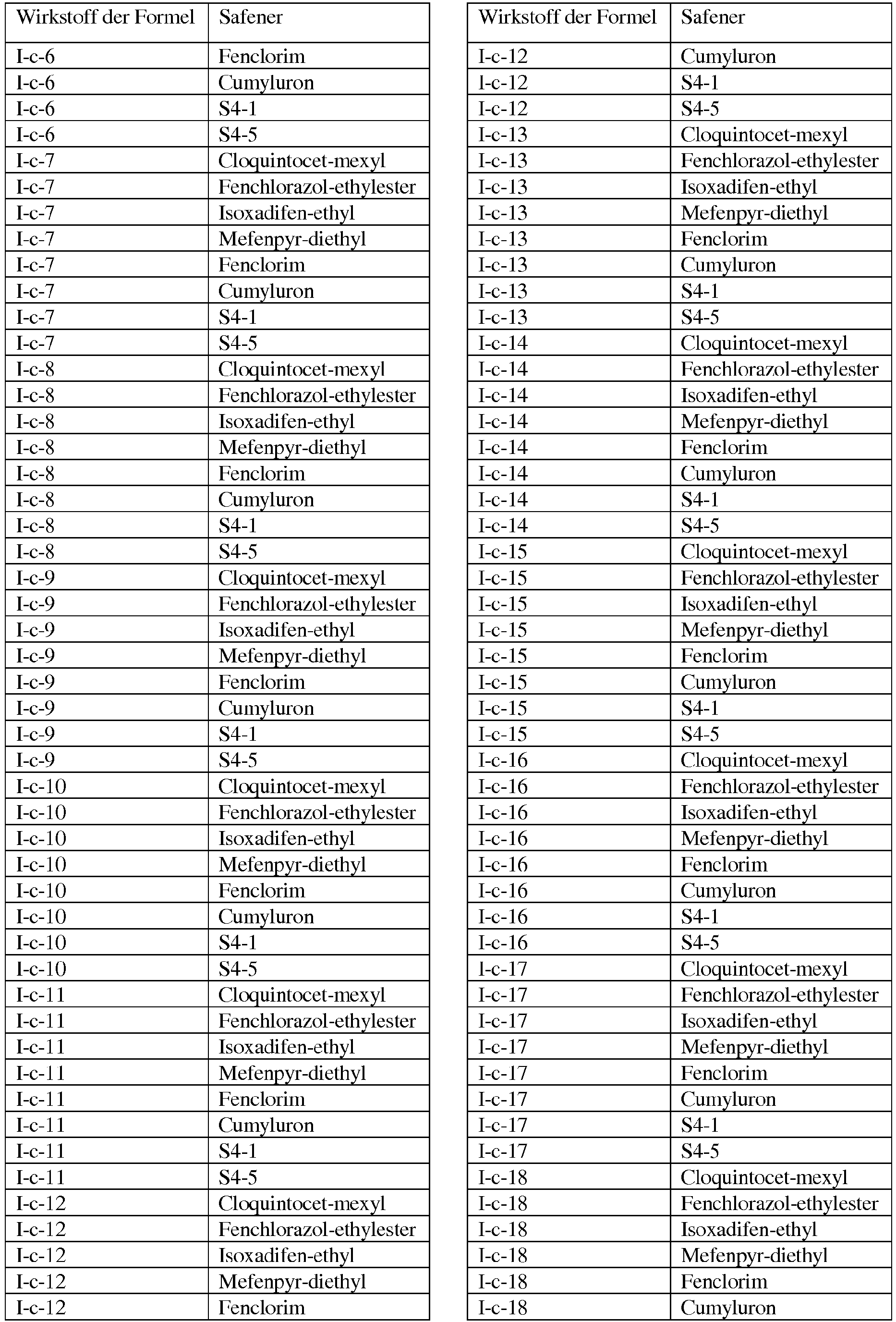

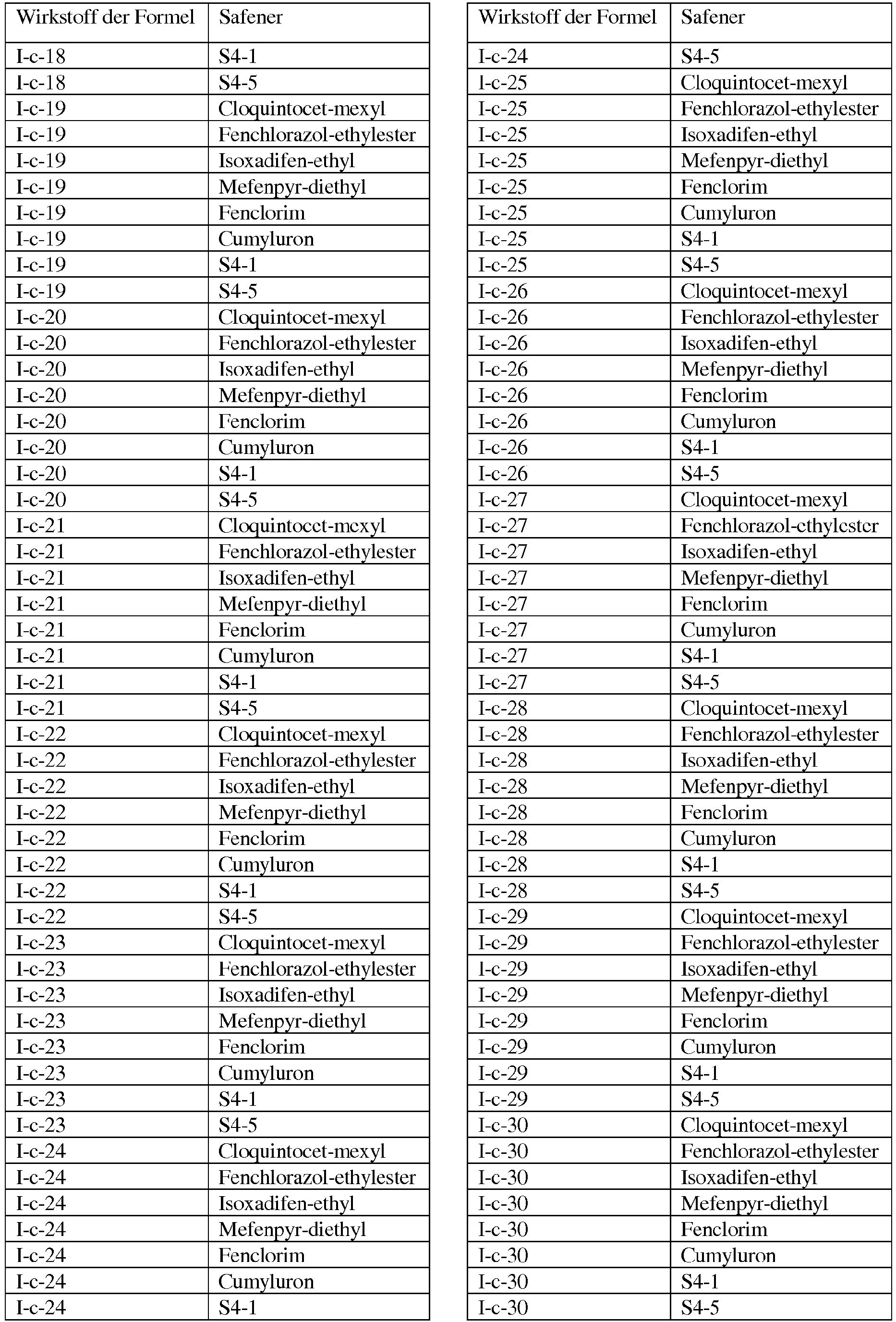

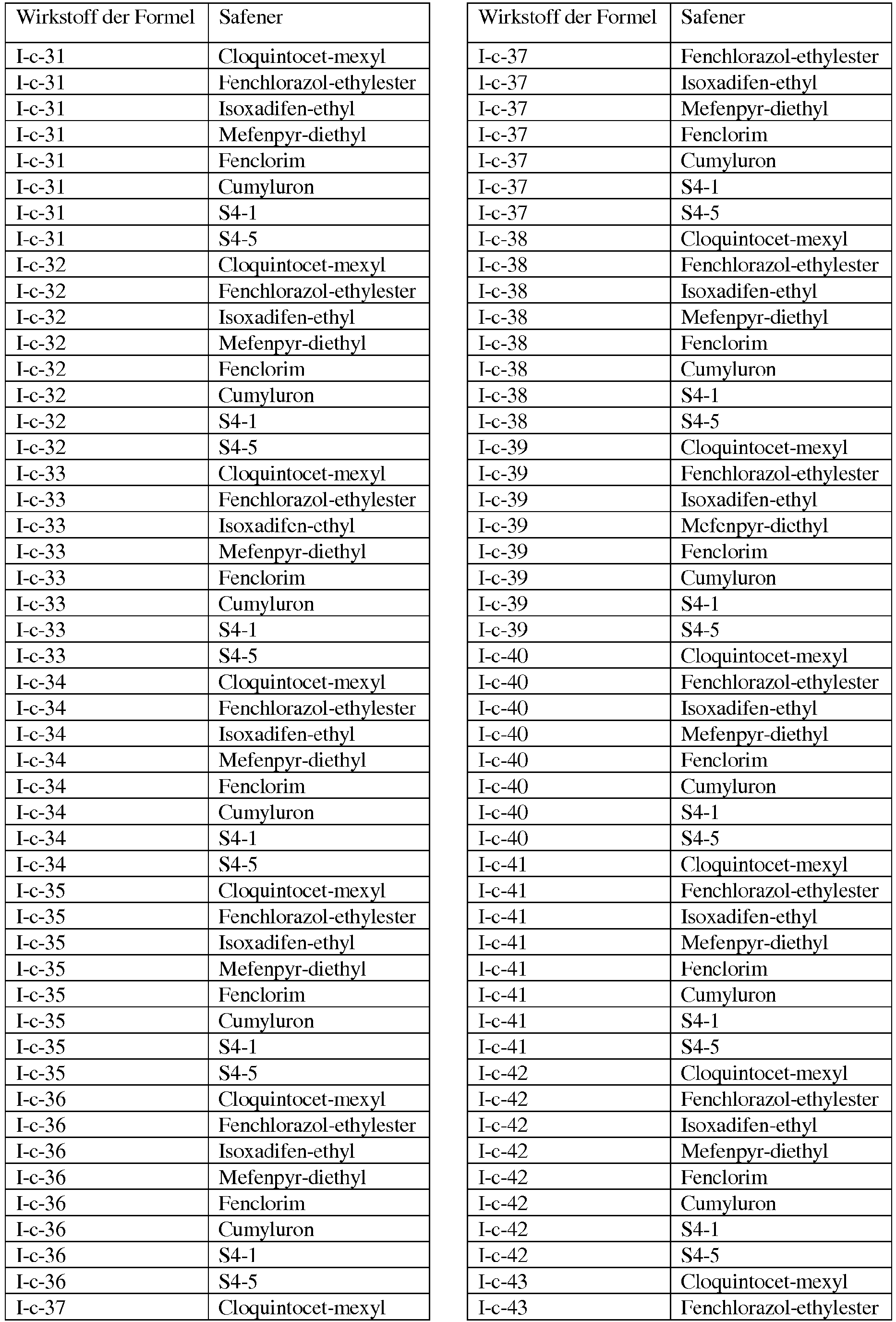

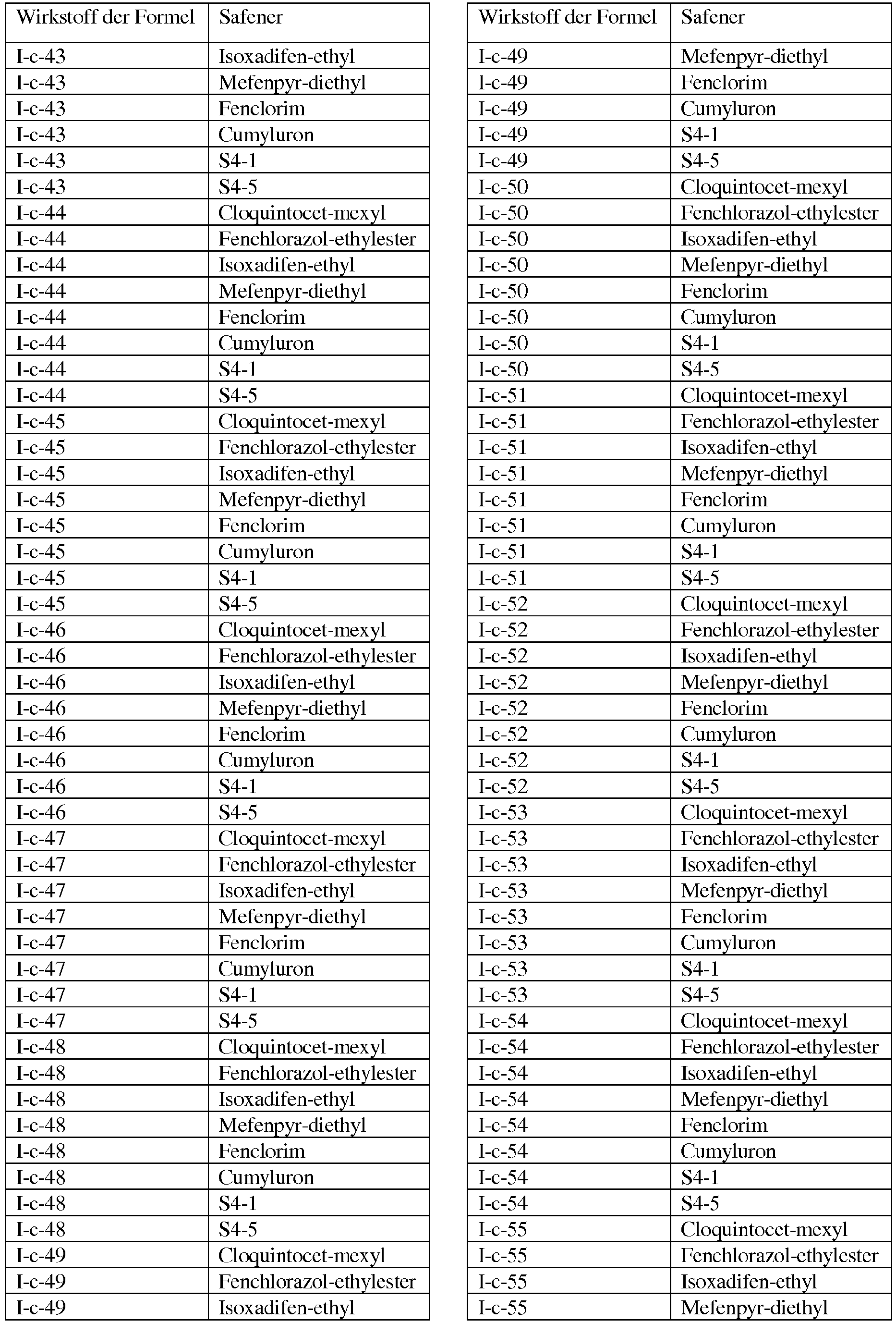

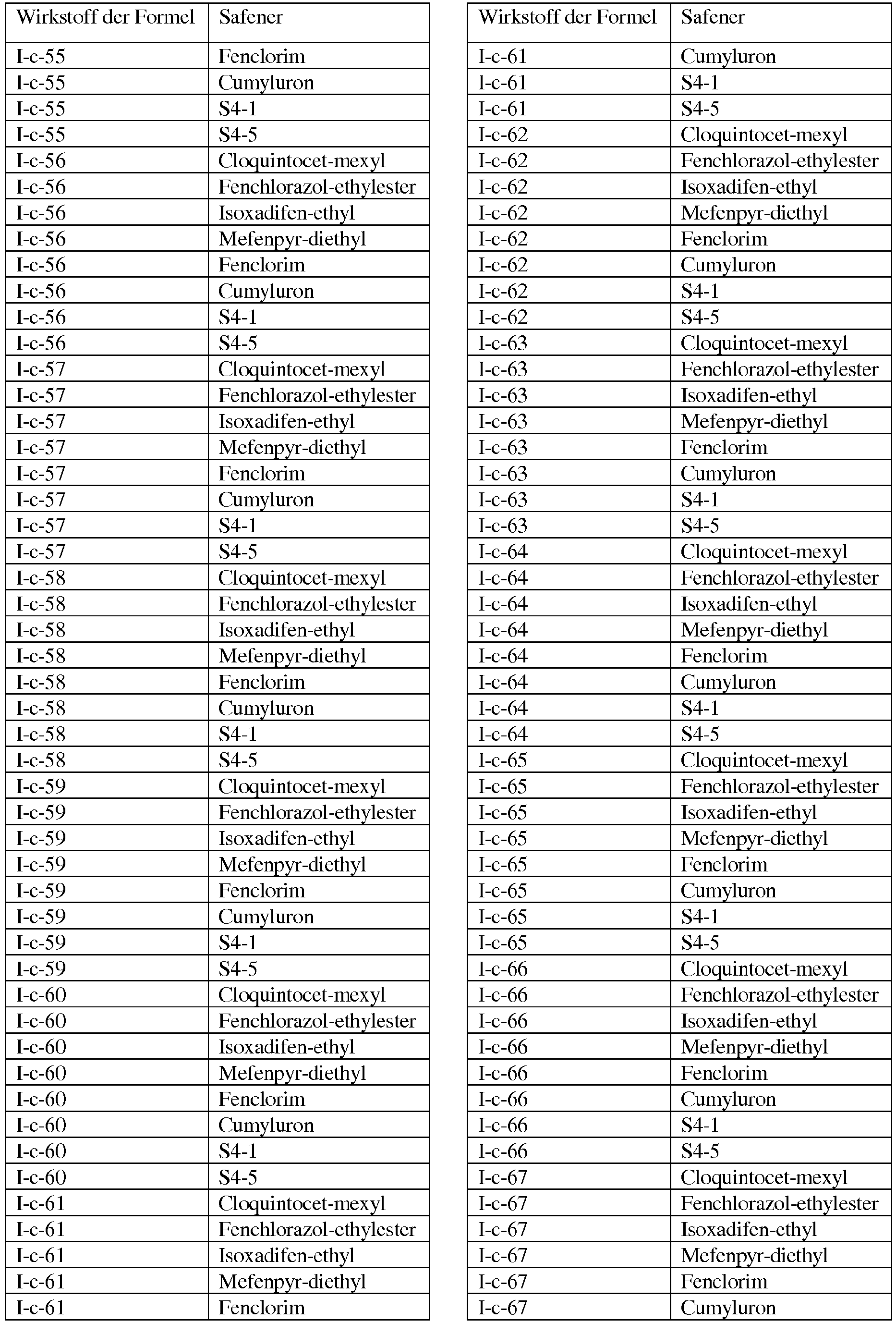

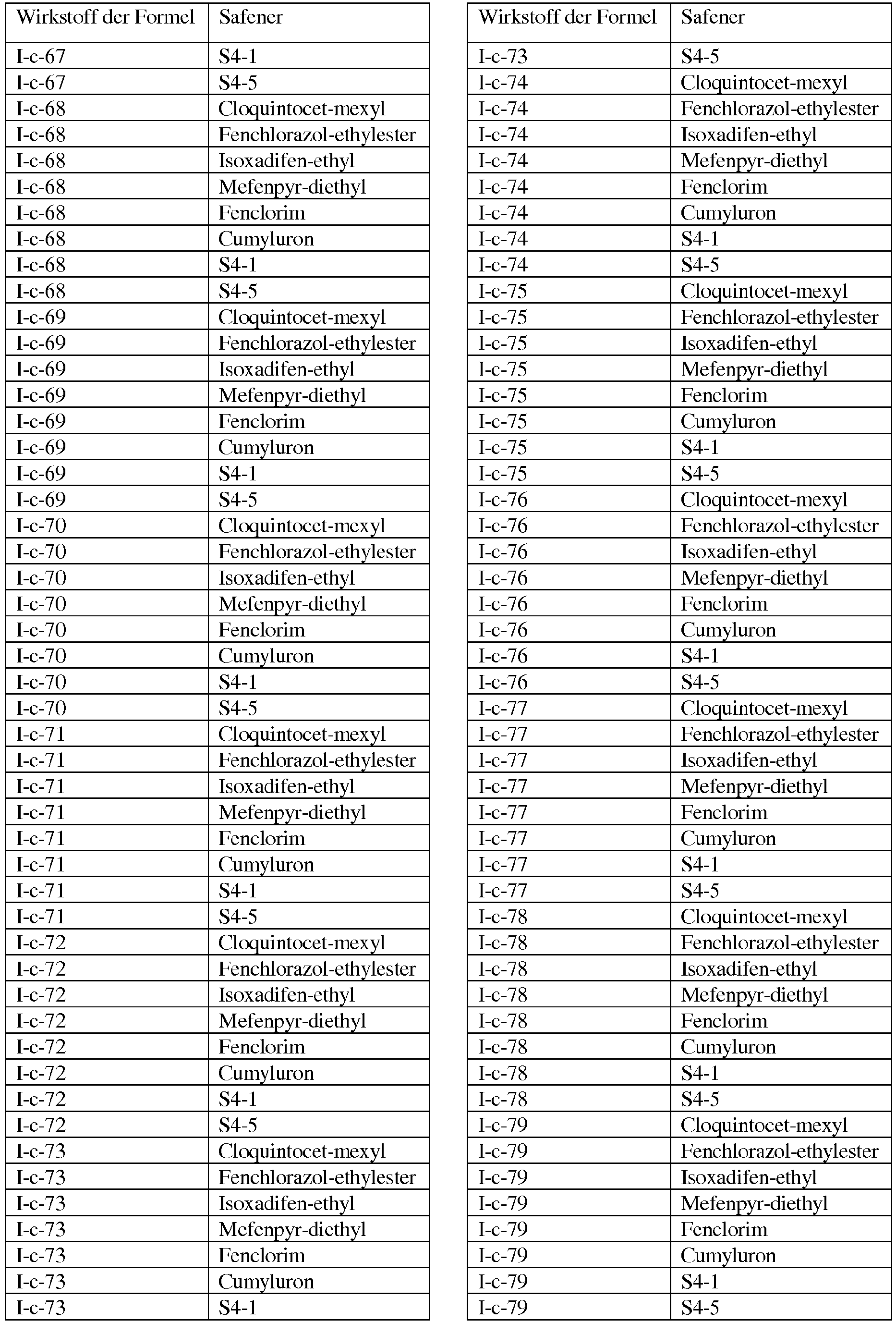

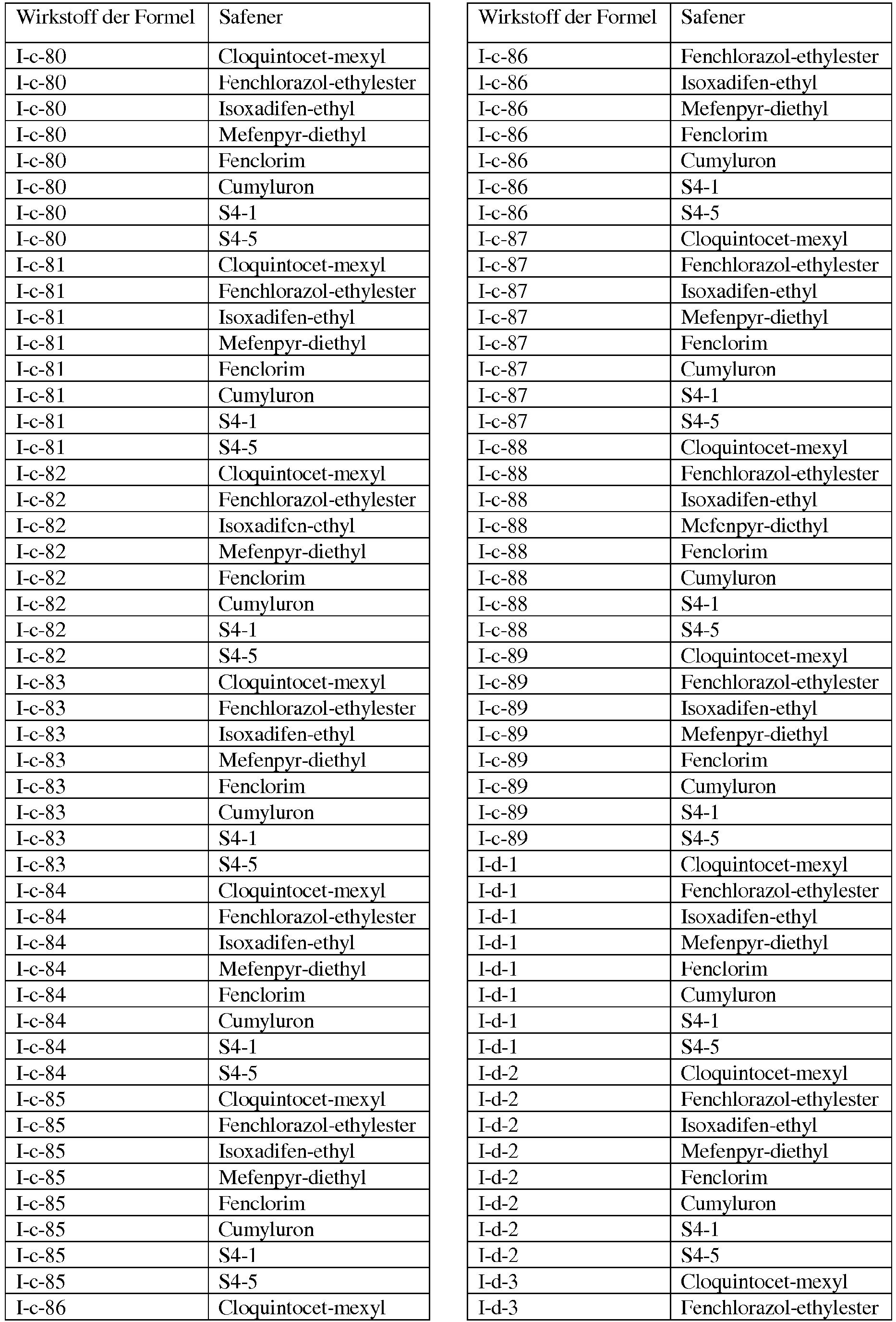

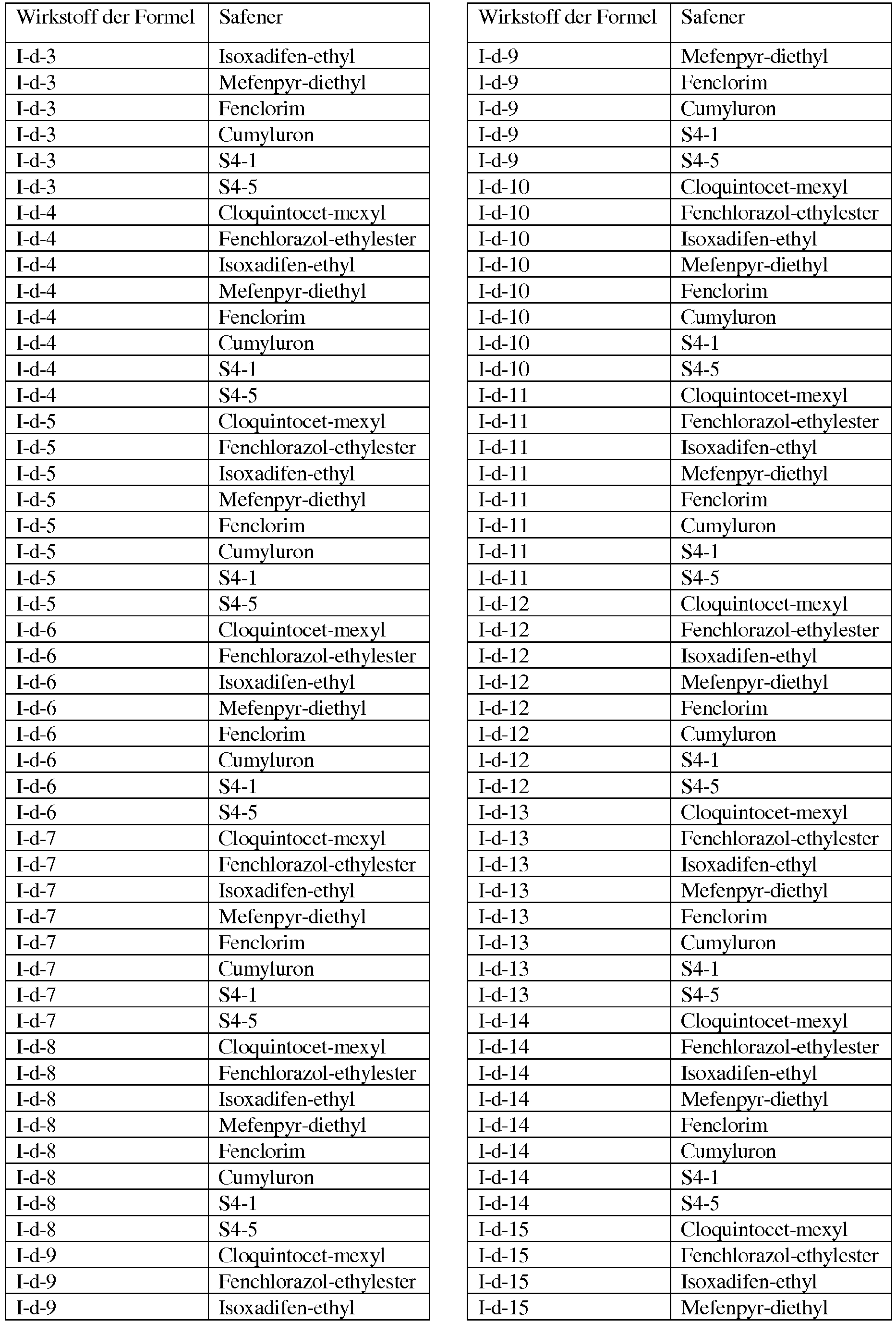

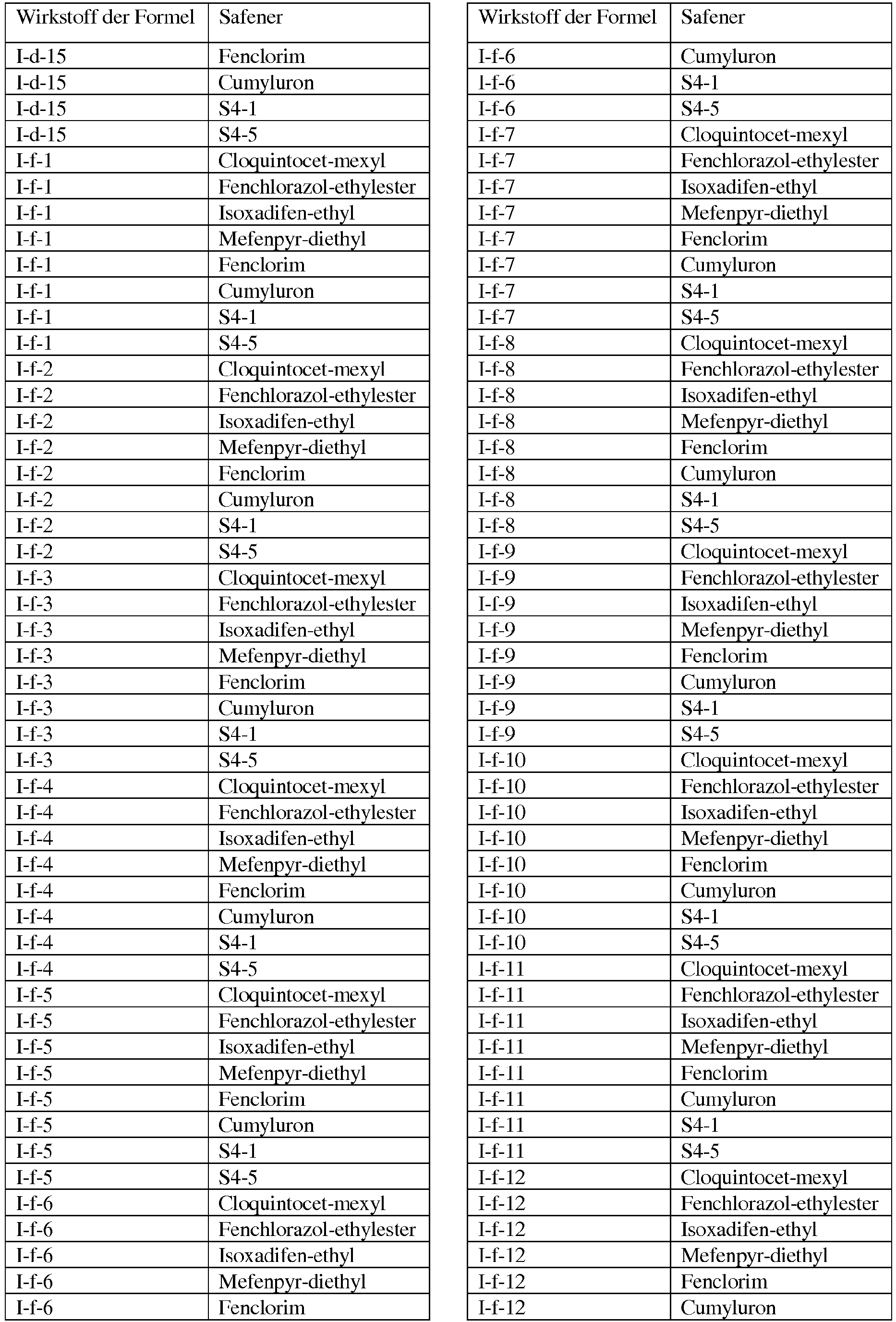

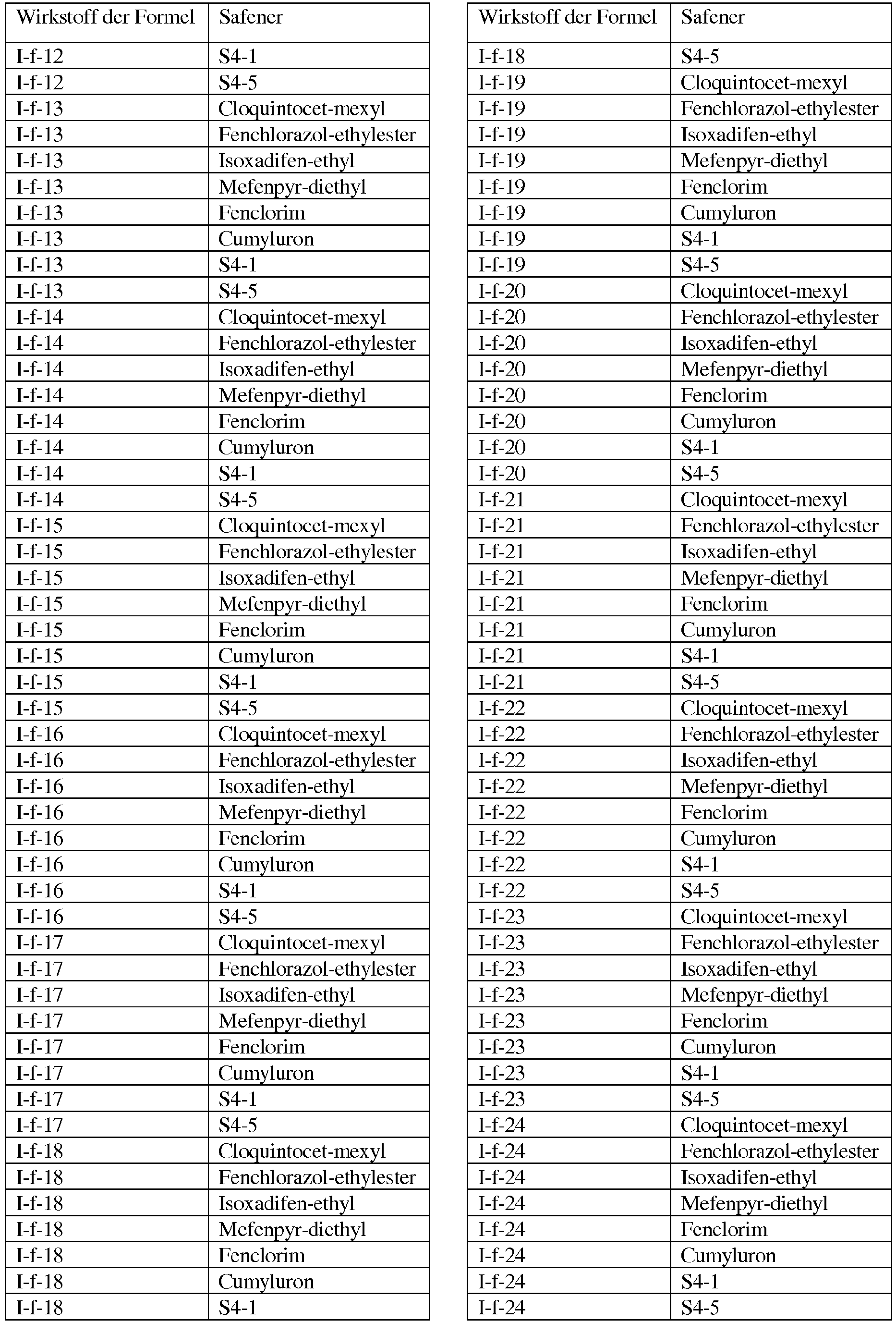

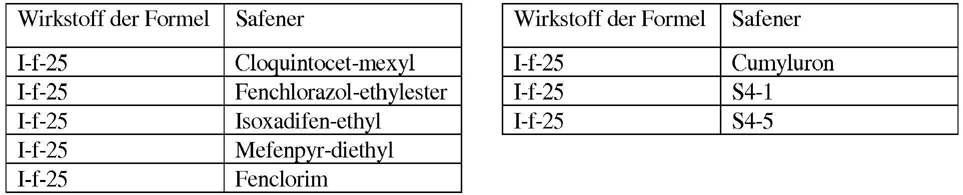

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht bevorzugt für C₁-C₄-Alkyl, C₃-C₆-Cycloalkyl oder C₁-C₄-Alkoxy,
- Y: steht bevorzugt für Wasserstoff, C₁-C₄-Alkyl, oder C₁-C₄-Alkoxy,
- Z: steht bevorzugt für Wasserstoff, C₁-C₄-Alkyl oder C₃-C₆-Cycloalkyl,
- A, B: und das Kohlenstoffatom an das sie gebunden sind, stehen bevorzugt für C₃-C₈-Cycloalkyl oder C₅-C₈-Cycloalkenyl, worin gegebenenfalls ein oder zwei Ringglieder durch Sauerstoff und/oder Schwefel ersetzt sind und welche gegebenenfalls ein- bis vierfach durch C₁-C₈-Alkyl, C₁-C₈-Alkenyl, C₁-C₄-Alkylen, C₁-C₈-Halogenalkyl, C₁-C₆-Alkoxy-C₁-C₄-alkyl, C₁-C₆-Alkoxy, C₁-C₆-Halogenalkoxy, Hydroxy, Hydroxy-C₁-C₄-alkyl oder Benzyloxy substituiert sind, oder einen weiteren ankondensierten C₃-C₈-Cycloalkylring, in dem gegebenenfalls ein- oder zwei Ringlieder durch Sauerstoff oder Schwefel ersetzt sind, oder welcher gegebenenfalls einfach oder zweifach durch C₁-C₈-Alkyl substituiert ist, enthalten, oder
- A, B: und das Kohlenstoffatom, an das sie gebunden sind, stehen bevorzugt für eine C₁-C₄-Alkylen-Gruppe, oder für eine =N-OR⁹-Gruppe,
G steht bevorzugt für Wasserstoff (a) oder für eine der Gruppen 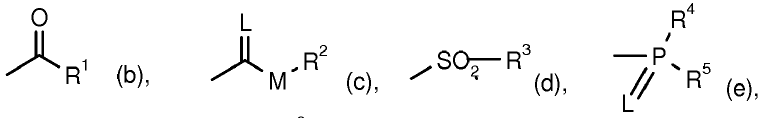 E (f) oder 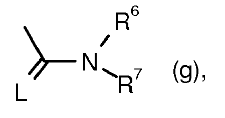 in welchen
E für ein Metallionäquivalent oder ein Ammoniumion steht,
L für Sauerstoff oder Schwefel steht und
M für Sauerstoff oder Schwefel steht,
R¹ steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkyl-sulfonyl substituiertes Phenyl, für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl),
R² steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
R³ steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
R⁴ und R⁵ stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio,
R⁶ und R⁷ stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls ein Kohlenstoffatom durch Sauerstoff oder Schwefel ersetzt ist,
R⁹ steht bevorzugt für Wasserstoff, für jeweils gegebenenfalls einfach bis fünffach durch Halogen substituiertes C₁-C₆-Alkyl, C₃-C₆-Cycloalkyl, CH₂C₃-C₆-Cycloalkyl, C₃-C₆-Alkenyl, C₃-C₆-Alkinyl oder jeweils gegebenenfalls einfach bis dreifach durch Alkyl, Halogen, Alkoxy, Halogenalkyl oder Halogenalkoxy substituiertes Phenyl-C₁-C₂-alkyl oder Hetaryl-C₁-C₂-alkyl.

In den als bevorzugt genannten Restedefinitionen steht Halogen für Fluor, Chlor, Brom und Jod, insbesondere für Fluor, Chlor und Brom. Mit den Bezeichnungen Alkyl, Alkyliden und Alkenyl sind sowohl geradkettige als auch verzweigte Kohlenwasserstofftreste gemeint.
- X: steht besonders bevorzugt für Methyl, Ethyl, Cyclopropyl, Methoxy oder Ethoxy,
- Y: steht besonders bevorzugt für Wasserstoff, Methyl oder Ethyl,
- Z: steht besonders bevorzugt für Wasserstoff, Methyl, Ethyl oder Cyclopropyl,
- A, B: und das Kohlenstoffatom an das sie gebunden sind, stehen besonders bevorzugt für C₃-C₈-Cycloalkyl oder C₅-C₈-Cycloalkenyl, worin gegebenenfalls ein oder zwei Ringglieder durch Sauerstoff und/oder Schwefel ersetzt sind und welches gegebenenfalls ein- bis vierfach durch C₁-C₄-Alkyl, C₁-C₄-Alkenyl, einer =CH₂-Gruppe, Trifluormethyl, C₁-C₄-Alkoxy, C₁-C₄-Alkoxy-C₁-C₂-alkyl oder mit einer der Gruppen 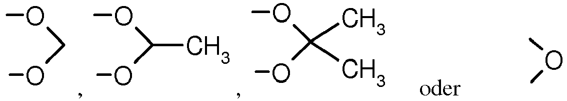
substituiert ist oder,
- A, B: und das Kohlenstoffatom, an das sie gebunden sind, stehen besonders bevorzugt für eine C₁-C₄-Alkylen-Gruppe oder für eine =N-OR⁹-Gruppe,
- G: steht besonders bevorzugt für Wasserstoff (a) oder für eine der Gruppen 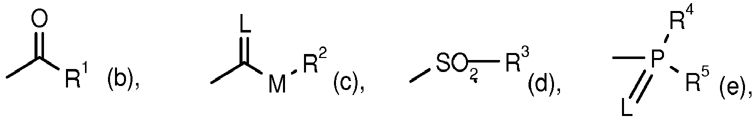 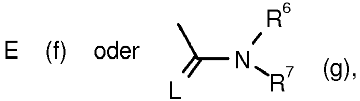 in welchen
E für ein Metallionäquivalent oder ein Ammoniumion steht,
L für Sauerstoff oder Schwefel steht und
M für Sauerstoff oder Schwefel steht,
- R¹: steht besonders bevorzugt für jeweils gegebenenfalls einfach bis dreifach durch Fluor oder Chlor substituiertes C₁-C₈-Alkyl, C₂-C₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl,
- R²: steht besonders bevorzugt für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,

- R³: steht besonders bevorzugt für gegebenenfalls einfach bis dreifach durch Fluor substituiertes C₁-C₆-Alkyl oder für gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl,
- R⁴: steht besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogen-alkylthio, C₁-C₃-Alkyl oder Trifluormethyl substituiertes Phenyl, Phenoxy oder Phenylthio,
- R⁵: steht besonders bevorzugt für gegebenenfalls einfach durch Chlor substituiertes C₁-C₆-Alkoxy oder C₁-C₆-Alkylthio,
- R⁶: steht besonders bevorzugt für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl,
- R⁷: steht besonders bevorzugt für C₁-C₆-Alkyl, C₃-C₆-Alkenyl oder C₁-C₆-Alkoxy-C₁-C₄-alkyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist,
- R⁹: steht besonders bevorzugt für Wasserstoff, für jeweils gegebenenfalls einfach bis dreifach durch Fluor oder Chlor substituiertes C₁-C₄-Alkyl, C₃-C₆-Cycloalkyl, CH₂C₃-C₆-Cycloalkyl, C₃-C₄-Alkenyl, C₃-C₄-Alkinyl, für jeweils gegebenenfalls einfach bis zweifach durch C₁-C₂-Alkyl, C₁-C₂-Alkoxy, Fluor, Chlor, Brom Trifluormethyl oder Trifluormethoxy substituiertes Benzyl oder Pyridinylmethyl.

In den als besonders bevorzugt genannten Restedefinitionen steht Halogen für Fluor, Chlor und Brom, insbesondere für Fluor und Chlor. Mit den Bezeichnungen Alkyl, Alkyliden und Alkenyl sind sowohl geradkettige als auch verzweigte Kohlenwasserstofftreste gemeint.
- X: steht ganz besonders bevorzugt für Methyl, Ethyl, Cyclopropyl, Methoxy oder Ethoxy, (hervorgehoben für Ethyl),
- Y: steht ganz besonders bevorzugt für Wasserstoff , Methyl oder Ethyl, (hervorgehoben für Methyl),
- Z: steht ganz besonders bevorzugt für Wasserstoff, Methyl, Ethyl oder Cyclopropyl, (hervorgehoben für Ethyl),
- A, B: und das Kohlenstoffatom an das sie gebunden sind, stehen ganz besonders bevorzugt für C₃-C₇-Cycloalkyl oder C₅-C₇-Cycloalkenyl, in welchem gegebenenfalls ein oder zwei Ringglieder durch Sauerstoff und/oder Schwefel ersetzt sind und welches gegebenenfalls ein-, zwei-, drei- oder vierfach durch Methyl, jeweils einfach durch Ethyl, eine =CH₂-Gruppe, Methoxy oder Ethoxy substituiert ist,
- A, B: und das Kohlenstoffatom, an das sie gebunden sind, stehen ganz besonders bevorzugt für eine =CH₂-Gruppe, für eine =CH₂-CH₃-Gruppe, für eine =CH₂-C₂H₅ -Gruppe oder für eine =N-OR⁹-Gruppe,
G steht ganz besonders bevorzugt für Wasserstoff (a) oder für eine der Gruppen 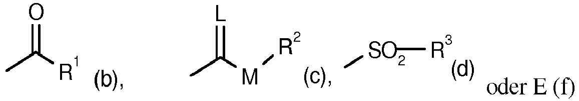 in welchen
E für ein Metallionenäquivalent (Na⁺ oder K⁺) steht,
L für Sauerstoff oder Schwefel steht und
M für Sauerstoff oder Schwefel steht,
R¹ steht ganz besonders bevorzugt für C₁-C₆-Alkyl, C₂-C₆-Alkenyl, C₁-C₂-Alkoxy-C₁-alkyl, C₁-C₂-Alkylthio-C₁-alkyl oder gegebenenfalls einfach durch Fluor, Chlor, Methyl oder Methoxy substituiertes C₃-C₆-Cyclopropyl oder für einfach durch Chlor substituiertes C₁-C₄-Alkyl,
für gegebenenfalls einfach durch Fluor, Chlor, Brom, Cyano, Nitro, Methyl, Methoxy, Trifluormethyl oder Trifluormethoxy substituiertes Phenyl,
R² steht ganz besonders bevorzugt für jeweils gegebenenfalls einfach bis dreifach durch Fluor substituiertes C₁-C₈-Alkyl, C₂-C₆-Alkenyl oder C₁-C₄-Alkoxy-C₂-C₃-alkyl, Phenyl oder Benzyl,
R³ steht ganz besonders bevorzugt für C₁-C₆-Alkyl oder für gegebenenfalls einfach durch Chlor oder C₁-C₄-Alkyl substituiertes Phenyl,
R⁹ steht ganz besonders bevorzugt für Wasserstoff, für jeweils gegebenenfalls durch Fluor substituiertes Methyl, Ethyl, Propyl, n-Butyl, i-Butyl, s-Butyl oder tert.-Butyl, für Allyl, Chlorallyl, Propinyl, Butinyl, Chlorbenzyl, Chlor-pyridylmethyl, CH(CH₃)-C≡CH, Cyclopropyl, Cyclobutyl, Cyclopentyl, CH₂Cyclopropyl, CH₂Cyclobutyl oder CH₂Cyclopentyl.
X steht insbesondere bevorzugt für Methyl, Ethyl oder Methoxy,
Y steht insbesondere bevorzugt für Methyl oder Ethyl,
Z steht insbesondere bevorzugt für Wasserstoff, Methyl oder Ethyl,
A, B und das Kohlenstoffatom an das sie gebunden sind, stehen insbesondere bevorzugt für C₅-C₇-Cycloalkyl oder C₇-Cycloalkenyl, in welchem gegebenenfalls zwei Ringglieder durch Sauerstoff und/oder Schwefel ersetzt sind und welches gegebenenfalls ein-, zwei-, oder vierfach durch Methyl, jeweils einfach durch Ethyl, eine =CH₂-Gruppe, Methoxy, Ethoxy oder Benzyloxy substituiert ist,
A, B und das Kohlenstoffatom, an das sie gebunden sind, stehen ganz besonders bevorzugt für eine =CH₂-Gruppe, oder für eine =N-OR⁹-Gruppe,
G steht insbesondere bevorzugt für Wasserstoff (a) oder für eine der Gruppen 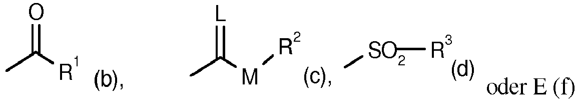 in welchen
E für ein Metallionenäquivalent (Na⁺) steht,
L für Sauerstoff steht und
M für Sauerstoff steht,
R¹ steht insbesondere bevorzugt für C₁-C₆-Alkyl, oder C₁-C₂-Alkoxy-C₁-alkyl, oder für einfach durch Chlor substituiertes C₁-C₄-Alkyl,
für gegebenenfalls einfach durch Chlor, Methyl oder Methoxy substituiertes Phenyl,
R² steht insbesondere bevorzugt für C₁-C₈-Alkyl,
R³ steht insbesondere bevorzugt für Methyl, Phenyl oder p-Methylphenyl,
R⁹ steht insbesondere bevorzugt für Wasserstoff, Methyl, i-Propyl, i-Butyl, tert.-Butyl, CH₂CF₃, Propinyl (CH₂-C≡CH), CH(CH₃)-C≡CH, Cyclopropyl, Cyclopentyl, CH₂Cyclopropyl, CH₂Cyclopentyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Besonders hervorgehoben steht G für Wasserstoff.

5 Im einzelnen seien außer den bei den Herstellungsbeispielen genannten Verbindungen die folgenden Verbindungen der Formel (I-1-a) genannt:

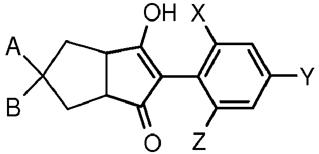

**Tabelle 1: X = CH₃, Y = CH₃, Z = CH₃**

| A | B |
|---|---|
| -O-(CH₂)₂-O- | |
| -O-(CH₂)₄-O- | |
| -O-CHCH₃-CH₂-O- | |
| -O-CHCH₃-CHCH₃-O- | |
| -O-(CH₂)₃-O- | |
| -O-CHCH₃-(CH₂)₂-O- | |
| -O-CHCH₃-CH₂-CHCH₃-O- | |
| -O-CH₂-CH(CH₃)-CH₂-O- | |
| -O-CH₂-CH(OCH₃)-CH₂-O- | |
| -O-CH₂-C(CH₃)₂-CH₂-O- | |
| 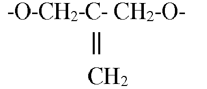 | |
| -O-CH₂-CH=CH-CH₂-O- | |
| -S-(CH₂)₂-S- | |
| -S-(CH₂)₃-S- | |
| -O-CH₂-O-CH₂- | |
| -O-C(CH₃)₂-O-CH₂- | |

| | | | |
|---|---|---|---|
| Tabelle 2: | A und B wie in Tabelle 1 genannt und | | |
| | X = C₂H₅; | Y = CH₃; | Z = CH₃ |
| Tabelle 3: | A und B wie in Tabelle 1 genannt und | | |
| | X = C₂H₅; | Y = CH₃; | Z = C₂H₅ |
| Tabelle 4: | A und B wie in Tabelle 1 genannt und | | |
| | X = C₂H₅; | Y =C₂H₅; | Z = C₂H₅ |
| Tabelle 5: | A und B wie in Tabelle 1 genannt und | | |
| | 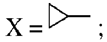 | Y = CH₃; | Z = CH₃ |
| Tabelle 6: | A und B wie in Tabelle 1 genannt und | | |
| | 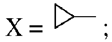 | Y = CH₃; | Z = C₂H₅ |
| Tabelle 7: | A und B wie in Tabelle 1 genannt und | | |
| | X = OCH₃; | Y = CH₃; | Z = CH₃ |
| Tabelle 8: | A und B wie in Tabelle 1 genannt und | | |
| | X = OC₂H₅; | Y = CH₃; | Z = CH₃ |
| Tabelle 9: | A und B wie in Tabelle 1 genannt und | | |
| | X = OCH₃; | Y = CH₃; | Z = C₂H₅ |
| Tabelle 10: | A und B wie in Tabelle 1 genannt und | | |
| | X = OC₂H₅; | Y = CH₃; | Z = C₂H₅ |
| Tabelle 11: | A und B wie in Tabelle 1 genannt und | | |
| | X = C₂H₅; | Y = CH₃; | Z=H |
| Tabelle 12: | A und B wie in Tabelle 1 genannt und | | |
| | 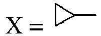 | Y = CH₃; | 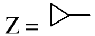 |
| Tabelle 13: | A und B wie in Tabelle 1 genannt und | | |
| | X = C₂H₅; | Y = C₂H₅; | Z = CH₃ |

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, WO 07/068427). Eine entsprechende Wirkungssteigerung bei Insektiziden wurde bereits durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Herbiziden aus der Klasse der phenylsubstituierten Bicyclooktan-1,3-dion-Derivate der Formel (I) durch den Zusatz von Ammonium- oder Phosphoniumsalzen zur Anwendungslösung oder durch den Einbau dieser Salze in eine Formulierung enthaltend phenylsubstituierte Bicyclooktan-1,3-dion-Derivate der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und/oder akarizid wirksame phenylsubstituierte Bicyclooktan-1,3-dion-Derivate der Formel (I) als Wirkstoff enthalten. Gegenstand der Erfindung sind ebenfalls Mittel, die herbizid und/oder akarizid und/oder insektizid wirksame phenylsubstituierte Bicyclooktan-1,3-dion-Derivate der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (III') definiert 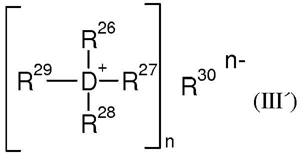 in welcher
D für Stickstoff oder Phosphor steht,
D bevorzugt für Stickstoff steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R²⁶, R²⁷, R²⁸ und R²⁹ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R²⁶, R²⁷, R²⁸ und R²⁹ besonders bevorzugt unabhängig voneinander für Wasserstoff, Methyl, Ethyl, n-Propyl, i-Propyl, n-Butyl, i-Butyl, s-Butyl oder t-Butyl stehen,
R²⁶, R²⁷, R²⁸ und R²⁹ ganz besonders bevorzugt für Wasserstoff stehen,
n für 1, 2, 3 oder 4 steht,
n bevorzugt für 1 oder 2 steht,
R³⁰ für ein anorganisches oder organisches Anion steht,
R³⁰ bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
R³⁰ besonders bevorzugt für Laktat, Sulfat, Nitrat, Thiosulfat, Thiocyanat, Oxalat oder Formiat steht,
R³⁰ ganz besonders bevorzugt für Sulfat steht.

Die Ammonium- und Phosphoniumsalze der Formel (III') können in einem breiten Konzentrationsbereich zur Steigerung der Wirkung von Pflanzenschutzmitteln enthaltend phenylsubstituierten Bicyclooktan-1,3-dion-Derivate der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder herbizid wirksame, phenylsubstituierte Bicyclooktan-1,3-dion-Derivate der Formel (I) als Wirkstoff enthalten. Gegenstand der Erfindung sind ebenfalls Mittel, die herbizid und/oder akarizid und/oder insektizid wirksame phenylsubstituierten Bicyclooktan-1,3-dion-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässrigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)_{V}-R' (IV')

in welcher
- R: für geradkettiges oder verzweigtes Alkyl mit 4 bis 20 Kohlenstoffatomen steht,

- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,
- PO: für 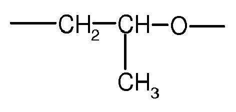 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 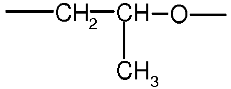 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R': die oben angegebenen Bedeutungen haben,
- EO: für -CH₂-CH₂-O- steht,
- BO: für 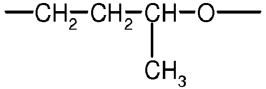 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R': die oben angegebenen Bedeutungen haben,
- BO: für 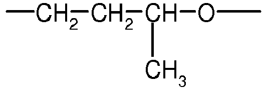 steht,
- EO: für -CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- ) t: für Zahlen von 8 bis 13 steht
- u: für Zahlen von 6 bis 17 steht.
In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 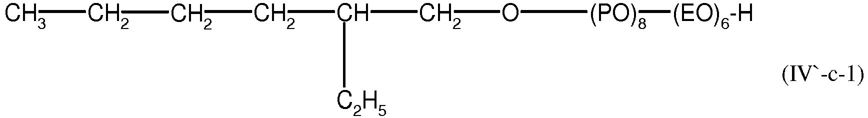 in welcher
- EO: für -CH₂-CH₂-O- steht,

- PO: für 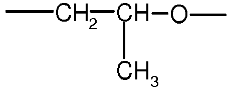 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für -CH₂-CH₂-O- steht,
- BO: für 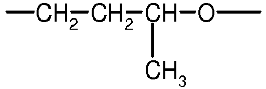 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,
Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Bezeichnung "Wirkstoffe" bzw. "Verbindungen" schließt immer auch die hier genannten Wirkstoffkombinationen mit ein.

Die Herstellung der Verbindungen mit der allgemeinen Struktur (I) kann erfindungsgemäß nach den Verfahren A bis H erfolgen.

Verwendet man beispielsweise gemäß Verfahren (A) 2,2-Dimethyl-8-[(2,6-diethyl-4-methyl)phenylacetyl]-1,3-dioxa-[4.4.0]-bicyclo-nonan-7-carbonsäuremethylester, so kann der Verlauf des erfindungsgemäßen Verfahrens durch folgendes Reaktionsschema wiedergegeben werden:

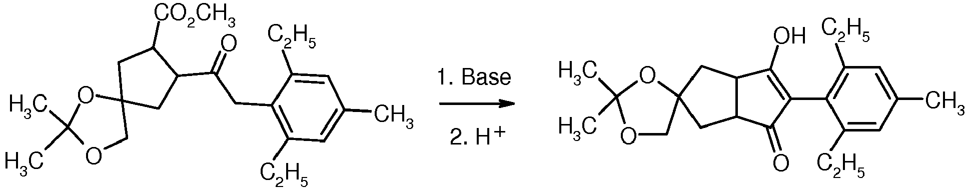

Verwendet man beispielsweise gemäß Verfahren (B) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Pivaloylchlorid als Ausgangsstoffe, so kann der Verlauf des erfindungsgemäßen Verfahrens durch folgendes Reaktionsschema wiedergegeben werden:

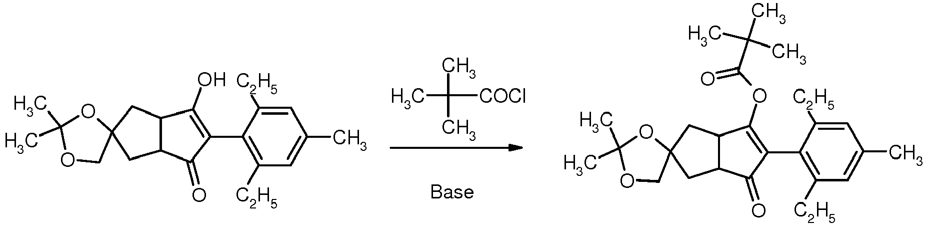

Verwendet man beispielsweise gemäß Verfahren (B) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Acetanhydrid als Ausgangsverbindungen, so kann der Verlauf des erfindungsgemäßen Verfahrens durch folgendes Reaktionsschema wiedergegeben werden:

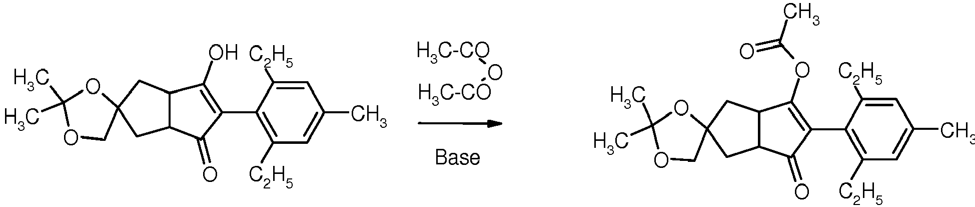

Verwendet man beispielsweise gemäß Verfahren (C) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Chlorameisensäureethylester als Ausgangsverbindungen, so kann der Verlauf des erfindungsgemäßen Verfahrens durch folgendes Reaktionsschema wiedergegeben werden:

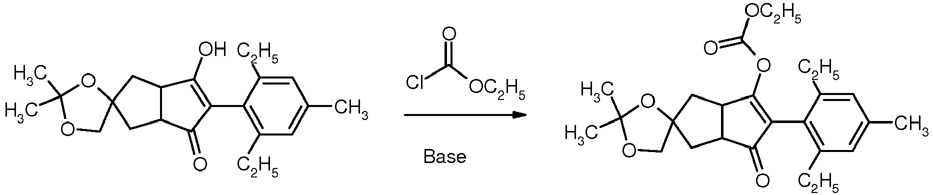

Verwendet man beispielsweise gemäß Verfahren (D) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Chlormonothioameisensäuremethylester als Ausgangsverbindungen, so kann der Verlauf des erfindungsgemäßen Verfahrens durch folgendes Reaktionsschema wiedergegeben werden:

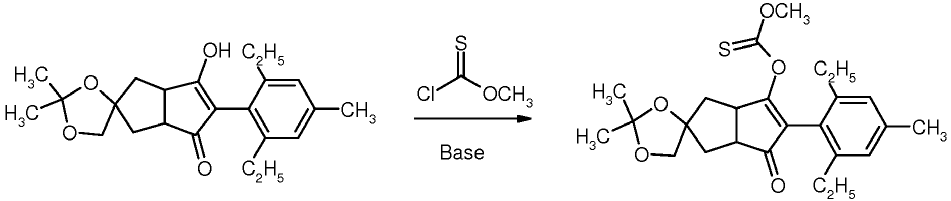

Verwendet man beispielsweise gemäß Verfahren (E) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Methansulfonsäurechlorid als Ausgangsprodukt, so kann der Reaktionsverlauf durch folgendes Reaktionsschema wiedergegeben werden:

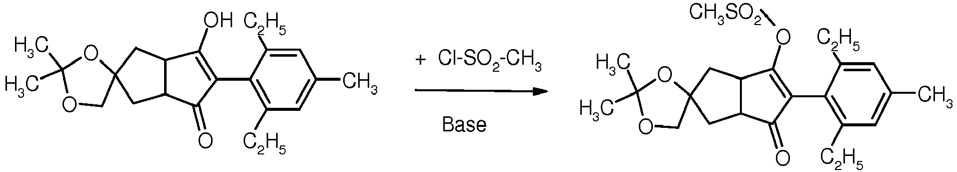

Verwendet man beispielsweise gemäß Verfahren (F) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Methanthio-phosphonsäurechlorid-(2,2,2-trifluorethylester) als Ausgangsprodukte, so kann der Reaktionsverlauf durch folgendes Reaktionsschema wiedergegeben werden:

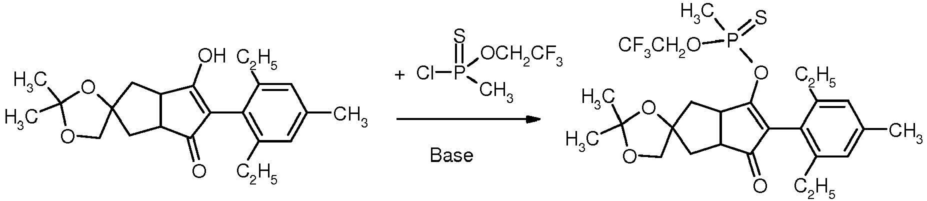

Verwendet man beispielsweise gemäß Verfahren (G) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und NaOH als Komponenten, so kann der Verlauf des erfindungsgemäßen Verfahrens durch folgendes Reaktionsschema wiedergegeben werden:

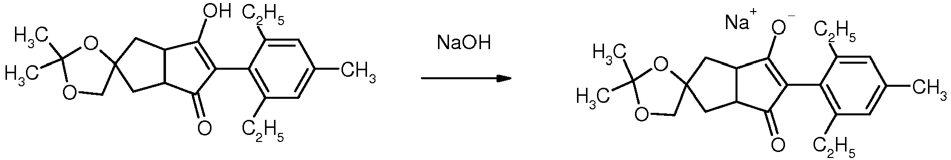

Verwendet man beispielsweise gemäß Verfahren (H) Variante α 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Ethylisocyanat als Ausgangsprodukte, so kann der Reaktionsverlauf durch folgendes Reaktionsschema wiedergegeben werden:

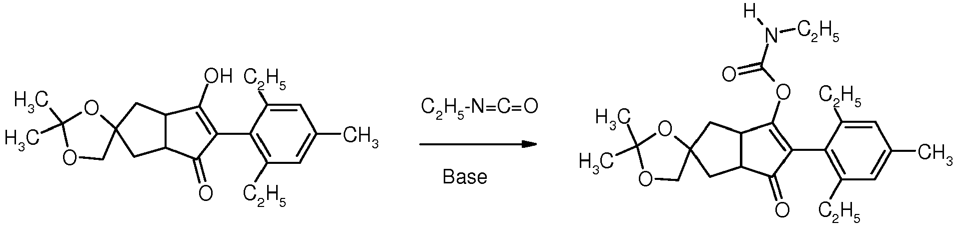

Verwendet man beispielsweise gemäß Verfahren (H) Variante ß 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Dimethylcarbamidsäurechlorid als Ausgangsprodukte, so kann der Reaktionsverlauf durch folgendes Schema wiedergegeben werden:

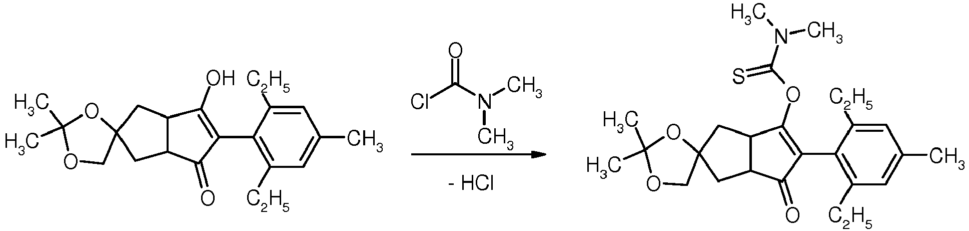

Die bei dem erfindungsgemäßen Verfahren (A) als Ausgangsstoffe benötigten Verbindungen der Formel (II) 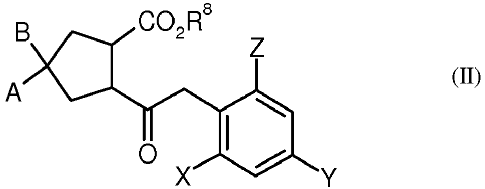 in welcher
A, B, X, Y, Z und R⁸ die oben angegebene Bedeutung haben,
sind neu.

Sie lassen sich nach im Prinzip bekannten Methoden herstellen.

Man erhält die 5-Aryl-4-ketocarbonsäureester der Formel (II) beispielsweise, wenn man 5-Aryl-4-ketocarbonsäuren der Formel (XIII) 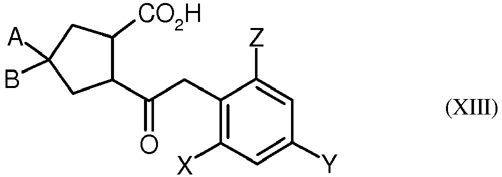 in welcher
X, Y, Z, A und B, die oben angegebene Bedeutung haben,
verestert (vgl. z.B. Organikum, 15. Auflage, Berlin, 1977, Seite 499) oder alkyliert (siehe Herstellungsbeispiel).

Die Aryl-ketocarbonsäuren der Formel (XIII) 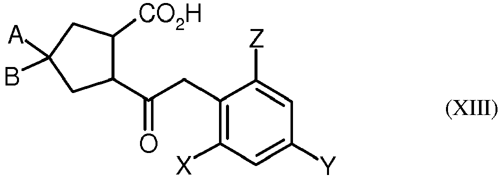 in welcher
A, B, X, Y und Z die oben angegebene Bedeutung haben,
sind neu, lassen sich aber nach im Prinzip bekannten Methoden herstellen (WO 07/080066, WO 96/01 798, WO 97/14667, WO 98/39281, WO 01/74770).

Man erhält die Aryl-ketocarbonsäuren der Formel (XIII) beispielsweise, wenn man 2-Phenyl-3-oxo-adipinsäureester der Formel (XIV) 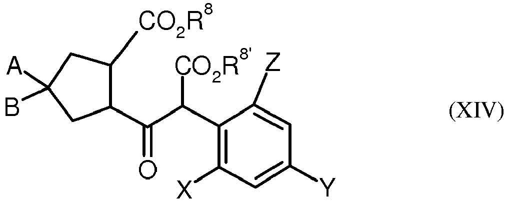 in welcher
A, B, X, Y und Z die oben angegebene Bedeutung haben und
R⁸ und R^{8'} für Alkyl (insbesondere C₁-C₈-Alkyl) stehen und
bei Einsatz der Verbindung der Formel (XVI) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XIV) 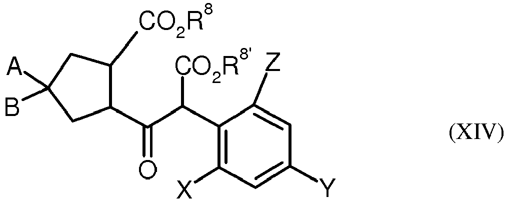 in welcher
A, B, X, Y, Z, R⁸, R^{8'} die oben angegebene Bedeutung haben und
bei Einsatz der Verbindung der Formel (XVI) R⁸ für Wasserstoff steht
sind neu.

Man erhält die Verbindungen der Formel (XIV) beispielsweise,
wenn man Dicarbonsäurehalbesterchloride der Formel (XV), 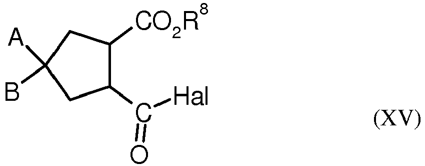 in welcher
A, B und R⁸ die oben angegebene Bedeutung haben und
Hal für Chlor oder Brom steht,
oder Carbonsäureanhydride der Formel (XVI) 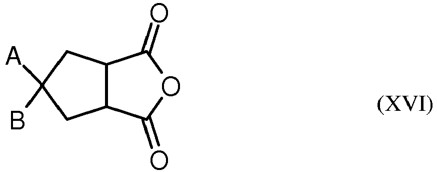 in welcher
A und B die oben angegebene Bedeutung haben,
mit einem Phenylessigsäureester der Formel (XVII) 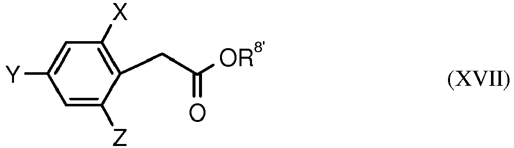 in welcher
X, Y, Z und R⁸ die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Eine weitere bewährte Methode zur Herstellung der beim Verfahren (A) als Ausgangsstoffe benötigten Verbindungen der Formel (II), in der A, B, X, Y, Z und R⁸ die oben angegebene Bedeutung haben, ist beispielsweise auch die Kupplung von Benzylzinkverbindungen der allgemeinen Formel (XVIII) 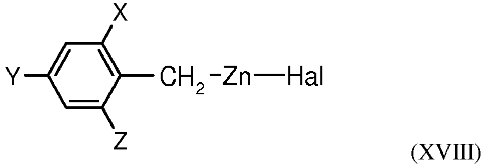 in welcher X, Y und Z die oben angegebene Bedeutung haben und Hal für ein Halogenatom, vorzugsweise Chlor oder Brom, steht,
gegebenenfalls in Gegenwart eines Katalysators, mit einem Dicarbonsäurehalbesterchlorid der allgemeinen Formel (XV) oder einem Carbonsäureanhydrid der allgemeinen Formel (XVI).

Sowohl Herstellung als auch Umsetzung von organischen Zinkverbindungen mit Carbonsäurechloriden bzw. mit Carbonsäureanhydriden sind im Prinzip bekannt und können in enger Anlehnung an die in der Literatur beschriebenen Verfahren durchgeführt werden. Weitere Einzelheiten dazu sind beispielsweise in Chem. Commun. 2008, 5824, WO 2007/113294, Tetrahedron Letters 30, 5069-5072 (1989) oder Chem. Rev. 1993, 93, 2117-2188 beschrieben.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) sind allgemein bekannte Verbindungen der Organischen bzw. Anorganischen Chemie.

Die Verbindungen der Formeln (XV), (XVI) und (XVII) sind teilweise bekannte Verbindungen der Organischen Chemie bzw. aus den eingangs zitierten Patentanmeldungen bekannt und/oder lassen sich nach im Prinzip bekannten Methoden in einfacher Weise herstellen bzw. lassen sich nach den in den eingangs zitierten Patentanmeldungen angegebenen Methoden herstellen.

Zur Herstellung von Benzylzinkverbindungen der Formel (XVIII) werden Benzylverbindungen der Formel (XIX) 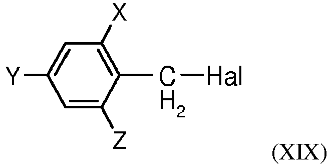 als Ausgangsmaterial eingesetzt, wobei X, Y und Z die oben angegebene Bedeutung besitzen und Hal für ein Halogenatom, vorzugsweise Chlor oder Brom steht. Benzylverbindungen der Formel (XIX) sind teilweise bekannt oder können nach bekannten Verfahren hergestellt werden (siehe beispielsweise Chem. Ber. 118, 1968 (1985), Monatshefte Chemie 135, 251 (2004), Acta Chem. Scand. 1963, 17 und Herstellungsbeispiele).

Das Verfahren (A) ist dadurch gekennzeichnet, dass man Verbindungen der Formel (II), in welcher A, B, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, molaren Mengen ein. Es ist jedoch auch möglich, die eine oder andere Komponente in einem größeren Überschuss (bis zu 3 Mol) zu verwenden.

Das Verfahren (B-α) ist dadurch gekennzeichnet, dass man Verbindungen der Formel (I-a) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (B-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (B-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (B-α) werden die Ausgangsstoffe der Formel (I-a) und das Carbonsäurehalogenid der Formel (III) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (B-ß) ist dadurch gekennzeichnet, dass man Verbindungen der Formel (I-a) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-ß) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (B-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (B-ß) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (B-ß) werden die Ausgangsstoffe der Formel (I-a) und das Carbonsäureanhydrid der Formel (IV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, dass man Verbindungen der Formel (I-a) jeweils mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (C) wird im Allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (V) im Allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (D) ist dadurch gekennzeichnet, dass man Verbindungen der Formel (I-a) jeweils mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, dass man Verbindungen der Formel (I-a) jeweils mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formel (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen der Formel (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, dass man Verbindungen der Formel (I-a) mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Iso-propanol, aber auch Wasser eingesetzt werden.

Das erfindungsgemäße Verfahren (G) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (H) ist dadurch gekennzeichnet, dass man Verbindungen der Formel (I-a) jeweils mit (H-α) Verbindungen der Formel (XI) gegebenenfalls in Gegenwart eines Verdünnungsmittels und gegebenenfalls in Gegenwart eines Katalysators oder (H-ß) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (H-α) setzt man pro Mol Ausgangsverbindung der Formel (I-a) ca. 1 Mol Isocyanat der Formel (XI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H-ß) setzt man pro Mol Ausgangsverbindung der Formel (I-a) ca. 1 Mol Carbamidsäurechlorid der Formel (XII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt. Die Reaktion kann bei Normaldruck oder unter erhöhtem Druck durchgeführt werden, vorzugsweise wird bei Normaldruck gearbeitet. Die Aufarbeitung geschieht nach üblichen Methoden. Die Herstellung und die Verwendung der erfindungsgemäßen Wirkstoffe geht aus den nachfolgenden Beispielen hervor.

**Herstellungsbeispiele** (Verbindungen, wobei A und B eine Carbonylgruppe bedeuten, sind nicht erfindungsgemäß.)

### Beispiel I-a-1 und I-a-2

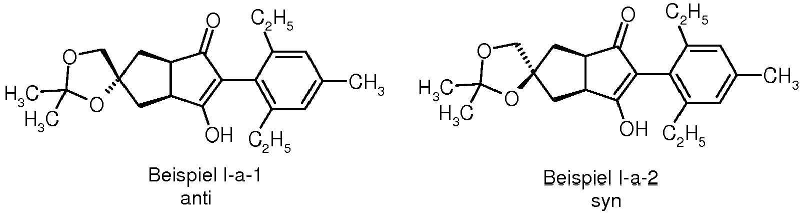

4.62 g (11.5 mMol) Methyl-8-[(2,6-diethyl-4-methylphenyl)acetyl]-2,2-dimethyl-1,3-dioxaspiro[4.4]nonan-7-carboxylat (gemäß Beispiel (II-5)) und 2.57 g Kalium-tert.-butylat werden in 50 ml wasserfreiem N,N-Dimethylformamid 2 h auf 50 °C erhitzt. Nach Abkühlen wird auf Eiswasser gegeben, mit konz. Salzsäure auf pH 3 angesäuert und dreimal mit Ethylacetat extrahiert. Nach Tocknen der organischen Phase (Magnesiumsulfat) wird das Lösemittel abdestilliert und der Rückstand an Kieselgel chromatographiert (Essigsäureethylester/Hexan = 50:50).
**Fraktion A:**
   anti-Isomer (I-a-1); Ausbeute 1.70 g (40%); farblose Kristalle mit Fp. 104-105 °C
**Fraktion B:**
   syn-Isomer (I-a-2); Ausbeute 1.40 g (34%); farblose Kristalle mit Fp. 88-89 °C

### Beispiel I-a-3

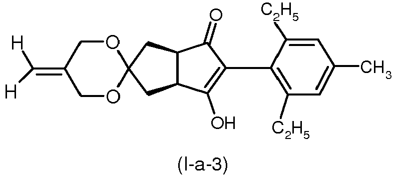

0.79 g (2.64 mMol) 2-(2,6-Diethyl-4-methylphenyl)-3-hydroxy-3a,4,6,6a-tetrahydro-pentalen-1,5-dion (gemäß Beispiel (I-a-4)), 0.58 g (6.6 mMol) 2-Methylidenpropan-1,3-diol und 20 mg p-Toluolsulfonsäure werden in 40 ml Toluol 3 h am Wasserabschneider erhitzt, danach wird einrotiert und in Essigsäureethylester aufgenommen. Nach Schütteln mit Bicarbonatlösung und Wasser, Trocknen (Magnesiumsulfat), Abdestillieren des Lösungsmittels und Chromatographie an Kieselgel (Essigsäureethylester/Hexan v:v=35:65) erhält man die Verbindung der Formel (I-a-3) in Form farbloser Kristalle.
Ausbeute: 0.52 g (53%)
¹H-NMR (400 MHz, CDCl₃): δ = 1.07 und 1.09 (je t, je 3H), 3.12 und 3.38 (je mc, je 1 H), 4.10-4.37 (m, 4 H), 4.39 (mc, 2 H) ppm

### Beispiel I-a-4

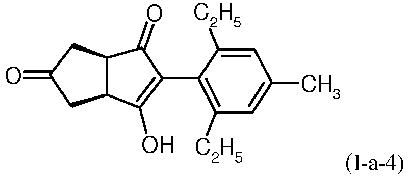

Zu 2.50 g (8.43 mmol) 2-(2,6-Diethyl-4-methylphenyl)-3-hydroxy-5-methyliden-4,5,6,6a-tetrahydropentalen-1(3aH)-on (gemäß Beispiel I-a-5) und 9.02 g (42.16 mmol) Natriummetaperiodat in 50 ml Wasser-tert.-Butanol-Gemisch (v/v=50:50) werden 2 ml einer 2.5%igen Lösung von Osmiumtetroxid in t-Butanol gegeben und 10 min bei Raumtemp. gerührt. Anschließend gibt man noch 50 ml Essigsäureethylester zu und rührt weitere 2 h bei Raumtemperatur. Die Reaktionsmischung wird danach auf Eis gegeben, in Essigsäureethylester aufgenommen und mit Wasser geschüttelt. Nach Trocknen (Magnesiumsulfat) und Abdestillieren des Lösemittels wird an Kieselgel mit Essigsäureethylester/Hexan (v/v=30:70) chromatographiert. Man erhält so 1.86 g (74.1 %) der Verbindung der Formel (I-a-4) als viskoses Öl.
¹H-NMR (400 MHz, CDCl₃): δ = 2.31 (s, 3H), 2.52 und 2.74 (je mc, breit, je 2H), 3.41 und 3.68 (je mc, breit, je 1 H) ppm

### Beispiel I-a-5

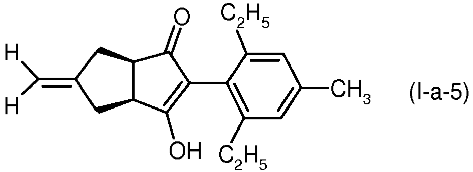

7.85 g (23.9 mmol) 2-[(2,6-Diethyl-4-methylphenyl)acetyl]-4-methylidencyclopentan-carbonsäuremethylester (gemäß Beispiel (II-1)) und 5.36 g (47.8 mmol) Kalium-tert.-butylat werden in 80 ml N,N-Dimethylformamid 2 h auf 50 °C erhitzt. Nach Abkühlen wird die Mischung auf Eiswasser gegeben, mit konz. Salzsäure auf pH 2 angesäuert und mit Essigsäureethylester extrahiert. Die organische Phase wird zweimal mit Wasser gewaschen, getrocknet (Magnesiumsulfat) und einrotiert. Nach chromatographischer Reinigung an Kieselgel (Laufmittel Ethylacetat/Hexan v:v = 40:60) erhält man 4.30 g (61%) der Verbindung der Formel (I-a-5) in Form farbloser Kristalle mit Schmelzpunkt 127-128 °C.
¹H-NMR (400 MHz, CDCl₃): δ = 0.94 und 1.08 (je t, je 3H), 2.28 (s, 3H), 2.51 (mc, 4H), 4.89 (s, 2 H) ppm

### Beispiel I-a-18

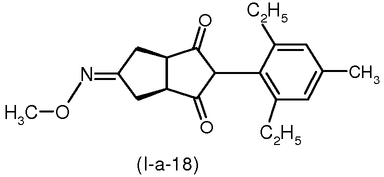

0.161 g (0.54 mmol) 2-(2,6-Diethyl-4-methylphenyl)-3-hydroxy-3a,4,6,6a-tetrahydropentalen-1,5-dion (Beispiel I-a-4) und 0.090 g (1.08 mmol) N-Methylhydroxylamin-hydrochlorid in 10 ml Acetonitril werden mit 0,164 g (1.62 mmol) Triethylamin versetzt und 6 h bei Raumtemp. gerührt. Man gibt anschließend auf Eis, nimmt in Essigsäureethylester auf , trennt die organische Phase ab und wäscht mit Wasser. Trocknen (Magnesiumsulfat), Abdestillieren des Lösungsmittels und Chromatographie an Kieselgel mit Essigsäureethylester/Hexan (v/v=25:75) ergibt schließlich 0.154 g (87%) der gewünschten Verbindung der Formel (I-a-18) als viskoses, gelbstichiges Öl.
¹H-NMR (400 MHz, CDCl₃): δ = 2.62 (mc, 1H), 3.20 und 3.48 (je mc, je 1 H), 3.82 (s, 3 H) ppm

In Analogie zu den Beispielen (I-a-1) bis (I-a-5) bzw. (I-a-18) und gemäß den allgemeinen Angaben zur Herstellung erhält man folgende Verbindungen der Formel (I-a):

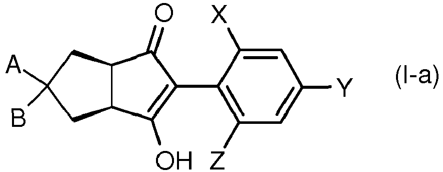

| **Bsp**.-**Nr.** | **X** | **Y** | **Z** | **A** | **B** | **Fp. [°C] oder ¹H-NMR (400 MHz, CDCl₃, δ in ppm)** | **Isomerie** |
|---|---|---|---|---|---|---|---|
| I-a-6 | C₂H₅ | CH₃ | C₂H₅ | -O-CH(C₂H₅)-O-CH₂- | | 181 | anti |
| I-a-7 | C₂H₅ | CH₃ | C₂H₅ | -O-CH(CH₃)-O-CH₂- | | δ =1.48 und 1.49 (2 s, insges. 3 H), 3.74 (d, 1 H), 3.87 (d, 1H), 5.09 (s, br, 1 H) | anti |
| I-a-8 | C₂H₅ | CH₃ | C₂H₅ | -O-(CH₂)₂-O- | | 2.05-2.22 (m, 4H), 2.30 (s, 3H), 3.10 (mc, 1H), 3.38 (mc, 1H), 3.88 (mc, 4H), 6.92 (s, 2H) | |
| I-a-9 | C₂H₅ | CH₃ | C₂H₅ | -O-CH₂-C(CH₃)₂-CH₂-O- | | 96-97 | |
| I-a-10 | C₂H₅ | CH₃ | C₂H₅ | -O-CH(CH₃)-CH₂-O- | | 89-90 | syn-/anti-Gemisch |
| I-a-11 | C₂H₅ | CH₃ | C₂H₅ | -O-(CH₂)₃-O- | | δ = 2.70-2.75 (m. 1H), 3.11 und 3.35 (je mc, je 1H), 3.78 (mc, 1H), 3.88 (mc, 3H) | |
| I-a-12 | C₂H₅ | CH₃ | C₂H₅ | -O-CH(CH₃)-CH₂-CH(CH₃)-O- | | 117-118 | Gemisch bezüglich der CH₃-Gruppen am Acetalring |
| I-a-13 | C₂H₅ | CH₃ | C₂H₅ | -O-(CH₂)₄- | | 3.05 und 3.31 (je mc, je 1H), 3.59 und 3.70 (je mc, je 2H), 7.02 und 7.06 (je s, je 1H) | |
| I-a-14 | C₂H₅ | CH₃ | C₂H₅ | -O-CH₂-CH=CH-CH₂-O- | | 3.10 und 3.39 (je mc, je 1H), 4.04-4.31 (m,4H); 5.67 (s,2H) | |
| I-a-15 | C₂H₅ | CH₃ | C₂H₅ | -O-CH(CH₃)-CH₂-CH(CH₃)-O- | | 79-80 | (R,R)-Konfiguration der CH₃-Gruppen am Acetalring |
| I-a-16 | C₂H₅ | CH₃ | C₂H₅ | -S-(CH₂)₂-S- | | δ = 2.61 (mc, 2H), 3.32 (mc, 1H), 6.98 (s, 1H) | |
| I-a-17 | C₂H₅ | CH₃ | C₂H₅ | -S-(CH₂)₃-S- | | δ = 2.75-3.01 (m, 4H), 3.49 und 3.61 (je mc, je 1H) | |
| I-a-18 | C₂H₅ | CH₃ | C₂H₅ | =N-OCH₃ | | δ = 2.62 (mc, 1H), 3.20 und 3.48 (je mc, je 1 H), 3.82 (s, 3 H) | |
| I-a-19 | C₂H₅ | CH₃ | C₂H₅ | -O-C(CH₃)₂-C(CH₃)₂-O- | | δ = 1.09 und 1.12 (je t, je 3 H), 1.14 und 1.15 (je s, je 6H), 2.10-2.21 (m, 4 H), 3.08 und 3.34 (je mc, je 1 H) | |
| I-a-20 | C₂H₅ | CH₃ | C₂H₅ | -S-(CH₂)₃-O- | | 93-94 | syn-/anti-Gemisch |
| I-a-21 | C₂H₅ | C₂H₅ | C₂H₅ | CH₂= | | 173-174 | |
| I-a-22 | C₂H₅ | C₂H₅ | C₂H₅ | O= | | 243-244 | |
| I-a-23 | C₂H₅ | C₂H₅ | C₂H₅ | -O-(CH₂)₂-O- | | 193-194 | |
| I-a-24 | C₂H₅ | C₂H₅ | C₂H₅ | -O-(CH₂)₃-O- | | 196-197 | |
| I-a-25 | C₂H₅ | CH₃ | C₂H₅ | -OCH₂CH(CH₃)CH₂O- | | 89-90 | |
| I-a-26 | C₂H₅ | CH₃ | C₂H₅ | -OCH₂CH(OCH₃)CH₂O- | | 171-172 | |
| I-a-27 | C₂H₅ | CH₃ | C₂H₅ | -OCH₂CH(OCH₂C₆H₅)CH₂O- | | 75-76 | |
| I-a-28 | C₂H₅ | CH₃ | C₂H₅ | -OCH₂CH(OC₂H₅)CH₂O- | | 75-76 | |
| I-a-29 | C₂H₅ | CH₃ | C₂H₅ | =NOi-C₃H₇ | | 143 | |
| I-a-30 | C₂H₅ | CH₃ | C₂H₅ | =NOCyclopropyl | | 79-80 | |
| I-a-31 | C₂H₅ | CH₃ | C₂H₅ | =NOCH₂Cyclopropyl | | 70-71 | |
| I-a-32 | C₂H₅ | CH₃ | C₂H₅ | =NOCH(CH₃)-C≡CH | | 70 | |
| I-a-33 | C₂H₅ | CH₃ | C₂H₅ | =NOCH₂-C≡CH | | 61-62 | |
| I-a-34 | C₂H₅ | CH₃ | C₂H₅ | -O-CH(CH₃)-CH₂-CH₂-O- | | 89-90 | syn-/anti-Gemisch |
| I-a-35 | C₂H₅ | CH₃ | C₂H₅ | -S-(CH₂)₂-O- | | 246 | syn-/anti-Gemisch |
| I-a-36 | CH₃ | CH₃ | CH₃ | =CH₂ | | 202-203 | |
| I-a-37 | CH₃ | CH₃ | CH₃ | -O-(CH₂)₃-O- | | 225-226 | |
| I-a-38 | CH₃ | CH₃ | CH₃ | -OCH₂CH(CH₃)CH₂O- | | 225 | |
| I-a-39 | CH₃ | CH₃ | CH₃ | -O-(CH₂)₂-O- | | 236 | |
| I-a-40 | CH₃ | CH₃ | CH₃ | -O-(CH₂)₄-O- | | 216-217 | |
| I-a-41 | CH₃ | CH₃ | CH₃ | -O-CH₂-CH=CH-CH₂-O- | | 203-204 | |
| I-a-42 | CH₃ | CH₃ | CH₃ | -O-C(CH₃)₂-O-CH₂- | | 186-187 | |
| I-a-43 | CH₃ | CH₃ | CH₃ | =NOCH₂-C≡CH | | 70-71 | |
| I-a-44 | CH₃ | CH₃ | CH₃ | =NOCH₃ | | 105-106 | |
| I-a-45 | OCH₃ | CH₃ | CH₃ | CH₂= | | 201-202 | |
| I-a-46 | OCH₃ | CH₃ | CH₃ | O= | | 230 | |
| I-a-47 | OCH₃ | CH₃ | CH₃ | -O-(CH₂)₃-O- | | 2.12 und 2.30 (je s, je 3H), 3.09 und 3.34 (je mc, je 1H), 3.27 (s, 3H) | |
| I-a-48 | OCH₃ | CH₃ | CH₃ | -O-(CH₂)₂-O- | | 144-145 | |
| I-a-49 | OCH₃ | CH₃ | CH₃ | -O-CH₂-CH=CH-CH₂-O- | | 184-185 | |
| I-a-50 | OCH₃ | CH₃ | CH₃ | -O-(CH₂)₄-O- | | 202-203 | |
| I-a-51 | OCH₃ | CH₃ | C₂H₅ | CH₂= | | 206 | |
| I-a-52 | OCH₃ | CH₃ | C₂H₅ | O= | | 244 | |
| I-a-53 | OCH₃ | CH₃ | C₂H₅ | -O-(CH₂)₂-O- | | 263 | |
| I-a-54 | OCH₃ | CH₃ | C₂H₅ | -O-(CH₂)₃-O- | | 78-79 | |
| I-a-55 | C₂H₅ | CH₃ | CH₃ | CH₂= | | 164-165 | |
| I-a-56 | C₂H₅ | CH₃ | CH₃ | O= | | 175 | |
| I-a-57 | C₂H₅ | CH₃ | CH₃ | -O-(CH₂)₂-O- | | 195 | |
| I-a-58 | C₂H₅ | CH₃ | CH₃ | -O-(CH₂)₃-O- | | 135-136 | |
| I-a-59 | C₂H₅ | CH₃ | CH₃ | -O-CH₂-C(CH₃)₂-CH₂-O- | | | |
| I-a-60 | C₂H₅ | CH₃ | CH₃ | -O-CH₂-CH=CH-CH₂-O- | | 198-199 | |
| I-a-61 | C₂H₅ | CH₃ | CH₃ | -O-(CH₂)₄-O- | | 208-209 | |
| I-a-62 | C₂H₅ | CH₃ | CH₃ | -O-C(CH₃)₂-O-CH₂- | | 81-82 | anti |
| I-a-63 | C₂H₅ | CH₃ | H | -O-C(CH₃)₂-O-CH₂- | | | |
| I-a-64 | C₂H₅ | CH₃ | H | -O-(CH₂)₄-O- | | 1.12 (t, 3H), 2.32 (s, 3H), 2.49 (1, 2H), 3.55 und 3.70 (je mc, je 2H), 6.91 (d, 1H), 7.02 (d, 2H), 7.11 (s, 1H) | |
| I-a-65 | C₂H₅ | CH₃ | H | -O-CH₂-CH=CH-CH₂-O- | | | |
| I-a-66 | C₂H₅ | CH₃ | H | -O-(CH₂)₃-O- | | 1.12 (t, 3H), 2.31 (s, 3H), 2.72 (mc, 1H), 3.08 (mc, 1H), 3.30 (mc, 1H), | |
| I-a-67 | C₂H₅ | CH₃ | H | -O-(CH₂)₂-O- | | 79-80 | |
| I-a-68 | C₂H₅ | CH₃ | C₂H₅ | =NOt-C₄H₉ | | 1.00 und 1.09 (je t, je 3H), 1.22 (s, 9H), 2.30 (s, 3H) | |
| I-a-69 | C₂H₅ | CH₃ | C₂H₅ | =NOi-C₄H₉ | | 0.90 (mc, 6H), 1.92 (hept, 1H), 3.20 und 3.39 (je mc, je 1H), 3.79 (mc, 2H), 6.91 und 6.93 (je s, je 1H) | |
| I-a-70 | C₂H₅ | CH₃ | C₂H₅ | =NOCH₂CF₃ | | 2.60-2.86 (m, 3H), 3.22 und 3.51 (je mc, je 1H), 4.48 (mc, 2H) | |
| I-a-71 | C₂H₅ | CH₃ | H | CH₂= | | 172 | |
| I-a-72 | C₂H₅ | CH₃ | C₂H₅ | =NOH | | 119-120 | |
| I-a-73 | C₂H₅ | CH₃ | H | O= | | 68-69 | |
| I-a-74 | C₂H₅ | CH₃ | C₂H₅ | -O-CH(C₂H₅)-CH₂-O | | | syn-/anti-Gemisch |
| I-a-75 | C₂H₅ | CH₃ | C₂H₅ | -O-CH(CH₃)-CH(CH₃)-O- | | | Gemisch bezüglich der CH₃-Gruppen am Acetalring |

### Beispiel I-b-1

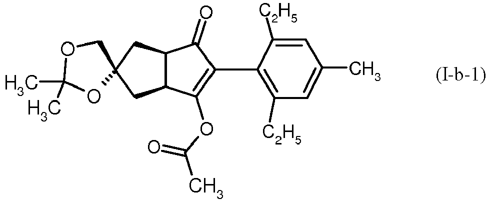

0.100 g (0.27 mmol) der erfindungsgemäßen Verbindung I-a-1 (anti-Isomer), 23.3 mg (0.297 mmol) Acetylchlorid und 82 mg (0.297 mmol) Triethylamin in 5 ml Dichlormethan werden 2 h bei Raumtemp. gerührt. Der Ansatz wird auf Eis gegeben, in Dichlormethan aufgenommen, mit Wasser gewaschen, getrocknet (Magnesiumsulfat) und einrotiert. Nach Chromatographie an Kieselgel (Laufmittel Essigsäureethylester/Hexan v:v= 30:70) erhält man die erfindungsgemäße Verbindung der Formel (I-b-1) als farbloses Öl. Ausbeute 76.6 mg (68 %).
¹H-NMR (400 MHz, CDCl₃): δ 1.39 (s, 6 H), 2.10 (s, 3 H), 2.21 (s, 3 H), 3.85 (dd, 2 H) ppm

In Analogie zu Beispiel (I-b-1) und gemäß den allgemeinen Angaben zur Herstellung erhält man folgende Verbindungen der Formel (I-b):

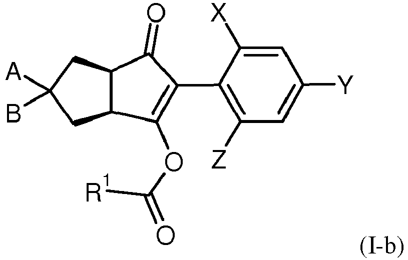

| **Bsp**.-**Nr.** | **X** | **Y** | **Z** | **A** | **B** | **R¹** | **Fp. [°C] oder ¹H-NMR (400 MHz, CDCl₃, δ in ppm)** | **Isomerie** |
|---|---|---|---|---|---|---|---|---|
| I-b-2 | C₂H₅ | CH₃ | C₂H₅ | -O-C(CH₃)₂-O-CH₂- | | C₂H₅ | 83 | anti |
| I-b-3 | C₂H₅ | CH₃ | C₂H₅ | -O-C(CH₃)₂-O-CH₂- | | i-C₃H₇ | 108-109 | anti |
| I-b-4 | C₂H₅ | CH₃ | C₂H₅ | -O-C(CH₃)₂-O-CH₂- | | H₃C-O-CH₂- | 3.29 (s, 3H), 3.39 (mc, 1 H), 3.88 (dd, 2H), 4.02 (s, 2 H) | anti |
| I-b-5 | C₂H₅ | CH₃ | C₂H₅ | -O-C(CH₃)₂-O-CH₂- | | t-C₄H₉ | 114-115 | anti |
| I-b-6 | C₂H₅ | CH₃ | C₂H₅ | -O-C(CH₃)₂-O-CH₂- | | C₂H₅ | 1.12 (t, 3 H), 2.49 (q, 2 H), 3.90 (dd, 2 H) | syn |
| I-b-7 | C₂H₅ | CH₃ | C₂H₅ | -O-C(CH₃)₂-O-CH₂- | | i-C₃H₇ | δ = 1.32 (d, 6H), 2.60 (hept, 1H), 3.19 (mc, 1H), 4.01 (mc, 1H) | syn |
| I-b-8 | C₂H₅ | CH₃ | C₂H₅ | -O-C(CH₃)₂-O-CH₂- | | t-C₄H₉ | 73 | syn |
| I-b-9 | C₂H₅ | CH₃ | C₂H₅ | -S-(CH₂)₃-S- | | C₂H₅ | δ = 1.01 (3 x t, 9 H), 2.70-2.98 (m, 5 H) | |
| I-b-10 | C₂H₅ | CH₃ | C₂H₅ | -S-(CH₂)₃-S- | | H₃C-O-CH₂ | δ = 2.62 (mc, 1 H), 2.70-2.97 (m, 5 H), 3.32 (s, 3 H), 4.02 (dd, 2H) | |
| I-b-11 | C₂H₅ | CH₃ | C₂H₅ | -S-(CH₂)₃-S- | | i-C₃H₇ | δ = 1.09 (d, 6 H), 2.58 (hept, 1 H), 3.48 und 4.20 (je mc, je 1H) | |
| I-b-12 | C₂H₅ | CH₃ | C₂H₅ | -O-CH₂-CH=CH-CH₂-O- | | i-C₃H₇ | 116-117 | |
| I-b-13 | C₂H₅ | CH₃ | C₂H₅ | 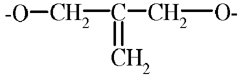 | | i-C₃H₇ | 108-109 | |
| I-b-14 | C₂H₅ | CH₃ | C₂H₅ | -OCH₂CH(CH₃)CH₂O- | | i-C₃H₇ | 116 | |
| I-b-15 | C₂H₅ | CH₃ | C₂H₅ | -O-CHCH₃-CH₂-CHCH₃-O- | | i-C₃H₇ | 1.00-1.26 (m, 18H), 2.58, hept, 1H), 3.20 (mc, 1H), 3.90-4.00 (m, 2+1H) | (R,R)-Konfiguration der CH₃-Gruppen am Acetalring |
| I-b-16 | C₂H₅ | CH₃ | C₂H₅ | -OCH₂CH(OC₂H₅)CH₂O- | | i-C₃H₇ | 2.59 (hept, 1H), 3.19 (mc, 1H), 3.40 mc, 1H), 3.50 (mc, 3H), 3.98 (mc, 2H), 4.22 | |
| | | | | | | | (mc, 1H) | |
| I-b-17 | C₂H₅ | CH₃ | C₂H₅ | -OCH₂CH(OCH₂C₆H₅)CH₂O- | | i-C₃H₇ | 1.09 m, 3H), 3.18 (mc, 1H), 3.40 (mc, 1H), 3.69-4.00 (m, 5H), 4.60 (s, 2H) | |
| I-b-18 | C₂H₅ | CH₃ | C₂H₅ | -O-(CH₂)₂-O- | | CH₃ | 132 | |
| I-b-19 | C₂H₅ | CH₃ | C₂H₅ | -S-(CH₂)₂-O- | | t-C₄H₉ | 129-130 | syn-/anti-Gemisch |
| I-b-20 | C₂H₅ | CH₃ | C₂H₅ | -S-(CH₂)₃-O- | | t-C₄H₉ | 1.07 (s, 9H), 2.98-3.03 (m, 2H), 3.76 (mc, 2H), 4.00 (mc, 1H) | syn-/anti-Gemisch |
| I-b-21 | C₂H₅ | CH₃ | C₂H₅ | -S-(CH₂)₂-O- | | i-C₃H₇ | 1.04 (mc, 6H), 2.60 (hept, 1H), 3.01 (mc, 1H), 3.95-4.12 (m, 3H) | syn-/anti-Gemisch |
| I-b-22 | C₂H₅ | CH₃ | C₂H₅ | -O-(CH₂)₃-O- | | t-C₄H₉ | 1.05 (s, 9H), 2.77 (mc, 1H), 3.18 (mc, 1H), 3.75-3.94 (m, 5H) | |
| I-b-23 | C₂H₅ | CH₃ | C₂H₅ | -O-(CH₂)₃-O- | | CH₃ | 2.11 und 2.32 (jes, je 3H), 3.18 (mc, 1H), 3.72-3.90 (m, 4H), 3.95 (mc, 1H) | |
| I-b-24 | C₂H₅ | CH₃ | C₂H₅ | -O-(CH₂)₃-O- | | C₂H₅ | 99-100 | |
| I-b-25 | C₂H₅ | CH₃ | C₂H₅ | -O-(CH₂)₃-O- | | C(CH₃)₂CH₂Cl | 1.10 und 1.15 (je s, je 3H), 1.58 (mc, 2H), 1.82 (mc, 1H), 2.03-2.10 (m. 2H), 3.40 und 3.48 (je d, je 1H) | |
| I-b-26 | C₂H₅ | C₂H₅ | C₂H₅ | CH₂= | | t-C₄H₉ | 1.05 (s, 9H), 2.22-2.40 (m, 5H), 2.54-2.74 (m, 5H), 4.91 (mc, 2H) | |
| I-b-27 | C₂H₅ | CH₃ | C₂H₅ | -O-(CH₂)₃-O- | | t-C₄H₉ | 77-78 | |
| I-b-28 | C₂H₅ | CH₃ | C₂H₅ | -O-(CH₂)₃-O- | | H₃C-O-CH₂- | 94-95 | |
| I-b-29 | C₂H₅ | CH₃ | C₂H₅ | -O-(CH₂)₃-O- | | i-C₃H₇ | 95-96 | |
| I-b-30 | C₂H₅ | CH₃ | C₂H₅ | -O-(CH₂)₃-O- | | -C(CH₃)₂C₂H₅ | 93-94 | |
| I-b-31 | C₂H₅ | CH₃ | C₂H₅ | -O-(CH₂)₂-O- | | i-C₃H₇ | 1,05 und 1.10 (je mc, je 6H), 2.59 (hept, 1H), 3.20 (mc, 1H), 3.90 (mc, 4H), 4.00 (mc, 1 H) | |
| I-b-32 | C₂H₅ | CH₃ | C₂H₅ | -O-(CH₂)₂-O- | | t-C₄H₉ | 108-109 | |
| I-b-33 | C₂H₅ | CH₃ | C₂H₅ | -O-(CH₂)₂-O- | | C₆H₅ | 2.28 (s, 3H), 3.90 (mc, 4H), 6.88 und 6.91 (je s, je 1H), 7.41 (t, 2H), 7.60 (t, 2H), 7.90 (d, 2H) | |
| I-b-34 | C₂H₅ | CH₃ | C₂H₅ | -O-(CH₂)₂-O- | | p-Cl-C₆H₅ | 2.30 (s, 3H), 3.88 (mc, 4H), 7.40 (d, 2H), 7.82 (d, 2H) | |
| I-b-35 | C₂H₅ | CH₃ | C₂H₅ | -O-(CH₂)₂-O- | | p-CH₃-C₆H₅ | 2.30 und 2.38 (je s, je 3H), 3.88 (mc, 4H), 4.21 (mc, 1H), 7.20 (d, 2H), 7.79 (d, 2H) | |
| I-b-36 | C₂H₅ | CH₃ | C₂H₅ | -O-(CH₂)₂-O- | | p-OCH₃-C₆H₅ | 120-121 | |
| I-b-37 | CH₃ | CH₃ | CH₃ | CH₂= | | t-C₄H₉ | 87 | |
| I-b-38 | CH₃ | CH₃ | CH₃ | CH₂= | | i-C₃H₇ | 1.03 und 1.11 (2 x d, Σ 6H), 2.00, 2.05 und 2.21 (je s, je 3H), 2.57 (hept, 1H), 4.90 (mc, 2H) | |
| I-b-39 | CH₃ | CH₃ | CH₃ | -O-(CH₂)₂-O- | | i-C₃H₇ | 1.02 und 1.08 (2 x d, Σ 6H), 2.03, 2.11 und 2.23 (je s, je 3H), 2.58 (hept, 1H), 3.88 (mc, 4H) | |
| I-b-40 | CH₃ | CH₃ | CH₃ | -O-(CH₂)₄-O- | | t-C₄H₉ | 134-135 | |
| I-b-41 | CH₃ | CH₃ | CH₃ | -O-(CH₂)₄-O- | | i-C₃H₇ | 106-107 | |
| I-b-42 | CH₃ | CH₃ | CH₃ | -O-CH₂-CH=CH-CH₂-O- | | i-C₃H₇ | 1.02 und 1.09 (2 x d, Σ 6H), 2.57 (hept, 1H), 3.88 (mc, 4H), 4.00-4.31 (m, 4H), 5.65 (mc, 2H) | |
| I-b-43 | CH₃ | CH₃ | CH₃ | -OCH₂CH(CH₃)CH₂O- | | i-C₃H₇ | 0.71 und 0.88 (je d, Σ 3H), 1.02 und 1.10 (je mc, je 3H), 2.58 (mc, 1H), 3.30-3.45 (m, 2H), 3.40-3.88 (m, 3H) | |
| I-b-44 | CH₃ | CH₃ | CH₃ | -O-C(CH₃)₂-O-CH₂- | | i-C₄H₉ | 0.88 (mc, 6H), 1.48 (s, 6H), 3.31 (mc, 1H), 3.83 und 3.89 (je d, je 1H), 6.82 (s, 2H) | syn-/anti-Gemisch |
| I-b-45 | CH₃ | CH₃ | CH₃ | -O-C(CH₃)₂-O-CH₂- | | -C(CH₃)₂C₂H₅ | 88 | syn-/anti-Gemisch |
| I-b-46 | CH₃ | CH₃ | CH₃ | -O-C(CH₃)₂-O-CH₂- | | i-C₃H₇ | 1.01 (mc, 6H), 1.38 (s, 6H), 2.58 (hept, 1H), 3.32 (mc, 1H), 3.85 und 3.90 (je d, je 2H), 3.88 (mc, 1H) | syn-/anti-Gemisch |
| I-b-47 | CH₃ | CH₃ | CH₃ | -O-C(CH₃)₂-O-CH₂- | | H₃C-O-CH₂- | 1.38 (s, 6H), 3.28 (s. 3H), 3.85 und 3.89 (Je d, je 1H), 3.98 und 4.04 (Je d, je 1H), 6.84 (s, 2H) | syn-/anti-Gemisch |
| I-b-48 | CH₃ | CH₃ | CH₃ | -O-C(CH₃)₂-O-CH₂- | | t-C₄H₉ | 97-98 | syn-/anti-Gemisch |
| I-b-49 | CH₃ | CH₃ | CH₃ | -O-C(CH₃)₂-O-CH₂- | | CH₃ | 1.40 (s, 6H), 2.05 (s, 3H), 2.10 (s, 6H), 2.26 (s, 3H), 3.84 und 3.88 (je d, je 1H) | syn-/anti-Gemisch |
| I-b-50 | CH₃ | CH₃ | CH₃ | -O-C(CH₃)₂-O-CH₂- | | C₂H₅ | 1.05 (t, 3H), 1.39 (s, 6H), 2.38 (q, 2H), 3.34 (mc, 1H), 3.85 und 3.90 (je d, je 1H), 3.92 (mc, 1H) | syn-/anti-Gemisch |
| I-b-51 | CH₃ | CH₃ | CH₃ | -O-C(CH₃)₂-O-CH₂- | | C(CH₃)₂CH₂Cl | 90-91 | syn-/anti-Gemisch |
| I-b-52 | CH₃ | CH₃ | CH₃ | O= | | C(CH₃)₂CH₂Cl | 1.12 und 1.17 (je s, je 3H), 3.43 und 3.46 (je d, je 3H), 6.83 (s, 2H) | |
| I-b-53 | CH₃ | CH₃ | CH₃ | O= | | H₃C-O-CH₂- | 1.99, 2.09 und 2.25 (je s, je 3H), 3.30 (s, 3H), 3.51 (mc, 1H), 4.03 (s, 2H) | |
| I-b-54 | CH₃ | CH₃ | CH₃ | O= | | i-C₃H₇ | 1.04 und 1.07 (je d, je 3H), 2.56 (hept, 1H), 3.50 und 4.12 (je mc, je 1H) | |
| I-b-55 | CH₃ | CH₃ | CH₃ | O= | | -C(CH₃)₂C₂H₅ | 0.61 (t, 3H), 1.42 (q, 2H), 2.39-2.85 (m, 4H), 6.83 (s, 2H) | |
| I-b-56 | CH₃ | CH₃ | CH₃ | O= | | i-C₄H₉ | 0.80 (mc, 6H), 1.95 (hept, 1H), 2.40-2.85 (m, 4H) | |
| I-b-57 | CH₃ | CH₃ | CH₃ | O= | | C₂H₅ | 1.05 (t, 3H), 2.40 (q, 2H), 3.49 und 4.16 (je mc, je 1H) | |
| I-b-58 | CH₃ | CH₃ | CH₃ | =NOCH₂-C≡CH | | i-C₃H₇ | 1.02 und 1.11 (je d, je 3H), 2.41 (mc, 1H), 2.55-2.94 (m, 5H), 4.51 (mc, 2H) | |
| I-b-59 | CH₃ | CH₃ | CH₃ | =NOCH₂-C≡CH | | t-C₄H₉ | 1.09 (s, 9H), 2.42 (mc, 1H), 2.63-3.08 (m, | |
| | | | | | | | 4H), 4.52 (mc, 1H) | |
| I-b-60 | CH₃ | CH₃ | CH₃ | =O | | t-C₄H₉ | 1.08 (s, 9H), 1.99, 2.06 und 2.22 (je s, je 3H), 3.50 und 4.11 (je mc, je 1H) | |
| I-b-61 | OCH₃ | CH₃ | CH₃ | =O | | i-C₃H₇ | 1.10 (mc, 6H), 2.30 (s, 3H), 3.48 (mc, 1H), 3.68 (s, 3H), 4.12-4.28 (m, 1H) | |
| I-b-62 | OCH₃ | CH₃ | CH₃ | =CH₂ | | i-C₃H₇ | 1.10 (mc, 6H), 2.00 und 2.29 (je s, je 3H), 3.65 (s, 3H), 4.90 (mc, 2H) | |
| I-b-63 | OCH₃ | CH₃ | CH₃ | -O-CH₂-CH=CH-CH₂-O- | | i-C₃H₇ | 1.05-1.14 (m, 5H), 2.60 (hept, 1H), 3.68 und 3.70 (je s, Σ 3H), 5.63 (mc, 2H) | |
| I-b-64 | OCH₃ | CH₃ | CH₃ | -O-(CH₂)₃-O- | | i-C₃H₇ | 1.08-1.15 (m, 6H), 2.61 (hept, 1H), 3.66 (s, 3H), 6.50 und 6.63 (je s, je 1H) | |
| I-b-65 | OCH₃ | CH₃ | CH₃ | -O-(CH₂)₂-O- | | i-C₃H₇ | 1.10 (mc, 6H), 2.62 (hept, 1H), 3.68 s, 3H), 3.88 (mc, 4H) | |
| I-b-66 | C₂H₅ | CH₃ | CH₃ | -O-C(CH₃)₂-O-CH₂- | | CH₃ | 1.07 und 1.10 (je t, Σ6H), 1.40 (s, 6H), 2.05 und 2.06 (je s, je 3H), 3.84 und 3.88 | anti |
| | | | | | | | (je d, je 1H) | |
| I-b-67 | C₂H₅ | CH₃ | CH₃ | -O-C(CH₃)₂-O-CH₂- | | C₂H₅ | 1.05 (mc, 3H), 1.10 (t, 3H), 2.05 und 2.28 (je s, je 3H), 2.34 (mc, 2H) | anti |
| I-b-68 | C₂H₅ | CH₃ | CH₃ | -O-C(CH₃)₂-O-CH₂- | | i-C₄H₉ | 0.79 (mc, 6H), 1.38 (s, 6H), 3.32 (mc, 1H), 3.85 (mc, 2H), 3.94 (mc, 1H) | anti |
| I-b-69 | C₂H₅ | CH₃ | CH₃ | -O-C(CH₃)₂-O-CH₂- | | i-C₃H₇ | 1.05 (mc, 6H), 1.39 (s, 6H), 2.55 (hept, 1H), 3.33 (mc, 1H), 3.89 (mc, 2H), 3.97 (mc, 1H) | anti |
| I-b-70 | C₂H₅ | CH₃ | CH₃ | -O-C(CH₃)₂-O-CH₂- | | t-C₄H₉ | 79-80 | anti |
| I-b-71 | C₂H₅ | CH₃ | CH₃ | O= | | t-C₄H₉ | 1.06 (s, 9H), 2.27 (s, 3H), 3.50 und 4.15 (je mc, je1H) | |
| I-b-72 | C₂H₅ | CH₃ | CH₃ | CH₂= | | i-C₃H₇ | 0.95-1.10 (m, 9H), 2.26 (s, 3H), 2.52-2.70 (m, 4H), 4.90 (mc, 2H) | |
| I-b-73 | C₂H₅ | CH₃ | CH₃ | CH₂= | | t-C₄H₉ | 0.98 und 1.03 (je t, Σ 3H), 1.06 (s, 9H), 3.18 und 3,88 (je mc, je 1H), 4.91 (mc, 2H) | |
| I-b-74 | C₂H₅ | CH₃ | CH₃ | -O-(CH₂)₃-O- | | t-C₄H₉ | 1.08 (s, 9H), 2.25-2.33 (m, 1H), 3.15 (mc, 1H), 3.70-3.90 (m, 5H) | |
| I-b-75 | C₂H₅ | CH₃ | CH₃ | -O-CH₂-CH=CH-CH₂-O- | | t-C₄H₉ | 103-104 | |
| I-b-76 | C₂H₅ | CH₃ | CH₃ | -O-CH₂-CH=CH-CH₂-O- | | i-C₃H₇ | 84-85 | |
| I-b-77 | C₂H₅ | CH₃ | CH₃ | -O-(CH₂)₄-O- | | i-C₃H₇ | 101 | |
| I-b-78 | C₂H₅ | CH₃ | CH₃ | -O-(CH₂)₄-O- | | t-C₄H₉ | 119-120 | |
| I-b-79 | C₂H₅ | CH₃ | CH₃ | -O-(CH₂)₂-O- | | i-C₃H₇ | 1.06 (mc, 6H), 2.47 (hept, 1H), 3.19 (mc, 1H), 3.85-3.95 (m, 5H) | |

### Beispiel I-c-1

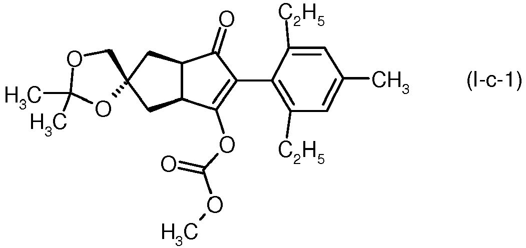

Zu 0.100 g (0.27 mmol) der erfindungsgemäßen Verbindung (I-a-1) und 28 mg (0.30 mmol) Chlorameisensäuremethylester in 5 ml Dichlormethan werden bei Raumtemp. 82 mg (0.81 mmol) Triethylamin zugegeben und anschließend noch 1 h nachgerührt. Der Ansatz wird auf Eiswasser gegeben, in Dichlormethan aufgenommen, mit Wasser gewaschen, getrocknet (Magnesiumsulfat) und das Lösemittel abdestilliert.

Chromatographische Aufreinigung an Kieselgel (Essigsäureethylester/Hexan v/v=20:80) ergibt 104 mg (90%) der gewünschten Verbindung der Formel (I-c-1) in Form farbloser Kristalle mit Schmelzpunkt 111-112°C.

### Beispiel I-c-17

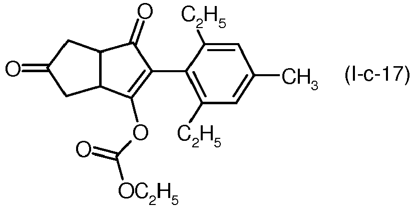

Zu 1.50 g (4.05 mmol) 2-(2,6-Diethyl-4-methylphenyl)-5-methyliden-3-oxo-3,3a,4,5,6,6a-hexahydropentalen-1-yl-ethylcarbonat (Beispiel I-c-5) und 4.33 g (20.24 mmol) Natriummetaperiodat in 150 ml Wasser-tert.-Butanol-Gemisch (v/v=50:50) werden 0.98 ml einer 2.5%igen Lösung von Osmiumtetroxid in n-Butanol gegeben und 10 min bei Raumtemp. gerührt. Anschließend gibt man noch 50 ml Essigsäureethylester zu und rührt weitere 2 h bei Raumtemperatur.

Die Reaktionsmischung wird auf Eis gegeben, in Essigsäureethylester aufgenommen und mit Wasser geschüttelt. Nach Trocknen (Magnesiumsulfat) und Abdestillieren des Lösemittels wird an Kieselgel mit Essigsäureethylester/Hexan (v/v=30:70) chromatographiert. Man erhält so 1.00 g (66%) der gewünschten Verbindung der Formel (I-c-17) als viskoses Öl.
¹H-NMR (400 MHz, CDCl₃): δ =1.26 (t, 3 H), 3.51 und 4.31 (jeweils mc, je 1 H), 4.20 (mc, 2 H) ppm

In Analogie zu den Beispielen (I-c-1) und (I-c-17) und gemäß den allgemeinen Angaben zur Herstellung erhält man folgende Verbindungen der Formel (I-c):

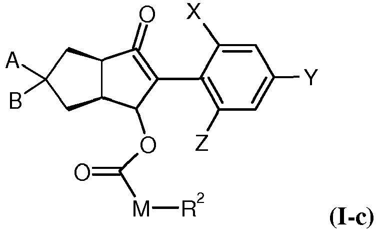

| **Bsp**.-**Nr.** | **X** | **Y** | **Z** | **A** | **B** | **M** | **R²** | **Fp**. [**°C**] **oder ¹H-NMR (400 MHz, CDCl₃, δ in ppm)** | **Isomerie** |
|---|---|---|---|---|---|---|---|---|---|
| I-c-2 | C₂H₅ | CH₃ | C₂H₅ | -O-C(CH₃)₂-O-CH₂- | | O | i-C₃H₇ | δ = 1.21 (d, 6 H), 1.88 und 1.98 (je mc, je 1 H), 3.89 (dd. 2 H), 4.81 (quint, 1H) | anti |
| I-c-3 | C₂H₅ | CH₃ | C₂H₅ | -O-C(CH₃)₂-O-CH₂- | | O | i-C₃H₇ | δ = 1.32 (d, 6 H), 1.92-2.13 (m, 3 H), 3.92 (dd, 2H), 4.83 (quint, 1 H) | syn |
| I-c-4 | C₂H₅ | CH₃ | C₂H₅ | -OC(CH₃)₂-OCH₂- | | O | CH₃ | 132-133 | syn |
| I-c-5 | C₂H₅ | CH₃ | C₂Hs | =CH₂ | | O | C₂H₅ | δ = 1.26 (t, 3H), 3.20 (mc, 1 H), 4.02 (mc, 1H), 4.82 (mc, 2 H). | |
| I-c-6 | C₂H₅ | CH₃ | C₂H₅ | -O-CH₂-C(CH₃)₂-CH₂-O- | | O | C₂H₅ | 125-126 | |
| I-c-7 | C₂H₅ | CH₃ | C₂H₅ | -O-(CH₂)₂-O- | | O | C₂H₅ | δ = 1.08, 1.09 und 1.22 (je t, je 3H), 3.20 (mc, 1 H), 3.89 (mc, 4 H), 4.04 (mc, 1H), 4.19 (mc, 2H) | |
| I-c-8 | C₂H₅ | CH₃ | C₂H₅ | -O-CHCH₃-CH₂-O- | | O | C₂H₅ | 79 | syn-/anti-Gemisch |
| I-c-9 | C₂H₅ | CH₃ | C₂H₅ | -O-(CH₂)₄-O- | | O | C₂H₅ | 113 | |
| I-c-10 | C₂H₅ | CH₃ | C₂H₅ | -O-CHCH₃-CH₂- CHCH₃-O- | | O | C₂H₅ | δ = 1.02-1.28 (m, 15 H), 3.20 (mc, 1H), 3.80-4.01 (m, 3 H), 4.18 (mc, 2H) | Isomerengemisch bezüglich der CH₃-Gruppen am Acetalring |
| I-c-11 | C₂H₅ | CH₃ | C₂H₅ | -O-CH₂-CH=CH-CH₂-O- | | O | C₂H₅ | δ = 1.08. 1.12 und 1.24 (je t, je 3 H), 4.01-4.32 (m, 7 H), 5.65 (s, 2 H) | |
| I-c-12 | C₂H₅ | CH₃ | C₂H₅ | 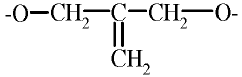 | | O | C₂H₅ | 119 | |
| I-c-13 | C₂H₅ | CH₃ | C₂H₅ | -O-CH(CH₃)-CH₂-CH(CH₃)-O- | | O | C₂H₅ | δ = 1.05-1.25 (m, 15H), 3.20 und 3.92 (je mc, je 1 H), 4.00 (mc, 2H), 4.18 | (R,R)-Konfiguration der CH₃-Gruppen am Acetalring |
| | | | | | | | | (mc, 2 H) | |
| I-c-14 | C₂H₅ | CH₃ | C₂H₅ | -O-(CH₂)₃-O- | | O | C₂H₅ | 110 | |
| I-c-15 | C₂H₅ | CH₃ | C₂H₅ | -S-(CH₂)₂-S- | | O | C₂H₅ | δ = 1.25 (t, 3H), 2.61 (mc, 2H), 3.35 (mc, 5H), 4.19 (mc, 3H), 6.90 (s, 2H) | |
| I-c-16 | C₂H₅ | CH₃ | C₂H₅ | -S-(CH₂)₃-S- | | O | C₂H₅ | δ = 1.26 (t, 3H), 2.83 (mc, 3H), 2.93 (mc, 3H), 4.20 (mc, 2H), 4.22 (mc, 1H) | |
| I-c-17 | C₂H₅ | CH₃ | C₂H₅ | =O | | O | C₂H₅ | 1.26 (t, 3 H), 3.51 und 4.31 (jeweils mc, je 1 H), 4.20 (mc, 2 H) | |
| I-c-18 | C₂H₅ | CH₃ | C₂H₅ | -O-C(CH₃)₂-C(CH₃)₂-O- | | O | C₂H₅ | 116-117 | |
| I-c-19 | C₂H₅ | CH₃ | C₂H₅ | -S-(CH₂)₃-O- | | O | C₂H₅ | 1.24 (t, 3H), 3.00 (mc, 2H), 3.72 (mc, 2H), 4.18 (mc, 1H) | syn-/anti-Gemisch |
| I-c-20 | C₂H₅ | CH₃ | C₂H₅ | -S-(CH₂)₃-S- | | O | CH₃ | 2.62 (mc, 1H), 2.80-2.98 (m, 5H), 3.49 (mc, 1H), 3.78 (s, 1H), 4.23 (mc, 1H) | |
| I-c-21 | C₂H₅ | CH₃ | C₂H₅ | -O-CH₂-CH=CH-CH₂-O- | | O | CH₃ | 1.08 und 1.12 (je t, je 3H), 3.75 (s, 3H), 4.02 (mc, 2H), 4.16-4.32 (m, 3H), 5.67 (s, 2H) | |
| I-c-22 | C₂H₅ | CH₃ | C₂H₅ | -O-CH₂-C(CH₃)₂-CH₂-O- | | O | C₂H₅ | 98-99 | |
| I-c-23 | C₂H₅ | CH₃ | C₂H₅ | -O-CH₂-C(CH₃)₂-CH₂-O- | | O | CH₃ | 126-127 | |
| I-c-24 | C₂H₅ | CH₃ | C₂H₅ | -O-CH(CH₃)-CH₂-CH(CH₃)O- | | O | CH₃ | 1.03-1.22 (m, 12H), 3.20 (mc, 1H), 3.75 (verdoppeltes Singulett, Σ 3H), 3.90-4.02 (m, 3H) | (R,R)-Konfiguration der CH₃-Gruppen am Acetalring |
| I-c-25 | C₂H₅ | CH₃ | C₂H₅ | -O-CH₂-C(OCH₃)-CH₂-O- | | O | C₂H₅ | 1.23 (t, 3H), 2.70-2.79 (m, 1H), 4.19 (mc, 2H), 3.10 (mc, 1H), 3.38 (s, 3H), | |
| I-c-26 | C₂H₅ | CH₃ | C₂H₅ | -O-CH₂CH(OC₂H₅)CH₂-O- | | O | C₂H₅ | 1.03-1.28 (m, 12H), 3.20 und 3.39 (je mc, je 1H), 3.95-4.05 (m, 2H), 4.11-4.25 (m, 3H) | |
| I-c-27 | C₂H₅ | CH₃ | C₂H₅ | -OCH₂-CH(OC₂H₅) CH₂-O- | | O | CH₃ | 1.00-1.21 (m, 12H), 3.76 (s, 3H), 3.92-4.05 (m, 2H), 4.21 (mc, 1H) | |
| I-c-28 | C₂H₅ | CH₃ | C₂H₅ | -OCH₂-CH(OCH₂C₆H₅)CH₂-O- | | O | C₂H₅ | 1.22 (t, 3H), 2.95 (mc, 2H), 4.15 (mc, 2H), 4.55 (s, 2H), 6.88 und 6.91 (je s, je 1H), 7.27-7.85 (m, 5H) | |
| I-c-29 | C₂H₅ | CH₃ | C₂Hs | -OCH₂-CH(O CH₂C₆H₅)CH₂-O- | | O | CH₃ | 2.05-2.12 (m, 2H), 3.41 (mc, 1H), 3.74 (s, 3H), 4.60 (s, 2H), | |
| I-c-30 | C₂H₅ | CH₃ | C₂H₅ | -S-(CH₂)₂-O- | | O | C₂H₅ | 94-95 | syn-/anti-Gemisch |
| I-c-31 | C₂H₅ | CH₃ | C₂H₅ | -S-(CH₂)₂-O- | | O | CH₃ | 3.00 (mc, 2H), 3.77 (s, 3H), 3.96 (mc, 1H), 4.02-4.10 (m, 2H) | syn-/anti-Gemisch |
| I-c-32 | C₂H₅ | CH₃ | C₂H₅ | -S-(CH₂)₃-O- | | O | CH₃ | 2.95-3.05 (m, 2H), 3.25 (mc, 1H), 3.72 (mc, 2H), 3.75 (s, 3H), 4.08 (mc, 1H) | syn-/anti-Gemisch |
| I-c-33 | C₂H₅ | CH₃ | C₂H₅ | -S-(CH₂)₂-O- | | O | i-C₃H₇ | 1.23 (6H), 3.01 (mc, 2H), 4.08 (mc, 2H), 4.81 (mc, 1H) | syn-/anti-Gemisch |
| I-c-34 | C₂H₅₆ | CH₃ | C₂H₅ | -O-(CH₂)₃-O- | | O | i-C₃H₇ | 1.22 (mc, 6H), 3.18 (mc, 1H), 3.79 (mc, 2H), 3.88 (mc, 2H), 3.99, (mc, 1H), 4.81 (hept, 1H) | |
| I-c-35 | C₂H₅ | CH₃ | C₂Hs | -O-(CH₂)₃-O- | | O | CH₃ | 2.00-2.12 (m, 2H), 3.19 (mc, 1H), 3.73 (s, 3H), 3.88 (mc, 1H), 3.99 (mc, 1H) | |
| I-c-36 | C₂H₅ | CH₃ | C₂H₅ | -O-(CH₂)-O- | | O | i-C₃H₇ | 110-111 | |
| I-c-37 | C₂H₅ | CH₃ | C₂H₅ | -O-(CH₂)₂-O- | | O | CH₃ | 116 | |
| I-c-38 | C₂H₅ | CH₃ | C₂H₅ | =NOtC₄H₉ | | O | C₂H₅ | 1.24 (s, 9H), 1.28 (mc, 3H), 2.62-2.90 (m, 4H), 4.15 (mc, 1H), 4.20 (mc, 2H) | |
| I-c-39 | C₂H₅ | CH₃ | C₂H₅ | =NOi-C₃H₇ | | O | C₂H₅ | 1.03 (mc, 6H), 1.15-1.30 (m, 9H), 2.35 (hept, 2H), 3.32 (mc, 1H), 4.20 (mc, 2H), 4.25 (mc, 1H) | |
| I-c-39 | C₂H₅ | CH₃ | C₂H₅ | =NOCyclopentyl | | O | C₂H₅ | 1.52-1.80 (m, 8H), 3.30 (mc, 1H), 4.12-4.22 (m, 3H), 4.62 (mc, 1H) | |
| I-c-40 | C₂H₅ | CH₃ | C₂H₅ | =NOCH₂Cyclopropyl | | O | C₂H₅ | 0.23 und 0.51 (je mc, je 2H), 3.19-3.49 (m, 1H), 3.82 (mc, 2H), 4.12-4.24 (m, 3H) | |
| I-c-41 | C₂H₅ | CH₃ | C₂H₅ | =NOCH₂C≡CH | | O | C₂H₅ | 1.25 (mc, 3H), 2.47 (mc, 1H), 4.15-4.25 (m, 3H), 4.61 (d, 2H) | |
| I-c-42 | C₂H₅ | CH₃ | C₂H₅ | O= | | O | CH₃ | 2.60-2.85 (m, 3H), 3.51 (mc, 1H), 3.79 (s, 3H), 4.30 (mc, 1H) | |
| I-c-43 | C₂H₅ | CH₃ | C₂H₅ | =NOCH(CH₃)-C≡CH | | O | C₂H₅ | 1.28 (t, 3H), 1.48 (mc, 3H), 2.43 (mc, 1H), 3.34 (mc, 1H), 4.80 (mc, 1H) | |
| I-c-44 | CH₃ | CH₃ | CH₃ | -O-(CH₂)₂-O- | | O | C₂H₅ | 1.21 (t, 3H), 2.05, 2.12 und 2.22 (je s, je 3H), 3.19 (mc, 1H), 3.89 (mc, 4H), 3.91 (mc, 1H), 4.12 (mc, 2H) | |
| I-c-45 | CH₃ | CH₃ | CH₃ | CH₂= | | O | C₂H₅ | 1.21 (t, 3H), 1.98, 2.06 und 2.24 (je s, je 3H), 4.15 (mc, 2H), 4.90 (mc, 2H) | |
| I-c-46 | CH₃ | CH₃ | CH₃ | CH₂= | | O | CH₃ | 1.99, 2.05 und 2.22 (je s, je 3H), 2.40-2.72 (m, 4H), 3.72 (s, 3H), 4.90 (mc, 2H) | |
| I-c-47 | CH₃ | CH₃ | CH₃ | -O-(CH₂)₃-O- | | O | C₂H₅ | 1.20 (t, 3H), 2.80 (mc, 1H), 3.16 (mc, 1H), 3.70-3.90 (m, 5H), 4.12 (mc, 2H) | |
| I-c-48 | CH₃ | CH₃ | CH₃ | -O-CH₂-CH(CH₃)-CH₂-O | | O | C₂H₅ | 0.71 und 0.82 (je d, Σ 3H), 2.45-2.50 (m, 1H), 3.28-3.45 (m, 2H), 4.12 (mc, 2H) | |
| I-c-49 | CH₃ | CH₃ | CH₃ | -O-(CH₂)₄-O- | | O | CH₃ | 122 | |
| I-c-50 | CH₃ | CH₃ | CH₃ | -O-(CH₂)₄-O- | | O | C₂H₅ | 131 | |
| I-c-51 | CH₃ | CH₃ | CH₃ | -O-CH₂-CH=CH-CH₂-O- | | O | C₂H₅ | 1.21 (t, 3H), 4.00-4.31 (m, 6H), 5.65 (mc, 2H), 6.85 (mc, 2H) | |
| I-c-52 | CH₃ | CH₃ | CH₃ | -O-CH₂-CH=CH-CH₂-O- | | O | CH₃ | 3.70 (s, 3H), 3.94 (mc, 1H), 3.96-4.30 (m, 4H), 5.64 (mc, 2H) | |
| I-c-53 | CH₃ | CH₃ | CH₃ | -O-CH₂-CH(CH₃)-CH₂-O- | | O | CH₃ | 0.70 und 0.82 (je t, Σ 3H), 2.48 (mc, 1H), 3.15 (mc, 1H), 3.29-3.45 (m, 2H), 3.71 (s, 3H) | |
| I-c-54 | CH₃ | CH₃ | CH₃ | -O-C(CH₃)₂-O-CH₂- | | O | C₂H₅ | 1.20 (t, 3H), 1.48 (s, 6H), 3.34 (mc, 1H), 3.85 und 3.89 (je d, je 1H), 4.12 (q, 2H) | syn-/anti-Gemisch |
| I-c-55 | CH₃ | CH₃ | CH₃ | -O-C(CH₃)₂-O-CH₂- | | O | i-C₃H₇ | 1.15 und 1.19 (je d, Σ 6H), 1.38 (s, 6H), 3.85 und 3.90 (je d, je 1H), 4.72 (hept, 1 H) | syn-/anti-Gemisch |
| I-c-56 | CH₃ | CH₃ | CH₃ | -O-C(CH₃)₂-O-CH₂- | | O | CH₃ | 1.38 (s, 6H), 3.36 (mc, 1H), 3.71 (s, 3H), 4.00 (mc, 1H) | syn-/anti-Gemisch |
| I-c-57 | CH₃ | CH₃ | CH₃ | =O | | O | CH₃ | 1.98, 2.10 und 2.26 (je s, je 3H), 3.50 (mc, 1H), 3.75 (s, 3H), 4.19 (mc, 1H) | |
| I-c-58 | CH₃ | CH₃ | CH₃ | =O | | O | C₂H₅ | 1.22 (t, 3H), 2.00, 2.11 und 2.26 (je s, je 3H), 2.42-2.85 (m, 4H), 4.10-4.22 (3H), | |
| I-c-59 | CH₃ | CH₃ | CH₃ | =NOCH₂C≡CH | | O | CH₃ | 2.45 (mc, 1H), 2.70-3.08 (m, 4H), 3.75 (s, 3H), 4.61 (mc, 2H), | |
| I-c-60 | CH₃ | CH₃ | CH₃ | =NOCH₂C≡CH | | O | C₂H₅ | 1.23 (mc, 3H), 2.44 (mc, 1H), 2.70-3.08 (m, 4H), 4.18 (mc, 2H), 4.61 (mc, 2H) | |
| I-c-61 | OCH₃ | CH₃ | CH₃ | CH₂= | | O | C₂H₅ | 2.30 (s, 3H), 2.39-2.70 (m, 4H), 3.70 (s, 3H), 4.18 (mc, 2H) | |
| I-c-62 | OCH₃ | CH₃ | CH₃ | CH₂= | | O | CH₃ | 2.38-2.70 (m, 4H), 3.70 und 3.75 (je s, je 1H), 4.89 (mc, 2H) | |
| I-c-62 | OCH₃ | CH₃ | CH₃ | O= | | O | C₂H₅ | 1.25 (mc, 3H), 2.30(s, 3H), 2.40-2.85 (m, 4H), 3.50 (mc, 1H), 4.10 (mc, 2H) | |
| I-c-63 | OCH₃ | CH₃ | CH₃ | -O-(CH₂)₃-O- | | O | C₂H₅ | 1.22 (t, 3H), 2.09 und 2.29 (je s, je 3H), 2.76-2.80 (m, 1H), 3.18 (mc, 1H), 3.70 (s, 3H) | |
| I-c-64 | OCH₃ | CH₃ | CH₃ | -O-(CH₂)₂-O- | | O | C₂H₅ | 21.12 und 2.30 (je s, je 3H), 3.70 (s, 3H), 3.85 (mc, 4H), 4.18 (q, 2H) | |
| I-c-65 | OCH₃ | CH₃ | CH₃ | -O-CH₂-CH=CH-CH₂-O- | | O | C₂H₅ | 1.25 (t, 3H), 3.70 (s, 3H), 3.86-430 (m, 7H), 5.64 (mc, 2H) | |
| I-c-66 | OCH₃ | CH₃ | CH₃ | -O-(CH₂)₄-O- | | O | C₂H₅ | 1.22 (t, 3H), 2.22 und 2.30 (je s, je 3H), 3.12 (mc, 1H), 3.70 (s, 3H), 4.16 (q, 2H) | |
| I-c-67 | OCH₃ | CH₃ | C₂H₅ | CH₂= | | O | C₂H₅ | 0.99 und 1.08 (je t, Σ 3H), 1.26 (mc, 3H), 3.61 und 3.68 (je s, Σ 3H), 4.89 (mc, 2H) | |
| I-c-68 | OCH₃ | CH₃ | C₂H₅ | O= | | O | C₂H₅ | 1.05 und 1.11 (je t, Σ 3H), 1.26 (mc, 3H), 2.25-2.83 (m, 4H), 2.30 (s, 3H), 4.20 (mc, 2H) | |
| I-c-69 | OCH₃ | CH₃ | C₂H₅ | -O-(CH₂)₂-O- | | O | C₂H₅ | 1.09 (t, 3H), 1.24 (t, 3H), 2.31 (s, 3H), 3.70 (s, 3H), 3.88 (mc, 4H), 4.19 (q, 2H) | |
| I-c-70 | OCH₃ | CH₃ | C₂H₅ | -O-(CH₂)₃-O- | | O | C₂H₅ | 1.10 und 1.15 (je t, je 3H), 3.70 (s, 3H), 3.70-4.00 (m, 4H), 4.18 (q, 2H) | |
| I-c-71 | C₂H₅ | CH₃ | CH₃ | -O-(CH₂)₂-O- | | O | C₂H₅ | 1.08 und 1.22 (je t, je 3H), 3.88 (mc, 4H), 3.96 (mc, 1H), 4.15 (mc, 2H) | |
| I-c-72 | C₂H₅ | CH₃ | CH₃ | -O-CH₂-CH=CH-CH₂-O- | | O | C₂H₅ | 124-125 | |
| I-c-73 | C₂H₅ | CH₃ | CH₃ | -O-CH₂-CH=CH-CH₂-O- | | O | CH₃ | 1.08 (t, 3H), 3.18 (mc, 1H), 3.71 (s, 3H), 3.95-4.31 (m, 5H), 5.65 (mc, 2H) | |
| I-c-74 | C₂H₅ | CH₃ | CH₃ | -O-CH₂-CH=CH-CH₂-O- | | O | i-C₃H₇ | 1.20 (mc, 6H), 3.91-4.32 (m, 5H), 4.79 (hept, 1H), 5.64 (mc, 2H) | |
| I-c-75 | C₂H₅ | CH₃ | CH₃ | -O-(CH₂)₄-O- | | O | C₂H₅ | 102 | |
| I-c-76 | C₂H₅ | CH₃ | CH₃ | CH₂= | | O | C₂H₅ | 0.96 und 1.08 (je t, Σ 3H), 1.22 (t, 3H), 2.26 (s, 3H), 4.15 (mc, 2H), 4.91 (mc, 1 H) | |
| I-c-77 | C₂H₅ | CH₃ | CH₃ | CH₂= | | O | CH₃ | 0.97 und 1.05 (je t, Σ 3H), 3.75 (s, 3H), 4.92 (mc, 2H) | |
| I-c-78 | C₂H₅ | CH₃ | CH₃ | -O-C(CH₃)₂-O-CH₂- | | O | i-C₃H₇ | 116-117 | syn-/anti-Gemisch |
| I-c-79 | C₂H₅ | CH₃ | CH₃ | -O-C(CH₃)₂-O-CH₂- | | O | CH₃ | 1.02-1.11 (m, 3H), 1.38 (s, 6H), 3.74 (s, 3H), 3.85 und 3.88 (je d, je 1H) | syn-/anti-Gemisch |
| I-c-80 | C₂H₅ | CH₃ | CH₃ | -O-C(CH₃)₂-O-CH₂- | | O | C₂H₅ | 1.20 (t, 3H), 1.38 (s, 6H), 2.30 (s, 3H), 3.86 und 4.00 (je d, je 1H), 4.15 (mc, 2H) | syn-/anti-Gemisch |
| I-c-81 | C₂H₅ | C₂H₅ | C₂H₅ | CH₂= | | O | C₂H₅ | 0.98 und 1.07 (je t, je 3H), 1.22-1.29 (m, 6H), 4.20 (mc, 2H), 4.92 (mc, 2H) | |
| I-c-82 | C₂H₅ | C₂H₅ | C₂H₅ | O= | | O | C₂H₅ | 103 | |
| I-c-83 | C₂H₅ | C₂H₅ | C₂H₅ | -O-(CH₂)₃-O- | | O | C₂H₅ | 122 | |
| I-c-84 | C₂H₅ | C₂H₅ | C₂H₅ | -O-(CH₂)₂-O- | | O | C₂H₅ | 98 | |
| I-c-85 | C₂H₅ | CH₃ | H | -O-(CH₂)₃-O- | | O | C₂H₅ | | |
| I-c-86 | C₂H₅ | CH₃ | H | -O-(CH₂)₃-O- | | O | CH₃ | | |
| I-c-87 | C₂H₅ | CH₃ | H | -O-(CH₂)₄-O- | | O | C₂H₅ | | |
| I-c-88 | C₂H₅ | CH₃ | H | -O-(CH₂)₂-O- | | O | C₂H₅ | | |
| I-c-89 | C₂H₅ | CH₃ | C₂H₅ | -O-CH(CH₃)-CH₂-CH₂-O- | | O | C₂H₅ | 1.05-1.28 (m, 12H), 2.32 (s, 3H), 3.70-4.05 (m, 4H), 4.18 (mc, 2H) | syn-/anti-Gemisch |

### Beispiel Id-1

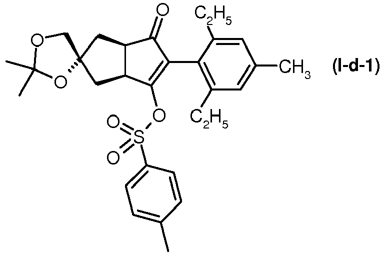

Zu 100 mg (0.27 mmol) der Verbindung (I-a-1) in 5 ml Dichlormethan werden bei Raumtemperatur 57 mg (0.29 mmol) p-Toluolsulfonsäurechlorid und 0.1 ml Triethylamin gegeben. Nach 1h Rühren wird mit Wasser verdünnt, abgetrennt und zweimal mit 1 N Salzsäure gewaschen. Trocknen mit Magnesiumsulfat und Abdestillieren des Lösungsmittels liefert 120 mg (87%) der gewünschten Substanz als farbloses Öl.
¹H-NMR (400 MHz, CDCl₃): δ = 0.98 und 1.03 (je t, je 3H), 2.30 und 2.39 (je s, je 1H), 3.15 und 3.90 (je mc, je 1H), 3.98-4.35 (m, 4H), 5.65 (s, 2H) ppm.

In Analogie zu Beispiel (I-d-1) und gemäß den allgemeinen Angaben zur Herstellung erhält man folgende Verbindungen der Formel (I-d):

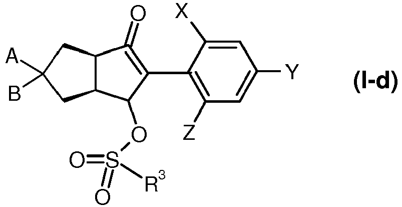

| **Bsp.-Nr.** | **X** | **Y** | **Z** | **A** | **B** | **R³** | **Fp. [°C] oder ¹H-NMR (400 MHz, d₆**-**DMSO, δ in ppm)** | **Isomerie** |
|---|---|---|---|---|---|---|---|---|
| **I-d-2** | C₂H₅ | CH₃ | C₂H₅ | -O-C(CH₃)₂-O-CH₂- | | C₆H₅ | 1.40 (s, 6H), 3.85 und 3.88 (je d, je 1H), 7.39 (mc, 2H), 7.61 (mc, 3H) | anti |
| **I-d-3** | C₂H₅ | CH₃ | C₂H₅ | -O-C(CH₃)₂-O-CH₂- | | CH₃ | 1.12 (t, 3H), 1.40 (s, 6H), 2.78 (s, 3H), 3.41 (mc, 1H), 3.88 und 3.91 (je d, je 1H), 3.93 (mc, 1H) | anti |
| **I-d-4** | CH₃ | CH₃ | CH₃ | -O-C(CH₃)₂-O-CH₂- | | CH₃ | 54-55 | syn-/anti-Gemisch |
| **I-d-5** | CH₃ | CH₃ | CH₃ | -O-C(CH₃)₂-O-CH₂- | | C₆H₅ | 140 | syn-/anti-Gemisch |
| **I-d-6** | CH₃ | CH₃ | CH₃ | -O-C(CH₃)₂-O-CH₂- | | pCH₃C₆H₅ | 144-145 | syn-/anti-Gemisch |
| **I-d-7** | C₂H₅ | CH₃ | C₂H₅ | -O-CH₂-CH=CH-CH₂-O- | | CH₃ | 133-134 | |
| **I-d-8** | C₂H₅ | CH₃ | C₂H₅ | -O-CH₂-CH=CH-CH₂-O- | | C₆H₅ | 129-130 | |
| **I-d-9** | C₂H₅ | CH₃ | C₂H₅ | -O-CH₂-CH=CH-CH₂-O- | | pCH₃C₆H₅ | 121-122 | |
| **I-d-10** | C₂H₅ | CH₃ | C₂H₅ | -O-(CH₂)₂-O- | | CH₃ | 146-147 | |
| **I-d-11** | C₂H₅ | CH₃ | C₂H₅ | -O-(CH₂)₂-O- | | C₆H₅ | 145-146 | |
| **I-d-12** | C₂H₅ | CH₃ | C₂H₅ | -O-(CH₂)₂-O- | | pCH₃C₆H₅ | 80 | |
| **I-d-13** | C₂H₅ | CH₃ | C₂H₅ | =NOCH₂C≡CH | | CH₃ | 143-144 | |
| **I-d-14** | C₂H₅ | CH₃ | C₂H₅ | =NOCH₂C≡CH | | C₆H₅ | | |
| **I-d-15** | C₂H₅ | CH₃ | C₂H₅ | =NOCH₂C≡CH | | pCH₃C₆H₅ | 2.30 und 2.42 (je s, je 3H), 2.45 (t, 1H), 3.99-4.08 (m, 1H), 4.60 (mc, 2H), 7.21 (d, 2H), 7.54 (d, 2H) | |

### Beispiel I-f-1

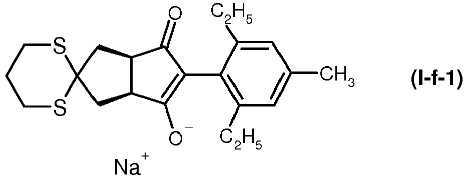

0.094 g (0.242 mmol) der Verbindung (I-a-17) in 3 ml absolutem Methanol werden mit 0.040 g (0.242 mmol) Natriummethanolat versetzt und noch 30 min bei Raumtemp. nachgerührt.

Abdestillieren des Lösungsmittel liefert 0.096 g (96%) der erfindungsgemäßen Verbindung der Formel (I-f-1) in Form farbloser Kristalle mit Schmelzpunkt > 300 °C.
¹H-NMR (400 MHz, d₆-DMSO): δ = 0.92 und 0.95 (je t, je 3 H), 2.22 (s, 3 H), 6.68 (mc, 2 H) ppm

In Analogie zu Beispiel (I-f-1) und gemäß den allgemeinen Angaben zur Herstellung erhält man folgende Verbindungen der Formel (I-f):

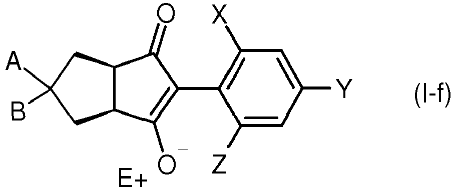

| **Bsp.-Nr.** | **X** | **Y** | **Z** | **A** | **B** | **E** | **Fp. [°C] oder ¹H-NMR (400 MHz, d₆-DMSO, δ in ppm)** | **Isomerie** |
|---|---|---|---|---|---|---|---|---|
| I-f-2 | C₂H₅ | CH₃ | C₂H₅ | -O-CH(CH₃)-CH₂- CH(CH₃)-O- | | Na⁺ | δ = 1.12 (d, 6H), 3.91 (mc, 2H), 6.68 (s, 2H) | (R,R)-Konfiguration der CH₃-Gruppen am Acetalring |
| I-f-3 | CH₃ | CH₃ | CH₃ | -O-CH(CH₃)-CH₂- CH(CH₃)-O- | | Na⁺ | 1.22 (s, 6H), 1.95, 2.00 und 2.12 (je s, je 3H) | (R,R)-Konfiguration der CH₃-Gruppen am Acetalring |
| I-f-4 | OCH₃ | CH₃ | CH₃ | -O-(CH₂)₃-O- | | Na⁺ | 6.42 und 4.46 (je s, je 1H) | |
| I-f-5 | OCH₃ | CH₃ | CH₃ | -O-(CH₂)₄-O- | | Na⁺ | 6.42 (s, 1H), 6.48 (s, 1H) | |
| I-f-6 | OCH₃ | CH₃ | CH₃ | -O-(CH₂)₂-O- | | Na⁺ | 3.52 (s, 1H), 6.42 (s, 1H), 6.45 (s, 1H) | |
| I-f-7 | OCH₃ | CH₃ | CH₃ | -O-CH₂-CH=CH-CH₂-O- | | Na⁺ | 6.43 und 6.47 (je s, je 1H) | |
| I-f-8 | CH₃ | CH₃ | CH₃ | -O-CH₂-CH=CH-CH₂-O- | | Na⁺ | 5.61 (s, 2H), 6.62 (s, 2H) | |
| I-f-9 | CH₃ | CH₃ | CH₃ | -O-CH₂-CH(CH₃)-CH₂-O- | | Na⁺ | 0.70 (d, 3H), 3.61 und 3.72 (je mc, je 2H, 6.62 (s, 2H) | |
| I-f-10 | CH₃ | CH₃ | CH₃ | -O-(CH₂)₄-O- | | Na⁺ | 3.48 und 3.55 (je mc, je 2H), 6.62 (s, 2H) | |
| I-f-11 | CH₃ | CH₃ | CH₃ | -O-(CH₂)₂-O- | | Na⁺ | 2.00 (s, 6H), 2.15 (s, 3H), 3.75 (mc, 4H), 6.62 (s, 2H) | |
| I-f-12 | C₂H₅ | CH₃ | C₂H₅ | -O-CH₂-CH=CH-CH₂-O- | | Na⁺ | 0.93 (t, 6H), 2.33 (q, 4H), 5.62 (s, 2H), 6.68 (s, 2H) | |
| I-f-13 | C₂H₅ | CH₃ | C₂H₅ | -O-CH₂-CH(OCH₃)-CH₂-O- | | Na⁺ | 0.91 (t, 6H), 3.25 (s, 3H), 3.55 und 3.62 (je mc, je 1H), 6.66 (s, 2H) | |
| I-f-14 | C₂H₅ | CH₃ | C₂H₅ | -O-CH₂-CH(OC₂H₅)-CH₂-O- | | Na⁺ | 168-169 | |
| I-f-15 | C₂H₅ | CH₃ | C₂H₅ | -O-(CH₂)₂-O- | | Na⁺ | 0.95 (mc, 6H), 1.70 und 1.88 (je mc, je 2H), 3.78 (mc, 4H), 6.68 (mc, 2H) | |
| I-f-16 | C₂H₅ | CH₃ | C₂H₅ | -O-CH₂-CH(OCH₂C₆H₅) -CH₂-O- | | Na⁺ | 4.52 (s, 2H), 6.55 (s, 2H, 7.25-7.37 (m, 5H) | |
| I-f-17 | C₂H₅ | CH₃ | C₂H₅ | -O-(CH₂)₄-O- | | Na⁺ | 288-289 | |
| I-f-18 | C₂H₅ | CH₃ | C₂H₅ | -O-CH(CH₃)-CH₂-CH(CH₃)-O- | | Na⁺ | 1.12 (mc, 6H), 1.50 (mc, 2H), 1.61 (mc, 1H), 3.91 (mc, 2H), 6.68 (s, 2H) | (R,R)-Konfiguration der CH₃-Gruppen am Acetalring |
| I-f-19 | C₂H₅ | CH₃ | C₂H₅ | -O-CH(CH₃)-CH₂-CH(CH₃)-O- | | Na⁺ | 1.03 und 1.12 (je d, ∑ 6H), 1.50-1.71 (m, 3H), 3.79 und 3.91 (je mc, ∑ 2H) | Isomerengemisch bezüglich der CH₃-Gruppen am Acetalring |
| I-f-20 | C₂H₅ | CH₃ | C₂H₅ | 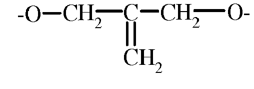 | | Na+ | 0.93 (t, 6H), 2.32 (q, 4H), 4.15, 4.22, 4.82 und 6.68 (je s, je 2H) | |
| I-f-21 | C₂H₅ | CH₃ | C₂H₅ | CH₂= | | Na+ | 117-118 | |
| I-f-22 | OCH₃ | CH₃ | CH₃ | CH₂= | | Na+ | 4.64 (s, 3H), 6.41 und 6.47 (je d, je 2H) | |
| I-f-23 | CH₃ | CH₃ | CH₃ | CH₂= | | Na+ | 1.90, 2.01 und 2.15 (je s, je 3H), 4.65 (s, 2H), 6.61 (mc, 2H) | |
| I-f-24 | OCH₃ | CH₃ | CH₃ | O= | | Na+ | 6.45 (mc, 2H) | |
| I-f-25 | C₂H₅ | CH₃ | C₂H₅ | -O-(CH₂)₃-O- | | Na+ | 6.62 (s, 2H) | |

### Herstellung von Ausgangsmaterialien

### 2-[(2,6-Diethyl-4-methylphenyl)acetyl]-4-methylidencyclohexancarbonsäure -

### Beispiel (XIII-1)

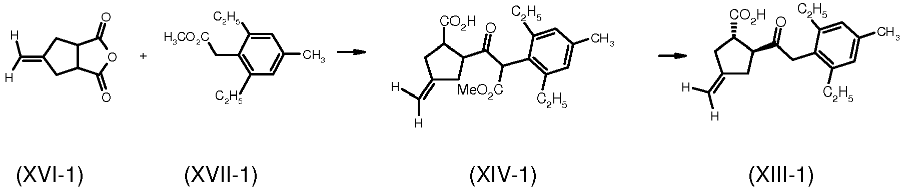

Zu einer Lösung von Lithiumdiisopropylamid in 250 ml THF, hergestellt aus 16,3 g (164 mmol) Diisopropylamin und der äquimolaren Menge einer Lösung von n-Butyllithium in Hexan, werden bei -30°C langsam 14.48 g (65.7 mmol) 2-Ethyl-4,6-dimethylphenylessigsäuremethylester (XVII-1) getropft und 45 min. bei Raumtemperatur nachgerührt. Danach werden 10.00 g (65.7 mmol) 4-Methylencyclopentan-1,2-dicarbonsäureanhydrid (XVI-1), gelöst in 20 ml THF, bei -20°C zugegeben und noch 12 h bei Raumtemperatur nachgerührt. Zur Aufarbeitung werden 100 ml ges. Ammoniumchloridlösung zugegeben, mit Essigsäurethylester überschichtet, mit Wasser gewaschen, getrocknet (Magnesiumsulfat) und einrotiert. Man erhält so 22,3 g der Verbindung (XIV-1) als rötliches Öl, das ohne weitere Reinigung umgesetzt werden kann.
¹H-NMR (400 MHz, CDCl₃): δ =1.11 und 1.12, je t, je 3H), 2.25-2.50 (m, 4 H), 2.35 (s, 3 H), 2.69 (mc, 2H), 2.72-2.90 (m, 4H), 3.66 (s, 3H), 4.85 (s, 2H) ppm

22.3 g der Zwischenstufe (XIV-1) werden mit 10 g Kaliumhydroxid in 100 ml Wasser versetzt und 24 h am Rückfluss erhitzt. Danach läßt man auf Raumtemperatur abkühlen, säuert mit 2N Salzsäure auf pH 2 an, rührt 1 h bei Raumtemp. nach und saugt den ausgefallenen Feststoff ab. Man erhält so 10.3 g (45 %) 2-[(2,6-Diethyl-4-methylphenyl)acetyl]-4-methylidencyclohexancarbonsäure (XIII-1) als gelblichen Feststoff, der ohne weitere Reinigung im folgenden Reaktionsschritt eingesetzt werden kann.
¹H-NMR (400 MHz, CDCl₃): δ = 1.12 (t, 6H), 2.45 (q, 4H), 3.90 (s, 2H), 4.91 (mc, 2H), 6.89 (s, 2H) ppm

Analog wurden folgende Zwischenstufen der allgemeinen Formel (XIII) hergestellt:

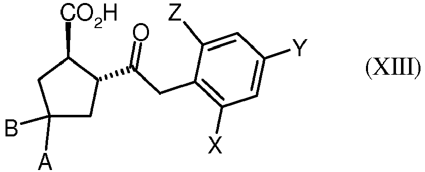

| **Bsp.-Nr.** | **X** | **Y** | **Z** | **A** | **B** | **Fp. [°C] oder ¹H-NMR (400 MHz, CDCl₃, δ in ppm)** |
|---|---|---|---|---|---|---|
| XIII-2 | OCH₃ | CH₃ | C₂H₅ | CH₂= | | 3.28-3.40 (m, 2H), 3.78 (mc, 2H), 3.72 und 3.80 (s, insges. 3H), 4.88 (mc, 2H) |
| XIII-3 | CH₃ | CH₃ | CH₃ | CH₂= | | 2.15 (s, 6H), 2.25 (s, 3H), 3.83 (mc, 2H), 4.90 (mc, 2H) |
| XIII-4 | C₂H₅ | CH₃ | CH₃ | CH₂= | | 1.20 (t, 3H), 2.12 (s, 3H), 2.22 (s, 6H), 3.70 (s, 2H), 4.90 (mc, 2H) |
| XIII-5 | OCH₃ | CH₃ | C₂H₅ | CH₂= | | 1.17 (mc, 3H), 3.28-3.40 (m, 2H), 3.72 und 3.79 (je s, he 3H), 4.89 (mc, 2H) |
| XIII-6 | C₂H₅ | CH₃ | H | CH₂= | | 96-100 |

Analog wurden folgende Zwischenstufen der allgemeinen Formel (XIV) hergestellt:

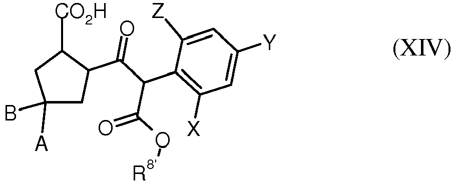

| **Bsp.-Nr.** | **X** | **Y** | **Z** | **A B** | **R⁸** | **Fp. [°C] oder ¹H-NMR (400 MHz, CDCl₃, δ in ppm)** |
|---|---|---|---|---|---|---|
| XIV-2 | CH₃ | CH₃ | CH₃ | CH₂= | C₂H₅ | 118-119 |
| XIV-3 | C₂H₅ | CH₃ | CH₃ | CH₂= | CH₃ | 167-168 |
| XIV-4 | OCH₃ | CH₃ | CH₃ | CH₂= | CH₃ | 2.20 und 2.31 (je s, je 3H), 3.13 und 3.58 (je s, je 3H), 4.85 (mc, 2H) |
| XIV-5 | OCH₃ | CH₃ | C₂H₅ | CH₂= | CH₃ | 1.08-1.18 (m, 3H), 2.69-3.00 (m, 4H), 4.88 (mc, 2H), 13.20 (s, br, 1H) |
| XIV-6 | C₂H₅ | CH₃ | H | CH₂= | CH₃ | 106-107 |

### 2-[(2,6-Diethyl-4-methylphenyl)acetyl]-4-methylidencyclopentancarbonsäure-methylester Beispiel (II-1)

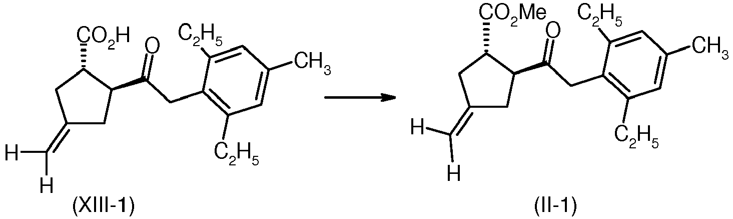

5,45g (17.33 mmol) 2-[(2,6-Diethyl-4-methylphenyl)acetyl]-4-methylidencyclohexancarbonsäure (XIII-1) werden zusammen mit 2.38 g Kaliumcarbonat und 2.62 g (20.8 mmol) Dimethylsulfat 5 h in 50 ml Aceton am Rückfluss gekocht und nach Abkühlen der Reaktionsmischung in Essigsäureethylester aufgenommen, mit Wasser geschüttelt, getrocknet (Magnesiumsulfat) und das Lösemittel abdestilliert. Nach Chromatographie an Kieselgel mit Essigsäureethylester/Hexan (v/v=30:70) erhält man 3.93 g (69 %) 2-[(2,6-Diethyl-4-methylphenyl)acetyl]-4-methylidencyclohexancarbonsäuremethylester der Formel (II-1).
¹H-NMR (400 MHz, CDCl₃): δ = 3.58 (s, 3 H), 3.89 (s, 2H), 4.88 (s, 2H), 6.89 (s, 2H) ppm

### 2-[(2-Ethyl-4,6-dimethylphenyl)acetyl]-4-oxocyclopentancarbonsäure-ethylester

### Beispiel (II-2)

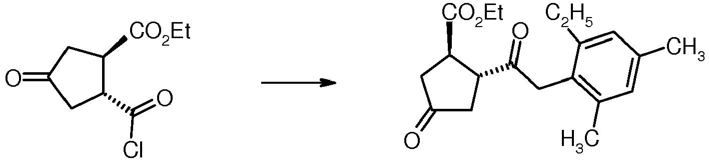

5.62 g (25.7 mmol) 2-Ethyl-4,6-dimethylbenzylchlorid in 30 ml THF werden zu einer Suspension von 3.85 g (28.3 mmol) trockenen Zinkchlorid, 1.56 g (64.2 mmol) Magnesiumspänen und 1.36 g (42.4 mmol) Lithiumchlorid so langsam zugetropft, daß die Innentemperatur 35 °C nicht überschreitet. Nach beendeter Zugabe wird noch 2 h bei Raumtemp. weitergerührt. Die entstandene Lösung wird bei Raumtemp. langsam zu einer Mischung von 5.62 g (25.7 mmol) Ethyl-2-(chlorcarbonyl)-4-oxocyclopentancarboxylat und 85 mg Bis(triphenylphosphin)-palladium(II)chlorid in 40 ml THF getropft und dann weitere 3 h bei Raumtemp. gerührt.

Man gibt den Ansatz auf Wasser, extrahiert zweimal mit je 50 ml Methyl-tert.-Butylether, trocknet (Magnesiumsulfat) und destilliert das Lösungsmittel ab. Chromatographie an Kieselgel mit Essigsäureethylester/Hexan (v/v=30:70) ergibt 4.89 g (57%) der gewünschten Zwischenstufe in Form farbloser Kristalle mit Fp. 75 °C.
¹H-NMR (400 MHz, CDCl₃): δ = 1.14 und 1.27 (je t, je 3H), 3.42-3.58 (m, 2H), 3.90 (s, 2H), 4.19 (q, 2H) ppm

### 2-[(2,4,6-trimethylphenyl)acetyl]-4-oxocyclopentancarbonsäureethylester

### Beispiel (II-3)

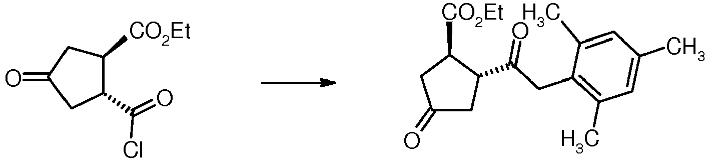

4.08 g (17.4 mmol) 2,4,6-Trimethylbenzylchlorid in 40 ml THF werden innerhalb von 2 h zu 1.70 g (26.1 mmol) mit Salzsäure aktiviertem und im Vakuum getrocknetem Zinkstaub und 0.50 g Trimethylsilylchlorid in 30 ml THF getropft und noch 2 h bei Raumtemp. nachgerührt.

1.56 g (17.4 mmol) Kupfer(I)cyanid und 1.65 g (38.8 mmol) Lithiumchlorid werden im Vakkum 8 h auf 150 °C erhitzt, nach Abkühlen mit Argon belüftet und in 25 ml THF aufgenommen. Dazu wird bei -25 °C innerhalb von 20 min. die oben hergestellte Lösung von 2,4,6-Trimethylbenzylzinkchlorid getropft und noch weitere 20 min. bei dieser Temperatur nachgerührt.

Man kühlt die Mischung auf -40 °C und gibt innerhalb von 30 min. eine Lösung von 3.77 g (17.3 mmol) Ethyl-2-(chlorcarbonyl)-4-oxocyclopentancarboxylat in 20 ml THF zu, läßt auf Raumtemp. kommen und rührt noch weitere 3 h.

Zur Aufarbeitung werden 100 ml Wasser zugegeben, mit Essigsäureethylester extrahiert, getrocknet (Magnesiumsulfat) und einrotiert. Chromatographie an Kieselgel (Essigsäureethylester/ Hexan v/v=30:70) ergibt 1.98 g (27%) des gewünschten Produktes.
¹H-NMR (400 MHz, CDCl₃): δ = 2.18 (s, 6H), 2.23 (s, 3H), 3.48 (mc, 2H), 3.82 und 3.88 (je d, je 1H), 4.18 (q, 2H) ppm

### Methyl-2-[(2,6-diethyl-4-methylphenyl)acetyl]-4-hydroxy-4-(hydroxymethyl)cyclopentancarboxylat Beispiel (II-4)

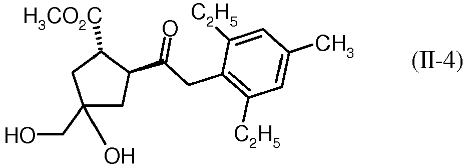

Zu 1.37 ml Osmiumtetroxidlösung (2.5 %ige Lösung in n-Butanol) und 1.02 g (8.7 mmol) N-Methylmorpholin-N-oxid in 25 ml Aceton/Wasser (v/v=5:1) werden 2.39 g (7.27 mmol) 2-[(2,6-Diethyl-4-methylphenyl)acetyl]-4-methylidencyclohexancarbonsäuremethylester gemäß Beispiel (II-2) gegeben und 14 h bei Raumtemp. gerührt. Danach werden 0.745 g (3.64 mmol) Natriumdithionit zugegeben, 10 min bei Raumtemperatur gerührt und anschließend das Lösungsmittel im Vakuum entfernt. Nach Aufnehmen des Rückstands in Essigsäureethylester, Extraktion mit Wasser, Trocknen (Magnesiumsulfat) wird einrotiert. Man erhält 2.45 g (92 %) gelbliches, viskoses Öl, das ohne weitere Reinigung weiter eingesetzt werden kann.
¹H-NMR (400 MHz, CDCl₃): δ = 1.70-2.25 (m, 4 H), 3.34 (mc, 1 H), 3.89 (dd, 2 H) ppm

### Methyl-8-[(2,6-diethyl-4-methylphenyl)acetyl]-2,2-dimethyl-1,3-dioxaspiro[4.4]nonan-7-carboxylat (Beispiel II-5)

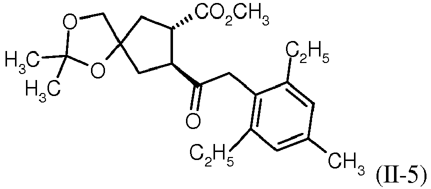

1.16 g (3.19 mmol) Methyl-2-[(2,6-diethyl-4-methylphenyl)acetyl]-4-hydroxy-4-(hydroxymethyl)cyclopentancarboxylat, 10 ml 2,2-Dimethoxypropan und 30 mg p-Toluolsulfonsäure werden 1 h am Rückfluß erhitzt. Nach Abkühlen wird die Mischung auf Eiswasser gegeben, in Essigsäureethylester aufgenommen, nacheinander mit 1 N Natriumhydrogencarbonatlösung und Wasser gewaschen, getrocknet (Magnesiumsulfat) und das Lösemittel im Vakuum abdestilliert. Chromatographie an Kieselgel (Laufmittel Essigsäureethylester/Hexan v/v=35:65) ergibt 0.92 g (71%) farbloses Öl. Lt. ¹H-NMR liegt ein endo-/exo-Isomerengemisch der Verbindung mit der Formel (II-5) vor.
¹H-NMR (400 MHz, CDCl₃): δ = 1.33-1.37 (Singuletts aufgesplittet, insges. 6 H), 2.26 (s, 3 H), 3.22 (mc, 1 H), 3.80-3.90 (m, 4 H), 6.89 (s, 2 H) ppm

### Ethyl-8-[(2-ethyl-4,6-dimethylphenyl)acetyl]-1,4-dioxaspiro[4.4]nonan-7-carboxylat (Beispiel II-16)

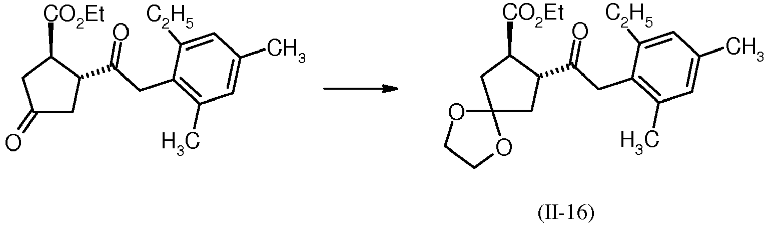

1.20 g (3.63 mmol) 2-[(2-Ethyl-4,6-dimethylphenyl)acetyl]-4-oxocyclopentancarbonsäureethylester gemäß Beispiel (II-2), 338 mg (5.4 mmol) Ethandiol, 578 mg (5.4 mmol) Orthoameisensäuretrimethylester und 20 mg p-Toluolsulfonsäure werden in 30 ml Toluol 24 h bei Raumtemp. gerührt. Das Lösemittel wird anschließend abdestilliert und der Rückstand an Kieselgel (Laufmittel Essigsäureethylester/Hexan v/v=15:85) chromatographiert. Man erhält 819 mg (60%) der gewünschten Verbindung in Form farbloser Kristalle mit Fp. 72-73 °C.

Analog wurden folgende Zwischenstufen der allgemeinen Formel (II) hergestellt

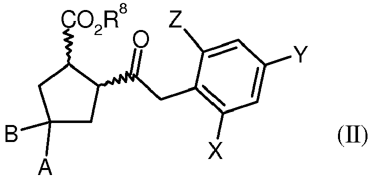

| **Bsp. No** | **X** | **Y** | **Z** | **A** | **B** | **R⁸** | **¹H-NMR (400 MHz, CDCl₃, δ in ppm)** | **Isomerie** |
|---|---|---|---|---|---|---|---|---|
| II-6 | OCH₃ | CH₃ | C₂H₅ | CH₂= | | CH₃ | 3.38 und 3.59 (je q, je 1H), 3.67 (s, 3H), 3.71 (mc, 2H), 3.75 (s, 3H), 4.86 (mc, 2H) | trans |
| II-7 | CH₃ | CH₃ | CH₃ | CH₂= | | CH₃ | 2.15 (s, 6H), 2.27 (s, 3H), 3.40 (mc, 2H), 3.74 (mc, 2H), 4.90 (mc, 2H) | trans |
| II-8 | CH₃ | CH₃ | C₂H₅ | CH₂= | | CH₃ | 1.15 (t, 3H), 3.28-3.40 (m, 2H), 3.67 (s, 2H), 3.85 (s, 3H), 4.88 (mc, 2H) | trans |
| II-9 | CH₃ | CH₃ | CH₃ | CH₂= | | CH₃ | 3.10 und 3.40 (je mc, je 1H), 3.62 (s, 3H), 3.78 und 3.90 (je q, je 1H) | cis |
| II-10 | C₂H₅ | CH₃ | C₂H₅ | CH₂= | | t-C₄H₉ | 1.42 (s, 9H), 3.16 und 3.38 (je q, je 1H), 3.88 (s, 2H), 4.86 (mc, 1H) | cis |
| II-11 | C₂H₅ | CH₃ | C₂H₅ | -OCH₂CH=CHCH₂O- | | C₂H₅ | 2.29 (s, 3H), 2.45 (q, 4H), 3.88 (s, 2H), 4.14-4.25 (m, 6H), 5.68 (mc, 2H) | trans |
| II-12 | C₂H₅ | CH₃ | C₂H₅ | -O(CH₂)₂O- | | C₂H₅ | 1.12 (mc, 6H), 1.24 (t, 3H), 3.39 und 3.49 (jemc, je 1H), 3.90 (mc, 4H), 4.14 (q, 2H) | trans |
| II-13 | C₂H₅ | CH₃ | CH₃ | -O(CH₂)₄O- | | C₂H₅ | 1.12 und 1.23 (je t, je 3H), 2.49 (q, 2H), 3.61 (mc, 4H), 3.82 (s, 2H), 4.12 (q, 2H) | trans |
| II-14 | C₂H₅ | CH₃ | CH₃ | -OCH₂CH=CHCH₂O- | | C₂H₅ | 3.35-3.50 (m, 2H), 3.85 (s, 2H), 4.10-4.25 (m, 6H), 5.68 (s, 2H) | trans |
| II-15 | C₂H₅ | CH₃ | CH₃ | -O(CH₂)₃O- | | C₂H₅ | 2.24 und 2.28 (je s, je 3H), 3.35-3.48 (m, 2H), 3.80-3.95 (m, 6H) | trans |
| II-16 | C₂H₅ | CH₃ | CH₃ | -O(CH₂)₂O- | | C₂H₅ | 72-73 | trans |
| II-17 | C₂H₅ | CH₃ | C₂H₅ | -O(CH₂)₃O- | | C₂H₅ | 1.24 (t, 3H), 3.31-3.40 (m, 2H), 3.80-3.95 (m, 6H), 4.15 (q, 2H) | trans |
| II-18 | C₂H₅ | CH₃ | C₂H₅ | O= | | C₂H₅ | 2.28 (s, 3H), 3.33-3.46 (m, 3H), 3.87 und 3.95 (je d, je 1H), 6.90 (s, 2H) | cis |
| II-19 | C₂H₅ | CH₃ | C₂H₅ | CH₂= | | C₂H₅ | 3.35-3.51 (m, 2H), 3.89 (s, 2H), 4.20-4.48 (m, 4H), 4.90 (s, 2H) | trans |
| II-20 | C₂H₅ | CH₃ | C₂H₅ | -O-CH(CH₃)-CH₂-CH(CH₃)-O- Gemisch bzgl. der CH₃-Gruppen am Acetal | | C₂H₅ | 1.10-1.28 (m, 15H), 3.35-3.58 (m, 2H), 3.70-4.00 (m, 4H), 4.12 (mc, 2H) | trans |
| II-21 | C₂H₅ | CH₃ | C₂H₅ | -S(CH₂)₃O- | | C₂H₅ | 3.40-3.63 (m, 2H), 3.82 (mc, 2H), 3.88 (mc, 2H), 4.14 (mc, 2H)6.89 (s, 2H) | trans |
| II-22 | C₂H₅ | CH₃ | C₂H₅ | -OCH₂CH(CH₃)CH₂O- | | C₂H₅ | 0.76 und 0.82 (je d, insges. 3 H), 2.00 (mc, 1H), 3.28-3.50 (m, 3H), 3.75-3.90 (m, 2H) | trans |
| II-23 | C₂H₅ | CH₃ | C₂H₅ | -S(CH₂)O- | | C₂H₅ | 3.05 (mc, 2H), 3.87 und 3.91 (je d, je 2H), 4.05 (mc, 2H), 4.12 (q, 2H) | trans |
| II-24 | C₂H₅ | CH₃ | C₂H₅ | 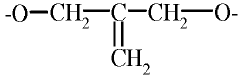 | | C₂H₅ | 3.38-3.50 (m, 2H), 3.89 (s, 2H), 4.20-4.39 (m, 4H), 4.90 (s, 2H) | trans |
| II-25 | OCH₃ | CH₃ | C₂H₅ | CH₂= | | CH₃ | 1.14 (t, 3H), 2.31 (s, 3H), 3.68 (s, 3H), 3.72 (s, 3H, 4.85 (mc, 2H), 6.52 und 6.65 (je s, je 1H) | trans |
| II-26 | C₂H₅ | CH₃ | H | CH₂= | | CH₃ | 1.14 (t, 3H), 2.31 (s, 3H), 3.68 (s, 3H), 3.72 (s, 3H, 4.85 (mc, 2H), 6.52 und 6.65 (je s, je 1H) | trans |

### 2,6 Diethyl-4-methylbenzylchlorid Beispiel (XIX-1)

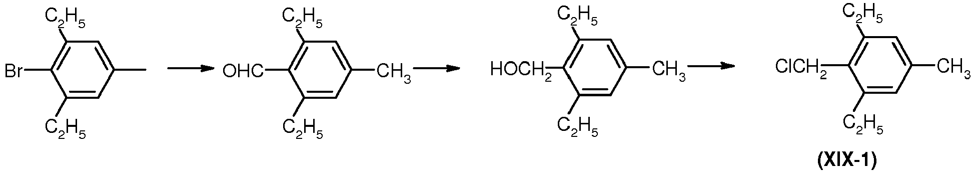

Zu einer Lösung von 2,6-Diethyl-4-methylphenylmagnesiumbromid, hergestellt aus 6.00 g Magnesiumspänen und 50 g 1- Brom-2,6 diethyl-4-methylbenzol in 220 ml THF werden unter Eiskühlung langsam 48.0 g (3 Äquiv.) N,N-Dimethylformamid, gelöst in 50 ml THF, getropft und noch weitere 3 h bei Raumtemp. weitergerührt. Man gießt auf ges. Ammoniumchloridlösung, extrahiert mit Essigsäureethylester, trocknet (Magnesiumsulfat) und erhält nach Abdestillieren des Lösemittel 38.70 g (99%) 2,6-Diethyl-4-methylbenzaldehyd als farbloses Öl, ¹H-NMR (400 MHz, CDCl₃): δ = 1.22 (t, 6H), 2.32 (s, 3H), 2.93 (q, 4H), 6.91 (s, 2H), 10.52 (s, 1H)

Analog wurden folgende Verbindungen hergestellt:
2-Ethyl-4,6-dimethylbenzaldehyd:
   ¹H-NMR (400 MHz, CDCl₃): 1.22 (t, 3H), 2.32 (s, 3H), 2.57 (s, 3H), 2.95 (q, 2H), 6.92 (mc, 2H), 10.55 (s, 1H) ppm
2-Methoxy-4,6-dimethylbenzaldehyd
2-Methoxy-6-Ethyl-4-methylbenzaldehyd
2-Cyclopropyl-6-Ethyl-4-methylbenzaldehyd
2,6-Cyclopropyl-4-methylbenzaldehyd

Zu einer Suspension von 2.10 g (55.4 mmol) Lithiumaluminiumhydrid in 200 ml Diethylether wird eine Lösung von 35.5 g (201.4 mmol) 2,6-Diethyl-4-methylbenzaldehyd, gelöst in 80 ml Diethylether, getropft. Danach wird noch 1 h am Rückfluß erhitzt. Nach Abkühlen wird mit Wasser und 10%iger Schwefelsäure hydrolysiert, abgetrennt und die wässrige Phase noch zweimal mit Diethylether extrahiert. Trocknen (Magnesiumsulfat), Abdestillieren des Lösungsmittels und Chromatographie des erhaltenen Rohprodukts an Kieselgel (Essigsäureethylester/Hexan = 30:70) ergibt 33.10 g (92%) 2,6 Diethyl-4-methylbenzylalkohol in Form farbloser Kristalle mit Fp. 71-72 °C. ¹H-NMR (400 MHz, CDCl₃): δ = 1.22 (t, 6H), 2.30 (s, 3H), 2.74 (q, 4H), 4.71 (s, 2H), 6.90 (s, 2H).

Direkte Herstellung von 2,6 Diethyl-4-methylbenzylalkohol aus 1-Brom-2,6-diethyl-4-methylbenzol

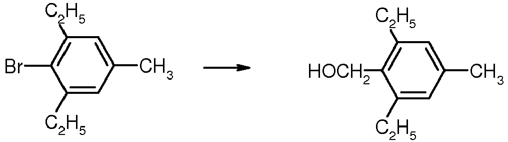

In eine Lösung von 2,6 Diethyl-4-methylphenylmagnesiumbromid, hergestellt aus 12.40 g (54.6 mmol) 1-Brom-2,6-diethyl-4-methylbenzol und 1.47 g (60.65 mmol) Magnesiumspänen in 50 ml THF wird unter Rühren über einen Zeitraum von 30 min. gasförmiges Formaldehyd (generiert aus 5.56 g Paraformaldehyd) eingeleitet und anschließend noch 2 h bei Raumtemp. nachgerührt. Abdestillieren des Lösungsmittels und Chromatographie an Kieselgel (s. oben) ergibt 6.88 g (71%) 2,6 Diethyl-4-methylbenzylalkohol.

Analog wurden folgende Verbindungen hergestellt:
2-Ethyl-4,6-dimethylbenzylalkohol: Fp 40-41 °C
2-Methoxy-4,6-dimethylbenzylalkohol: Fp 63-65 °C
2-Cyclopropyl-6-ethyl-4-methylbenzylalkohol
2,6-Cyclopropyl-4-methylbenzylalkohol

Zu 15.26 g (85.6 mmol) 2,6 Diethyl-4-methylbenzylalkohol in 200 ml Dichlormethan tropft man langsam 11.20 g (94.16 mmol) Thionylchlorid und erhitzt anschließend 2 h am Rückfluß. Nach Abkühlung wird 10 min. mit 50 ml Wasser gerührt, die organische Phase abgetrennt, getrocknet (Magnesiumsulfat), einrotiert und an Kieselgel (Essigsäureethylester/Hexan = 15:85) chromatographiert. Man erhält 16.18 g (96 %) 2,6 Diethyl-4-methylbenzylchlorid als farbloses Öl. ¹H-NMR (400 MHz, CDCl₃): δ = 1.26 (t, 6H), 2.30 (s, 3H), 2.76 (q, 4H), 4.70 (s, 2H), 6.88 (s, 2H).

### 2,6 Diethyl-4-methylbenzylbromid Beispiel (XIX-2)

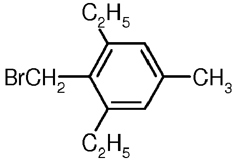

14.7 g (82.5 mmol) 2,6-Diethyl-4-methylbenzylalkohol werden in 150 ml Bromwasserstoffsäure 4 h bei 100 °C gerührt. Nach Abkühlen wird mit Dichlormethan extrahiert, mit Wasser gewaschen, getrocknet (Magnesiumsulfat) und einrotiert. Nach Destillation (110 °C Badtemperatur, 0.3 mbar) werden 17.82 g (89%) 2,6 Diethyl-4-methylbenzylbromid als farbloses Öl erhalten. ¹H-NMR (400 MHz, CDCl₃): δ = 1.28 (t, 6H), 2.30 (s, 3H), 2.73 (q, 4H), 4.61 (s, 2H), 6.90 (s, 2H).

In analoger Weise wurden folgende Vor- bzw. Zwischenstufen der Formel (XIX) hergestellt:

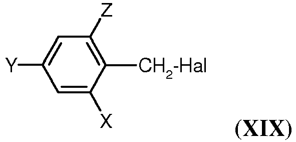

| **Bsp.-Nr.** | **Hal** | **X** | **Y** | **Z** | **Fp. °C oder ¹H-NMR (400 MHz, CDCl₃, δ in ppm)** |
|---|---|---|---|---|---|
| XIX-3 | Cl | C₂H₅ | CH₃ | CH₃ | 1.26 (t, 3H), 2.28 (s, 3H), 2.41 (s, 3H), 2.72 (q, 2H), 4.69 (s, 2H) |
| XIX-4 | Br | C₂H₅ | CH₃ | CH₃ | 2.28 (s, 3H), 2.39 (s, 3H), 2.73 (q, 2H), 4.60 (s, 2H), 6.85 und 6.88 (je s, je 1H) |
| XIX-5 | Cl | OCH₃ | CH₃ | CH₃ | 2.21 und 2.38 (je s, je 3H), 3.75 (s, 3H), 4.72 (s, 2H), 6.59 und 6.62 (je s, je 1H) |
| XIX-6 | Cl | Cyclopropyl | CH₃ | C₂H₅ | |
| XIX-7 | Br | Cyclopropyl | CH₃ | C₂H₅ | |

### Beispiel Nr. 1

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 1/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Alopecurus myosuroides, Echinocloa crus-qalli, Lolium multiflorum und Setaria viridis eine Wirkung von ≥ 80 %: I-a-3, I-a-6, I-a-7, I-a-8, I-a-9, I-a-10, I-a-11, I-a-12, I-a-13, I-a-14, I-b-4, I-b-5, I-b-6, I-b-7, I-b-8, I-c-3, I-c-4, I-c-5, I-c-7, I-c-9, I-c-10, I-c-11, I-c-12, I-c-14

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Alopecurus myosuroides, Echinocloa crus-galli, Lolium multiflorum und Setaria viridis eine Wirkung von ≥ 80%: I-a-2, I-a-3, I-a-6, I-a-7, I-a-8, I-a-10, I-a-11, I-a-12, I-a-13, I-a-14, I-b-2, I-b-6, I-b-7, I-c-3, I-c-7, I-c-8, I-c-9, I-c-10, I-c-11, I-c-12, I-c-14, I-c-15, I-f-1

### Verwendung von Safenern:

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

### Gefäßversuche mit Getreide im Gewächshaus

### Mefenpyr 1 Tag vor Herbizidapplikation

| | | 28 Tage nach Applikation |
|---|---|---|
| | Aufwandmenge g a.i./ha | Sommerweizen beobachtet (%) |
| I-a-2 | 50 | 60 |
| | 25 | 50 |
| | 12,5 | 40 |
| I-a-2 | 50 + 50 | 5 |
| + Mefenpyr | 25 + 50 | 5 |
| | 12,5 + 50 | 0 |

| | | 10 Tage nach Applikation |
|---|---|---|
| | Aufwandmenge g a.i./ha | Sommerweizen beobachtet (%) |
| I-a-10 | 50 | 60 |
| | 25 | 50 |
| | 12,5 | 30 |
| I-a-10 | 50 + 50 | 5 |
| + Mefenpyr | 25 + 50 | 3 |
| | 12,5 + 50 | 2 |

| | | 10 Tage nach Applikation |
|---|---|---|
| | Aufwandmenge g a.i./ha | Sommerweizen beobachtet (%) |
| I-c-7 | 50 | 60 |
| | 25 | 50 |
| | 12,5 | 40 |
| I-c-7 | 50 + 50 | 5 |
| + Mefenpyr | 25 + 50 | 5 |
| | 12,5 + 50 | 3 |

| | | 10 Tage nach Applikation |
|---|---|---|
| | Aufwandmenge g a.i./ha | Sommerweizen beobachtet (%) |
| I-a-11 | 50 | 70 |
| | 25 | 50 |
| | 12,5 | 30 |
| I-a-11 | 50 + 50 | 10 |
| + Mefenpyr | 25 + 50 | 10 |
| | 12,5 + 50 | 8 |

| | | 10 Tage nach Applikation |
|---|---|---|
| | Aufwandmenge g a.i./ha | Sommerweizen beobachtet (%) |
| I-a-14 | 50 | 60 |
| | 25 | 60 |
| | 12,5 | 50 |
| I-a-14 | 50 + 50 | 10 |
| + Mefenpyr | 25 + 50 | 10 |
| | 12,5 + 50 | 5 |

| | | 10 Tage nach Applikation |
|---|---|---|
| | Aufwandmenge g a.i./ha | Sommerweizen beobachtet (%) |
| I-a-13 | 50 | 60 |
| | 25 | 50 |
| | 12,5 | 40 |
| I-a-13 | 50 + 50 | 5 |
| + Mefenpyr | 25 + 50 | 2 |
| | 12,5 + 50 | 2 |

| | | 28 Tage nach Applikation |
|---|---|---|
| | Aufwandmenge g a.i./ha | Sommerweizen beobachtet (%) |
| I-c-9 | 100 | 50 |
| | 50 | 40 |
| | 25 | 30 |
| I-c-9 | 100 + 50 | 10 |
| + Mefenpyr | 50 + 50 | 5 |
| | 25 + 50 | 0 |

| | Aufwandmenge | Sommerweizen | |
|---|---|---|---|
| | g a.i./ha | beobachtet (%) | |
| | | 10 Tage nach Applikation | |
| | | | |
| I-a-8 | 100 | 60 | |
| | 50 | 50 | |
| | 25 | 40 | |
| | 12,5 | 20 | |
| | | | |
| I-a-8 | 100 + 50 | 5 | |
| +Mefenpyr | 50 + 50 | 5 | |
| | 25+50 | 2 | |
| | 12,5 + 50 | 0 | |

| | Aufwandmenge | Sommergerste | Sommerweizen |
|---|---|---|---|
| | g a.i./ha | beobachtet (%) | beobachtet (%) |
| | | 10 Tage nach Applikation | 10 Tage nach Applikation |
| | | | |
| I-a-8 | 100 | 20 | 30 |
| | 50 | 20 | 30 |
| | 25 | 10 | 20 |
| | | | |
| I-a-8 | 100 + 50 | 10 | 5 |
| +Mefenpyr | 50 + 50 | 8 | 5 |
| | 25+50 | 5 | 3 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-c-14 | 100 | 20 | 70 |
| | 50 | 10 | 60 |
| | 25 | | 50 |
| | 12,5 | | 40 |
| | | | |
| I-c-14 | 100 + 50 | 5 | 5 |
| + Mefenpyr | 50 + 50 | 0 | 5 |
| | 25+50 | | 5 |
| | 12,5 + 50 | | 0 |

| | Aufwandmenge | Sommerweizen | |
|---|---|---|---|
| | g a.i./ha | beobachtet (%) | |
| | | 28 Tage nach Applikation | |
| | | | |
| I-c-7 | 100 | 30 | |
| | 50 | 30 | |
| | 25 | 30 | |
| | 12,5 | 10 | |
| | | | |
| I-c-7 | 100 + 50 | 2 | |
| + Mefenpyr | 50 + 50 | 2 | |
| | 25+50 | 0 | |
| | 12,5 + 50 | 0 | |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-a-11 | 100 | 10 | 70 |
| | 50 | 8 | 60 |
| | 25 | | 50 |
| | 12,5 | | 10 |
| | | | |
| I-a-11 | 100 + 50 | 0 | 2 |
| +Mefenpyr | 50 + 50 | 0 | 0 |
| | 25+50 | | 0 |
| | 12,5 + 50 | | 0 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-c-3 | 100 | 95 | 99 |
| | 50 | 30 | 60 |
| | 25 | 15 | 20 |
| | 12,5 | | 10 |
| | | | |
| I-c-3 | 100 + 50 | 20 | 15 |
| + Mefenpyr | 50 + 50 | 15 | 10 |
| | 25+50 | 10 | 5 |
| | 12,5 + 50 | | 5 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-a-3 | 100 | 70 | 85 |
| | 50 | 60 | 70 |
| | 25 | 20 | 60 |
| | 12,5 | | 10 |
| | | | |
| I-a-3 | 100 + 50 | 20 | 20 |
| + Mefenpyr | 50 + 50 | 10 | 10 |
| | 25+50 | 10 | 8 |
| | 12,5 + 50 | | 5 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-a-14 | 100 | 20 | 70 |
| | 50 | 10 | 30 |
| | 25 | 8 | 30 |
| | 12,5 | 5 | 10 |
| | | | |
| I-a-14 | 100 + 50 | 8 | 8 |
| + Mefenpyr | 50 + 50 | 5 | 5 |
| | 25+50 | 0 | 5 |
| | 12,5 + 50 | 0 | 0 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-c-10 | 100 | 95 | |
| | 50 | 50 | 95 |
| | 25 | | 50 |
| | 12,5 | | 15 |
| | | | |
| I-c-10 | 100 + 50 | 30 | |
| + Mefenpyr | 50 + 50 | 20 | 40 |
| | 25+50 | | 20 |
| | 12,5 + 50 | | 10 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-a-25 | 100 | 85 | 85 |
| | 50 | 60 | 70 |
| | 25 | | 30 |
| | 12,5 | | 10 |
| | | | |
| I-a-25 | 100 + 50 | 20 | 10 |
| + Mefenpyr | 50 + 50 | 10 | 8 |
| | 25 + 50 | | 5 |
| | 12,5 + 50 | | 0 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-b-13 | 100 | 50 | 85 |
| | 50 | 20 | 80 |
| | 25 | | 40 |
| | 12,5 | | 20 |
| | | | |
| I-b-13 | 100 + 50 | 10 | 10 |
| +Mefenpyr | 50 + 50 | 5 | 10 |
| | 25 + 50 | | 5 |
| | 12,5 + 50 | | 0 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-f-19 | 100 | 50 | 99 |
| | 50 | 20 | 97 |
| | 25 | 10 | 90 |
| | 12,5 | 10 | 20 |
| | | | |
| I-f-19 | 100 + 50 | 10 | 20 |
| + Mefenpyr | 50 + 50 | 5 | 10 |
| | 25+50 | 0 | 10 |
| | 12,5 + 50 | 0 | 10 |

| | Aufwandmenge | Sommergerste | Sommerweizen |
|---|---|---|---|
| | g a.i./ha | beobachtet (%) | beobachtet (%) |
| | | 28 Tage nach Applikation | 28 Tage nach Applikation |
| | | | |
| I-a-58 | 100 | 15 | 60 |
| | 50 | 10 | 20 |
| | 25 | 5 | 15 |
| | 12,5 | | 10 |
| | | | |
| I-a-58 | 100 + 50 | 5 | 5 |
| + Mefenpyr | 50 + 50 | 0 | 0 |
| | 25+50 | 0 | 0 |
| | 12,5 + 50 | | 0 |

### Vorauflauf

| 80 g/ha | | | | | |
|---|---|---|---|---|---|
| Beispiel | ALOMY | AVEFA | ECHCG | LOLMU | SETVI |
| I-a-1 | 90 | 80 | 50 | 100 | 60 |
| I-a-2 | 90 | 50 | 90 | 100 | 50 |
| I-a-3 | 100 | 80 | 100 | 100 | 100 |
| I-a-5 | 60 | 60 | 80 | 90 | 70 |
| I-a-6 | 100 | 60 | 60 | 100 | 60 |
| I-a-7 | 100 | 80 | 70 | 90 | 70 |
| I-a-8 | 100 | 60 | 90 | 100 | 90 |
| I-a-9 | 90 | 70 | 100 | 100 | 90 |
| I-a-10 | 70 | 50 | 80 | 100 | 60 |
| I-a-11 | 90 | 50 | 100 | 100 | 70 |
| I-a-12 | 100 | 80 | 100 | 100 | 90 |
| I-a-13 | 90 | 80 | 100 | 100 | 90 |
| I-a-14 | 100 | 80 | 100 | 100 | 90 |
| I-a-15 | 80 | 40 | 70 | 100 | 70 |
| I-a-19 | 90 | 20 | 0 | 100 | 20 |
| I-a-25 | 100 | 70 | 90 | 100 | 90 |
| I-a-26 | 100 | 70 | 100 | 100 | 100 |
| I-a-27 | 90 | 80 | 100 | 100 | 90 |
| I-a-28 | 100 | 80 | 100 | 100 | 90 |
| I-a-29 | 90 | | 70 | 80 | 20 |
| I-a-30 | 80 | 0 | | 70 | 70 |
| I-a-31 | 80 | 40 | 100 | 100 | 90 |
| I-a-32 | 90 | 60 | 40 | 100 | 20 |
| I-a-35 | 80 | 80 | 70 | 100 | 70 |
| I-a-36 | 60 | 70 | 80 | 90 | 80 |
| I-a-38 | 100 | 40 | 40 | 100 | 90 |
| I-a-39 | 80 | 70 | 0 | 100 | 30 |
| I-a-40 | 50 | 40 | 80 | 100 | 50 |
| I-a-41 | 60 | 60 | 50 | 100 | |
| I-a-45 | 80 | 60 | 100 | 80 | 100 |
| I-a-48 | 80 | 60 | 40 | 80 | 70 |
| I-a-49 | 100 | 90 | 100 | 100 | 90 |
| I-a-50 | 100 | 60 | 80 | 100 | 70 |
| I-a-55 | 60 | 60 | 100 | 50 | 90 |
| I-a-57 | 80 | 60 | 100 | 100 | 90 |
| I-a-58 | 70 | 70 | 100 | 100 | 100 |
| I-a-61 | 70 | 60 | 80 | 100 | 60 |
| I-a-62 | 100 | 40 | 100 | 100 | 90 |
| I-a-68 | 40 | 40 | 50 | 80 | 0 |
| I-a-69 | 50 | 70 | 20 | 70 | 80 |
| I-a-70 | 80 | 60 | 80 | 80 | 60 |
| I-b-1 | 90 | 40 | 40 | 100 | 70 |
| I-b-2 | 90 | 50 | 30 | 100 | 40 |
| I-b-3 | 90 | 60 | 0 | 100 | 50 |
| I-b-4 | 90 | 50 | 70 | 100 | 60 |
| I-b-5 | 90 | | 10 | 100 | 60 |
| I-b-6 | 100 | 80 | 30 | 100 | 60 |
| I-b-7 | 100 | 80 | 60 | 100 | 70 |
| I-b-8 | 100 | 80 | 90 | 100 | 90 |
| I-b-12 | 100 | 70 | 100 | 100 | 90 |
| I-b-13 | 100 | 80 | 100 | 100 | 100 |
| I-b-14 | 100 | 70 | 100 | 100 | 100 |
| I-b-15 | 80 | 50 | 100 | 80 | |
| I-b-16 | 100 | 80 | 100 | 100 | 100 |
| I-b-17 | 90 | 80 | 100 | 100 | 90 |
| I-b-18 | 100 | 70 | 80 | 100 | 70 |
| I-b-19 | 100 | | 100 | 100 | 90 |
| I-b-20 | 100 | 90 | 80 | 100 | 90 |
| I-b-21 | 80 | 80 | 80 | 100 | 20 |
| I-b-22 | 100 | 70 | 100 | 100 | 80 |
| I-b-23 | 90 | 70 | 100 | 100 | 50 |
| I-b-24 | 70 | 70 | 100 | 100 | 40 |
| I-b-25 | 90 | 70 | 100 | 100 | 60 |
| I-b-27 | | 70 | 100 | 100 | 70 |
| I-b-28 | 90 | 50 | 100 | 100 | 70 |
| I-b-29 | | 70 | 100 | 100 | 80 |
| I-b-30 | 100 | 70 | 100 | 100 | 90 |
| I-b-37 | 90 | 40 | 70 | 70 | 70 |
| I-b-38 | 80 | 70 | 80 | 60 | 80 |
| I-b-39 | 60 | 50 | 10 | 80 | 0 |
| I-b-40 | 90 | 50 | 50 | 100 | 30 |
| I-b-41 | 50 | 40 | 0 | 100 | 50 |
| I-b-42 | | 70 | 60 | 100 | 90 |
| I-b-43 | 90 | 40 | 20 | 90 | 60 |
| I-b-45 | 90 | 40 | 20 | 60 | 30 |
| I-b-46 | 90 | 60 | 0 | 100 | 0 |
| I-b-48 | 80 | 20 | 0 | 70 | 0 |
| I-b-49 | 90 | 70 | 50 | 70 | 0 |
| I-b-51 | 80 | 30 | 30 | 70 | 0 |
| I-b-62 | 50 | 40 | 70 | 70 | 80 |
| I-b-63 | 70 | | 100 | 100 | 70 |
| I-b-64 | 60 | 50 | 0 | 100 | 90 |
| I-b-65 | 60 | 70 | 90 | 100 | 70 |
| I-b-66 | 80 | 80 | 100 | 100 | 80 |
| I-b-67 | 90 | 90 | 100 | 100 | 90 |
| I-b-69 | 100 | 80 | 100 | 100 | 80 |
| I-b-70 | 100 | 80 | 100 | 100 | 90 |
| I-b-72 | 80 | 60 | 100 | 100 | 100 |
| I-b-73 | 80 | 80 | 90 | 100 | 100 |
| I-b-74 | 60 | 60 | 100 | 100 | 70 |
| I-b-75 | 60 | 60 | 100 | 100 | 70 |
| I-b-76 | 100 | 60 | 100 | 100 | 70 |
| I-b-77 | 60 | 40 | | 90 | 60 |
| I-b-78 | 100 | 60 | 100 | 100 | 60 |
| I-b-79 | 70 | 70 | 100 | 100 | 80 |
| I-c-2 | 90 | 50 | 40 | 100 | 60 |
| I-c-3 | 100 | 80 | 40 | 100 | 70 |
| I-c-4 | 90 | 80 | 60 | 90 | 50 |
| I-c-5 | 80 | 80 | 90 | 80 | 80 |
| I-c-6 | 50 | 10 | 90 | 80 | 60 |
| I-c-7 | 100 | 70 | 100 | 90 | 70 |
| I-c-8 | 60 | 40 | 60 | 100 | 50 |
| I-c-9 | 100 | 70 | 100 | 100 | 70 |
| I-c-10 | 100 | 80 | 100 | 100 | 90 |
| I-c-11 | 90 | 70 | 100 | 90 | 90 |
| I-c-12 | 100 | 80 | 100 | 100 | 100 |
| I-c-13 | 90 | 50 | 60 | 80 | 60 |
| I-c-14 | 90 | 70 | 90 | 100 | 70 |
| I-c-18 | 90 | 0 | 100 | 100 | 10 |
| I-c-19 | 100 | 70 | 50 | 90 | 90 |
| I-c-21 | 90 | 70 | 100 | 100 | 100 |
| I-c-22 | 100 | 70 | 100 | 100 | 100 |
| I-c-23 | 100 | 70 | 100 | 100 | 100 |
| I-c-24 | 90 | | 70 | 100 | 70 |
| I-c-25 | 100 | 90 | 100 | 100 | 80 |
| I-c-26 | 100 | 80 | 100 | 100 | 100 |
| I-c-27 | 100 | 80 | 100 | 100 | 90 |
| I-c-28 | 90 | 60 | 100 | | 50 |
| I-c-30 | 100 | 90 | 80 | 100 | 60 |
| I-c-31 | 90 | 80 | 60 | 0 | 60 |
| I-c-32 | 80 | 70 | 100 | 100 | 70 |
| I-c-33 | 90 | 90 | 90 | 100 | 60 |
| I-c-34 | 90 | 60 | 100 | 100 | 80 |
| I-c-35 | 80 | 70 | | 100 | 70 |
| I-c-39 | 100 | 70 | 80 | 60 | 70 |
| I-c-39 | 20 | 20 | 100 | 80 | 20 |
| I-c-40 | 80 | 60 | 0 | 80 | 40 |
| I-c-41 | 90 | 40 | 60 | 100 | 40 |
| I-c-43 | 80 | 40 | 80 | 80 | 0 |
| I-c-44 | 50 | 50 | 0 | 100 | 0 |
| I-c-45 | 60 | 80 | 80 | 80 | 80 |
| I-c-46 | 80 | 50 | 80 | 70 | 80 |
| I-c-50 | 30 | 0 | 0 | 100 | 0 |
| I-c-51 | 100 | 60 | 30 | 90 | 90 |
| I-c-52 | 60 | 70 | 50 | 100 | 70 |
| I-c-53 | 100 | 50 | 0 | 90 | 60 |
| I-c-54 | 90 | 40 | 0 | 90 | 0 |
| I-c-56 | 90 | 20 | 20 | 90 | 0 |
| I-c-61 | 70 | 60 | 90 | 50 | 90 |
| I-c-62 | 60 | 50 | 80 | 80 | 90 |
| I-c-63 | 80 | 70 | 50 | 100 | 50 |
| I-c-64 | 60 | 70 | 50 | 100 | 70 |
| I-c-65 | 100 | 60 | 80 | 100 | 90 |
| I-c-66 | 100 | 70 | 100 | 100 | 60 |
| I-c-71 | 70 | 70 | 100 | 100 | 90 |
| I-c-72 | 60 | 50 | 100 | 100 | 80 |
| I-c-73 | 70 | 40 | 50 | 100 | 60 |
| I-c-74 | 70 | 60 | 100 | 100 | 100 |
| I-c-75 | 70 | 60 | 100 | 100 | 60 |
| I-c-76 | 80 | 20 | 100 | 90 | 90 |
| I-c-77 | 80 | 80 | 80 | 90 | 100 |
| I-c-78 | 100 | 90 | 100 | 100 | 80 |
| I-c-79 | 90 | 80 | 100 | 100 | 80 |
| I-c-80 | 100 | 60 | 100 | 100 | 60 |
| I-f-2 | 90 | 50 | 90 | 100 | 60 |
| I-f-4 | 0 | 50 | 90 | 100 | 50 |
| I-f-5 | 90 | 70 | 70 | 90 | 90 |
| I-f-6 | 60 | 90 | 70 | 90 | 70 |
| I-f-7 | 100 | 70 | 80 | 100 | 90 |
| I-f-8 | 30 | 40 | 0 | 100 | 70 |
| I-f-9 | 30 | 50 | 60 | 100 | 50 |
| I-f-11 | 40 | 50 | 0 | 100 | 20 |
| I-f-12 | 100 | 70 | 100 | 70 | 90 |
| I-f-13 | 100 | 0 | 100 | 100 | 90 |
| I-f-14 | 100 | 70 | 100 | 100 | 100 |
| I-f-15 | 100 | 60 | 100 | 100 | 90 |
| I-f-16 | 80 | 80 | 60 | 90 | 90 |
| I-f-17 | 80 | 60 | 90 | 100 | 90 |
| I-f-19 | 100 | 60 | 100 | 100 | 100 |
| I-f-20 | 100 | 70 | 100 | 100 | 100 |
| I-f-21 | 90 | 70 | 100 | 100 | 90 |
| I-f-22 | 80 | 70 | | 80 | 100 |

### Nachauflauf

| 80 g/ha | | | | | |
|---|---|---|---|---|---|
| Beispiel | ALOMY | AVEFA | ECHCG | LOLMU | SETVI |
| I-a-1 | 100 | 90 | 100 | 100 | |
| I-a-2 | 100 | 70 | 100 | 100 | 90 |
| I-a-3 | 90 | 90 | 100 | 100 | 100 |
| I-a-4 | 60 | 30 | 100 | 20 | 100 |
| I-a-5 | 60 | 50 | 100 | 90 | 100 |
| I-a-6 | 90 | 70 | 100 | 100 | 90 |
| I-a-7 | 90 | 60 | 100 | 100 | 90 |
| I-a-8 | 100 | 100 | 100 | 100 | 100 |
| I-a-9 | 60 | 60 | 100 | 90 | 90 |
| I-a-10 | 90 | 80 | 100 | 100 | 90 |
| I-a-11 | 90 | 90 | 100 | 100 | 100 |
| I-a-12 | 100 | 100 | 100 | 100 | 100 |
| I-a-13 | 90 | 90 | 100 | 100 | 90 |
| I-a-14 | 90 | 90 | 100 | 100 | 100 |
| I-a-15 | 90 | 90 | 100 | 100 | 90 |
| I-a-16 | 30 | 30 | 90 | 80 | 80 |
| I-a-17 | 30 | 40 | 90 | 90 | 80 |
| I-a-18 | 90 | 60 | 100 | 100 | 90 |
| I-a-19 | 80 | 0 | 90 | 100 | 50 |
| I-a-20 | 90 | 80 | 100 | 100 | 90 |
| I-a-25 | 100 | 100 | 100 | 100 | 100 |
| I-a-26 | 100 | 100 | 100 | 100 | 100 |
| I-a-27 | 100 | 100 | 100 | 100 | 90 |
| I-a-28 | 100 | 100 | 100 | 100 | 100 |
| I-a-29 | 100 | 60 | 100 | 100 | 80 |
| I-a-30 | 90 | 0 | 100 | 90 | 50 |
| I-a-31 | 100 | 80 | 100 | 100 | 100 |
| I-a-32 | 80 | 0 | 100 | 100 | 20 |
| I-a-33 | 100 | 0 | 100 | 100 | 90 |
| I-a-35 | 100 | 80 | 100 | 100 | 90 |
| I-a-36 | 50 | 0 | 90 | 90 | 90 |
| I-a-37 | 60 | 90 | 100 | 100 | 60 |
| I-a-38 | 100 | 90 | 80 | 100 | 90 |
| I-a-39 | 100 | 20 | 90 | 100 | 90 |
| I-a-40 | 80 | 60 | 90 | 90 | 80 |
| I-a-41 | 100 | 40 | 90 | 100 | 100 |
| I-a-43 | 20 | 0 | 90 | 40 | 40 |
| I-a-44 | 20 | 0 | 100 | 20 | 80 |
| I-a-45 | 0 | 0 | 80 | 0 | 80 |
| I-a-46 | 0 | 0 | 100 | 0 | 40 |
| I-a-47 | 20 | 80 | 100 | 100 | 100 |
| I-a-48 | 80 | 90 | 100 | 100 | 100 |
| I-a-49 | 100 | 100 | 100 | 100 | 100 |
| I-a-50 | 100 | 100 | 100 | 100 | 100 |
| I-a-55 | 20 | 0 | 100 | 40 | 100 |
| I-a-56 | 0 | 0 | 100 | 0 | 100 |
| I-a-57 | 100 | 100 | 100 | 100 | 100 |
| I-a-58 | 100 | | 100 | 100 | 100 |
| I-a-60 | 100 | 90 | 100 | 100 | 100 |
| I-a-61 | 90 | 90 | 100 | 100 | 90 |
| I-a-62 | 100 | 80 | 100 | 100 | 90 |
| I-a-68 | 90 | 0 | 100 | 100 | 40 |
| I-a-69 | 90 | 30 | 100 | 100 | 80 |
| I-a-70 | 90 | 0 | 100 | 100 | 60 |
| I-b-1 | 100 | 60 | 100 | 100 | 40 |
| I-b-2 | 100 | 60 | 90 | 100 | 80 |
| I-b-3 | 100 | 40 | 100 | 100 | 20 |
| I-b-4 | 100 | 90 | 100 | 100 | 60 |
| I-b-5 | 90 | 20 | 80 | 100 | 0 |
| I-b-6 | 90 | 100 | 100 | 100 | 90 |
| I-b-7 | 90 | 100 | 100 | 100 | 90 |
| I-b-8 | 90 | 70 | 80 | 100 | 60 |
| I-b-9 | 90 | 0 | 90 | 40 | 40 |
| I-b-10 | 90 | 20 | 90 | 70 | 70 |
| I-b-12 | 90 | 100 | 100 | 100 | 100 |
| I-b-13 | 100 | 100 | 100 | 100 | 100 |
| I-b-14 | 100 | 100 | 100 | 100 | 100 |
| I-b-15 | 100 | 90 | 100 | 100 | 100 |
| I-b-16 | 100 | 90 | 100 | 100 | 100 |
| I-b-17 | 90 | 90 | 100 | 100 | 90 |
| I-b-18 | 100 | 100 | 100 | 100 | 100 |
| I-b-19 | 20 | 30 | 100 | 40 | 80 |
| I-b-20 | 0 | 30 | 100 | 60 | 0 |
| I-b-21 | 90 | 90 | 100 | 100 | 90 |
| I-b-22 | 100 | 100 | 100 | 100 | 100 |
| I-b-23 | 100 | 100 | 100 | 100 | 100 |
| I-b-24 | 100 | 100 | 100 | 100 | 100 |
| I-b-25 | 100 | 100 | 100 | 100 | 100 |
| I-b-27 | 100 | 100 | 100 | 100 | 100 |
| I-b-28 | 100 | 100 | 100 | 100 | 100 |
| I-b-29 | 100 | 90 | 100 | 100 | 100 |
| I-b-30 | 20 | 100 | 100 | 100 | 90 |
| I-b-37 | 0 | 0 | 90 | 70 | 90 |
| I-b-38 | 40 | 0 | 90 | 70 | 90 |
| I-b-39 | 60 | 20 | 100 | 100 | 90 |
| I-b-40 | 40 | 20 | 90 | 100 | 80 |
| I-b-41 | 40 | 0 | 100 | 100 | 80 |
| I-b-42 | 100 | 80 | 100 | 100 | 100 |
| I-b-43 | 100 | 80 | 90 | 100 | 100 |
| I-b-44 | 80 | 80 | 80 | 100 | 60 |
| I-b-45 | 80 | 0 | 90 | 90 | 40 |
| I-b-46 | 90 | 40 | 90 | 100 | 80 |
| I-b-47 | 90 | 80 | 100 | 90 | 0 |
| I-b-48 | 90 | 40 | 90 | 90 | 80 |
| I-b-49 | 90 | 80 | 100 | 100 | 60 |
| I-b-50 | 100 | 0 | 90 | 100 | 80 |
| I-b-51 | 90 | 0 | 100 | 60 | 40 |
| I-b-52 | 0 | 0 | 100 | 0 | 90 |
| I-b-53 | 20 | 0 | 100 | 0 | 90 |
| I-b-54 | 20 | 0 | 100 | 0 | 100 |
| I-b-55 | 0 | 0 | 90 | 0 | 90 |
| I-b-56 | 20 | 0 | 100 | 0 | 100 |
| I-b-57 | 0 | 0 | 100 | 0 | 100 |
| I-b-58 | 0 | 0 | 80 | 60 | 20 |
| I-b-59 | 20 | 0 | 80 | 80 | 0 |
| I-b-60 | 0 | 0 | 100 | 0 | 80 |
| I-b-62 | 30 | 0 | 100 | 90 | 90 |
| I-b-63 | 100 | 100 | 100 | 100 | 100 |
| I-b-64 | 100 | 100 | 100 | 100 | 100 |
| I-b-65 | 90 | 90 | 100 | 100 | 100 |
| I-b-66 | 100 | 90 | 100 | 100 | 80 |
| I-b-67 | 100 | 70 | 100 | 100 | 90 |
| I-b-69 | 90 | 10 | 100 | 100 | 60 |
| I-b-70 | 70 | 0 | 100 | 100 | 40 |
| I-b-71 | 0 | 0 | 100 | 0 | 100 |
| I-b-72 | 0 | 0 | 100 | 0 | 90 |
| I-b-73 | 0 | 0 | 100 | 70 | 100 |
| I-b-74 | 80 | 80 | 100 | 100 | 80 |
| I-b-75 | 100 | 90 | 100 | 100 | 100 |
| I-b-76 | 90 | 90 | 100 | 100 | 100 |
| I-b-77 | 100 | 60 | 100 | 100 | 90 |
| I-b-78 | 100 | 70 | 100 | 100 | 70 |
| I-b-79 | 90 | 100 | 100 | 100 | 100 |
| I-c-1 | 100 | 60 | 60 | 100 | 40 |
| I-c-2 | 90 | 40 | 40 | 100 | 40 |
| I-c-3 | 90 | 80 | 90 | 100 | 80 |
| I-c-4 | 90 | 90 | 100 | 100 | 0 |
| I-c-5 | 20 | 30 | 100 | 90 | 90 |
| I-c-7 | 90 | 70 | 100 | 100 | 80 |
| I-c-8 | 90 | 60 | 100 | 100 | 90 |
| I-c-9 | 90 | 80 | 100 | 100 | 90 |
| I-c-10 | 90 | 100 | 100 | 100 | 90 |
| I-c-11 | 80 | 80 | 100 | 90 | 90 |
| I-c-12 | 90 | 90 | 100 | 100 | 100 |
| I-c-13 | 70 | 80 | 100 | 100 | 90 |
| I-c-14 | 90 | 70 | 100 | 100 | 100 |
| I-c-15 | 90 | 60 | 90 | 90 | 90 |
| I-c-16 | 0 | 30 | 90 | 80 | 30 |
| I-c-17 | 30 | 0 | 100 | 40 | 90 |
| I-c-18 | 60 | 0 | 80 | 90 | 0 |
| I-c-19 | 100 | 100 | 100 | 100 | 100 |
| I-c-20 | 90 | 90 | 100 | 100 | 100 |
| I-c-21 | 90 | 100 | 100 | 100 | 100 |
| I-c-22 | 100 | 100 | 100 | 100 | 100 |
| I-c-23 | 100 | 100 | 100 | 100 | 100 |
| I-c-24 | 100 | 80 | 100 | 100 | 100 |
| I-c-25 | 100 | 100 | 100 | 100 | 100 |
| I-c-26 | 100 | 100 | 100 | 100 | 100 |
| I-c-27 | 100 | 100 | 100 | 100 | 100 |
| I-c-28 | 100 | 100 | 100 | 100 | 100 |
| I-c-30 | 80 | 40 | 100 | 100 | 70 |
| I-c-31 | 70 | 90 | 100 | 100 | 90 |
| I-c-32 | 80 | 90 | 90 | 90 | 80 |
| I-c-33 | 80 | 90 | 100 | 90 | 80 |
| I-c-34 | 100 | 100 | 100 | 100 | 90 |
| I-c-35 | 100 | 100 | 100 | 100 | 100 |
| I-c-38 | 70 | 0 | 80 | 90 | 30 |
| I-c-39 | 90 | 0 | 100 | 100 | 70 |
| I-c-39 | 20 | 0 | 100 | 100 | 20 |
| I-c-40 | 100 | 0 | 100 | 100 | 100 |
| I-c-41 | 100 | 80 | 100 | 100 | 20 |
| I-c-42 | 0 | 0 | 100 | 0 | 100 |
| I-c-43 | 100 | 0 | 100 | 100 | 0 |
| I-c-44 | 40 | 80 | 100 | 100 | 90 |
| I-c-45 | 40 | 0 | 90 | 100 | 90 |
| I-c-46 | 40 | 0 | 80 | 90 | 90 |
| I-c-47 | 100 | 90 | 100 | 100 | 60 |
| I-c-48 | 20 | 20 | 90 | 100 | 80 |
| I-c-49 | 100 | 80 | 90 | 100 | 80 |
| I-c-50 | 60 | 0 | 80 | 100 | 80 |
| I-c-51 | 100 | 40 | 100 | 100 | 100 |
| I-c-52 | 100 | 80 | 90 | 100 | 100 |
| I-c-53 | 100 | 80 | 90 | 100 | 90 |
| I-c-54 | 90 | 0 | 90 | 80 | 40 |
| I-c-55 | 80 | 20 | 100 | 100 | 30 |
| I-c-56 | 90 | 20 | 80 | 80 | 40 |
| I-c-57 | 90 | 0 | 100 | 0 | 90 |
| I-c-58 | 0 | 0 | 100 | 0 | 100 |
| I-c-59 | 0 | 0 | 100 | 60 | 20 |
| I-c-60 | 20 | 0 | 100 | 80 | 0 |
| I-c-61 | 40 | 0 | 90 | 70 | 90 |
| I-c-62 | 0 | 50 | 100 | 90 | 100 |
| I-c-63 | 100 | 100 | 100 | 100 | 100 |
| I-c-64 | 80 | 80 | 100 | 100 | 100 |
| I-c-65 | 100 | 100 | 100 | 100 | 100 |
| I-c-66 | 100 | 100 | 100 | 100 | 100 |
| I-c-71 | 80 | 90 | | 100 | 100 |
| I-c-72 | 100 | 70 | 100 | 100 | 100 |
| I-c-73 | 100 | 0 | 100 | 100 | 100 |
| I-c-74 | 60 | 70 | 100 | 100 | 100 |
| I-c-75 | 60 | 50 | 100 | 100 | 100 |
| I-c-76 | 10 | 0 | 100 | 90 | 100 |
| I-c-77 | 0 | 0 | 100 | 90 | 90 |
| I-c-78 | 100 | 0 | 90 | 100 | 80 |
| I-c-79 | 100 | 70 | 100 | 100 | 90 |
| I-c-80 | 90 | 70 | 100 | 100 | 90 |
| I-f-1 | 80 | 40 | 90 | 90 | 80 |
| I-f-2 | 80 | 90 | 100 | 100 | 90 |
| I-f-3 | 90 | 0 | 90 | 90 | 80 |
| I-f-4 | 100 | 90 | 100 | 100 | 100 |
| I-f-5 | 100 | 100 | 100 | 100 | 100 |
| I-f-6 | 100 | 80 | 100 | 100 | 100 |
| I-f-7 | 100 | 100 | 100 | 100 | 100 |
| I-f-8 | 100 | 90 | 100 | 100 | 100 |
| I-f-9 | 80 | 90 | 80 | 100 | 100 |
| I-f-10 | 100 | 80 | 90 | 100 | 90 |
| I-f-11 | 60 | 40 | 90 | 100 | 80 |
| I-f-12 | 100 | 100 | 100 | 100 | 100 |
| I-f-13 | 100 | 100 | 100 | 100 | 100 |
| I-f-14 | 100 | 100 | 100 | 100 | 100 |
| I-f-15 | 80 | 90 | 100 | 100 | 100 |
| I-f-16 | 100 | 100 | 100 | 100 | 100 |
| I-f-17 | 100 | 100 | 100 | 100 | 100 |
| I-f-19 | 100 | 100 | 100 | 100 | 100 |
| I-f-20 | 100 | 100 | 100 | 100 | 100 |
| I-f-21 | 80 | 80 | 100 | 100 | 100 |
| I-f-22 | 30 | 40 | 100 | 80 | 100 |
| I-f-23 | 0 | 0 | 90 | 60 | 80 |

| | | | | | |
|---|---|---|---|---|---|
| ALOMY: Alopecurus myosuroide AVEFA: Avena fatua ECHCG: Echinochloa crus-galli LOLMU: Lolium multiflorum SETVI: Setaria viridis | | | | | |

### Beispiel Nr. 3

Methode-Kulturverträglichkeit Kleinparzellenversuch Freiland:
Praxisübliche Aussaat mit Einzelkorn Sämaschine
Parzellengröße: 11,25m² / 2facher Wiederholung

Die Behandlung erfolgte mit einem Parzellenspritzgerät. Der Safener wurde zusammen mit der Testsubstanz als Tankmischung mit 300 1/ha Wasser appliziert (Angabe der Herbizid- und Safenermenge in g ai /ha). Die Nachauflaufapplikation erfolgte im fünf Blatt Stadium des Maises. Die Kulturverträglichkeit wurde 14 und 27 Tage nach der Behandlung visuell (prozentual im Vergleich zu Unbehandelt) bonitiert, 100% Schaden = Pflanzen sind abgestorben, 0 % Schaden = wie Kontrollpflanzen).

### Freilandversuch mit Mais (Magixx Duo ist Cycloxidim tolerant)

### Nachauflauf

| | Sorte | Magixx Duo | Oldham | | Magixx Duo | Oldham |
|---|---|---|---|---|---|---|
| | Tage nach Applikation / % Schaden | | | | | |
| | g ai/ha | 14 Tage | 14 Tage | | 27 Tage | 27 Tage |
| Bsp. I-a-8 SL 450 | 60 | 0% | 94 % | | 0% | 93 % |
| Bsp. I-a-8 + Cyprosulfamid WP 50 | 60 + 60 | 0% | 23 % | | 0% | 13 % |

### Beispiel Nr. 4

### Myzus-Test (MYZUPE Spritzbehandlung)

Lösungsmittel: 78,0 Gewichtsteile Aceton 1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp: I-a-10, I-c-10, I-c-14

### Beispiel Nr. 5

### Heliothis virescens - Test - Behandlung transgener Pflanzen

Lösungsmittel: 7 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel Nr. 6

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel Nr. 7

Steigerung der Penetration in die Pflanze durch Ammonium- oder Phosphoniumsalze und synergistische Steigerung der Penetration in die Pflanze durch Ammonium- / Phosphoniumsalze in Kombination mit Penetrationsförderern

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 µl der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 8 bis 10 Messungen.

| Wirkstoff | | | | | |
|---|---|---|---|---|---|
| | Penetration nach 24h / % | | | | |
| | a.i. | | a.i. + | a.i. + | a.i. + |
| | | | AS | Edenor Me SU 500 | Edenor ME SU 500 EW + |
| | | | | EW | AS |
| Beispiel I-a-1 | 2.5 | | 4 | 11.5 | 13 |
| Beispiele I-a-14 | 0 | | 3 | 5 | 16 |
| Beispiel I-a-42 | 0 | | 2 | 23 | 39 |

| | | | | | |
|---|---|---|---|---|---|
| a.i. (0.2 g/l) AS = Ammoniumsulfat (0.7 g/l) Edenor Me SU 500 EW (2 g/l) | | | | | |

## Patentansprüche

1. Verbindungen der Formel (I) 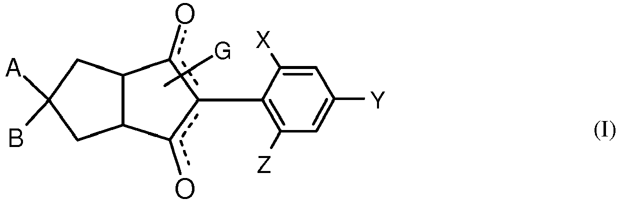 in welcher
X für Alkyl, Cycloalkyl oder Alkoxy steht,
Y für Wasserstoff, Alkyl oder Alkoxy steht,
Z für Wasserstoff, Alkyl oder Cycloalkyl steht,
wobei
A und B gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
oder
A und B gemeinsam mit dem Kohlenstoffatom an das sie gebunden sind für eine C₁-C₄-Alkylen-Gruppe oder eine =N-OR⁹-Gruppe stehen,
G für Wasserstoff (a) oder für eine der Gruppen 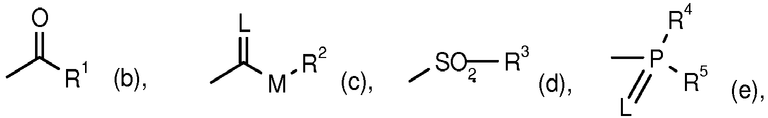 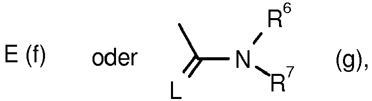 steht, worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen,
R⁹ für Wasserstoff, für jeweils gegebenenfalls substituiertes Alkyl, Cycloalkyl, CH₂Cycloalkyl, Alkenyl, Alkinyl, Arylalkyl oder Hetarylalkyl steht.

2. Verbindungen der Formel (I) gemäß Anspruch 1, in welcher
X für C₁-C₄-Alkyl, C₃-C₆-Cycloalkyl oder C₁-C₄-Alkoxy steht,
Y für Wasserstoff, C₁-C₄-Alkyl, oder C₁-C₄-Alkoxy steht,
Z für Wasserstoff, C₁-C₄-Alkyl oder C₃-C₆-Cycloalkyl steht,
A, B und das Kohlenstoffatom an das sie gebunden sind, für C₃-C₈-Cycloalkyl oder C₅-C₈-Cycloalkenyl, worin gegebenenfalls ein oder zwei Ringglieder durch Sauerstoff und/oder Schwefel ersetzt sind und welche gegebenenfalls ein- bis vierfach durch C₁-C₈-Alkyl, C₁-C₈-Alkenyl, C₁-C₄-Alkylen, C₁-C₈-Halogenalkyl, C₁-C₆-Alkoxy-C₁-C₄-alkyl, C₁-C₆-Alkoxy, C₁-C₆-Halogenalkoxy, Hydroxy, Hydroxy-C₁-C₄-alkyl oder Benzyloxy substituiert sind, oder einen weiteren ankondensierten C₃-C₈-Cycloalkylring, in dem gegebenenfalls ein- oder zwei Ringlieder durch Sauerstoff oder Schwefel ersetzt sind, oder welcher gegebenenfalls einfach oder zweifach durch C₁-C₈-Alkyl substituiert ist, enthalten, oder
A, B und das Kohlenstoffatom, an das sie gebunden sind, für eine C₁-C₄-Alkylen-Gruppe, oder für eine =N-OR⁹-Gruppe stehen,
G für Wasserstoff (a) oder für eine der Gruppen 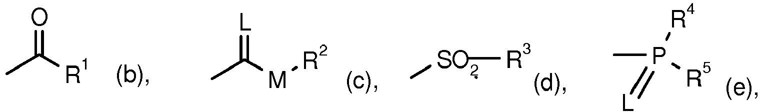 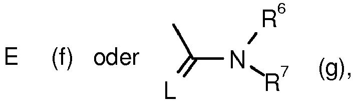 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5-oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen sub stituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
R⁹ für Wasserstoff, für jeweils gegebenenfalls einfach bis fünffach durch Halogen substituiertes C₁-C₆-Alkyl, C₃-C₆-Cycloalkyl, CH₂C₃-C₆-Cycloalkyl, C₃-C₆-Alkenyl, C₃-C₆-Alkinyl oder jeweils gegebenenfalls einfach bis dreifach durch Alkyl, Halogen, Alkoxy, Halogenalkyl oder Halogenalkoxy substituiertes Phenyl-C₁-C₂-alkyl oder Hetaryl-C₁-C₂-alkyl steht.

3. Verbindungen der Formel (I) gemäß Anpruch 1, in welcher
X für Methyl, Ethyl, Cyclopropyl, Methoxy oder Ethoxy steht,
Y für Wasserstoff, Methyl oder Ethyl steht,
Z für Wasserstoff, Methyl, Ethyl oder Cyclopropyl steht,
A, B und das Kohlenstoffatom an das sie gebunden sind, für C₃-C₈-Cycloalkyl oder C₅-C₈-Cycloalkenyl, worin gegebenenfalls ein oder zwei Ringglieder durch Sauerstoff und/oder Schwefel ersetzt sind und welches gegebenenfalls ein- bis vierfach durch C₁-C₄-Alkyl, C₁-C₄-Alkenyl, einer =CH₂-Gruppe, Trifluormethyl, C₁-C₄-Alkoxy, C₁-C₄-Alkoxy-C₁-C₂-alkyl oder mit einer der Gruppen 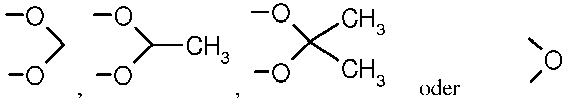
substituiert ist oder,
A, B und das Kohlenstoffatom, an das sie gebunden sind, für eine C₁-C₄-Alkylen-Gruppe oder für eine =N-OR⁹-Gruppe stehen,
G für Wasserstoff (a) oder für eine der Gruppen 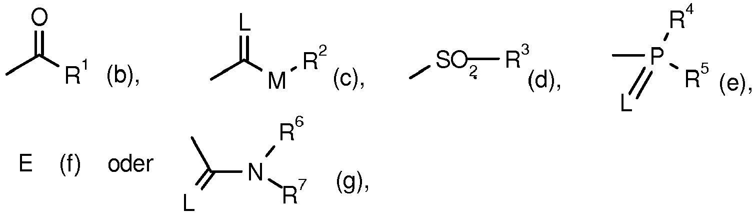 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₈-Alkyl, C₂-C₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind, für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl steht,
R² für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl steht,
R³ für gegebenenfalls einfach bis dreifach durch Fluor substituiertes C₁-C₆-Alkyl oder für gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio steht,
R⁵ für gegebenenfalls einfach durch Chlor substituiertes C₁-C₆-Alkoxy oder C₁-C₆-Alkylthio steht,
R⁶ für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl steht,
R⁷ für C₁-C₆-Alkyl, C₃-C₆-Alkenyl oder C₁-C₆-Alkoxy-C₁-C₄-alkyl steht,
R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist,
R⁹ für Wasserstoff, für jeweils gegebenenfalls einfach bis dreifach durch Fluor oder Chlor substituiertes C₁-C₄-Alkyl, C₃-C₆-Cycloalkyl, CH₂ C₃-C₆-Cycloalkyl, C₃-C₄-Alkenyl, C₃-C₄-Alkinyl, für jeweils gegebenenfalls einfach bis zweifach durch C₁-C₂-Alkyl, C₁-C₂-Alkoxy, Fluor, Chlor, Brom Trifluormethyl oder Trifluormethoxy substituiertes Benzyl oder Pyridinylmethyl steht.

4. Verbindungen der Formel (I) gemäß Anspruch 1, in welcher
X für Methyl, Ethyl, Cyclopropyl, Methoxy oder Ethoxy steht,
Y für Wasserstoff, Methyl oder Ethyl steht,
Z für Wasserstoff, Methyl, Ethyl oder Cyclopropyl steht,
A, B und das Kohlenstoffatom an das sie gebunden sind, für C₃-C₇-Cycloalkyl oder C₅-C₇-Cycloalkenyl, in welchem gegebenenfalls ein oder zwei Ringglieder durch Sauerstoff und/oder Schwefel ersetzt sind und welches gegebenenfalls ein-, zwei-, drei- oder vierfach durch Methyl, jeweils einfach durch Ethyl, eine =CH₂-Gruppe, Methoxy oder Ethoxy substituiert ist,
A, B und das Kohlenstoffatom, an das sie gebunden sind, für eine =CH₂-Gruppe, für eine =CH₂-CH₃-Gruppe, für eine =CH₂-C₂H₅ -Gruppe oder für eine =N-OR⁹-Gruppe stehen,
G für Wasserstoff (a) oder für eine der Gruppen 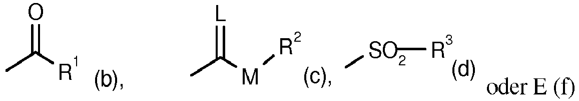 steht, in welchen
E für ein Metallionenäquivalent (Na⁺ oder K⁺) steht,
L für Sauerstoff oder Schwefel steht und
M für Sauerstoff oder Schwefel steht,
R¹ für C₁-C₆-Alkyl, C₂-C₆-Alkenyl, C₁-C₂-Alkoxy-C₁-alkyl, C₁-C₂-Alkylthio-C₁-alkyl oder gegebenenfalls einfach durch Fluor, Chlor, Methyl oder Methoxy substituiertes C₃-C₆-Cyclopropyl oder für einfach durch Chlor substituiertes C₁-C₄-Alkyl,
für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach bis dreifach durch Fluor substituiertes C₁-C₈-Alkyl, C₂-C₆-Alkenyl oder C₁-C₄-Alkoxy-C₂-C₃-alkyl, Phenyl oder Benzyl steht,
R³ für C₁-C₆-Alkyl oder für gegebenenfalls einfach durch Chlor oder C₁-C₄-Alkyl substituiertes Phenyl steht,
R⁹ für Wasserstoff, für jeweils gegebenenfalls durch Fluor substituiertes Methyl, Ethyl, Propyl, n-Butyl, i-Butyl, s-Butyl oder tert.-Butyl, für Allyl, Chlorallyl, Propinyl, Butinyl, Chlorbenzyl, Chlor-pyridylmethyl, CH(CH₃)-C≡CH, Cyclopropyl, Cyclobutyl, Cyclopentyl, CH₂Cyclopropyl, CH₂Cyclobutyl oder CH₂Cyclopentyl steht.

5. Verbindungen der Formel (I) gemäß Anspruch1, in welcher
X für Methyl, Ethyl oder Methoxy steht,
Y für Methyl oder Ethyl steht,
Z für Wasserstoff, Methyl oder Ethyl steht,
A, B und das Kohlenstoffatom an das sie gebunden sind, für C₅-C₇-Cycloalkyl oder C₇-Cycloalkenyl, in welchem gegebenenfalls zwei Ringglieder durch Sauerstoff und/oder Schwefel ersetzt sind und welches gegebenenfalls ein-, zwei-, oder vierfach durch Methyl, jeweils einfach durch Ethyl, eine =CH₂-Gruppe, Methoxy, Ethoxy oder Benzyloxy substituiert ist,
A, B und das Kohlenstoffatom, an das sie gebunden sind, für eine =CH₂-Gruppe, oder für eine =N-OR⁹-Gruppe stehen,
G für Wasserstoff (a) oder für eine der Gruppen 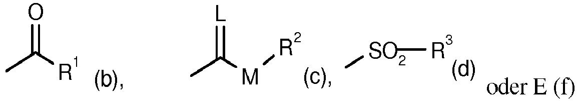 steht, in welchen
E für ein Metallionenäquivalent steht,
L für Sauerstoff steht und
M für Sauerstoff steht,
R¹ für C₁-C₆-Alkyl, oder C₁-C₂-Alkoxy-C₁-alkyl, oder für einfach durch Chlor substituiertes C₁-C₄-Alkyl,
für gegebenenfalls einfach durch Chlor, Methyl oder Methoxy substituiertes Phenyl steht,
R² für C₁-C₈-Alkyl steht,
R³ für Methyl, Phenyl oder p-Methylphenyl steht,
R⁹ für Wasserstoff, Methyl, i-Propyl, i-Butyl, tert.-Butyl, CH₂CF₃, Propinyl (CH₂-C≡CH), CH(CH₃)-C≡CH, Cyclopropyl, Cyclopentyl, CH₂Cyclopropyl, CH₂Cyclopentyl steht.

6. Verfahren zur Herstellung von Verbindungen der Formel (I) gemäß Anspruch 1, **dadurch gekennzeichnet, dass** man zum Erhalt von
(A) Verbindungen der Formel (I-a) 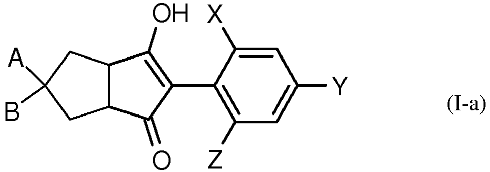 in welcher
A, B, X, Y und Z die oben angegebene Bedeutung haben,
Ketocarbonsäureester der Formel (II) 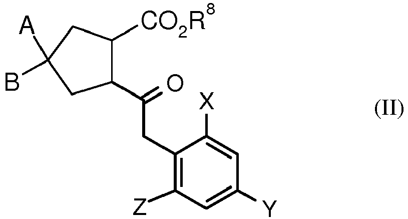 in welcher
A, B, X, Y und Z die oben angegebene Bedeutung haben, und
R⁸ für Alkyl steht,
gegebenenfalls in Gegenwart eines Verdünnungsmittels und in Gegenwart einer Base intramolekular cyclisiert,
(B) Verbindungen der oben gezeigten Formel (I-b), in welcher A, B, R¹, X, Y und Z die oben angegebenen Bedeutungen haben, Verbindungen der oben gezeigten Formel (I-a), in welcher A, B, X, Y und Z die oben angegebenen Bedeutungen haben, jeweils
(α) mit Säurehalogeniden der Formel (III) 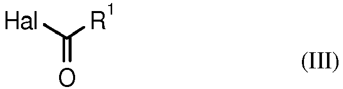 in welcher
R¹ die oben angegebene Bedeutung hat und
Hal für Halogen steht
oder
(β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(C) Verbindungen der oben gezeigten Formel (I-c), in welcher A, B, R², M, X, Y und Z die oben angegebenen Bedeutungen haben und L für Sauerstoff steht, Verbindungen der oben gezeigten Formel (I-a), in welcher A, B, X, Y und Z die oben angegebenen Bedeutungen haben, jeweils
mit Chlorameisensäureestern oder Chlorameisensäurethioestern der Formel (V)
R²-M-CO-Cl (V)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(D) Verbindungen der oben gezeigten Formel (I-c), in welcher A, B, R², M, X, Y und Z die oben angegebenen Bedeutungen haben und L für Schwefel steht, Verbindungen der oben gezeigten Formel (I-a), in welchen A, B, X, Y und Z die oben angegebenen Bedeutungen haben, jeweils
mit Chlormonothioameisensäureestern oder Chlordithioameisensäureestern der Formel (VI) 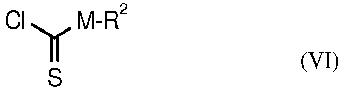 in welcher
M und R² die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formel (I-d), in welcher A, B, R³, X, Y und Z die oben angegebenen Bedeutungen haben, Verbindungen der oben gezeigten Formel (I-a), in welcher A, B, X, Y und Z die oben angegebenen Bedeutungen haben, jeweils mit Sulfonsäurechloriden der Formel (VII)
R³-SO₂-Cl (VII)
in welcher
R³ die oben angegebene Bedeutung hat,
gegebenenfalls in Gegenwart eines Verdünnungsmittels und gegebenenfalls in Gegenwart eines Säurebindemittels umsetzt,
(F) Verbindungen der oben gezeigten Formel (I-e), in welcher A, B, L, R⁴, R⁵, X, Y und Z die oben angegebenen Bedeutungen haben, Verbindungen der oben gezeigten Formel (I-a), in welchen A, B, X, Y und Z die oben angegebenen Bedeutungen haben, jeweils
mit Phosphorverbindungen der Formel (VIII) 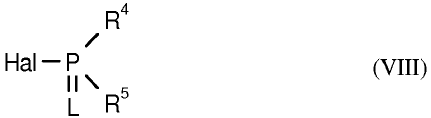 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(G) Verbindungen der oben gezeigten Formel (I-f), in welcher A, B, E, X, Y und Z die oben angegebenen Bedeutungen haben, Verbindungen der Formel (I-a), in welcher A, B, X, Y und Z die oben angegebenen Bedeutungen haben, jeweils
mit Metallverbindungen oder Aminen der Formeln (IX) oder (X)
Me(OR¹⁰)ₜ (IX) 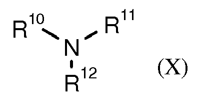
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(H) Verbindungen der oben gezeigten Formel (I-g), in welcher A, B, L, R⁶, R⁷, X, Y und Z die oben angegebenen Bedeutungen haben, Verbindungen der oben gezeigten Formel (I-a), in welcher A, B, X, Y und Z die oben angegebenen Bedeutungen haben, jeweils
(α) mit Isocyanaten oder Isothiocyanaten der Formel (XI)
R⁶-N=C=L (XI)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(ß) mit Carbamidsäurechloriden oder Thiocarbamidsäurechloriden der Formel (XII) 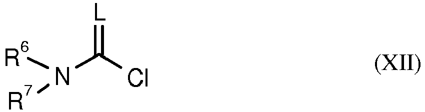 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

7. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

8. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

9. Verwendung von Verbindungen der Formel (I) gemäß Anspruch 1 zur Bekämpfung von tierischen Schädlingen und/oder unerwünschtem Pflanzenbewuchs.

10. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen und/oder unerwünschtem Pflanzenwuchs.

12. Mittel enthaltend einen wirksamen Gehalt an einer Wirkstoffkombination umfassend als Komponenten
(a') mindestens eine Verbindung der Formel (I), in welcher A, B, G, X, Y und Z die oben angegebene Bedeutung haben
und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14, S15;
S1) Verbindungen der Formel (S1), 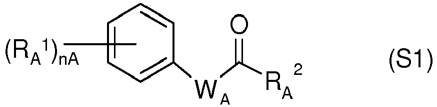 wobei die Symbole und Indizes folgende Bedeutungen haben:
n_{A} ist eine natürliche Zahl von 0 bis 5;
R_{A}¹ ist Halogen, (C₁-C₄)Alkyl, (C₁-C₄)Alkoxy, Nitro oder (C₁-C₄)Haloalkyl;
W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist,
m_{A} ist 0 oder 1;
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist;
R_{A}³ ist Wasserstoff oder ein unsubstituierter oder substituierter aliphatischer Kohlenwasserstoffrest;
R_{A}⁴ ist Wasserstoff, (C₁-C₆)Alkyl, (C₁-C₆)Alkoxy oder substituiertes oder unsubstituiertes Phenyl;
R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl.
S2) Chinolinderivate der Formel (S2), 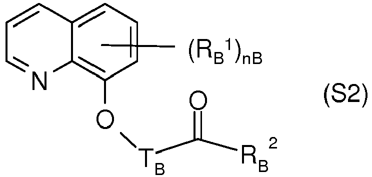 wobei die Symbole und Indizes folgende Bedeutungen haben:
R_{B}¹ ist Halogen, (C₁-C₄)Alkyl, (C₁-C₄)Alkoxy, Nitro oder (C₁-C₄)Haloalkyl;
n_{B} ist eine natürliche Zahl von 0 bis 5;
R_{B}² ist OR_{B}³, SR_{B}³ oder NR_{B}³R_{B}⁴ oder ein gesättigter
oder ungesättigter 3- bis 7-gliedriger Heterocyclus mit mindestens einem N-Atom und bis zu 3 Heteroatomen, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist;
R_{B}³ ist Wasserstoff oder ein unsubstituierter oder substituierter aliphatischer Kohlenwasserstoffrest;
R_{B}⁴ ist Wasserstoff, (C₁-C₆)Alkyl, (C₁-C₆)Alkoxy oder substituiertes oder unsubstituiertes Phenyl;
T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist.
S3) Verbindungen der Formel (S3) 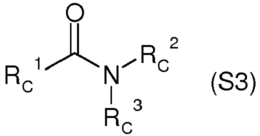 wobei die Symbole und Indizes folgende Bedeutungen haben:
R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl;
R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring.
S4) N-Acylsulfonamide der Formel (S4) und ihre Salze, 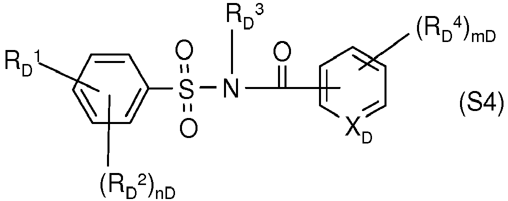 worin die Symbole und Indizes folgende Bedeutungen haben:
X_{D} ist CH oder N;
R_{D}¹ ist CO-NR_{D}⁵R_{D}⁶ oder NHCO-R_{D}⁷_{;}
R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)_{A}lkylcarbonyl;
R_{D}³ ist Wasserstoff, (C₁-C₄)Alkyl, (C₂-C₄)Alkenyl oder (C₂-C₄)Alkinyl;
R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkyl-sulfinyl, (C₁-C₄)Alkylsulfonyl, (C₁-C₄)Alkoxycarbonyl oder (C₁-C₄)Alkylcarbonyl;
R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
R_{D}⁵ und R_{D}⁶ gemeinsam mit dem dem sie tragenden Stickstoffatom einen Pyrrolidinyl- oder Piperidinyl-Rest bilden;
R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
n_{D} ist 0, 1 oder 2;
m_{D} ist 1 oder 2;
v_{D} ist 0, 1, 2 oder 3.
S5) Folgende Wirkstoffe aus der Klasse der Hydroxyaromaten und der aromatisch-aliphatischen Carbonsäurederivate (S5): 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure.
S6) Folgende Wirkstoffe aus der Klasse der 1,2-Dihydrochinoxalin-2-one (S6): 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on.
S7) Verbindungen der Formel (S7), 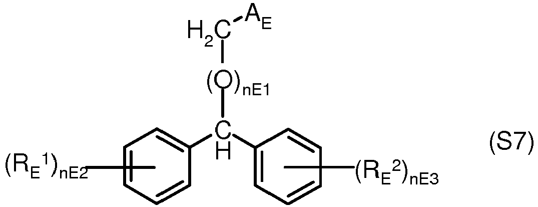 worin die Symbole und Indizes folgende Bedeutungen haben:
R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
A_{E} ist COOR_{E}³ oder COSR_{E}⁴
R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
n_{E}¹ ist 0 oder 1
n_{E}², n_{E}³ sind unabhängig voneinander 0, 1 oder 2.
S8) Verbindungen der Formel (S8),
R 2 worin
X_{F} CH oder N,
n_{F} für den Fall, dass X_{F}=N ist, eine ganze Zahl von 0 bis 4 und für den Fall, dass X_{F}=CH ist, eine ganze Zahl von 0 bis 5 ,
R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
R_{F}² Wasserstoff oder (C₁-C₄)Alkyl
R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
S9) Folgende aus der Klasse der 3-(5-Tetrazolylcarbonyl)-2-chinolone (S9): 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8).
S10) Verbindungen der Formeln (S10^{a}) oder (S10^{b}), 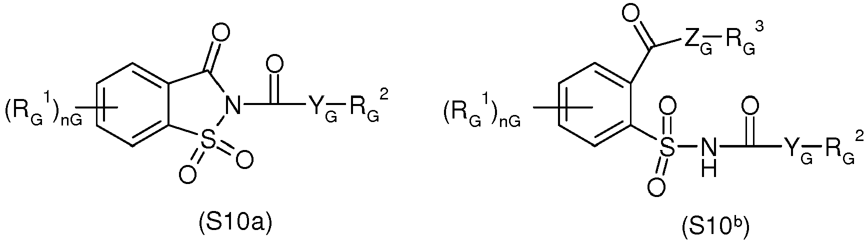 worin
R_{G}¹ Halogen, (C₁-C₄)Alkyl, Methoxy, Nitro, Cyano, CF₃, OCF₃
Y_{G,} Z_{G} unabhängig voneinander O oder S,
n_{G} eine ganze Zahl von 0 bis 4,
R_{G}² (C₁-C₁₆)Alkyl, (C₂-C₆)Alkenyl, (C₃-C₆)Cycloalkyl, Aryl; Benzyl, Halogenbenzyl,
R_{G}³ Wasserstoff oder (C₁-C₆)Alkyl bedeutet.
S11) Folgende Wirkstoffe vom Typ der Oxyimino-Verbindungen (S11), die als Saatbeizmittel bekannt sind:
"Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
"Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
"Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Folgende Wirkstoffe aus der Klasse der Isothiochromanone (S12): Methyl-[(3-oxo-1H-2-benzothiopyran-4(3H)-yliden)methoxy]acetate (CAS-Reg.Nr. 205121-04-6) (S12-1).
S13) Eine oder mehrere Verbindungen aus Gruppe (S13):
"Naphthalic anhydrid" (1,8-Naphthafindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist,
"MG-838" (CAS-Reg.Nr. 133993-74-5) (2-propenyl 1-oxa-4-azaspiro[4.5]decane-4-carbodithioate) (S13-6) der Firma Nitrokemia,
"Disulfoton" (O,O-Diethyl S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).
S14) Folgende Wirkstoffe, die neben einer herbiziden Wirkung gegen Schadpflanzen auch Safenerwirkung an Kulturpflanzen wie Reis aufweisen:
"Dimepiperate" oder "MY-93" (S-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-harnstoff), das als Safener für Reis gegen Schäden des Herbizids Imazosulfuron bekannt ist,
"Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Folgende Wirkstoffe, die vorrangig als Herbizide eingesetzt werden, jedoch auch Safenerwirkung auf Kulturpflanzen aufweisen:
(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba), 1-(Ethoxycarbonyl)ethyl-3,6-dichlor-2-methoxybenzoat (Lactidichlor-ethyl).

13. Mittel gemäß Anspruch 12, bei denen die Kulturpflanzen-Verträglichkeit verbessernde Verbindung Cloquintocet-mexyl ist.

14. Mittel gemäß Anspruch 12, bei denen die Kulturpflanzen-Verträglichkeit verbessernde Verbindung Mefenpyr-diethyl ist.

15. Mittel gemäß Anspruch 12, bei denen die Kulturpflanzen-Verträglichkeit verbessernde Verbindung Cyprosulfamid ist.

16. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

17. Verwendung eines Mittels gemäß Anspruch 12 zum Bekämpfen von unerwünschten Pflanzenwuchs.

18. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

19. Verbindungen der Formel (II) 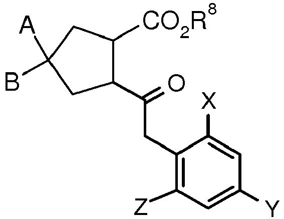 in welcher
A, B, X, Y, Z und R⁸ die oben angegebene Bedeutung haben.

20. Verbindungen der Formel (XIII) 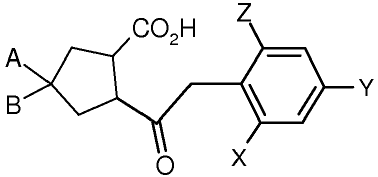 in welcher
A, B, X, Y, und Z die oben angegebene Bedeutung haben.

21. Verbindungen der Formel (XIV) 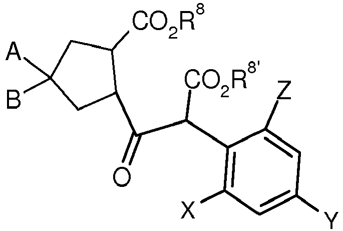 in welcher
A, B, X, Y, Z, R⁸ und R⁸ die oben angegebene Bedeutung haben.

22. Verbindungen der Formel (XIX) 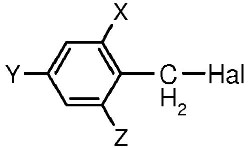 in welcher
X, Y, Z und Hal die in der Tabelle angegebenen Bedeutungen haben
| Hal | X | Y | Z |
|---|---|---|---|
| | | | |
| | | | |
| Cl | Cyclopropyl | CH₃ | C₂H₅ |
| Br | Cyclopropyl | CH₃ | C₂H₅ |

## Claims

1. Compounds of the formula (I) 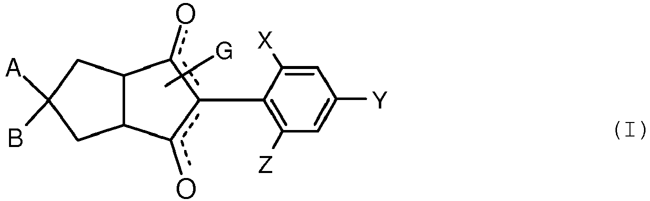 in which
X represents alkyl, cycloalkyl or alkoxy,
Y represents hydrogen, alkyl or alkoxy,
Z represents hydrogen, alkyl or cycloalkyl,
where
A and B together with the carbon atom to which they are attached represent a saturated or unsaturated unsubstituted or substituted cycle which optionally contains at least one heteroatom,
or
A and B together with the carbon atom to which they are attached represent a C₁-C₄-alkylene group or a =N-OR⁹ group,
G represents hydrogen (a) or represents one of the groups 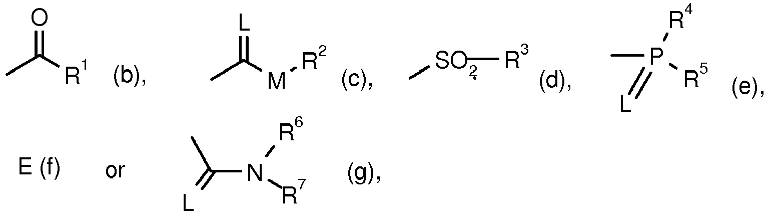 in which
E represents a metal ion equivalent or an ammonium ion,
L represents oxygen or sulphur,
M represents oxygen or sulphur,
R¹ represents in each case optionally halogen-substituted alkyl, alkenyl, alkoxyalkyl, alkylthioalkyl, polyalkoxyalkyl or optionally halogen-, alkyl- or alkoxy-substituted cycloalkyl which may be interrupted at least one heteroatom, in each case optionally substituted phenyl, phenylalkyl, hetaryl, phenoxyalkyl or hetaryloxyalkyl,
R² represents in each case optionally halogen-substituted alkyl, alkenyl, alkoxyalkyl, polyalkoxyalkyl or represents in each case optionally substituted cycloalkyl, phenyl or benzyl,
R³, R⁴ and R⁵ independently of one another represent in each case optionally halogen-substituted alkyl, alkoxy, alkylamino, dialkylamino, alkylthio, alkenylthio, cycloalkylthio or represent in each case optionally substituted phenyl, benzyl, phenoxy or phenylthio,
R⁶ and R⁷ independently of one another represent hydrogen, in each case optionally halogen-substituted alkyl, cycloalkyl, alkenyl, alkoxy, alkoxyalkyl, represent optionally substituted phenyl, represent optionally substituted benzyl, or together with the nitrogen atom to which they are attached represent a cycle which is optionally interrupted by oxygen or sulphur,
R⁹ represents hydrogen, represents in each case optionally substituted alkyl, cycloalkyl, CH₂-cycloalkyl, alkenyl, alkinyl, arylalkyl or hetarylalkyl.

2. Compounds of the formula (I) according to Claim 1 in which
X represents C₁-C₄-alkyl, C₃-C₆-cycloalkyl or C₁-C₄-alkoxy,
Y represents hydrogen, C₁-C₄-alkyl or C₁-C₄-alkoxy,
Z represents hydrogen, C₁-C₄-alkyl or C₃-C₆-cycloalkyl,
A, B and the carbon atom to which they are attached represent C₃-C₈-cycloalkyl or C₅-C₈-cycloalkenyl, in which optionally one or two ring members are replaced by oxygen and/or sulphur and which are optionally mono- to tetrasubstituted by C₁-C₈-alkyl, C₁-C₈-alkenyl, C₁-C₄-alkylene, C₁-C₈-halogenalkyl, C₁-C₆-alkoxy-C₁-C₄-alkyl, C₁-C₆-alkoxy, C₁-C₆-halogenalkoxy, hydroxyl, hydroxy-C₁-C₄-alkyl or benzyloxy, or a further fused-on C₃-C₈-cycloalkyl ring in which optionally one or two ring members are replaced by oxygen or sulphur, or which is optionally mono- or disubstituted by C₁-C₈-alkyl, or
A, B and the carbon atom to which they are attached represent a C₁-C₄-alkylene group or represent a =N-OR⁹ group,
G represents hydrogen (a) or represents one of the groups 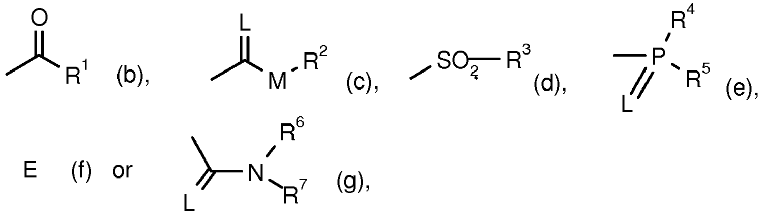 in which
E represents a metal ion equivalent or an ammonium ion,
L represents oxygen or sulphur, and
M represents oxygen or sulphur,
R¹ represents in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur,
represents optionally halogen-, cyano-, nitro-, C₁-C₆-alkyl-, C₁-C₆-alkoxy-, C₁-C₆-halogenalkyl-, C₁-C₆-halogenalkoxy-, C₁-C₆-alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆-halogenalkyl- or C₁-C₆-halogen-alkoxy-substituted phenyl-C₁-C₆-alkyl,
represents optionally halogen- or C₁-C₆-alkyl-substituted 5- or 6-membered hetaryl,
represents optionally halogen- or C₁-C₆-alkyl-substituted phenoxy-C₁-C₆-alkyl or
represents optionally halogen-, amino-or C₁-C₆-alkyl-substituted 5- or 6-membered hetaryloxy-C₁-C₆-alkyl,
R² represents in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl,
represents optionally halogen-, C₁-C₆-alkyl- or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-halogenalkyl- or C₁-C₆-halogenalkoxy-substituted phenyl or benzyl,
R3 represents optionally halogen-substituted C₁-C₈-alkyl or represents in each case optionally halogen-, C₁-C₆-alkyl-, C₁-C₆-alkoxy-, C₁-C₄-halogenalkyl-, C₁-C₄-halogenalkoxy-, cyano- or nitro-substituted phenyl or benzyl,
R⁴ and R⁵ independently of one another represent in each case optionally halogen-substituted C₁-C₈-alkyl, C₁-C₈-alkoxy, C₁-C₈-alkylamino, Di- (C₁-C₈-lkyl) amino, C₁-C₈-alkylthio, C₂-C₈-alkenylthio, C₃-C₇-Cycloalkyl-thio or represent in each case optionally halogen-, nitro-, cyano-, C₁-C₄-alkoxy-, C₁-C₄-halogenalkoxy-, C₁-C₄-alkylthio-, C₁-C₄-halo-genalkylthio-, C₁-C₄-alkyl- or C₁-C₄-halogen-alkyl-substituted phenyl, phenoxy or phenyl-thio,
R⁶ and R⁷ independently of one another represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈-alkyl, represent optionally halogen-, C₁-C₈-halogenalkyl-, C₁-C₈-alkyl- or C₁-C₈-alkoxy-substituted phenyl, optionally halogen-, C₁-C₈-alkyl-, C₁-C₈-halogenalkyl- or C₁-C₈-alkoxy-substituted benzyl or together represent an optionally C₁-C₄-alkyl-substituted C₃-C₆-alkylene radical in which optionally one carbon atom is replaced by oxygen or sulphur,
R⁹ represents hydrogen, represents C₁-C₆-alkyl, C₃-C₆-cycloalkyl, CH₂C₃-C₆-cycloalkyl, C₃-C₆-alkenyl, C₃-C₆-alkinyl, each of which is optionally mono- to pentasubstituted by halogen, or represents phenyl-C₁-C₂-alkyl or hetaryl-C₁-C₂-alkyl, each of which is optionally mono- to trisubstituted by alkyl, halogen, alkoxy, halogenalkyl or halogenalkoxy.

3. Compounds of the formula (I) according to Claim 1 in which
X represents methyl, ethyl, cyclopropyl, methoxy or ethoxy,
Y represents hydrogen, methyl or ethyl,
Z represents hydrogen, methyl, ethyl or cyclopropyl,
A, B and the carbon atom to which they are attached represent C₃-C₈-cycloalkyl or C₅-C₈-cycloalkenyl in which optionally one or two ring members are replaced by oxygen and/or sulphur and which is optionally mono- to tetrasubstituted by C₁-C₄-alkyl, C₁-C₄-alkenyl, a =CH₂ group, trifluoromethyl, C₁-C₄-alkoxy, C₁-C₄-alkoxy-C₁-C₂-alkyl or by one of the groups 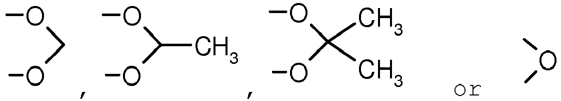
or
A, B and the carbon atom to which they are attached represent a C₁-C₄-alkylene group or represent a =N-OR⁹ group,
G represents hydrogen (a) or represents one of the groups 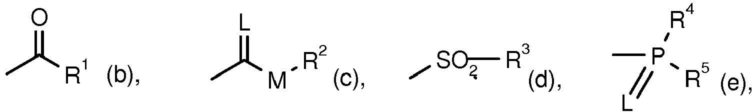 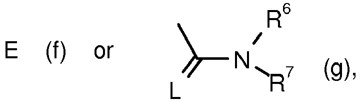
in which
E represents a metal ion equivalent or an ammonium ion,
L represents oxygen or sulphur and
M represents oxygen or sulphur,
R¹ represents C₁-C₈-alkyl, C₂-C₈-alkenyl, C₁-C₄-alkoxy-C₁-C₂-alkyl, C₁-C₄-alkylthio-C₁-C₂-alkyl, each of which is optionally mono- to trisubstituted by fluorine or chlorine, or represents C₃-C₆-cycloalkyl which is optionally mono- or disubstituted by fluorine, chlorine, C₁-C₂-alkyl or C₁-C₂-alkoxy and in which optionally one or two not directly adjacent ring members are replaced by oxygen,
represents phenyl which is optionally mono- or disubstituted by fluorine, chlorine, bromine, cyano, nitro, C₁-C₄-alkyl, C₁-C₄-alkoxy, C₁-C₂-haloalkyl or C₁-C₂-haloalkoxy,
R² represents C₁-C₈-alkyl, C₂-C₈-alkenyl or C₁-C₄-alkoxy-C₂-C₄-alkyl, each of which is optionally mono- to trisubstituted by fluorine,
represents C₃-C₆-cycloalkyl which is optionally monosubstituted by C₁-C₂-alkyl or C₁-C₂-alkoxy or
represents phenyl or benzyl, each of which is optionally mono- or disubstituted by fluorine, chlorine, bromine, cyano, nitro, C₁-C₄-alkyl, C₁-C₃-alkoxy, trifluoromethyl or trifluoromethoxy,
R³ represents C₁-C₆-alkyl which is optionally mono- to trisubstituted by fluorine or represents phenyl which is optionally monosubstituted by fluorine, chlorine, bromine, C₁-C₄-alkyl, C₁-C₄-alkoxy, trifluoromethyl, trifluoro-methoxy, cyano or nitro,
R⁴ represents C₁-C₆-alkyl, C₁-C₆-alkoxy, C₁-C₆-alkylamino, Di-(C₁-C₆-alkyl)amino, C₁-C₆-alkylthio, C₃-C₄-alkenylthio, C₃-C₆-cycloalkylthio, each of which is optionally mono- to trisubstituted by fluorine or chlorine, or represents phenyl, phenoxy or phenylthio, each of which is optionally monosubstituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or trifluoromethyl,
R⁵ represents C₁-C₆-alkylthio or C₁-C₆-alkoxy which is optionally monosubstituted by chlorine,
R⁶ represents hydrogen, C₁-C₆-alkyl, C₃-C₆-cycloalkyl, C₁-C₆-alkoxy, C₃-C₆-alkenyl, C₁-C₆-alkoxy-C₁-C₄-alkyl, represents phenyl which is optionally mono-substituted by fluorine, chlorine, bromine, trifluoromethyl, C₁-C₄-alkyl or C₁-C₄-alkoxy, represents benzyl which is optionally monosubstituted by fluorine, chlorine, bromine, C₁-C₄-alkyl, tri-fluoromethyl or C₁-C₄-alkoxy,
R⁷ represents C₁-C₆-alkyl, C₃-C₆-alkenyl or C₁-C₆-alkoxy-C₁-C₄-alkyl,
R⁶ and R⁷ together represent an optionally methyl- or ethyl-substituted C₄-C₅-alkylene radical in which optionally one methylene group is replaced by oxygen or sulphur,
R⁹ represents hydrogen, represents C₁-C₄-alkyl, C₃-C₆-cycloalkyl, CH₂-C₃-C₆-cyclo-alkyl, C₃-C₄-alkenyl, C₃-C₄-alkinyl, each of which is optionally mono- to trisubstituted by fluorine or chlorine, represents benzyl or pyridinylmethyl, each of which is optionally mono- or disubstituted by C₁-C₂-alkyl, C₁-C₂-alkoxy, fluorine, chlorine, bromine, trifluoromethyl or trifluoromethoxy.

4. Compounds of the formula (I) according to Claim 1 in which
X represents methyl, ethyl, cyclopropyl, methoxy or ethoxy,
Y represents hydrogen, methyl or ethyl,
Z represents hydrogen, methyl, ethyl or cyclopropyl,
A, B and the carbon atom to which they are attached represent C₃-C₇-cycloalkyl or C₅-C₇-cycloalkenyl in which optionally one or two ring members are replaced by oxygen and/or sulphur and which is optionally mono-, di-, tri- or tetrasubstituted by methyl, in each case monosubstituted by ethyl, a =CH₂ group, methoxy or ethoxy,
A, B and the carbon atom to which they are attached represent a =CH₂ group, represent a =CH₂-CH₃ group, represent a =CH₂-C₂H₅ group or represent a =N-OR⁹ group,
G represents hydrogen (a) or represents one of the groups 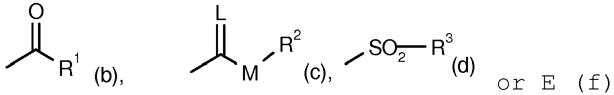
in which
E represents a metal ion equivalent (Na⁺ or K⁺),
L represents oxygen or sulphur and
M represents oxygen or sulphur,
R¹ represents C₁-C₆-alkyl, C₂-C₆-alkenyl, C₁-C₂-alkoxy-C₁-alkyl, C₁-C₂-alkylthio-C₁-alkyl or C₃-C₆-cyclopropyl which is optionally monosubstituted by fluorine, chlorine, methyl or methoxy or represents C₁-C₄-alkyl which is monosubstituted by chlorine,
represents phenyl which is optionally monosubstituted by fluorine, chlorine, bromine, cyano, nitro, methyl, methoxy, trifluoromethyl or trifluoromethoxy,
R² represents C₁-C₈-alkyl, C₂-C₆-alkenyl or C₁-C₄-alkoxy-C₂-C₃-alkyl, each of which is optionally mono- to trisubstituted by fluorine, represents phenyl or benzyl,
R³ represents C₁-C₆-alkyl or represents phenyl which is optionally monosubstituted by chlorine or C₁-C₄-alkyl,
R⁹ represents hydrogen, represents in each case optionally fluorine-substituted methyl, ethyl, propyl, n-butyl, isobutyl, s-butyl or tert-butyl, represents allyl, chloroallyl, propinyl, butinyl, chlorobenzyl, chloro-pyridylmethyl, CH(CH₃)-C≡CH, cyclopropyl, cyclobutyl, cyclopentyl, CH₂-cyclopropyl, CH₂-cyclobutyl or CH₂-cyclopentyl.

5. Compounds of the formula (I) according to Claim 1 in which
X represents methyl, ethyl or methoxy,
Y represents methyl or ethyl,
Z represents hydrogen, methyl or ethyl,
A, B and the carbon atom to which they are attached represent C₅-C₇-cycloalkyl or C₇-cycloalkenyl which optionally two ring members are replaced by oxygen and/or sulphur and which is optionally mono-, di- or tetrasubstituted by methyl, in each case monosubstituted by ethyl, a =CH₂ group, methoxy, ethoxy or benzyloxy,
A, B and the carbon atom to which they are attached represent a =CH₂ group or represent a =N-OR⁹ group,
G represents hydrogen (a) or represents one of the groups 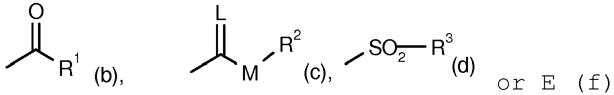
in which
E represents a metal ion equivalent,
L represents oxygen and
M represents oxygen,
R¹ represents C₁-C₆-alkyl or C₁-C₂-alkoxy-C₁-alkyl or C₁-C₄-alkyl which is monosubstituted by chlorine,
represents phenyl which is optionally monosubstituted by chlorine, methyl or methoxy,
R² represents C₁-C₈-alkyl,
R³ represents methyl, phenyl or p-methyl-phenyl,
R⁹ represents hydrogen, methyl, isopropyl, isobutyl, tert-butyl, CH₂CF₃, propinyl (CH₂-C≡CH), CH(CH₃)-C≡CH, cyclopropyl, cyclopentyl, CH₂-cyclopropyl, CH₂-cyclopentyl.

6. Process for preparing compounds of formula (I) according to Claim 1, **characterized in that**, to obtain
(A) Compounds of the formula (I-a) 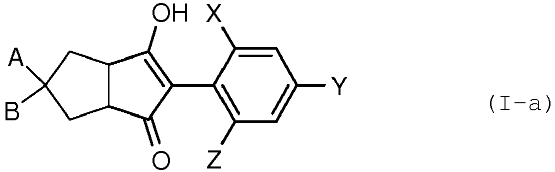 in which
A, B, X, Y and Z have the meaning given above,
ketocarboxylic esters of the formula (II) 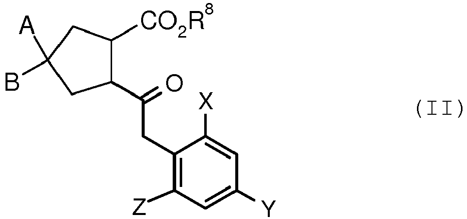 in which
A, B, X, Y and Z have the meaning given above, and
R⁸ represents alkyl,
are cyclised intramolecularly, if appropriate in the presence of a diluent and in the presence of a base,
(B) Compounds of the formula (I-b) shown above in which A, B, R¹, X, Y and Z have the meanings given above, compounds of the formula (I-a) shown above in which A, B, X, Y and Z have the meanings given above are in each case reacted
(α) with acid halides of the formula (III) 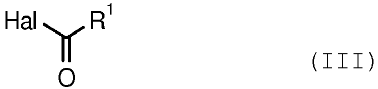 in which
R¹ has the meaning given above and
Hal represents halogen
or
(β) with carboxylic anhydrides of the formula (IV)
R¹-CO-O-CO-R¹ (IV)
in which
R¹ has the meaning given above,
if appropriate in the presence of a diluent and if appropriate in the presence of an acid binder;
(C) Compounds of the formula (I-c) shown above in which A, B, R², M, X, Y and Z have the meanings given above and L represents oxygen, compounds of the formula (I-a) shown above in which A, B, X, Y and Z have the meanings given above are in each case reacted with chloroformic esters or chloroformic thio esters of the formula (V)
R²-M-CO-Cl (V)
in which
R² and M have the meanings given above,
if appropriate in the presence of a diluent and if appropriate in the presence of an acid binder;
(D) Compounds of the formula (I-c) shown above in which A, B, R², M, X, Y and Z have the meanings given above and L represents sulphur, compounds of the formula (I-a) shown above in which A, B, X, Y and Z have the meanings given above are in each case reacted
with chloromonothioformic esters or chlorodithio-formic esters of the formula (VI) 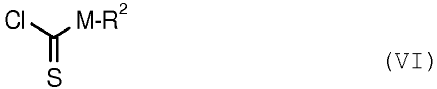 in which
M and R² have the meanings given above,
if appropriate in the presence of a diluent and if appropriate in the presence of an acid binder;
(E) Compounds of the formula (I-d) shown above in which A, B, R³, X, Y and Z have the meanings given above, compounds of the formula (I-a) shown above in which A, B, X, Y and Z have the meanings given above are in each case reacted,
with sulphonyl chlorides of the formula (VII)
R³-SO₂-Cl (VII)
in which
R³ has the meaning given above,
if appropriate in the presence of a diluent and if appropriate in the presence of an acid binder,
(F) Compounds of the formula (I-e) shown above in which A, B, L, R⁴, R⁵, X, Y and Z have the meanings given above, compounds of the formula (I-a) shown above in which A, B, X, Y and Z have the meanings given above are in each case reacted
with phosphorus compounds of the formula (VIII) 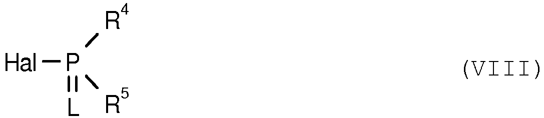 in which
L, R⁴ and R⁵ have the meanings given above and
Hal represents halogen,
if appropriate in the presence of a diluent and if appropriate in the presence of an acid binder,
(G) Compounds of the formula (I-f) shown above in which A, B, E, X, Y and Z have the meanings given above, compounds of the formula (I-a) in which A, B, X, Y and Z have the meanings given above are in each case reacted with metal compounds or amines of the formulae (IX) and (X) respectively,
Me(OR¹⁰)ₜ (IX) 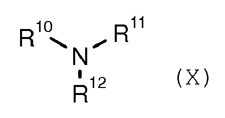
in which
Me represents a mono- or divalent metal,
t represents the number 1 or 2 and
R¹⁰, R¹¹, R¹² independently of one another represent hydrogen or alkyl,
if appropriate in the presence of a diluent,
(H) Compounds of the formula (I-g) shown above in which A, B, L, R⁶, R⁷, X, Y and Z have the meanings given above, compounds of the formula (I-a) shown above in which A, B, X, Y and Z have the meanings given above are in each case reacted
(α) with isocyanates or isothiocyanates of the formula (XI)
R⁶-N=C=L (XI)
in which
R⁶ and L have the meanings given above,
if appropriate in the presence of a diluent and if appropriate in the presence of a catalyst, or
(β) with carbamoyl chlorides or thiocarbamoyl chlorides of the formula (XII) 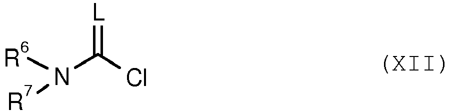 in which
L, R⁶ and R⁷ have the meanings given above,
if appropriate in the presence of a diluent and if appropriate in the presence of an acid binder.

7. Compositions for controlling pests and/or unwanted vegetation, **characterized in that** they comprise at least one compound of the formula (I) according to Claim 1.

8. Method for controlling animal pests and/or unwanted vegetation, **characterized in that** compounds of the formula (I) according to Claim 1 are allowed to act on pests, unwanted vegetation and/or their habitat.

9. Use of compounds of the formula (I) according to Claim 1 for controlling animal pests and/or unwanted vegetation.

10. Process for preparing compositions for controlling pests and/or unwanted vegetation, **characterized in that** compounds of the formula (I) according to Claim 1 are mixed with extenders and/or surfactants.

11. Use of compounds of the formula (I) according to Claim 1 for preparing compositions for controlling pests and/or unwanted vegetation.

12. Compositions, comprising an effective amount of an active compound combination comprising, as components,
(a') at least one compound of the formula (I) in which A, B, G, X, Y and Z have the meaning given above
and
(b') at least one crop plant compatibility-improving compound from the following group of compounds: S1, S2, S3, S4, S5, S6, S7, S8, S9, S10, S11, S12, S13, S14, S15;
S1) Compounds of the formula (S1) 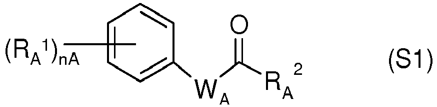 where the symbols and indices have the following meanings:
n_{A} is a natural number from 0 to 5;
R_{A}¹ is halogen, (C₁-C₄)-alkyl, (C₁-C₄)-alkoxy, nitro or (C₁-C₄)-haloalkyl;
W_{A} is an unsubstituted or substituted divalent heterocyclic radical from the group consisting of partially unsaturated or aromatic five-membered heterocycles having 1 to 3 hetero ring atoms from the group consisting of N and O, where at least one nitrogen atom and at most one oxygen atom is present in the ring,
m_{A} is 0 or 1;
R_{A}² is OR_{A}³, SR_{A}³ or NR_{A}³R_{A}⁴ or a saturated or unsaturated 3- to 7-membered heterocycle having at least one nitrogen atom and up to 3 heteroatoms which is attached via the nitrogen atom to the carbonyl group in (S1) and which is unsubstituted or substituted by radicals from the group consisting of (C₁-C₄)-alkyl, (C₁-C₄)-alkoxy and optionally substituted phenyl;
R_{A}³ is hydrogen or an unsubstituted or substituted aliphatic hydrocarbon radical;
R_{A}⁴ is hydrogen, (C₁-C₆)-alkyl, (C₁-C₆)-alkoxy or substituted or unsubstituted phenyl;
R_{A}⁵ is H, (C₁-C₈)-alkyl, (C₁-C₈)-haloalkyl, (C₁-C₄)-alkoxy-(C₁-C₈)-alkyl, cyano or COOR_{A}⁹ where R_{A}⁹ is hydrogen, (C₁-C₈)-alkyl, (C₁-C₈)-haloalkyl, (C₁-C₄)-alkoxy-(C₁-C₄)-alkyl, (C₁-C₆) -hydroxyalkyl, (C₃-C₁₂) -cycloalkyl or tri-(C₁-C₄)-alkylsilyl;
R_{A}⁶, R_{A}⁷, R_{A}⁸ are identical or different and are hydrogen, (C₁-C₈)-alkyl, (C₁-C₈)-haloalkyl, (C₃-C₁₂)-cycloalkyl or substituted or unsubstituted phenyl.
S2) Quinoline derivatives of the formula (S2) 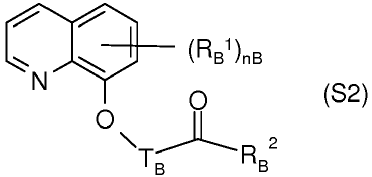 where the symbols and indices have the following meanings:
R_{B}¹ is halogen, (C₁-C₄)-alkyl, (C₁-C₄)-alkoxy, nitro or (C₁-C₄)-haloalkyl;
n_{B} is a natural number from 0 to 5;
R_{B}² is OR_{B}³, SR_{B}³ or NR_{B}³R_{B}⁴ or a saturated or unsaturated 3- to 7-membered heterocycle having at least one nitrogen atom and up to 3 heteroatoms which is attached via the nitrogen atom to the carbonyl group in (S2) and which is unsubstituted or substituted by radicals from the group consisting of (C₁-C₄)-alkyl, (C₁-C₄)-alkoxy and optionally substituted phenyl;
R_{B}³ is hydrogen or an unsubstituted or substituted aliphatic hydrocarbon radical;
R_{B}⁴ is hydrogen, (C₁-C₆)-alkyl, (C₁-C₆)-alkoxy or substituted or unsubstituted phenyl;
T_{B} is a (C₁- or C₂)-alkanediyl chain which is unsubstituted or substituted by one or two (C₁-C₄)-alkyl radicals or by [(C₁-C₃)-alkoxy]carbonyl.
S3) Compounds of the formula (S3) 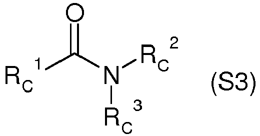 where the symbols and indices have the following meanings:
R_{c}¹ is (C₁-C₄)-alkyl, (C₁-C₄)-haloalkyl, (C₂-C₄)-alkenyl, (C₂-C₄)-haloalkenyl, (C₃-C₇)-cycloalkyl;
R_{c}², R_{c}³ are identical or different and are hydrogen, (C₁-C₄)-alkyl, (C₂-C₄) -alkenyl, (C₂-C₄)-alkynyl, (C₁-C₄)-haloalkyl, (C₂-C₄)-haloalkenyl, (C₁-C₄)-alkylcarbamoyl-(C₁-C₄)-alkyl, (C₂-C₄) -alkenylcarbamoyl- (C₁-C₄) -alkyl, (C₁-C₄)-alkoxy-(C₁-C₄)-alkyl, dioxolanyl-(C₁-C₄)-alkyl, thiazolyl, furyl, furylalkyl, thienyl, piperidyl, substituted or unsubstituted phenyl, or R_{C}² and R_{c}³ together form a substituted or unsubstituted heterocyclic ring.
S4) N-Acylsulphonamides of the formula (S4) and their salts 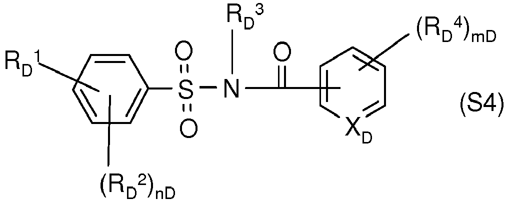 where the symbols and indices have the following meanings:
X_{D} is CH or N;
R_{D}¹ is CO-NR_{D}⁵R_{D}⁶ or NHCO-R_{D}⁷;
R_{D}² is halogen, (C₁-C₄)-haloalkyl, (C₁-C₄)-haloalkoxy, nitro, (C₁-C₄)-alkyl, (C₁-C₄)-alkoxy, (C₁-C₄)-alkylsulphonyl, (C₁-C₄)-alkoxycarbonyl or (C₁-C₄)-alkylcarbonyl;
R_{D}³ is hydrogen, (C₁-C₄)-alkyl, (C₂-C₄) -alkenyl or (C₂-C₄) -alkynyl;
R_{D}⁴ is halogen, nitro, (C₁-C₄)-alkyl, (C₁-C₄)-haloalkyl, (C₁-C₄)-haloalkoxy, (C₃-C₆)-cycloalkyl, phenyl, (C₁-C₄)-alkoxy, cyano, (C₁-C₄)-alkylthio, (C₁-C₄)-alkylsulphinyl, (C₁-C₄) -alkylsulphonyl, (C₁-C₄) -alkoxycarbonyl or (C₁-C₄)-alkylcarbonyl;
R_{D}⁵ is hydrogen, (C₁-C₆)-alkyl, (C₃-C₆)-cycloalkyl, (C₂-C₆)-alkenyl, (C₂-C₆) -alkynyl, (C₅-C₆)-cycloalkenyl, phenyl or 3- to 6-membered heterocyclyl which contains v_{D} heteroatoms from the group consisting of nitrogen, oxygen and sulphur, where the seven last-mentioned radicals are substituted by v_{D} substituents from the group consisting of halogen, (C₁-C₆)-alkoxy, (C₁-C₆) -haloalkoxy, (C₁-C₂)-alkylsulphinyl, (C₁-C₂)-alkylsulphonyl, (C₃-C₆) -cycloalkyl, (C₁-C₄) -alkoxycarbonyl, (C₁-C₄) -alkylcarbonyl and phenyl and, in the case of cyclic radicals, also (C₁-C₄)-alkyl and (C₁-C₄)-haloalkyl;
R_{D}⁶ is hydrogen, (C₁-C₆)-alkyl, (C₂-C₆) -alkenyl or (C₂-C₆)-alkynyl, where the three last-mentioned radicals are substituted by v_{D} radicals from the group consisting of halogen, hydroxy, (C₁-C₄)-alkyl, (C₁-C₄)-alkoxy and (C₁-C₄)-alkylthio, or
R_{D}⁵ and R_{D}⁶ together with the nitrogen atom carrying them form a pyrrolidinyl or piperidinyl radical;
R_{D}⁷ is hydrogen, (C₁-C₄)-alkylamino, di-(C₁-C₄)-alkylamino, (C₁-C₆)-alkyl, (C₃-C₆)-cycloalkyl, where the 2 last-mentioned radicals are substituted by v_{D} substituents from the group consisting of halogen, (C₁-C₄)-alkoxy, halo-(C₁-C₆)-alkoxy and (C₁-C₄)-alkylthio and, in the case of cyclic radicals, also (C₁-C₄)-alkyl and (C₁-C₄)-haloalkyl;
n_{D} is 0, 1 or 2;
m_{D} is 1 or 2;
v_{D} is 0, 1, 2 or 3.
S5) The following active compounds from the class of the hydroxyaromatics and aromatic-aliphatic carboxylic acid derivatives (S5):
ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 1,2-dihydro-2-oxo-6-trifluoromethylpyridine-3-carboxamide, 2,4-dichlorocinnamic acid.
S6) The following active compounds from the class of the 1,2-dihydroquinoxalin-2-ones (S6):
1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-[2-(diethylamino)ethyl]-6,7-dimethyl-3-thiophen-2-ylquinoxalin-2(1H)-one, 1-(2-methylsulphonylaminoethyl)-3-(2-thienyl)-1,2-dihydroquinoxalin-2-one.
S7) Compounds of the formula (S7) 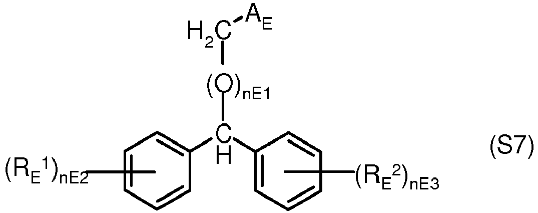 where the symbols and indices have the following meanings:
R_{E}¹, R_{E}² independently of one another are halogen, (C₁-C₄)-alkyl, (C₁-C₄)-alkoxy, (C₁-C₄)-haloalkyl, (C₁-C₄)-alkylamino, di- (C₁-C₄)-alkylamino, nitro;
A_{E} is COOR_{E}³ or COSR_{E}⁴
R_{E}³, R_{E}⁴ independently of one another are hydrogen, (C₁-C₄)-alkyl, (C₂-C₆)-alkenyl, (C₂-C₄)-alkynyl, cyanoalkyl, (C₁-C₄)-haloalkyl, phenyl, nitrophenyl, benzyl, halobenzyl, pyridinylalkyl or alkylammonium,
n_{E}¹ is 0 or 1;
n_{E}², n_{E}³ independently of one another are 0, 1 or 2.
S8) Compounds of the formula (S8), 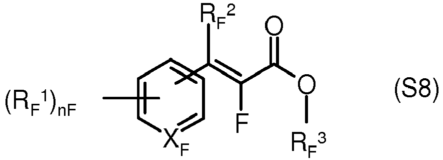
in which
X_{F} is CH or N,
n_{F} is, if X_{F}=N, an integer from 0 to 4 and
is, if X_{F}=CH, an integer from 0 to 5,
R_{F}¹ is halogen, (C₁-C₄)-alkyl, (C₁-C₄)-haloalkyl, (C₁-C₄)-alkoxy, (C₁-C₄)-haloalkoxy, nitro, (C₁-C₄)-alkylthio, (C₁-C₄)-alkylsulphonyl, (C₁-C₄)-alkoxycarbonyl, optionally substituted phenyl, optionally substituted phenoxy,
R_{F}² is hydrogen or (C₁-C₄)-alkyl,
R_{F}³ is hydrogen, (C₁-C₈)-alkyl, (C₂-C₄) -alkenyl, (C₂-C₄)-alkynyl or aryl, where each of the carbon-containing radicals mentioned above is unsubstituted or substituted by one or more, preferably by up to three, identical or different radicals from the group consisting of halogen and alkoxy; or salts thereof,
S9) The following from the class of the 3-(5-tetrazolylcarbonyl)-2-quinolones (S9):
1,2-dihydro-4-hydroxy-1-ethyl-3-(5-tetrazolylcarbonyl)-2-quinolone (CAS Reg. No.: 219479-18-2), 1,2-dihydro-4-hydroxy-1-methyl-3-(5-tetrazolylcarbonyl)-2-quinolone (CAS Reg. No.: 95855-00-8).
S10) Compounds of the formula (S10^{a}) or (S10^{b}) 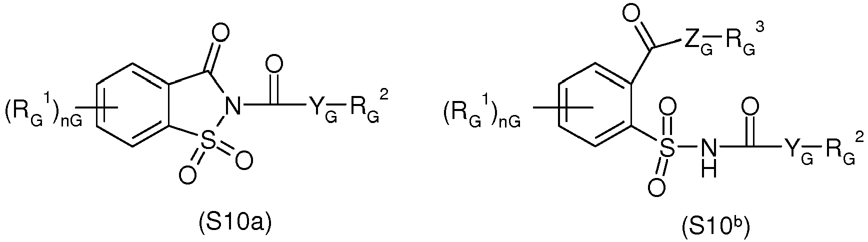 in which
R_{G}¹ is halogen, (C₁-C₄)-alkyl, methoxy, nitro, cyano, CF₃, OCF₃
Y_{G}, Z_{G} independently of one another are O or S,
n_{G} is an integer from 0 to 4,
R_{G}² is (C₁-C₁₆)-alkyl, (C₂-C₆) -alkenyl, (C₃-C₆)-cycloalkyl, aryl; benzyl, halobenzyl,
R_{G}³ is hydrogen or (C₁-C₆)-alkyl.
S11) The following active compounds of the type of the oxyimino compounds (S11), which are known as seed dressings:
"oxabetrinil" ((Z)-1,3-dioxolan-2-ylmethoxyimino(phenyl)acetonitrile) (S11-1), which is known as seed dressing safener for millet against metolachlor damage,
"fluxofenim" (1-(4-chlorophenyl)-2,2,2-trifluoro-1-ethanone O-(1,3-dioxolan-2-ylmethyl)oxime) (S11-2), which is known as seed dressing safener for millet against metolachlor damage, and
"cyometrinil" or "CGA-43089" ((Z)-cyanomethoxyimino(phenyl)acetonitrile) (S11-3), which is known as seed dressing safener for millet against metolachlor damage.
S12) The following active compounds from the class of the isothiochromanones (S12):
methyl [(3-oxo-1H-2-benzothiopyran-4(3H)-ylidene)methoxy]acetate (CAS Reg. No.: 205121-04-6) (S12-1).
S13) One or more compounds from group (S13):
"naphthalic anhydride" (1,8-naphthalenedicarboxylic anhydride) (S13-1), which is known as seed dressing safener for corn against thiocarbamate herbicide damage,
"fenclorim" (4,6-dichloro-2-phenylpyrimidine) (S13-2), which is known as safener for pretilachlor in sown rice,
"flurazole" (benzyl 2-chloro-4-trifluoromethyl-1,3-thiazole-5-carboxylate) (S13-3), which is known as seed dressing safener for millet against alachlor and metolachlor damage,
"CL-304415" (CAS Reg. No.: 31541-57-8) (4-carboxy-3,4-dihydro-2H-1-benzopyran-4-acetic acid) (S13-4) from American Cyanamid, which is known as safener for corn against imidazolinone damage,
"MG-191" (CAS Reg. No.: 96420-72-3) (2-dichloromethyl-2-methyl-1,3-dioxolane) (S13-5) from Nitrokemia, which is known as safener for corn,
"MG-838" (CAS Reg. No.: 133993-74-5) (2-propenyl 1-oxa-4-azaspiro[4.5]decane-4-carbodithioate) (S13-6) from Nitrokemia,
"disulfoton" (O,O-diethyl S-2-ethylthioethyl phosphorodithioate) (S13-7),
"dietholate" (O,O-diethyl 0-phenyl phosphorothioate) (S13-8),
"mephenate" (4-chlorophenyl methylcarbamate) (S13-9).
S14) The following active compounds which, besides a herbicidal effect against harmful plants, also have a safener effect on crop plants such as rice:
"dimepiperate" or "MY-93" (*S*-1-methyl-1-phenylethyl piperidine-1-carbothioate), which is known as safener for rice against molinate herbicide damage,
"daimuron" or "SK 23" (1-(1-methyl-1-phenylethyl)-3-p-tolylurea), which is known as safener for rice against imazosulphuron herbicide damage,
"cumyluron" = "JC-940" (3-(2-chlorophenylmethyl)-1-(1-methyl-1-phenylethyl)urea, see JP-A-60087254), which is known as safener for rice against some herbicide damage,
"methoxyphenone" or "NK 049" (3,3'-dimethyl-4-methoxybenzophenone), which is known as safener for rice against some herbicide damage,
"CSB" (1-bromo-4-(chloromethylsulphonyl)-benzene) from Kumiai, (CAS Reg. No. 54091-06-4), which is known as safener against some herbicide damage in rice.
S15) The following active compounds which are primarily used as herbicides, but also have safener effect on crop plants:
(2,4-dichlorophenoxy)acetic acid (2,4-D),
(4-chlorophenoxy)acetic acid,
(R,S)-2-(4-chloro-o-tolyloxy)propionic acid (mecoprop),
4-(2,4-dichlorophenoxy)butyric acid (2,4-DB),
(4-chloro-o-tolyloxy)acetic acid (MCPA),
4-(4-chloro-o-tolyloxy)butyric acid,
4-(4-chlorophenoxy)butyric acid,
3,6-dichloro-2-methoxybenzoic acid (dicamba),
1-(ethoxycarbonyl)ethyl 3,6-dichloro-2-methoxybenzoate (lactidichlor-ethyl).

13. Compositions according to Claim 12 in which the crop plant compatibility-improving compound is cloquintocet-mexyl.

14. Compositions according to Claim 12 in which the crop plant compatibility-improving compound is mefenpyr-diethyl.

15. Compositions according to Claim 12 in which the crop plant compatibility-improving compound is cyprosulfamide.

16. Method for controlling unwanted vegetation, **characterized in that** a composition according to Claim 12 is allowed to act on the plants or their surroundings.

17. Use of a composition according to Claim 12 for controlling unwanted vegetation.

18. Method for controlling unwanted vegetation, **characterized in that** a compound of the formula (I) according to Claim 1 and the crop plant compatibility-improving compound according to Claim 12 are allowed to act separately in close temporal succession on the plants or their surroundings.

19. Compounds of the formula (II) 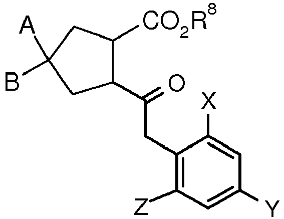 in which
A, B, X, Y, Z and R⁸ have the meanings given above.

20. Compounds of the formula (XIII) 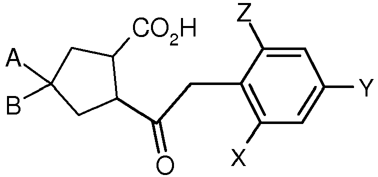 in which
A, B, X, Y, and Z have the meanings given above.

21. Compounds of the formula (XIV) 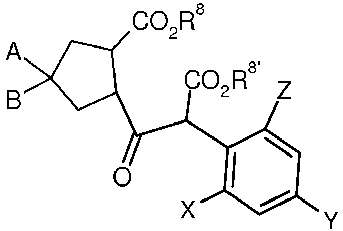 in which
A, B, X, Y, Z, R⁸ and R⁸ have the meanings given above.

22. Compounds of the formula (XIX) 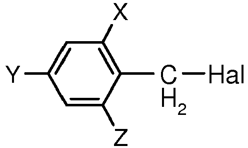 in which
X, Y, Z and Hal have the meanings given in the table
| Hal | X | Y | Z |
|---|---|---|---|
| | | | |
| | | | |
| Cl | cyclopropyl | CH₃ | C₂H₅ |
| Br | cyclopropyl | CH₃ | C₂H₅ |

## Revendications

1. Composés de formule (I) 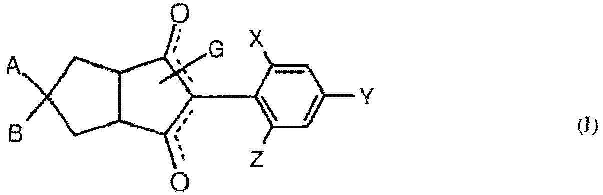 dans lesquels
X représente alkyle, cycloalkyle ou alcoxy,
Y représente hydrogène, alkyle ou alcoxy,
Z représente hydrogène, alkyle ou cycloalkyle,
A et B représentent ensemble avec l'atome de carbone auquel ils sont reliés un cycle saturé ou insaturé, contenant éventuellement au moins un hétéroatome, non substitué ou substitué,
ou
A et B représentent ensemble avec l'atome de carbone auquel ils sont reliés un groupe alkylène en C₁-C₄ ou un groupe =N-OR⁹,
G représente l'hydrogène (a) ou un des groupes 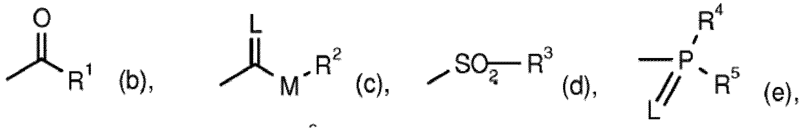 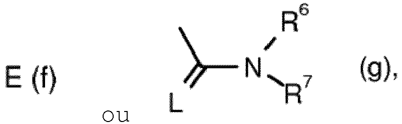
E représentant un équivalent d'ion métallique ou un ion ammonium,
L représentant oxygène ou soufre,
M représentant oxygène ou soufre,
R¹ représentant alkyle, alcényle, alcoxyalkyle, alkylthioalkyle, polyalcoxyalkyle, chacun éventuellement substitué par halogène ; ou cycloalkyle éventuellement substitué par halogène, alkyle ou alcoxy, qui peut être interrompu par au moins un hétéroatome ; phényle, phénylalkyle, hétaryle, phénoxyalkyle ou hétaryloxyalkyle, chacun éventuellement substitué,
R² représentant alkyle, alcényle, alcoxyalkyle, polyalcoxyalkyle, chacun éventuellement substitué par halogène ; ou cycloalkyle, phényle ou benzyle, chacun éventuellement substitué,
R³, R⁴ et R⁵ représentant indépendamment les uns des autres alkyle, alcoxy, alkylamino, dialkylamino, alkylthio, alcénylthio, cycloalkylthio, chacun éventuellement substitué par halogène; ou phényle, benzyle, phénoxy ou phénylthio, chacun éventuellement substitué,
R⁶ et R⁷ représentent indépendamment l'un de l'autre hydrogène ; alkyle, cycloalkyle, alcényle, alcoxy, alcoxyalkyle, chacun éventuellement substitué par halogène ; phényle éventuellement substitué ; benzyle éventuellement substitué, ou représentent ensemble avec l'atome N auquel ils sont reliés un cycle éventuellement interrompu par oxygène ou soufre,
R⁹ représente hydrogène; alkyle, cycloalkyle, CH₂-cycloalkyle, alcényle, alcynyle, arylalkyle ou hétarylalkyle, chacun éventuellement substitué.

2. Composés de formule (I) selon la revendication 1, dans lesquels
X représente alkyle en C₁-C₄, cycloalkyle en C₃-C₆ ou alcoxy en C₁-C₄,
Y représente hydrogène, alkyle en C₁-C₄ ou alcoxy en C₁-C₄,
Z représente hydrogène, alkyle en C₁-C₄ ou cycloalkyle en C₃-C₆,
A, B et l'atome de carbone auquel ils sont reliés représentent un cycloalkyle en C₃-C₈ ou cycloalcényle en C₅-C₈, dans lesquels un ou deux éléments de cycle sont éventuellement remplacés par oxygène et/ou soufre, et qui sont éventuellement substitués une à quatre fois par alkyle en C₁-C₈, alcényle en C₁-C₈, alkylène en C₁-C₄, halogénoalkyle en C₁-C₈, alcoxy en C₁-C₆-alkyle en C₁-C₄, alcoxy en C₁-C₆, halogénoalcoxy en C₁-C₆, hydroxy, hydroxy-alkyle en C₁-C₄ ou benzyloxy, ou un cycle cycloalkyle en C₃-C₈ condensé, dans lequel un ou deux éléments de cycle sont éventuellement remplacés par oxygène ou soufre, ou qui est éventuellement substitué une ou deux fois par alkyle en C₁-C₈, ou
A, B et l'atome de carbone auquel ils sont reliés représentent un groupe alkylène en C₁-C₄ ou un groupe =N-OR⁹,
G représente l'hydrogène (a) ou un des groupes 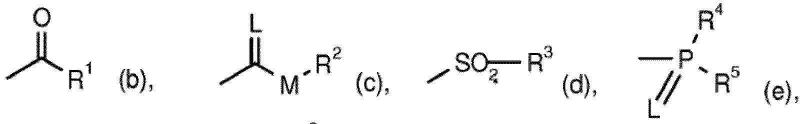 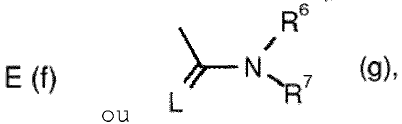
E représentant un équivalent d'ion métallique ou un ion ammonium,
L représentant oxygène ou soufre, et
M représentant oxygène ou soufre,
R¹ représentant alkyle en C₁-C₂₀, alcényle en C₂-C₂₀, alcoxy en C₁-C₈-alkyle en C₁-C₈, alkylthio en C₁-C₈-alkyle en C₁-C₈, poly-alcoxy en C₁-C₈-alkyle en C₁-C₈, chacun éventuellement substitué par halogène; ou cycloalkyle en C₃-C₈ éventuellement substitué par halogène, alkyle en C₁-C₆ ou alcoxy en C₁-C₆, dans lequel un ou plusieurs éléments de cycle non directement voisins sont éventuellement remplacés par oxygène et/ou soufre,
phényle éventuellement substitué par halogène, cyano, nitro, alkyle en C₁-C₆, alcoxy en C₁-C₆, halogénoalkyle en C₁-C₆, halogénoalcoxy en C₁-C₆, alkylthio en C₁-C₆ ou alkylsulfonyle en C₁-C₆,
phényl-alkyle en C₁-C₆ éventuellement substitué par halogène, nitro, cyano, alkyle en C₁-C₆, alcoxy en C₁-C₆, halogénoalkyle en C₁-C₆ ou halogénoalcoxy en C₁-C₆,
hétaryle à 5 ou 6 éléments éventuellement substitué par halogène ou alkyle en C₁-C₆,
phénoxy-alkyle en C₁-C₆ éventuellement substitué par halogène ou alkyle en C₁-C₆ ou
hétaryloxy-alkyle en C₁-C₆ à 5 ou 6 éléments éventuellement substitué par halogène, amino ou alkyle en C₁-C₆,
R² représentant alkyle en C₁-C₂₀, alcényle en C₂-C₂₀, alcoxy en C₁-C₈-alkyle en C₂-C₈, poly-alcoxy en C₁-C₈-alkyle en C₂-C₈, chacun éventuellement substitué par halogène,
cycloalkyle en C₃-C₈ éventuellement substitué par halogène, alkyle en C₁-C₆ ou alcoxy en C₁-C₆, ou
phényle ou benzyle, chacun éventuellement substitué par halogène, cyano, nitro, alkyle en C₁-C₆, alcoxy en C₁-C₆, halogénoalkyle en C₁-C₆ ou halogénoalcoxy en C₁-C₆,
R³ représente alkyle en C₁-C₈ éventuellement substitué par halogène ; ou phényle ou benzyle, chacun éventuellement substitué par halogène, alkyle en C₁-C₆, alcoxy en C₁-C₆, halogénoalkyle en C₁-C₄, halogénoalcoxy en C₁-C₄, cyano ou nitro,
R⁴ et R⁵ représentent indépendamment l'un de l'autre alkyle en C₁-C₈, alcoxy en C₁-C₈, alkylamino en C₁-C₈, di-(alkyle en C₁-C₈)-amino, alkylthio en C₁-C₈, alcénylthio en C₂-C₈, cycloalkylthio en C₃-C₇, chacun éventuellement substitué par halogène ; ou phényle, phénoxy ou phénylthio, chacun éventuellement substitué par halogène, nitro, cyano, alcoxy en C₁-C₄, halogénoalcoxy en C₁-C₄, alkylthio en C₁-C₄, halogénoalkylthio en C₁-C₄, alkyle en C₁-C₄ ou halogénoalkyle en C₁-C₄,
R⁶ et R⁷ représentent indépendamment l'un de l'autre hydrogène ; alkyle en C₁-C₈, cycloalkyle en C₃-C₈, alcoxy en C₁-C₈, alcényle en C₃-C₈, alcoxy en C₁-C₈-alkyle en C₁-C₈, chacun éventuellement substitué par halogène ; phényle éventuellement substitué par halogène, halogénoalkyle en C₁-C₈, alkyle en C₁-C₈ ou alcoxy en C₁-C₈; benzyle éventuellement substitué par halogène, alkyle en C₁-C₈, halogénoalkyle en C₁-C₈ ou alcoxy en C₁-C₈, ou représentent ensemble un radical alkylène en C₃-C₆ éventuellement substitué par alkyle en C₁-C₄, dans lequel un atome de carbone est éventuellement remplacé par oxygène ou soufre,
R⁹ représente hydrogène ; alkyle en C₁-C₆, cycloalkyle en C₃-C₆, CH₂-cycloalkyle en C₃-C₆, alcényle en C₃-C₆, alcynyle en C₃-C₆, chacun éventuellement substitué une à cinq fois avec halogène ; ou phényl-alkyle en C₁-C₂ ou hétaryl-alkyle en C₁-C₂, chacun éventuellement substitué une à trois fois par alkyle, halogène, alcoxy, halogénoalkyle ou halogénoalcoxy.

3. Composés de formule (I) selon la revendication 1, dans lesquels
X représente méthyle, éthyle, cyclopropyle, méthoxy ou éthoxy,
Y représente hydrogène, méthyle ou éthyle,
Z représente hydrogène, méthyle, éthyle ou cyclopropyle,
A, B et l'atome de carbone auquel ils sont reliés représentent un cycloalkyle en C₃-C₈ ou cycloalcényle en C₅-C₈, dans lesquels ou deux éléments de cycle sont éventuellement remplacés par oxygène et/ou soufre, et qui sont éventuellement substitués une à quatre fois par alkyle en C₁-C₄, alcényle en C₁-C₄, un groupe =CH₂, trifluorométhyle, alcoxy en C₁-C₄, alcoxy en C₁-C₄-alkyle en C₁-C₂, ou avec un des groupes 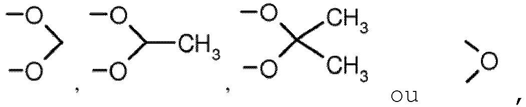 ou
A, B et l'atome de carbone auquel ils sont reliés représentent un groupe alkylène en C₁-C₄ ou un groupe =N-OR⁹,
G représente l'hydrogène (a) ou un des groupes 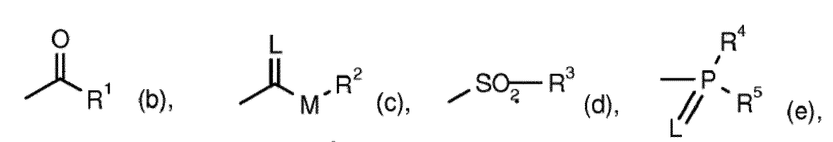 E (f) ou 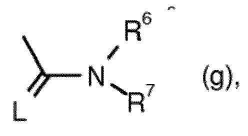
E représentant un équivalent d'ion métallique ou un ion ammonium,
L représentant oxygène ou soufre,
M représentant oxygène ou soufre,
R¹ représentant alkyle en C₁-C₈, alcényle en C₂-C₈,
alcoxy en C₁-C₄-alkyle en C₁-C₂, alkylthio en C₁-C₄-alkyle en C₁-C₂, chacun éventuellement substitué une à trois fois par fluor ou chlore ; ou cycloalkyle en C₃-C₆ éventuellement substitué une à deux fois par fluor, chlore, alkyle en C₁-C₂ ou alcoxy en C₁-C₂, dans lequel un ou deux éléments de cycle non directement voisins sont éventuellement remplacés par oxygène,
phényle éventuellement substitué une à deux fois par fluor, chlore, brome, cyano, nitro, alkyle en C₁-C₄, alcoxy en C₁-C₄, halogénoalkyle en C₁-C₂ ou halogénoalcoxy en C₁-C₂,
R² représentant alkyle en C₁-C₈, alcényle en C₂-C₈ ou alcoxy en C₁-C₄-alkyle en C₂-C₄, chacun éventuellement substitué une à trois fois par fluor,
cycloalkyle en C₃-C₆ éventuellement substitué une fois par alkyle en C₁-C₂ ou alcoxy en C₁-C₂, ou
phényle ou benzyle, chacun éventuellement substitué une à deux fois par fluor, chlore, brome, cyano, nitro, alkyle en C₁-C₄, alcoxy en C₁-C₃, trifluorométhyle ou trifluorométhoxy,
R³ représente alkyle en C₁-C₆ éventuellement substitué une à trois fois par fluor ; ou phényle éventuellement substitué une fois par fluor, chlore, brome, alkyle en C₁-C₄, alcoxy en C₁-C₄, trifluorométhyle, trifluorométhoxy, cyano ou nitro,
R⁴ représente alkyle en C₁-C₆, alcoxy en C₁-C₆, alkylamino en C₁-C₆, di-(alkyle en C₁-C₆)-amino, alkylthio en C₁-C₆, alcénylthio en C₃-C₄, cycloalkylthio en C₃-C₆, chacun éventuellement substitué une à trois fois par fluor ou chlore ; ou phényle, phénoxy ou phénylthio, chacun éventuellement substitué une fois par fluor, chlore, brome, nitro, cyano, alcoxy en C₁-C₃, halogénoalcoxy en C₁-C₃, alkylthio en C₁-C₃, halogénoalkylthio en C₁-C₃, alkyle en C₁-C₃ ou trifluorométhyle,
R⁵ représente alcoxy en C₁-C₆ ou alkylthio en C₁-C₆, éventuellement substitué une fois par chlore,
R⁶ représente hydrogène, alkyle en C₁-C₆, cycloalkyle en C₃-C₆, alcoxy en C₁-C₆, alcényle en C₃-C₆, alcoxy en C₁-C₆-alkyle en C₁-C₄; phényle éventuellement substitué une fois par fluor, chlore, brome, trifluorométhyle, alkyle en C₁-C₄ ou alcoxy en C₁-C₄; benzyle éventuellement substitué une fois par fluor, chlore, brome, alkyle en C₁-C₄, trifluorométhyle ou alcoxy en C₁-C_{4,}
R⁷ représente alkyle en C₁-C₆, alcényle en C₃-C₆ ou alcoxy en C₁-C₆-alkyle en C₁-C₄,
R⁶ et R⁷ représentent ensemble un radical alkylène en C₄-C₅ éventuellement substitué par méthyle ou éthyle, dans lequel un groupe méthylène est éventuellement remplacé par oxygène ou soufre,
R⁹ représente hydrogène ; alkyle en C₁-C₄, cycloalkyle en C₃-C₆, CH₂-cycloalkyle en C₃-C₆, alcényle en C₃-C₄, alcynyle en C₃-C₄, chacun éventuellement substitué une à trois fois par fluor ou chlore ; benzyle ou
pyridinylméthyle, chacun éventuellement substitué une à deux fois par alkyle en C₁-C₂, alcoxy en C₁-C₂, fluor, chlore, brome, trifluorométhyle ou trifluorométhoxy.

4. Composés de formule (I) selon la revendication 1, dans lesquels
X représente méthyle, éthyle, cyclopropyle, méthoxy ou éthoxy,
Y représente hydrogène, méthyle ou éthyle,
Z représente hydrogène, méthyle, éthyle ou cyclopropyle,
A, B et l'atome de carbone auquel ils sont reliés représentent un cycloalkyle en C₃-C₇ ou cycloalcényle en C₅-C₇, dans lesquels ou deux éléments de cycle sont éventuellement remplacés par oxygène et/ou soufre, et qui sont éventuellement substitués une, deux, trois ou quatre fois par méthyle, à chaque fois une fois par éthyle, un groupe =CH₂, méthoxy ou éthoxy, A, B et l'atome de carbone auquel ils sont reliés représentent un groupe =CH₂, un groupe =CH₂-CH₃, un groupe =CH₂-C₂H₅ ou un groupe =N-OR⁹,
G représente l'hydrogène (a) ou un des groupes 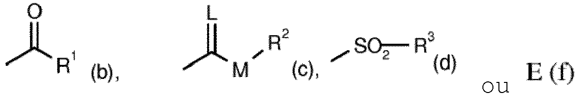
E représentant un équivalent d'ion métallique (Na⁺ ou K⁺),
L représentant oxygène ou soufre, et
M représentant oxygène ou soufre,
R¹ représentant alkyle en C₁-C₆, alcényle en C₂-C₆, alcoxy en C₁-C₂-alkyle en C₁, alkylthio en C₁-C₂-alkyle en C₁ ; ou cyclopropyle en C₃-C₆ éventuellement substitué une fois par fluor, chlore, méthyle ou méthoxy ; ou alkyle en C₁-C₄ substitué une fois par chlore,
phényle éventuellement substitué une fois par fluor, chlore, brome, cyano, nitro, méthyle, méthoxy, trifluorométhyle ou trifluorométhoxy,
R² représentant alkyle en C₁-C₈, alcényle en C₂-C₆ ou alcoxy en C₁-C₄-alkyle en C₂-C₃, chacun éventuellement substitué une à trois fois par fluor ; phényle ou benzyle
R³ représente alkyle en C₁-C₆ ; ou phényle éventuellement substitué une fois par chlore ou alkyle en C₁-C₄,
R⁹ représente hydrogène ; méthyle, éthyle, propyle, n-butyle, i-butyle, s-butyle ou tert.-butyle, chacun éventuellement substitué par fluor ; allyle, chloroallyle, propinyle, butinyle, chlorobenzyle, chloro-pyridylméthyle, CH(CH3)-C≡CH, cyclopropyle, cyclobutyle, cyclopentyle, CH₂-cyclopropyle, CH₂-cyclobutyle ou CH₂-cyclopentyle.

5. Composés de formule (I) selon la revendication 1, dans lesquels
X représente méthyle, éthyle ou méthoxy,
Y représente méthyle ou éthyle,
Z représente hydrogène, méthyle ou éthyle, A, B et l'atome de carbone auquel ils sont reliés représentent un cycloalkyle en C₅-C₇ ou cycloalcényle en C₇, dans lesquels deux éléments de cycle sont éventuellement remplacés par oxygène et/ou soufre, et qui sont éventuellement substitués une, deux ou quatre fois par méthyle, à chaque fois une fois par éthyle, un groupe =CH₂, méthoxy, éthoxy ou benzyloxy, A, B et l'atome de carbone auquel ils sont reliés représentent un groupe =CH₂ ou un groupe =N-OR⁹,
G représente l'hydrogène (a) ou un des groupes 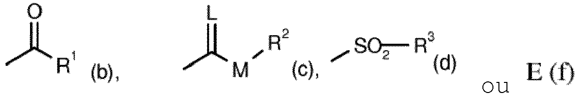
E représentant un équivalent d'ion métallique,
L représentant oxygène et
M représentant oxygène,
R¹ représentant alkyle en C₁-C₆ ou alcoxy en C₁-C₂-alkyle en C₁, ou alkyle en C₁-C₄ substitué une fois par chlore,
phényle éventuellement substitué une fois par chlore, méthyle ou méthoxy,
R² représentant alkyle en C₁-C₈,
R³ représente méthyle, phényle ou p-méthylphényle,
R⁹ représente hydrogène, méthyle, i-propyle, i-butyle, tert.-butyle, CH₂CF₃, propinyle, (CH₂-C≡CH), CH(CH₃)-C≡CH, cyclopropyle, cyclopentyle, CH₂-cyclopropyle, CH₂-cyclopentyle.

6. Procédé de fabrication de composés de formule (I) selon la revendication 1, **caractérisé en ce que**, pour l'obtention de
(A) des composés de formule (I-a) 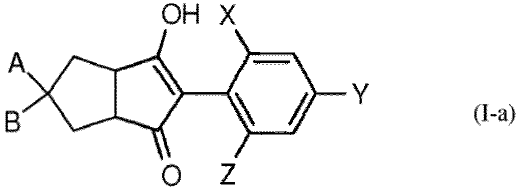 dans laquelle
A, B, X, Y et Z ont la signification indiquée précédemment,
des esters d'acide cétocarboxylique de formule (II) 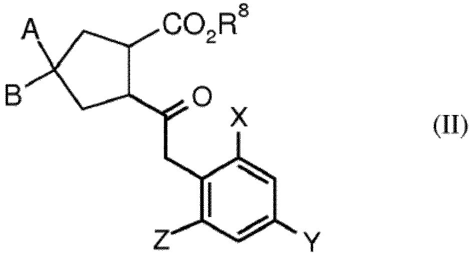 dans laquelle
A, B, X, Y et Z ont la signification indiquée précédemment, et
R⁸ représente alkyle,
sont cyclisés intramoléculairement éventuellement en présence d'un diluant et en présence d'une base,
(B) des composés de la formule (I-b) représentée précédemment, dans laquelle A, B, R¹, X, Y et Z ont les significations indiquées précédemment, des composés de la formule (I-a) représentée précédemment, dans laquelle A, B, X, Y et Z ont les significations indiquées précédemment, sont mis en réaction respectivement
(α) avec des halogénures d'acide de formule (III) 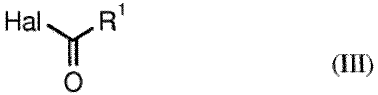 dans laquelle
R¹ a la signification indiquée précédemment, et
Hal représente halogène,
ou
(β) avec des anhydrides d'acide carboxylique de formule (IV)
R¹-CO-O-CO-R¹ (IV)
dans laquelle
R¹ a la signification indiquée précédemment, éventuellement en présence d'un diluant et éventuellement en présence d'un liant d'acide ;
(C) des composés de la formule (I-C) représentée précédemment, dans laquelle A, B, R², M, X, Y et Z ont les significations indiquées précédemment et L représente oxygène, des composés de la formule (I-a) représentée précédemment, dans laquelle A, B, X, Y et Z ont les significations indiquées précédemment, sont mis en réaction respectivement
avec des esters de l'acide chloroformique ou des thioesters de l'acide chloroformique de formule (V)
R²-M-CO-Cl (V)
dans laquelle
R² et M ont les significations indiquées précédemment, éventuellement en présence d'un diluant et éventuellement en présence d'un liant d'acide ;
(D) des composés de la formule (I-c) représentée précédemment, dans laquelle A, B, R², M, X, Y et Z ont les significations indiquées précédemment et L représente soufre, des composés de la formule (I-a) représentée précédemment, dans laquelle A, B, X, Y et Z ont les significations indiquées précédemment, sont mis en réaction respectivement avec des esters de l'acide chloromonothioformique ou des esters de l'acide chlorodithioformique de formule (VI) 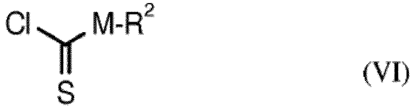 dans laquelle
M et R² ont les significations indiquées précédemment, éventuellement en présence d'un diluant et éventuellement en présence d'un liant d'acide ;
(E) des composés de la formule (I-d) représentée précédemment, dans laquelle A, B, R³, X, Y et Z ont les significations indiquées précédemment, des composés de la formule (I-a) représentée précédemment, dans laquelle A, B, X, Y et Z ont les significations indiquées précédemment, sont mis en réaction respectivement
avec des chlorures d'acide sulfonique de formule (VII)
R³-SO₂-Cl (VII)
dans laquelle
R³ a la signification indiquée précédemment, éventuellement en présence d'un diluant et éventuellement en présence d'un liant d'acide,
(F) des composés de la formule (I-e) représentée précédemment, dans laquelle A, B, L, R⁴, R⁵, X, Y et Z ont les significations indiquées précédemment, des composés de la formule (I-a) représentée précédemment, dans laquelle A, B, X, Y et Z ont les significations indiquées précédemment, sont mis en réaction respectivement
avec des composés de phosphore de formule (VIII) 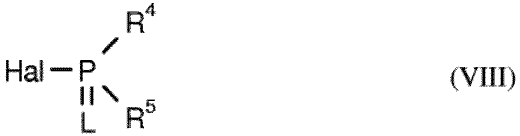 dans laquelle
L, R⁴ et R⁵ ont les significations indiquées précédemment, et
Hal représente halogène,
éventuellement en présence d'un diluant et éventuellement en présence d'un liant d'acide,
(G) des composés de la formule (I-f) représentée précédemment, dans laquelle A, B, E, X, Y et Z ont les significations indiquées précédemment, des composés de la formule (I-a), dans laquelle A, B, X, Y et Z ont les significations indiquées précédemment, sont mis en réaction respectivement
avec des composés métalliques ou des amines de formule (IX) ou (X) 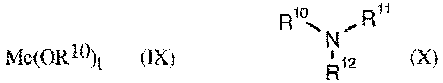 dans lesquelles
Me représente un métal mono- ou bivalent,
t représente le nombre 1 ou 2, et
R¹⁰, R¹¹, R¹² représentent indépendamment les uns des autres hydrogène ou alkyle,
éventuellement en présence d'un diluant,
(H) des composés de la formule (I-g) représentée précédemment, dans laquelle A, B, L, R⁶, R⁷, X, Y et Z ont les significations indiquées précédemment, des composés de la formule (I-a) représentée précédemment, dans laquelle A, B, X, Y et Z ont les significations indiquées précédemment, sont mis en réaction respectivement
(α) avec des isocyanates ou des isothiocyanates de formule (XI)
R⁶-N=C=L (XI)
dans laquelle
R⁶ et L ont les significations indiquées précédemment, éventuellement en présence d'un diluant et éventuellement en présence d'un catalyseur, ou
(β) avec des chlorures d'acide carbamique ou des chlorures d'acide thiocarbamique de formule (XII) 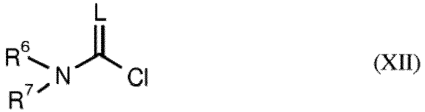 dans laquelle
L, R⁶ et R⁷ ont les significations indiquées précédemment,
éventuellement en présence d'un diluant et éventuellement en présence d'un liant d'acide.

7. Agent de lutte contre des nuisibles et/ou une végétation indésirable, **caractérisé par** une teneur en au moins un composé de formule (I) selon la revendication 1.

8. Procédé de lutte contre des animaux nuisibles et/ou une végétation indésirable, **caractérisé en ce que** des composés de formule (I) selon la revendication 1 sont laissés agir sur des nuisibles, une végétation indésirable et/ou leur habitat.

9. Utilisation de composés de formule (I) selon la revendication 1 pour lutter contre des animaux nuisibles et/ou une végétation indésirable.

10. Procédé de fabrication d'agents de lutte contre des nuisibles et/ou une végétation indésirable, **caractérisé en ce que** des composés de formule (I) selon la revendication 1 sont mélangés avec des extendeurs et/ou des substances tensioactives.

11. Utilisation de composés de formule (I) selon la revendication 1 pour la fabrication d'agents pour lutter contre des nuisibles et/ou une végétation indésirable.

12. Agent contenant un teneur efficace en une combinaison d'agents actifs comprenant en tant que composants :
(a') au moins un composé de formule (I), dans laquelle A, B, G, X, Y et Z ont la signification indiquée précédemment,
et
(b') au moins un composé améliorant la compatibilité avec les plantes cultivées du groupe suivant de composés : S1, S2, S3, S4, S5, S6, S7, S8, S9, S10, S11, S12, S13, S14, S15 :
S1) les composés de formule (S1) 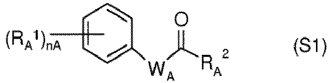 dans laquelle les symboles et les indices ont les significations suivantes :
n_{A} est un nombre naturel de 0 à 5 ;
R_{A}¹ représente halogène, alkyle en (C₁-C₄), alcoxy en (C₁-C₄), nitro ou haloalkyle en (C₁-C₄) ;
W_{A} représente un radical hétérocyclique bivalent non substitué ou substitué du groupe des hétérocycles à cinq éléments partiellement insaturés ou aromatiques contenant 1 à 3 hétéroatomes de cycle du groupe constitué par N et O, au moins un atome N et au plus un atome O étant contenus dans le cycle,
m_{A} représente 0 ou 1 ;
R_{A}² représente OR_{A}³, SR_{A}³ ou NR_{A}³R_{A}⁴ ou un hétérocycle saturé ou insaturé de 3 à 7 éléments contenant au moins un atome N et jusqu'à 3 hétéroatomes, qui est relié par l'atome N avec le groupe carbonyle dans (S1) et qui est non substitué ou substitué par des radicaux du groupe constitué par alkyle en (C₁-C₄), alcoxy en (C₁-C₄) ou
phényle éventuellement substitué ;
R_{A}³ représente l'hydrogène ou un radical hydrocarboné aliphatique non substitué ou substitué ;
R_{A}⁴ représente hydrogène, alkyle en (C₁-C₆), alcoxy en (C₁-C₆) ou phényle substitué ou non substitué ;
R_{A}⁵ représente H, alkyle en (C₁-C₈), haloalkyle en (C₁-C₈), alcoxy en (C₁-C₄)-alkyle en (C₁-C₈), cyano ou COOR_{A}⁹,
R_{A}⁹ représentant hydrogène, alkyle en (C₁-C₈), haloalkyle en (C₁-C₈), alcoxy en (C₁-C₄)-alkyle en (C₁-C₄),
hydroxyalkyle en (C₁-C₆), cycloalkyle en (C₃-C₁₂) ou trialkyle en (C₁-C₄)-silyle ;
R_{A}⁶, R_{A}⁷, R_{A}⁸ sont identiques ou différents, et représentent hydrogène, alkyle en (C₁-C₈), haloalkyle en (C₁-C₈), cycloalkyle en (C₃-C₁₂) ou phényle substitué ou non substitué,
S2) les dérivés de quinoline de formule (S2) 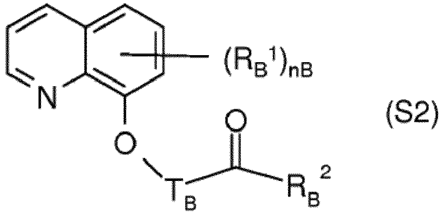 dans laquelle les symboles et les indices ont les significations suivantes :
R_{B}¹ représente halogène, alkyle en (C₁-C₄), alcoxy en (C₁-C₄), nitro ou haloalkyle en (C₁-C₄) ;
n_{B} est un nombre naturel de 0 à 5 ;
R_{B}² représente OR_{B}³, SR_{B}³ ou NR_{B}³R_{B}⁴ ou un hétérocycle saturé ou insaturé de 3 à 7 éléments, contenant au moins un atome N et jusqu'à 3 hétéroatomes, qui est relié par l'atome N avec le groupe carbonyle dans (S2) et qui est non substitué ou substitué par des radicaux du groupe constitué par alkyle en (C₁-C₄), alcoxy en (C₁-C₄) ou phényle éventuellement substitué ;
R_{B}³ représente l'hydrogène ou un radical hydrocarboné aliphatique non substitué ou substitué ;
R_{B}⁴ représente hydrogène, alkyle en (C₁-C₆), alcoxy en (C₁-C₆) ou phényle substitué ou non substitué ;
T_{B} représente une chaîne alcanediyle en C₁ ou C₂, qui est non substituée ou substituée avec un ou deux radicaux alkyle en (C₁-C₄) ou avec [alcoxy en (C₁-C₃)]-carbonyle,
S3) les composés de formule (S3) 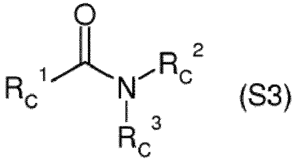 dans laquelle les symboles et les indices ont les significations suivantes :
R_{c}¹ représente alkyle en (C₁-C₄), haloalkyle en (C₁-C₄), alcényle en (C₂-C₄), haloalcényle en (C₂-C₄), cycloalkyle en (C₃-C₇);
R_{c}², R_{c}³ sont identiques ou différents, et représentent hydrogène, alkyle en (C₁-C₄), alcényle en (C₂-C₄), alcynyle en (C₂-C₄), haloalkyle en (C₁-C₄), haloalcényle en (C₂-C₄), alkyle en (C₁-C₄)-carbamoyle-alkyle en (C₁-C₄), alcényle en (C₂-C₄) -carbamoyle-alkyle en (C₁-C₄), alcoxy en (C₁-C₄)-alkyle en (C₁-C₄), dioxolanyl-alkyle en (C₁-C₄), thiazolyle, furyle, furylalkyle, thiényle, pipéridyle, phényle substitué ou non substitué, ou R_{c}² et R_{c}³ forment ensemble un cycle hétérocyclique substitué ou non substitué,
S4) les N-acylsulfonamides de formule (S4) et leurs sels 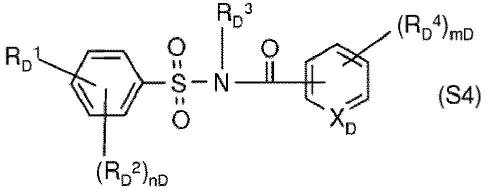 dans laquelle les symboles et les indices ont les significations suivantes :
X_{D} représente CH ou N ;
R_{D}¹ représente CO-NR_{D}⁵R_{D}⁶ ou NHCO-R_{D}⁷ ;
R_{D}² représente halogène, haloalkyle en (C₁-C₄), haloalcoxy en (C₁-C₄), nitro, alkyle en (C₁-C₄), alcoxy en (C₁-C₄), alkylsulfonyle en (C₁-C₄), alcoxycarbonyle en (C₁-C₄) ou alkylcarbonyle en (C₁-C₄) ;
R_{D}³ représente hydrogène, alkyle en (C₁-C₄), alcényle en (C₂-C₄) ou alcynyle en (C₂-C₄) ;
R_{D}⁴ représente halogène, nitro, alkyle en (C₁-C₄), haloalkyle en (C₁-C₄), haloalcoxy en (C₁-C₄), cycloalkyle en (C₃-C₆), phényle, alcoxy en (C₁-C₄), cyano, alkylthio en (C₁-C₄), alkylsulfinyle en (C₁-C₄), alkylsulfonyle en (C₁-C₄), alcoxycarbonyle en (C₁-C₄) ou alkylcarbonyle en (C₁-C₄) ;
R_{D}⁵ représente hydrogène, alkyle en (C₁-C₆), cycloalkyle en (C₃-C₆), alcényle en (C₂-C₆), alcynyle en (C₂-C₆), cycloalcényle en (C₅-C₆), phényle ou hétérocyclyle de 3 à 6 éléments, contenant v_{D} hétéroatomes du groupe constitué par azote, oxygène et soufre, les sept derniers radicaux mentionnés étant substitués par v_{D} substituants du groupe constitué par halogène, alcoxy en (C₁-C₆), haloalcoxy en (C₁-C₆), alkylsulfinyle en (C₁-C₂), alkylsulfonyle en (C₁-C₂), cycloalkyle en (C₃-C₆), alcoxycarbonyle en (C₁-C₄), alkylcarbonyle en (C₁-C₄) et phényle, et, dans le cas de radicaux cycliques, également alkyle en (C₁-C₄) et haloalkyle en (C₁-C₄) ;
R_{D}⁶ représente hydrogène, alkyle en (C₁-C₆), alcényle en (C₂-C₆) ou alcynyle en (C₂-C₆), les trois derniers radicaux mentionnés étant substitués par v_{D} radicaux du groupe constitué par halogène, hydroxy, alkyle en (C₁-C₄), alcoxy en (C₁-C₄) et alkylthio en (C₁-C₄), ou
R_{D}⁵ et R_{D}⁶ forment ensemble avec l'atome d'azote qui les porte un radical pyrrolidinyle ou pipéridinyle ;
R_{D}⁷ représente hydrogène, alkylamino en (C₁-C₄), dialkylamino en (C₁-C₄), alkyle en (C₁-C₆), cycloalkyle en (C₃-C₆), les 2 derniers radicaux mentionnés étant substitués par v_{D} substituants du groupe constitué par halogène, alcoxy en (C₁-C₄), haloalcoxy en (C₁-C₆) et alkylthio en (C₁-C₄), et, dans le cas de radicaux cycliques, également alkyle en (C₁-C₄) et haloalkyle en (C₁-C₄) ;
n_{D} représente 0, 1 ou 2 ;
m_{D} représente 1 ou 2 ;
v_{D} représente 0, 1, 2 ou 3,
S5) les agents actifs suivants de la classe des composés hydroxyaromatiques et des dérivés d'acides carboxyliques aromatiques-aliphatiques (S5) :
l'ester éthylique de l'acide 3,4,5-triacétoxybenzoïque, l'acide 3,5-diméthoxy-4-hydroxybenzoïque, 3,5-dihydroxybenzoïque, l'acide 4-hydroxysalicylique, l'acide 4-fluorosalicylique, l'acide 4-hydroxysalicylique, l'acide 4-fluorosalicylique, l'acide 2-hydroxycinnamique, le 1,2-dihydro-2-oxo-6-trifluorométhylpyridine-3-carboxamide, l'acide 2,4-dichlorocinnamique,
S6) les agents actifs suivants de la classe des 1,2-dihydroquinoxalin-2-ones (S6) : la 1-méthyl-3-(2-thiényl)-1,2-dihydroquinoxalin-2-one, la 1-méthyl-3-(2-thiényl)-1,2-dihydroquinoxaline-2-thione, le chlorhydrate de 1-(2-aminoéthyl)-3-(2-thiényl)-1,2-dihydro-quinoxalin-2-one, la 1-[2-(diéthylamino)éthyl]-6,7-diméthyl-3-thiophén-2-ylquinoxalin-2(1H)-one, la 1-(2-méthylsulfonylaminoéthyl)-3-(2-thiényl)-1,2-dihydro-quinoxalin-2-one,
S7) les composés de formule (S7) 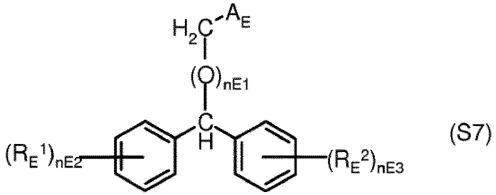 dans laquelle les symboles et les indices ont les significations suivantes :
R_{E}¹, R_{E}² représentent indépendammnt l'un de l'autre halogène, alkyle en (C₁-C₄), alcoxy en (C₁-C₄), haloalkyle en (C₁-C₄), alkylamino en (C₁-C₄), dialkylamino en (C₁-C₄), nitro;
A_{E} représente COOR_{E}³ ou COSR_{E}⁴ ;
R_{E}³, R_{E}⁴ représentent indépendamment l'un de l'autre hydrogène, alkyle en (C₁-C₄), alcényle en (C₂-C₆), alcynyle en (C₂-C₄), cyanoalkyle, haloalkyle en (C₁-C₄), phényle, nitrophényle, benzyle, halobenzyle, pyridinylalkyle et alkylammonium,
n_{E}¹ représente 0 ou 1,
n_{E}², n_{E}³ représentent indépendamment l'un de l'autre 0, 1 ou 2,
S8) les composés de formule (S8) 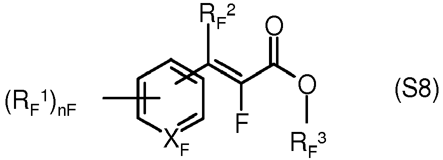 dans laquelle
X_{F} représente CH ou N,
n_{F} lorsque X_{F} = N, un nombre entier de 0 à 4, et lorsque X_{F} = CH, un nombre entier de 0 à 5,
R_{F}¹ représente halogène, alkyle en (C₁-C₄), haloalkyle en (C₁-C₄), alcoxy en (C₁-C₄), haloalcoxy en (C₁-C₄), nitro, alkylthio en (C₁-C₄), alkylsulfonyle en (C₁-C₄), alcoxycarbonyle en (C₁-C₄), phényle éventuellement substitué, phénoxy éventuellement substitué,
R_{F}² représente hydrogène ou alkyle en (C₁-C₄),
R_{F}³ représente hydrogène, alkyle en (C₁-C₈), alcényle en (C₂-C₄), alcynyle en (C₂-C₄) ou aryle, chacun des radicaux contenant C sunsmentionnés étant non substitué ou substitué par un ou plusieurs, de préférence jusqu'à trois, radicaux identiques ou différents du groupe constitué par halogène et alcoxy, ou leurs sels,
S9) les agents actifs suivants de la classe des 3-(5-tétrazolylcarbonyl)-2-quinolones (S9) :
la 1,2-dihydro-4-hydroxy-1-éthyl-3-(5-tétrazolylcarbonyl)-2-quinolone (n° CAS 219479-18-2), la 1,2-dihydro-4-hydroxy-1-méthyl-3-(5-tétrazolylcarbonyl)-2-quinolone (n° CAS 95855-00-8),
S10) les composés de formule (S10^{a}) ou (S10^{b}) 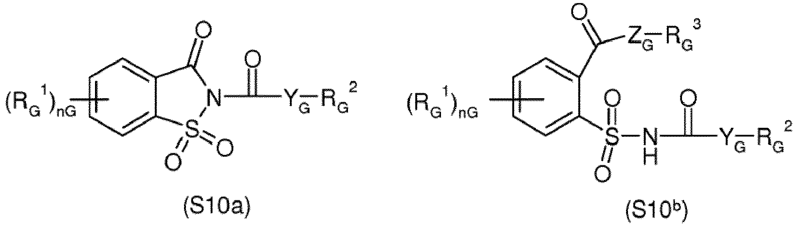 dans lesquelles
R_{G}¹ représente halogène, alkyle en (C₁-C₄), méthoxy, nitro, cyano, CF₃, OCF₃,
Y_{G}, Z_{G} représentent indépendamment l'un de l'autre O ou S,
n_{G} représente un nombre entier de 0 à 4,
R_{G}² représente alkyle en (C₁-C₁₆), alcényle en (C₂-C₆), cycloalkyle en (C₃-C₆), aryle, benzyle, halogénobenzyle, R_{G}³ représente hydroène ou alkyle en (C₁-C₆),
S11) les agents actifs suivants du type des composés oxyimino (S11), qui sont connus en tant qu'agents de traitement de graines :
« oxabénitrile » ((Z)-1,3-dioxolan-2-ylméthoxyimino(phényl)acétonitrile) (S11-1), qui est connu en tant qu'agent protecteur de graines pour le millet contre les dégâts causés par le métolachlore,
« le fluxofénim » (1-(4-chlorophényl)-2,2,2-trifluoro-1-éthanone-O-(1,3-dioxolan-2-ylméthyl)-oxime) (S11-2), qui est connu en tant qu'agent protecteur de graines pour le millet contre les dégâts causés par le métolachlore, et
« le cyométrinil » ou « CGA-43089 » ((Z)-cyanométhoxyimino(phényl)acétonitrile) (S11-3), qui est connu en tant qu'agent protecteur de graines pour le millet contre les dégâts causés par le métolachlore ;
S12) les agents actifs suivants de la classe des isothiochromanones (S12) :
le [(3-oxo-1H-2-benzothiopyran-4(3H)-ylidène)méthoxy]acétate de méthyle (n° CAS 205121-04-6) (S12-1) ;
S13) un ou plusieurs composés du groupe (S13) :
« l'anhydride naphtalique » (anhydride de l'acide 1,8-naphtalinedicarboxylique) (S13-1), qui est connu en tant qu'agent protecteur de graines pour le maïs contre les dégâts causés par les herbicides thiocarbamate,
« le fenclorim » (4,6-dichloro-2-phénylpyrimidine) (S13-2), qui est connu en tant qu'agent protecteur pour le prétilachlore dans les semences de riz,
« le flurazole » (2-chloro-4-trifluorométhyl-1,3-thiazol-5-carboxylate de benzyle) (S13-3), qui est connu en tant qu'agent protecteur de graines pour le millet contre les dégâts causés par l'alachlore et le métolachlore,
« CL 304415 » (n° CAS 31541-57-8) (acide 4-carboxy-3,4-dihydro-2H-1-benzopyrane-4-acétique) (S13-4) de la société American Cyanamid, qui est connu en tant qu'agent protecteur pour le maïs contre les dégâts causés par les imidazolinones,
« MG 191 » (n° CAS 96420-72-3) (2-dichlorométhyl-2-méthyl-1,3-dioxolane) (S13-5) de la société Nitrokemia, qui est connu en tant qu'agent protecteur pour le maïs,
« MG-838 » (n° CAS 133993-74-5) (1-oxa-4-azaspiro[4.5]décane-4-carbodithioate de 2-propényle) (S13-6) de la société Nitrokemia,
« le disulfoton » (phosphorodithioate de O,O-diéthyl-S-2-éthylthioéthyle) (S13-7),
« le diétholate » (phosphorothioate de O,O-diéthyl-O-phényle) (S13-8),
« le méphénate » (carbamate de 4-chlorophényl-méthyle) (S13-9) ;
S14) les agents actifs suivants qui, en plus d'une action herbicide contre les plantes nocives, présentent également un effet protecteur sur les plantes cultivées, telles que le riz :
le « dimépipérate » ou « MY-93 » (1-carbothioate de S-1-méthyl-1-phényléthyl-pipéridine), qui est connu en tant qu'agent protecteur pour le riz contre les dégâts de l'herbicide molinate,
« le daimuron » ou « SK 23 » (1-(1-méthyl-1-phényléthyl)-3-p-tolyl-urée), qui est connu en tant qu'agent protecteur pour le riz contre les dégâts de l'herbicide imazosulfurone,
« le cumyluron » = « JC-940 » (3-(2-chlorophénylméthyl)-1-(1-méthyl-1-phényl-éthyl)-urée, voir JP-A-60087254), qui est connu en tant qu'agent protecteur pour le riz contre les dégâts de plusieurs herbicides,
« la méthoxyphénone » ou « NK 049 » (3,3'-diméthyl-4-méthoxy-benzophénone), qui est connue en tant qu'agent protecteur pour le riz contre les dégâts de plusieurs herbicides,
« CSB » (1-bromo-4-(chlorométhylsulfonyl)benzène) de Kumiai (n° CAS 54091-06-4), qui est connu en tant qu'agent protecteur pour le riz contre les dégâts de plusieurs herbicides ;
S15) les agents actifs qui sont principalement utilisés en tant qu'herbicides, mais qui présentent toutefois également un effet protecteur sur les plantes cultivées :
l'acide (2,4-dichlorophénoxy)acétique (2,4-D),
l'acide (4-chlorophénoxy)acétique,
l'acide (R,S)-2-(4-chloro-o-tolyloxy)propionique (Mecoprop),
l'acide 4-(2,4-dichlorophénoxy)butyrique (2,4-DB),
l'acide (4-chloro-o-tolyloxy)acétique (MCPA),
l'acide 4-(4-chloro-o-tolyloxy)butyrique,
l'acide 4-(4-chlorophénoxy)butyrique,
l'acide 3,6-dichloro-2-méthoxybenzoïque (Dicamba),
le 3,6-dichloro-2-méthoxybenzoate de 1-(éthoxycarbonyl)éthyle (Lactidichlor-éthyle).

13. Agent selon la revendication 12, dans lequel le composé améliorant la compatibilité avec les plantes cultivées est le cloquintocet-mexyl.

14. Agent selon la revendication 12, dans lequel le composé améliorant la compatibilité avec les plantes cultivées est le méfenpyr-diéthyle.

15. Agent selon la revendication 12, dans lequel le composé améliorant la compatibilité avec les plantes cultivées est le cyprosulfamide.

16. Procédé de lutte contre une végétation indésirable, **caractérisé en ce qu'**un agent selon la revendication 12 est laissé agir sur les plantes ou leur environnement.

17. Utilisation d'un agent selon la revendication 12 pour lutter contre une végétation indésirable.

18. Procédé de lutte contre une végétation indésirable, **caractérisé en ce qu'**un composé de formule (I) selon la revendication 1 et le composé améliorant la compatibilité avec les plantes cultivées selon la revendication 12 sont laissés agir séparément sur les plantes ou leur environnement en succession temporelle proche.

19. Composés de formule (II) 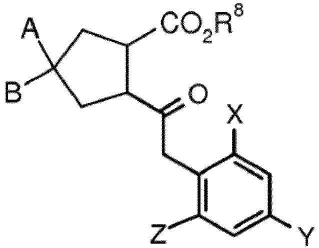 dans laquelle
A, B, X, Y, Z et R⁸ ont la signification indiquée précédemment.

20. Composés de formule (XIII) 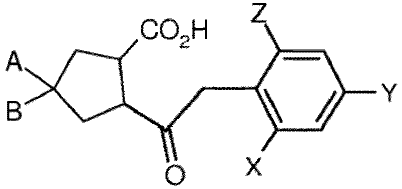 dans laquelle
A, B, X, Y et Z ont la signification indiquée précédemment.

21. Composés de formule (XIV) 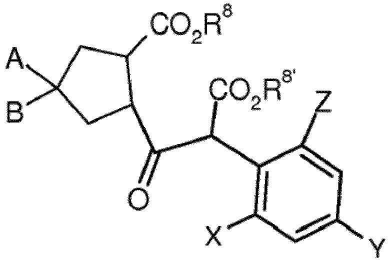 dans laquelle
A, B, X, Y, Z, R⁸ et R^{8'} ont la signification indiquée précédemment.

22. Composés de formule (XIX) 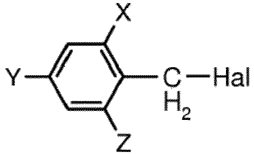 dans laquelle
X, Y, Z et Hal ont les significations indiquées dans le tableau
| Hal | X | Y | Z |
|---|---|---|---|
| | | | |
| | | | |
| Cl | Cyclopropyle | CH₃ | C₂H₅ |
| Br | Cyclopropyle | CH₃ | C₂H₅ |
